# EUROPEAN PATENT APPLICATION

(11) **EP 2 116 245 A2**
(43) Date of publication of application: **11.11.2009**
(21) Application number: 09167644.5
(22) Date of filing: 03.08.2005
(51) Int. Cl.: A61K 31/47, A61K 31/4709, A61K 31/517, A61K 31/519, A61K 39/395, A61K 45/06, A61P 1/00, A61P 11/00

(54) **EGFR kinase inhibitor combinations for treating respiratory and gastrointestinal disorders**

(30) Priority: 07.08.2004 EP 04018808
(62) Divisional of application: 05773706.6
(71) Applicant: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: Jung, Birgit, 88471, LAUPHEIM (DE); Himmelsbach, Frank, 88441, MITTELBIBERACH (DE)
(74) Representative: Hammann, Heinz

(57) **Abstract**

The present invention relates to novel pharmaceutical compositions comprising at least one EGFR kinase inhibitor and at least one additional active compound selected from beta-2 mimetics, steroids, PDE-IV inhibitors, p38 MAP kinase inhibitors, NK₁ antagonists and endothelin-antagonists, processes for preparing the compositions and the use thereof as medicament in the treatment of respiratory or gastrointestinal complaints, as well as inflammatory diseases of the joints, the skin or the eyes.

## Description

The present invention relates to novel pharmaceutical compositions comprising one or more, preferably one, selected EGFR kinase inhibitors **1**, and at least one additional active compound **2,** processes for preparing them and their use as medicament in the treatment of respiratory or gastrointestinal complaints, as well as inflammatory diseases of the joints, the skin or the eyes.

### Detailed description of the invention

In a first aspect the present invention relates to pharmaceutical compositions comprising at least one EGFR kinase inhibitor **1** selected from the group consisting of
(**1.1**) 4-[(3-chloro-4-fluorophenyl)amino]-6-{[4-(morpholin-4-yl)-1-oxo-2-buten-1-yl]-amino}-7-cyclopropylmethoxy-quinazoline,
(**1.2**) 4-[(3-chloro-4-fluorophenyl)amino]-6-{[4-(N,N-diethylamino)-1-oxo-2-buten-1-yl]amino}-7-cyclopropylmethoxy-quinazoline,
(**1.3**) 4-[(3-chloro-4-fluorophenyl)amino]-6-{[4-(N,N-dimethylamino)-1-oxo-2-buten-1-yl]amino}-7-cyclopropylmethoxy-quinazoline,
(**1.4**) 4-[(R)-(1-phenyl-ethyl)amino]-6-{[4-(morpholin-4-yl)-1-oxo-2-buten-1-yl]-amino}-7-cyclopentyloxy-quinazoline,
(**1.5**) 4-[(3-chloro-4-fluoro-phenyl)amino]-6-{[4-((R)-6-methyl-2-oxo-morpholin-4-yl)-1-oxo-2-buten-1-yl]amino}-7-cyclopropylmethoxy-quinazoline,
(**1.6**) 4-[(3-chloro-4-fluoro-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-quinazoline,
(**1.7**) 4-[(3-chloro-4-fluoro-phenyl)amino]-6-{[4-((R)-2-methoxymethyl-6-oxo-morpholin-4-yl)-1-oxo-2-buten-1-yl]amino}-7-cyclopropylmethoxy-quinazoline,
(**1.8**) 4-[(3-chloro-4-fluoro-phenyl)amino]-6-[2-((S)-6-methyl-2-oxo-morpholin-4-yl)-ethoxy]-7-methoxy-quinazoline,
(**1.9**) 4-[(3-chloro-4-fluorophenyl)amino]-6-({4-[N-(2-methoxy-ethyl)-N-methyl-amino]-1-oxo-2-buten-1-yl}amino)-7-cyclopropylmethoxy-quinazoline,
(**1.10**) 4-[(3-chloro-4-fluorophenyl)amino]-6-{[4-(N,N-dimethylamino)-1-oxo-2-buten-1-yl]amino}-7-cyclopentyloxy-quinazoline,
(**1.11**) 4-[(R)-(1-phenyl-ethyl)amino]-6-{[4-(N,N-bis-(2-methoxy-ethyl)-amino)-1-oxo-2-buten-1-yl]amino}-7-cyclopropylmethoxy-quinazoline,
(**1.12**) 4-[(R)-(1-phenyl-ethyl)amino]-6-({4-[N-(2-methoxy-ethyl)-N-ethyl-amino]-1-oxo-2-buten-1-yl}amino)-7-cyclopropylmethoxy-quinazoline,
(**1.13**) 4-[(R)-(1-phenyl-ethyl)amino]-6-({4-[N-(2-methoxy-ethyl)-N-methyl-amino]-1-oxo-2-buten-1-yl}amino)-7-cyclopropylmethoxy-quinazoline,
(**1.14**) 4-[(R)-(1-phenyl-ethyl)amino]-6-({4-[N-(tetrahydropyran-4-yl)-N-methyl-amino]-1-oxo-2-buten-1-yl}amino)-7-cyclopropylmethoxy-quinazoline,
(**1.15**) 4-[(3-chloro-4-fluorophenyl)amino]-6-{[4-(N,N-dimethylamino)-1-oxo-2-buten-1-yl]amino}-7-((R)-tetrahydrofuran-3-yloxy)-quinazoline,
(**1.16**) 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline,
(**1.17**) 4-[(3-chloro-4-fluorophenyl)amino]-6-({4-[N-(2-methoxy-ethyl)-N-methylamino]-1-oxo-2-buten-1-yl}amino)-7-cyclopentyloxy-quinazoline,
(**1.18**) 4-[(3-chloro-4-fluorophenyl)amino]-6-{[4-(N-cyclopropyl-N-methyl-amino)-1-oxo-2-buten-1-yl]amino}-7-cyclopentyloxy-quinazoline,
(**1.19**) 4-[(3-chloro-4-fluorophenyl)amino]-6-{[4-(N,N-dimethylamino)-1-oxo-2-buten-1-yl]amino}-7-[(R)-(tetrahydrofuran-2-yl)methoxy]-quinazoline,
(**1.20**) 4-[(3-chloro-4-fluorophenyl)amino]-6-{[4-(N,N-dimethylamino)-1-oxo-2-buten-1-yl]amino}-7-[(S)-(tetrahydrofuran-2-yl)methoxy]-quinazoline,
(**1.21**) 4-[(3-ethinyl-phenyl)amino]-6,7-bis-(2-methoxy-ethoxy)-quinazoline,
(**1.22**) 4-[(3-chloro-4-fluorophenyl)amino]-7-[3-(morpholin-4-yl)-propyloxy]-6-[(vinylcarbonyl)amino]-quinazoline,
(**1.23**) 4-[(R)-(1-phenyl-ethyl)amino]-6-(4-hydroxy-phenyl)-7H-pyrrolo[2,3-d]pyrimidine,
(**1.24**) 3-cyano-4-[(3-chlor-4-fluorophenyl)amino]-6-{[4-(N,N-dimethylamino)-1-oxo-2-buten-1-yl]amino}-7-ethoxy-quinoline,
(**1.25**) 3-cyano-4-[(3-chlor-4-(pyridin-2-yl-methoxy)-phenyl)amino]-6-{[4-(N,N-dimethylamino)-1-oxo-2-buten-1-yl]amino}-7-ethoxy-quinoline,
(**1.26**) 4-{[3-chloro-4-(3-fluoro-benzyloxy)-phenyl]amino}-6-(5-{[(2-methansulfonylethyl)amino]methyl}-furan-2-yl)quinazoline,
(**1.27**) 4-[(R)-(1-phenyl-ethyl)amino]-6-{[4-((R)-6-methyl-2-oxo-morpholin-4-yl)-1-oxo-2-buten-1-yl]amino}-7-methoxy-quinazoline,
(**1.28**) 4-[(3-chloro-4-fluorophenyl)amino]-6-{[4-(morpholin-4-yl)-1-oxo-2-buten-1-yl]-amino}-7-[(tetrahydrofuran-2-yl)methoxy]-quinazoline,
(**1.29**) 4-[(3-chloro-4-fluorophenyl)amino]-6-({4-[N,N-bis-(2-methoxy-ethyl)-amino]-1-oxo-2-buten-1-yl}amino)-7-[(tetrahydrofuran-2-yl)methoxy]-quinazoline,
(**1.30**) 4-[(3-ethinyl-phenyl)amino]-6-{[4-(5,5-dimethyl-2-oxo-morpholin-4-yl)-1-oxo-2-buten-1-yl]amino}-quinazoline,
(**1.31**) 4-[(3-chloro-4-fluoro-phenyl)amino]-6-[2-(2,2-dimethyl-6-oxo-morpholin-4-yl)-ethoxy]-7-methoxy-quinazoline,
(**1.32**) 4-[(3-chloro-4-fluoro-phenyl)amino]-6-[2-(2,2-dimethyl-6-oxo-morpholin-4-yl)-ethoxy]-7-[(R)-(tetrahydrofuran-2-yl)methoxy]-quinazoline,
(**1.33**) 4-[(3-chloro-4-fluoro-phenyl)amino]-7-[2-(2,2-dimethyl-6-oxo-morpholin-4-yl)-ethoxy]-6-[(S)-(tetrahydrofuran-2-yl)methoxy]-quinazoline,
(**1.34**) 4-[(3-chloro-4-fluoro-phenyl)amino]-6-{2-[4-(2-oxo-morpholin-4-yl)-piperidin-1-yl]-ethoxy}-7-methoxy-quinazoline,
(**1.35**) 4-[(3-chloro-4-fluoro-phenyl)amino]-6-[1-(tert.-butyloxycarbonyl)-piperidin-4-yloxy]-7-methoxy-quinazoline,
(**1.36**) 4-[(3-chloro-4-fluoro-phenyl)amino]-6-(trans-4-amino-cyclohexan-1-yloxy)-7-methoxy-quinazoline,
(**1.37**) 4-[(3-chloro-4-fluoro-phenyl)amino]-6-(trans-4-methansulfonylamino-cyclohexan-1-yloxy)-7-methoxy-q u inazol ine,
(**1.38**) 4-[(3-chloro-4-fluoro-phenyl)amino]-6-(tetrahydropyran-3-yloxy)-7-methoxyquinazoline,
(**1.39**) 4-[(3-chloro-4-fluoro-phenyl)amino]-6-(1-methyl-piperidin-4-yloxy)-7-methoxyquinazoline,
(**1.40**) 4-[(3-chloro-4-fluoro-phenyl)amino]-6-{1-[(morpholin-4-yl)carbonyl]-piperidin-4-yloxy}-7-methoxy-quinazoline,
(**1.41**) 4-[(3-chloro-4-fluoro-phenyl)amino]-6-{1-[(methoxymethyl)carbonyl]-piperidin-4-yloxy}-7-methoxy-quinazoline,
(**1.42**) 4-[(3-chloro-4-fluoro-phenyl)amino]-6-(piperidin-3-yloxy)-7-methoxyquinazoline,
(**1.43**) 4-[(3-chloro-4-fluoro-phenyl)amino]-6-[1-(2-acetylamino-ethyl)-piperidin-4-yl-oxy]-7-methoxy-quinazoline,
(**1.44**) 4-[(3-chloro-4-fluoro-phenyl)amino]-6-(tetrahydropyran-4-yloxy)-7-ethoxy-quinazoline
(**1.45**) 4-[(3-chloro-4-fluoro-phenyl)amino]-6-((S)-tetrahydrofuran-3-yloxy)-7-hydroxy-quinazoline,
(**1.46**) 4-[(3-chloro-4-fluoro-phenyl)amino]-6-(tetrahydropyran-4-yloxy)-7-(2-methoxy-ethoxy)-q u inazol ine,
(**1.47**) 4-[(3-chloro-4-fluoro-phenyl)amino]-6-{trans-4-[(dimethylamino)sulfonyl-amino]-cyclohexan-1-yloxy}-7-methoxy-quinazoline,
(**1.48**) 4-[(3-chloro-4-fluoro-phenyl)amino]-6-{trans-4-[(morpholin-4-yl)carbonylamino]-cyclohexan-1-yloxy}-7-methoxy-quinazoline,
(**1.49**) 4-[(3-chloro-4-fluoro-phenyl)amino]-6-{trans-4-[(morpholin-4-yl)sulfonylamino]-cyclohexan-1-yloxy}-7-methoxy-quinazoline,
(**1.50**) 4-[(3-chloro-4-fluoro-phenyl)amino]-6-(tetrahydropyran-4-yloxy)-7-(2-acetylamino-ethoxy)-quinazoline,
(**1.51**) 4-[(3-chloro-4-fluoro-phenyl)amino]-6-(tetrahydropyran-4-yloxy)-7-(2-methan-sulfonylamino-ethoxy)-quinazoline,
(**1.52**) 4-[(3-chloro-4-fluoro-phenyl)amino]-6-{1-[(piperidin-1-yl)carbonyl]-piperidin-4-yloxy}-7-methoxy-quinazoline,
(**1.53**) 4-[(3-chloro-4-fluoro-phenyl)amino]-6-(1-aminocarbonylmethyl-piperidin-4-yl-oxy)-7-methoxy-quinazoline,
(**1.54**) 4-[(3-chloro-4-fluoro-phenyl)amino]-6-(cis-4-{N-[(tetrahydropyran-4-yl)carbonyl]-N-methyl-amino}-cyclohexan-1-yloxy)-7-methoxy-quinazoline,
(**1.55**) 4-[(3-chloro-4-fluoro-phenyl)amino]-6-(cis-4-{N-[(morpholin-4-yl)carbonyl]-N-methyl-amino}-cyclohexan-1-yloxy)-7-methoxy-quinazoline,
(**1.56**) 4-[(3-chloro-4-fluoro-phenyl)amino]-6-(cis-4-{N-[(morpholin-4-yl)sulfonyl]-N-methyl-amino}-cyclohexan-1-yloxy)-7-methoxy-quinazoline,
(**1.57**) 4-[(3-chloro-4-fluoro-phenyl)amino]-6-(trans-4-ethansulfonylamino-cyclohexan-1-yloxy)-7-methoxy-quinazoline,
(**1.58**) 4-[(3-chloro-4-fluoro-phenyl)amino]-6-(1-methansulfonyl-piperidin-4-yloxy)-7-ethoxy-quinazoline,
(**1.59**) 4-[(3-chloro-4-fluoro-phenyl)amino]-6-(1-methansulfonyl-piperidin-4-yloxy)-7-(2-methoxy-ethoxy)-quinazoline,
(**1.60**) 4-[(3-chloro-4-fluoro-phenyl)amino]-6-[1-(2-methoxy-acetyl)-piperidin-4-yloxy]-7-(2-methoxy-ethoxy)-quinazoline,
(**1.61**) 4-[(3-chloro-4-fluoro-phenyl)amino]-6-(cis-4-acetylamino-cyclohexan-1-yloxy)-7-methoxy-quinazoline,
(**1.62**) 4-[(3-ethinyl-phenyl)amino]-6-[1-(tert.-butyloxycarbonyl)-piperidin-4-yloxy]-7-methoxy-quinazoline,
(**1.63**) 4-[(3-ethinyl-phenyl)amino]-6-(tetrahydropyran-4-yloxy]-7-methoxyquinazoline,
(**1.64**) 4-[(3-chloro-4-fluoro-phenyl)amino]-6-(cis-4-{N-[(piperidin-1-yl)carbonyl]-N-methyl-amino}-cyclohexan-1-yloxy)-7-methoxy-quinazoline,
(**1.65**) 4-[(3-chloro-4-fluoro-phenyl)amino]-6-(cis-4-{N-[(4-methyl-piperazin-1-yl)-carbonyl]-N-methyl-amino}-cyclohexan-1-yloxy)-7-methoxy-quinazoline,
(**1.66**) 4-[(3-chloro-4-fluoro-phenyl)amino]-6-{cis-4-[(morpholin-4-yl)carbonylamino]-cyclohexan-1-yloxy}-7-methoxy-quinazoline,
(**1.67**) 4-[(3-chloro-4-fluoro-phenyl)amino]-6-{1-[2-(2-oxopyrrolidin-1-yl)ethyl]-piperidin-4-yloxy}-7-methoxy-quinazoline,
(**1.68**) 4-[(3-chloro-4-fluoro-phenyl)amino]-6-{1-[(morpholin-4-yl)carbonyl]-piperidin-4-yloxy}-7-(2-methoxy-ethoxy)-quinazoline,
(**1.69**) 4-[(3-ethinyl-phenyl)amino]-6-(1-acetyl-piperidin-4-yloxy)-7-methoxyquinazoline,
(**1.70**) 4-[(3-ethinyl-phenyl)amino]-6-(1-methyl-piperidin-4-yloxy)-7-methoxyquinazoline,
(**1.71**) 4-[(3-ethinyl-phenyl)amino]-6-(1-methansulfonyl-piperidin-4-yloxy)-7-methoxy-quinazoline,
(**1.72**) 4-[(3-chloro-4-fluoro-phenyl)amino]-6-(1-methyl-piperidin-4-yloxy)-7(2-methoxy-ethoxy)-quinazoline,
(**1.73**) 4-[(3-chloro-4-fluoro-phenyl)amino]-6-(1-isopropyloxycarbonyl-piperidin-4-yl-oxy)-7-methoxy-quinazoline,
(**1.74**) 4-[(3-chloro-4-fluoro-phenyl)amino]-6-(cis-4-methylamino-cyclohexan-1-yloxy)-7-methoxy-quinazoline,
(**1.75**) 4-[(3-chloro-4-fluoro-phenyl)amino]-6-{cis-4-[N-(2-methoxy-acetyl)-N-methylamino]-cyclohexan-1-yloxy}-7-methoxy-quinazoline,
(**1.76**) 4-[(3-ethinyl-phenyl)amino]-6-(piperidin-4-yloxy)-7-methoxy-quinazoline,
(**1.77**) 4-[(3-ethinyl-phenyl)amino]-6-[1-(2-methoxy-acetyl)-piperidin-4-yloxy]-7-methoxy-quinazoline,
(**1.78**) 4-[(3-ethinyl-phenyl)amino]-6-{1-[(morpholin-4-yl)carbonyl]-piperidin-4-yloxy}-7-methoxy-quinazoline,
(**1.79**) 4-[(3-chloro-4-fluoro-phenyl)amino]-6-{1-[(cis-2,6-dimethyl-morpholin-4-yl)carbonyl]-piperidin-4-yloxy}-7-methoxy-quinazoline,
(**1.80**) 4-[(3-chloro-4-fluoro-phenyl)amino]-6-{1-[(2-methyl-morpholin-4-yl)carbonyl]-piperidin-4-yloxy}-7-methoxy-quinazoline,
(**1.81**) 4-[(3-chloro-4-fluoro-phenyl)amino]-6-{1-[(S,S)-(2-oxa-5-aza-bicyclo[2.2.1]-hept5-yl)carbonyl]-piperidin-4-yloxy}-7-methoxy-quinazoline,
(**1.82**) 4-[(3-chloro-4-fluoro-phenyl)amino]-6-{1-[(N-methyl-N-2-methoxyethyl-amino)carbonyl]-piperidin-4-yloxy}-7-methoxy-quinazoline,
(**1.83**) 4-[(3-chloro-4-fluoro-phenyl)amino]-6-(1-ethyl-piperidin-4-yloxy)-7-methoxyquinazoline,
(**1.84**) 4-[(3-chloro-4-fluoro-phenyl)amino]-6-{1-[(2-methoxyethyl)carbonyl]-piperidin-4-yloxy}-7-methoxy-quinazoline,
(**1.85**) 4-[(3-chloro-4-fluoro-phenyl)amino]-6-{1-[(3-methoxypropyl-amino)-carbonyl]-piperidin-4-yloxy}-7-methoxy-quinazoline,
(**1.86**) 4-[(3-chloro-4-fluoro-phenyl)amino]-6-[cis-4-(N-methansulfonyl-N-methyl-amino)-cyclohexan-1-yloxy]-7-methoxy-quinazoline,
(**1.87**) 4-[(3-chloro-4-fluoro-phenyl)amino]-6-[cis-4-(N-acetyl-N-methyl-amino)-cyclo-hexan-1-yloxy]-7-methoxy-qu inazol ine,
(**1.88**) 4-[(3-chloro-4-fluoro-phenyl)amino]-6-(trans-4-methylamino-cyclohexan-1-yl-oxy)-7-methoxy-quinazoline
(**1.89**) 4-[(3-chloro-4-fluoro-phenyl)amino]-6-[trans-4-(N-methansulfonyl-N-methylamino)-cyclohexan-1-yloxy]-7-methoxy-quinazoline,
(**1.90**) 4-[(3-chloro-4-fluoro-phenyl)amino]-6-(trans-4-dimethylamino-cyclohexan-1-yloxy)-7-methoxy-quinazoline ,
(**1.91**) 4-[(3-chloro-4-fluoro-phenyl)amino]-6-(trans-4-{N-[(morpholin-4-yl)carbonyl]-N-methyl-amino}-cyclohexan-1-yloxy)-7-methoxy-quinazoline,
(**1.92**) 4-[(3-chloro-4-fluoro-phenyl)amino]-6-{1-[2-(2-oxo-3-methyl-imidazolidin-1-yl)-ethyl]-piperidin-4-yloxy}-7-methoxy-quinazoline
(**1.93**) 4-[(3-chloro-4-fluoro-phenyl)amino]-6-{1-[2-(2-oxo-hexahydropyrimidin-1-yl)-ethyl]-piperidin-4-yloxy}-7-methoxy-quinazoline,
(**1.94**) 4-[(3-chloro-4-fluoro-phenyl)amino]-6-[2-(2,2-dimethyl-6-oxo-morpholin-4-yl)-ethoxy]-7-[(S)-(tetrahydrofuran-2-yl)methoxy]-quinazoline,
(**1.95**) 4-[(3-chloro-4-fluoro-phenyl)amino]-6-(1-methansulfonyl-piperidin-4-yloxy)-7-methoxy-quinazoline,
(**1.96**) 4-[(3-chloro-4-fluoro-phenyl)amino]-6-(1-cyano-piperidin-4-yloxy)-7-methoxyquinazoline,
**(1.97)** 4-[(3-chloro-4-fluoro-phenyl)amino]-6-(tetrahydropyran-4-yloxy)-7-methoxyquinazoline,
**(1.98)** 4-[(3-chloro-4-fluoro-phenyl)amino]-6-(1-methylcarbonyl-piperidin-4-yloxy)-7-methoxy-quinazoline,
**(1.99)** 4-[(3-chloro-4-fluoro-phenyl)amino]-6-(1-dimethylaminoacetyl-piperidin-4-yloxy)-7-methoxy-quinazoline,
**(1.100)** 4-[(3-chloro-4-fluoro-phenyl)amino]-6-{1-[(dimethylamino)carbonylmethyl]-piperidin-4-yloxy}-7-methoxy-quinazoline,
**(1.101)** 4-[(3-chloro-4-fluoro-phenyl)amino]-6-(1-methansulfonyl-piperidin-4-yloxy)-quinazoline,
(**1.102**) Cetuximab, (**1.103**) Trastuzumab, (**1.104**) ABX-EGF and (**1.105**) Mab ICR-62,
optionally in the form of tautomers, racemates, enantiomers, diastereomers, pharmacologically acceptable acid addition salts, solvates or hydrates thereof,
and further comprising one or more additional active compounds **2** selected from the groups consisting of beta-2 mimetics **2a,** steroids **2b,** PDE-IV inhibitors **2c,** p38 MAP kinase inhibitors **2d,** NK₁ antagonists **2e** and endothelin-antagonists **2f,** optionally together with one or more pharmaceutically acceptable excipients or carriers. All active components should be present in effective amounts.

The active compounds **1.1** to **1.105** are disclosed in the prior art, e.g. in WO 96/30347; WO 97/02266; WO 99/35146; WO 00/31048; WO 00/78735; WO 01/34574; WO 01/61816; WO 01/77104; WO02/18351; WO 02/18372; WO 02/18373; WO 02/18376; WO 02/50043; WO 03/082290; Cancer Research 2004, 64:11 (3958-3965); Am J Health-Syst Pharm 2000, 57(15), 2063-2076; Clinical Therapeutics 1999, 21(2),309-318; WO 98/50433; and WO 95/20045.

In the pharmaceutical compositions according to the present invention the EGFR kinase inhibitors **1** may be contained in a form selected from tautomers, optical isomers, enantiomers, racemates, diastereomers, pharmacologically acceptable acid addition salts, solvates or hydrates, as far as such forms exist, depending on the individual compound. Pharmaceutical compositions comprising one or more, preferably one, compound **1** in form of a substantially pure enantiomer are preferred.

Pharmacological acceptable acid addition salts of EGFR kinase inhibitors **1** comprise salts selected from the group consisting of the hydrochloride, hydrobromide, hydroiodide, hydrosulphate, hydrophosphate, hydromethanesulphonate, hydronitrate, hydromaleate, hydroacetate, hydrobenzoate, hydrocitrate, hydrofumarate, hydrotartrate, hydrolactate, hydrooxalate, hydrosuccinate, hydrobenzoate and hydro-p-toluolsulphonate, preferably hydrochloride, hydrobromide, hydrosulphate, hydrophosphate, hydromaleate, hydrofumarate and hydromethansulphonate. Some of the compounds **1** may add more than one equivalent acid, e.g. two equivalents. The salts of hydrochloric acid, methanesulphonic acid, maleic acid, benzoic acid and acetic acid are especially preferred.

The pharmaceutical compositions according to the invention comprising at least one EGFR kinase inhibitor **1** and at least one additonal active compound **2** are not restricted to binary combinations of actives. The combinations disclosed exemplary below comprising an EGFR kinase inhibitor **1** together with an additional active compound **2** may comprise a third or a third and a fourth, preferably a third active compound, also selected from the group consisting of beta-2 mimetics **2a,** steroids **2b,** PDE-IV inhibitors **2c,** p38 MAP kinase inhibitors **2d,** NK₁ antagonists **2e** and endothelin-antagonists **2f.** All components **2a** to **2f** mentioned specifically hereinafter are described in the prior art.

In a first preferred embodiment of the invention the pharmaceutical combination is binary, comprising an EGFR kinase inhibitor **1** and an active compound selected from one of the classes **2a, 2b, 2c, 2d, 2e** and **2f.**

In a second preferred embodiment of the invention the pharmaceutical combination is ternary, comprising an EGFR kinase inhibitor **1,** an active compound selected from the class of beta-2 mimetics **2a** and an active drug selected from the class of steroids **2b.**

In a third embodiment of the invention the pharmaceutical combination is ternary, comprising two EGFR kinase inhibitors **1** and an active compound selected from one of the classes **2a, 2b, 2c, 2d, 2e** and **2f,** preferably selected from one of the classes **2b, 2d** and **2e.**

In a fourth embodiment of the invention the pharmaceutical combination is quarternary, comprising two EGFR kinase inhibitors **1** and two active compounds selected from either one or from two different classes of **2a, 2b, 2c, 2d, 2e** and **2f,** preferably selected from either one or from two different classes of **2b, 2d** and **2e.**

Any reference to an EGFR kinase inhibitor **1** within the scope of the present invention should be understood as a reference to any specific EGFR kinase inhibitor selected from compounds **1.1** to **1.105.** mentioned hereinbefore. Analogously, any reference to an active compound selected from the classes **2a, 2b, 2c, 2d, 2e** and **2f** within the scope of the present invention should be understood as a reference to any active compound of these classes mentioned specifically hereinbelow.

In the pharmaceutical combinations according to the invention the active substances may be combined in a single preparation, e.g. as a fixed dose combination comprising the active ingredients in one formulation together, or contained in two or more separate formulations, e.g. as a kit of parts adapted for simultaneous, separate or sequential administration. Pharmaceutical compositions containing the active substances 1 and 2 in a single preparation are preferred according to the invention.

In all embodiments of the invention the EGFR kinase inhibitors **1.1** to **1.101** are preferred, especially the EGFR kinase inhibitors
**1.1, 1.4, 1.6, 1.8, 1.9, 1.14, 1.17, 1.19, 1.21, 1.23, 1.24, 1.27, 1.28, 1.30, 1.34, 1.35, 1.37, 1.38, 1.40, 1.42, 1.43, 1.44, 1.48, 1.52, 1.55, 1.57, 1.59, 1.60, 1.63, 1.64, 1.66, 1.67, 1.69, 1.70, 1.71, 1.72, 1.78, 1.82, 1.83, 1.84, 1.88, 1.90, 1.91, 1.94** and **1.95.**

All pharmaceutical compositions of the present invention can be advantageously used in the following indications (A):
for the prevention and treatment of diseases of the airways and lungs which are accompanied by increased or altered production of mucus and / or in inflammatory and/or obstructive diseases of the airways such as
   acute bronchitis, chronic bronchitis, chronic obstructive bronchitis (COPD), cough, pulmonary emphysema,
   allergic or non-allergic rhinitis or sinusitis, chronic sinusitis or rhinitis, nasal polyposis, chronic rhinosinusitis, acute rhinosinusitis,
   asthma, allergic bronchitis, alveolitis, Farmers'disease, hyperreactive airways,
   bronchitis or pneumonits caused by infection, e.g. by bacteria or viruses or helminthes or fungi or protozoons or other pathogens,
   pediatric asthma, bronchiectasis,
   pulmonary fibrosis,
   adult respiratory distress syndrome, bronchial and pulmonary edema,
   bronchitis or pneumonitis or interstitial pneumonitis caused by different origins, e.g. aspiration, inhalation of toxic gases, vapors,
   bronchitis or pneumonitis or interstitial pneumonitis caused by heart failure, X-rays, radiation, chemotherapy,
   bronchitis or pneumonitis or interstitial pneumonitis associated with collagenosis, e.g. lupus erythematodes, systemic scleroderma,
   lung fibrosis, idiopathic pulmonary lung fibrosis (IPF), interstitial lung diseases or interstitial pneumonitis of different origin, including asbestosis, silicosis, M. Boeck or sarcoidosis, granulomatosis,
   cystic fibrosis or mucoviscidosis, or α1-antitrypsin deficiency.

Pharmaceutical compositions of the present invention comprising at least one EGFR kinase inhibitor **1** and further comprising one or more additional active compounds **2** selected from the groups consisting of steroids **2b,** p38 MAP kinase inhibitors **2d** and NK₁ antagonists **2e,** e.g.
binary compositions comprising an EGFR kinase inhibitor **1** and an active compound selected from one of the classes **2b, 2d** and **2e,**
ternary compositions comprising two EGFR kinase inhibitors **1** and an active compound selected from one of the classes **2b, 2d** and **2e,** or
quarternary compositions comprising two EGFR kinase inhibitors **1** and two active compounds selected from either one or from two different classes of **2b, 2d** and **2e,**
can be advantageously used in the following indications (B):
for treatment of inflammatory or hypersecretory diseases of the gastrointestinal tract of various origins or polyps of the gastrointestinal tract of various origins such as
   villous or adenomatous polyps of the large intestine, but also polyps in familial polyposis coli, in intestinal polyps in Gardner's syndrome, in polyps throughout the entire gastro-intestinal tract in Peutz-Jeghers Syndrome, in inflammatory pseudopolyps, in juvenile polyps, in colitis cystica profunda and in pneumatosis cystoides intestinales, acute or chronic inflammatory changes such as cholecystitis, Crohn's disease, ulcerative colitis, and ulcers or polyposis in the gastrointestinal tract or such as may occur in diseases of the gastrointestinal tract which are associated with increased secretions, such as Ménétrier's disease, secreting adenomas and protein loss syndromes, or
   diseases of the bile duct and gall bladder, e.g. gall stones or biliary concretion,
and also for treating inflammatory diseases of the joints, such as rheumatoid arthritis,
or inflammatory diseases of the skin or the eyes.

Preferred fields of application are inflammatory and/or obstructive diseases of the respiratory organs or of the intestine, such as chronic (obstructive) bronchitis (COPD), chronic sinusitis, chronic rhinosinusitis, nasal polyposis, asthma, Crohn's disease, ulcerative colitis or polyposis of the intestines.

Particularly preferred fields of application are inflammatory diseases of the airways or lungs such as chronic (obstructive) bronchitis (COPD) or asthma and chronic rhinosinusitis.

Thus a second aspect of the invention is a method of treating any of the indications mentioned hereinbefore comprising administering to a patient in need thereof a pharmaceutical composition according to the invention, comprising at least one of the selected EGFR kinase inhibitors **1** in combination with one or more additional active compounds **2** selected from the groups consisting of beta-2 mimetics **2a,** steroids **2b,** PDE-IV inhibitors **2c,** p38 MAP kinase inhibitors **2d,** NK₁ antagonists **2e** and endothelin-antagonists **2f,** optionally together with one or more pharmaceutically acceptable excipients. The expression "patient" is meant to comprise the mammal animal body, preferably the human body. The method of treatment is meant to encompass simultaneous as well as successive administration of the active components.

A third aspect of the invention is the use of any of the selected EGFR kinase inhibitors **1** in combination with one or more additional active compounds **2** selected from the groups consisting of beta-2 mimetics **2a,** steroids **2b,** PDE-IV inhibitors **2c,** p38 MAP kinase inhibitors **2d,** NK₁ antagonists **2e** and endothelin-antagonists 2f, optionally together with one or more pharmaceutically acceptable excipients, for the manufacture of a pharmaceutical composition for treating any of the indications mentioned hereinbefore in a patient in need thereof. This aspect encompasses the preparation of all pharmaceutical compositions according to the invention mentioned hereinbefore or below.

Preferred embodiments of the pharmaceutical compositions of the invention as well as the indications to be treated apply analogously regarding to the second and third aspect of the invention.

### Pharmaceutical compositions comprising an EGFR kinase inhibitor 1 and a beta-2 mimetic 2a:

One embodiment of the invention is a pharmaceutical composition comprising an EGFR kinase inhibitor **1** and a beta-2 mimetic **2a.** Binary compositions containing only one active **1** and one active **2a,** optionally together with one or more pharmaceutically acceptable excipients or carriers, are preferred. In the pharmaceutical combinations according to the invention preferred beta₂ agonists **2a** are selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, CHF 4226 (TA2005), HOKU-81, KUL-1248, 3-(4-{6-[2-hydroxy-2-(4-hydroxy-3-hydroxymethylphenyl)-ethylamino]-hexyloxy}-butyl)-benzenesulfoneamide, 5-[2-(5,6-diethyl-indan-2-ylamino)-1-hydroxy-ethyl]-8-hydroxy-1*H*-quinolin-2-one, 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butyl-amino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)1,2,4-triazol-3-yl]-2-methyl]-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof. According to the instant invention more preferred beta₂ agonists **2a** are selected from the group consisting of bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4-{6-[2-hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one, 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]-ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

More preferably, the betamimetics **2a** used as within the compositions according to the invention are selected from among fenoterol, formoterol, salmeterol, 3-(4-{6-[2-hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H-*quinolin-2-one, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, optionally in the form of the racemates, the enantiomers, the diastereomers and optionally the pharmacologically acceptable acid addition salts thereof, and the hydrates thereof.

Of the betamimetics mentioned above the compounds formoterol **2a.1,** salmeterol **2a.2,** 3-(4-{6-[2-hydroxy-2-(4-hydroxy-3-hydroxymethyl-phenyl)-ethylamino]-hexyloxy}-butyl)-benzenesulfoneamide **2a.3,** and 5-[2-(5,6-diethyl-indan-2-ylamino)-1-hydroxy-ethyl]-8-hydroxy-1*H*-quinolin-2-one **2a.4** are particularly preferred, optionally in the form of the racemates, the enantiomers, the diastereomers and optionally the pharmacologically acceptable acid addition salts thereof, and the hydrates thereof.

Of the betamimetics mentioned above the compounds formoterol and salmeterol are particularly preferred, optionally in the form of the racemates, the enantiomers, the diastereomers and optionally the pharmacologically acceptable acid addition salts thereof, and the hydrates thereof.

Examples of pharmacologically acceptable acid addition salts of the betamimetics **2a** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, 4-phenylcinnamic acid, 5-(2.4-difluorophenyl)salicylic acid or maleic acid. If desired, mixtures of the abovementioned acids may also be used to prepare the salts of **2a.**

According to the invention, the salts of the betamimetics **2a** selected from among the hydrochloride, hydrobromide, sulphate, phosphate, fumarate, methanesulphonate, 4-phenylcinnamate, 5-(2.4-difluorophenyl)salicylate, maleate and xinafoate are preferred. Particularly preferred are the salts of **2a** in the case of salmeterol selected from among the hydrochloride, sulphate, 4-phenylcinnamate, 5-(2.4-difluorophenyl)salicylate and xinafoate, of which the 4-phenylcinnamate, 5-(2.4-difluorophenyl)salicylate and especially xinafoate are particularly important. Particularly preferred are the salts of **2a** in the case of formoterol selected from the hydrochloride, sulphate and fumarate, of which the hydrochloride and fumarate are particularly preferred. Of exceptional importance according to the invention is formoterol fumarate.

Salts of salmeterol, formoterol, 3-(4-{6-[2-hydroxy-2-(4-hydroxy-3-hydroxymethylphenyl)-ethylamino]-hexyloxy}-butyl)-benzenesulfoneamide, and 5-[2-(5,6-diethylindan-2-ylamino)-1-hydroxy-ethyl]-8-hydroxy-1*H*-quinolin-2-one, are preferably used as the betamimetics **2a** according to the invention. Of particular importance according to the invention are salmeterol and formoterol salts. Any reference to the term betamimetics **2a** also includes a reference to the relevant enantiomers or mixtures thereof.

In the pharmaceutical compositions according to the invention, the compounds **2a** may be present in the form of their racemates, enantiomers or mixtures thereof. The separation of the enantiomers from the racemates may be carried out using methods known in the art (e.g. by chromatography on chiral phases, etc.). If the compounds **2a** are used in the form of their enantiomers, it is particularly preferable to use the enantiomers in the R configuration at the C-OH group.

As an example, any reference to the most preferred compounds **2a** according to the invention, the salts of salmeterol and formoterol, also includes the relevant enantiomeric salts of *R*-salmeterol, *S*-salmeterol, *R,R*-formoterol, *S,S*-formoterol, *R,S*-formoterol, *S,R*-formoterol and the mixtures thereof, while the enantiomeric salts of *R*-salmeterol and *R,R*-formoterol are of particular importance. The compounds **2a** may also be present according to the invention in the form of the hydrates or solvates thereof.

Preferred betamimetics **2a** according to the invention which are not in salt form include the free base of formoterol, salmeterol whereas the particularly preferred compounds **2a** according to the invention are salmeterol xinafoate or formoterol fumarate.

Within the scope of the present invention the betamimetics **2a** may possibly also be referred to as sympathomimetics or beta-₂-agonists (β₂-agonists). All these terms are to be regarded as interchangeable for the purposes of the present invention.

Besides therapeutically effective quantities of **1** and **2a** the pharmaceutical compositions may contain in addition a pharmaceutically acceptable carrier. The present invention encompasses both pharmaceutical compositions with or without pharmaceutically acceptable carriers.

Especially preferred pharmaceutical compositions according to the invention comprise the following specific combinations of EGFR kinase inhibitors **1** and beta-2 mimetics **2a,** either as free bases or pharmacologically acceptable acid addition salts:
**1.1** and **2a.1, 1.4** and **2a.1, 1.6** and **2a.1, 1.8** and **2a.1, 1.9** and **2a.1, 1.14** and **2a.1, 1.17** and **2a.1, 1.19** and **2a.1, 1.21** and **2a.1, 1.23** and **2a.1, 1.24** and **2a.1, 1.27** and **2a.1, 1.28** and **2a.1, 1.30** and **2a.1, 1.34** and **2a.1, 1.35** and **2a.1, 1.37** and **2a.1, 1.38** and **2a.1, 1.40** and **2a.1, 1.42** and **2a.1, 1.43** and **2a.1, 1.44** and **2a.1, 1.48** and **2a.1, 1.52** and **2a.1, 1.55** and **2a.1, 1.57** and **2a.1, 1.59** and **2a.1, 1.60** and **2a.1, 1.63** and **2a.1, 1.64** and **2a.1, 1.66** and **2a.1, 1.67** and **2a.1, 1.69** and **2a.1, 1.70** and **2a.1, 1.71** and **2a.1, 1.72** and **2a.1, 1.78** and **2a.1, 1.82** and **2a.1, 1.83** and **2a.1, 1.84** and **2a.1, 1.88** and **2a.1, 1.90** and **2a.1, 1.91** and **2a.1, 1.94** and **2a.1, 1.95** and **2a.1;**
1.1 and **2a.2, 1.4** and **2a.2, 1.6** and **2a.2, 1.8** and **2a.2, 1.9** and **2a.2, 1.14** and **2a.2, 1.17** and **2a.2, 1.19** and **2a.2, 1.21** and **2a.2, 1.23** and **2a.2, 1.24** and **2a.2, 1.27** and **2a.2, 1.28** and **2a.2, 1.30** and **2a.2, 1.34** and **2a.2, 1.35** and **2a.2, 1.37** and **2a.2, 1.38** and **2a.2, 1.40** and **2a.2, 1.42** and **2a.2, 1.43** and **2a.2, 1.44** and **2a.2, 1.48** and **2a.2, 1.52** and **2a.2, 1.55** and **2a.2, 1.57** and **2a.2, 1.59** and **2a.2, 1.60** and **2a.2, 1.63** and **2a.2, 1.64** and **2a.2, 1.66** and **2a.2, 1.67** and **2a.2, 1.69** and **2a.2, 1.70** and **2a.2, 1.71** and **2a.2, 1.72** and **2a.2, 1.78** and **2a.2, 1.82** and **2a.2, 1.83** and **2a.2, 1.84** and **2a.2, 1.88** and **2a.2, 1.90** and **2a.2, 1.91** and **2a.2, 1.94** and **2a.2, 1.95** and **2a.2;**
**1.1** and **2a.3, 1.4** and **2a.3, 1.6** and **2a.3, 1.8** and **2a.3, 1.9** and **2a.3, 1.14** and **2a.3, 1.17** and **2a.3, 1.19** and **2a.3, 1.21** and **2a.3, 1.23** and **2a.3, 1.24** and **2a.3, 1.27** and **2a.3, 1.28** and **2a.3, 1.30** and **2a.3, 1.34** and **2a.3, 1.35** and **2a.3, 1.37** and **2a.3, 1.38** and **2a.3, 1.40** and **2a.3, 1.42** and **2a.3, 1.43** and **2a.3, 1.44** and **2a.3, 1.48** and **2a.3, 1.52** and **2a.3, 1.55** and **2a.3, 1.57** and **2a.3, 1.59** and **2a.3, 1.60** and **2a.3, 1.63** and **2a.3, 1.64** and **2a.3, 1.66** and **2a.3, 1.67** and **2a.3, 1.69** and **2a.3, 1.70** and **2a.3, 1.71** and **2a.3, 1.72** and **2a.3, 1.78** and **2a.3, 1.82** and **2a.3, 1.83** and **2a.3, 1.84** and **2a.3, 1.88** and **2a.3, 1.90** and **2a.3, 1.91** and **2a.3, 1.94** and **2a.3, 1.95** and **2a.3;**
**1.1** and **2a.4, 1.4** and **2a.4, 1.6** and **2a.4, 1.8** and **2a.4, 1.9** and **2a.4, 1.14** and **2a.4, 1.17** and **2a.4, 1.19** and **2a.4, 1.21** and **2a.4, 1.23** and **2a.4, 1.24** and **2a.4, 1.27** and **2a.4, 1.28** and **2a.4, 1.30** and **2a.4, 1.34** and **2a.4, 1.35** and **2a.4, 1.37** and **2a.4, 1.38** and **2a.4, 1.40** and **2a.4, 1.42** and **2a.4, 1.43** and **2a.4, 1.44** and **2a.4, 1.48** and **2a.4, 1.52** and **2a.4, 1.55** and **2a.4, 1.57** and **2a.4, 1.59** and **2a.4, 1.60** and **2a.4, 1.63** and **2a.4, 1.64** and **2a.4, 1.66** and **2a.4, 1.67** and **2a.4, 1.69** and **2a.4, 1.70** and **2a.4, 1.71** and **2a.4, 1.72** and **2a.4, 1.78** and **2a.4, 1.82** and **2a.4, 1.83** and **2a.4, 1.84** and **2a.4, 1.88** and **2a.4, 1.90** and **2a.4, 1.91** and **2a.4, 1.94** and **2a.4, 1.95** and **2a.4.**

The proportions in which the active substances **1** and **2a** may be used in the active substance combinations according to the invention are variable. Active substances **1** and **2a** may possibly be present in the form of salts, solvates or hydrates. Depending on the choice of the compounds **1** and **2a,** the weight ratios which may be used within the scope of the present invention vary on the basis of the different molecular weights of the various salt forms. The pharmaceutical combinations according to the invention may contain **1** and **2a** generally in ratios by weight ranging from 15 000 : 1 to 1 : 10, preferably from 6 000 : 1 to 10 : 1, e.g. 3 000 : 1 to 100 : 1.

The weight ratios specified hereinbefore and below are based on the free bases of the actives.

The pharmaceutical combinations according to the invention may contain **1** and formoterol **2a.1,** for example, in ratios by weight ranging from 15 000 : 1 to 1 : 1, preferably from 10 000 : 1 to 100 : 1, more preferably from 5000 : 1 to 500 : 1, e.g. from 5000 : 1 to 1 000 : 1.

For example, without restricting the scope of the invention thereto, combinations of **1** and **2** according to the invention may contain the EGFR-inhibitor **1** and formoterol **2a.1** in the following weight ratios: 15000:1, 14500:1, 14000:1, 13500:1, 13000:1, 12500:1, 12000:1, 11500:1, 11000:1, 10500:1, 10000:1, 9500:1, 9000:1, 8500:1, 8000:1, 7500:1, 7000:1, 6500:1, 6000:1, 5500:1, 5000:1, 4500:1, 4000:1, 3500:1, 3000:1, 2500:1, 2000:1, 1500:1, 1000:1, 900:1, 800:1, 700:1, 600:1, 500:1, 400:1, 300:1, 200:1.

The pharmaceutical compositions according to the invention containing the combinations of **1** and **2a.1** are normally administered so that **1** and formoterol **2a.1** are present together in doses of 5 to 15000µg, preferably from 10 to 10000µg, more preferably from 15 to 5000µg, better still from 20 to 2000µg per single dose.

For example, combinations of any of EGFR-inhibitors **1.1** to **1.101**, especially those characterized as preferred hereinbefore, and **2a.1** according to the invention contain a quantity of the actives such that the total dosage per single dose is about 100µg, 105µg, 110µg, 115µg, etc. (add stepwise 5µg) up to 15000µg.

It is clear to anyone skilled in the art that the suggested dosages per single dose specified above are not to be regarded as being limited to the numerical values actually stated. Fluctuations of about ± 2.5 µg, particularly in the decimal range, are also included, as will be apparent to the skilled man. In these dosage ranges, the active substances **1** and **2a** may be present in the weight ratios given above.

For example, without restricting the scope of the invention thereto, the combinations in which any of the preferred EGFR inhibitors **1.1, 1.4, 1.6, 1.8, 1.9, 1.14, 1.17, 1.19, 1.21, 1.23, 1.24, 1.27, 1.28, 1.30, 1.34, 1.35, 1.37, 1.38, 1.40, 1.42, 1.43, 1.44, 1.48, 1.52, 1.55, 1.57, 1.59, 1.60, 1.63, 1.64, 1.66, 1.67, 1.69, 1.70, 1.71, 1.72, 1.78, 1.82, 1.83, 1.84, 1.88, 1.90, 1.91, 1.94** and **1.95** is used and in which **2a** denotes formoterol fumarate, the pharmaceutical compositions according to the invention may contain for instance the following quantities for each single dose: 10µg of **1** and 2.9µg of **2a,** 10µg of **1** and 5.7µg of **2a,** 10µg of **1** and 11.5µg of **2a,** 10µg of **1** and 17.2µg of **2a,** 10µg of **1** and 22.9µg of **2a,** 10µg of **1** and 28.5µg of **2a,**
100µg of **1** and 2.9µg of **2a,** 100µg of **1** and 5.7µg of **2a,** 100µg of **1** and 11.5µg of **2a,** 100µg of **1** and 17.2µg of **2a,** 100µg of **1** and 22.9µg of **2a,** 100µg of **1** and 28.5µg of **2a,**
500µg of **1** and 2.9mg of **2a,** 500µg of **1** and 5.7µg of **2a,** 500µg of **1** and 11.5µg of **2a,** 500µg of **1** and 17.2µg of **2a,** 500µg of **1** and 22.9µg of **2a,** 500µg of **1** and 28.5µg of **2a,**
1000µg of **1** and 2.9µg of **2a,** 1000µg of **1** and 5.7µg of **2a,** 1000µg of **1** and 11.5µg of **2a,** 1000µg of **1** and 17.2µg of **2a,** 1000µg of **1** and 22.9µg of **2a,** 1000µg of **1** and 28.5µg of **2a,** 1000µg of **1** and 2.9µg of **2a,**
2000µg of **1** and 5.7µg of **2a,** 2000µg of **1** and 11.5µg of **2a,** 2000µg of **1** and 17.2µg of **2a,** 2000µg of **1** and 22.9µg of **2a,** 2000µg of **1** and 28.5µg of **2a,**
3000µg of **1** and 5.7µg of **2a,** 3000µg of **1** and 11.5µg of **2a,** 3000µg of **1** and 17.2µg of **2a,** 3000µg of **1** and 22.9µg of **2a,** 3000µg of **1** and 28.5µg of **2a,**
4000µg of **1** and 5.7µg of **2a,** 4000µg of **1** and 11.5µg of **2a,** 4000µg of **1** and 17.2µg of **2a,** 4000µg of **1** and 22.9µg of **2a,** 4000µg of **1** and 28.5µg of **2a,**
5000µg of **1** and 5.7µg of **2a,** 5000µg of **1** and 11.5µg of **2a,** 5000µg of **1** and 17.2µg of **2a,** 5000µg of **1** and 22.9µg of **2a,** 5000µg of **1** and 28.5µg of **2a,**
6000µg of **1** and 5.7µg of **2a,** 6000µg of **1** and 11.5µg of **2a,** 6000µg of **1** and 17.2µg of **2a,** 6000µg of **1** and 22.9µg of **2a,** 6000µg of **1** and 28.5µg of **2a,**
7000µg of **1** and 5.7µg of **2a,** 7000µg of **1** and 11.5µg of **2a,** 7000µg of **1** and 17.2µg of **2a,** 7000µg of **1** and 22.9µg of **2a,** 7000µg of **1** and 28.5µg of **2a,**
8000µg of **1** and 5.7µg of **2a,** 8000µg of **1** and 11.5µg of **2a,** 8000µg of **1** and 17.2µg of **2a,** 8000µg of **1** and 22.9µg of **2a,** 8000µg of **1** and 28.5µg of **2a,**
9000µg of **1** and 5.7µg of **2a,** 9000µg of **1** and 11.5µg of **2a,** 9000µg of **1** and 17.2µg of **2a,** 9000µg of **1** and 22.9µg of **2a,** 9000µg of **1** and 28.5µg of **2a,**
10000µg of **1** and 5.7µg of **2a,** 10000µg of **1** and 11.5µg of **2a,** 10000µg of **1** and 17.2µg of **2a,** 10000µg of **1** and 22.9µg of **2a,** 10000µg of **1** and 28.5µg of **2a,** 12500µg of **1** and 5.7µg of **2a,** 12500µg of **1** and 11.5µg of **2a,** 12500µg of **1** and 17.2µg of **2a,** 12500µg of **1** and 22.9µg of **2a,** 12500µg of **1** and 28.5µg of **2a,** 15000µg of **1** and 5.7µg of **2a,** 15000µg of **1** and 11.5µg of **2a,** 15000µg of **1** and 17.2µg of **2a,** 15000µg of **1** and 22.9µg of **2a,** 15000µg of **1** and 28.5µg of **2a.**

For example, the active substance combinations according to the invention may contain the component **1** together with salmeterol **2a.2** in ratios by weight in the range from about 5000:1 to 20:1, preferably 2500:1 to 50:1, more preferably 1000:1 to 100:1, most preferred from 500:1 to 150:1.

For example, without restricting the scope of the invention thereto, preferred combinations of **1** and **2a.2** according to the invention may contain any of EGFR-inhibitors **1.1** to **1.101,** especially those characterized as preferred hereinbefore, and salmeterol **2a.2** in the following ratios by weight:
5000:1, 4500:1, 4000:1, 3500:1, 3000:1, 2500:1, 2000:1, 1500:1, 1000:1, 900:1, 800:1, 700:1, 600:1, 500:1, 400:1, 300:1, 200:1, 100:1, 90:1, 80:1,70:1, 60:1, 50:1, 40:1, 30:1, 20:1.

The pharmaceutical compositions according to the invention containing the combinations of **1** and **2a.2** are usually administered so that **1** and salmeterol **2a.2** are present together in dosages of 100µg to 100000µg, preferably from 500µg to 50000µg, more preferably from 1000µg to 10000µg per single dose.

For example, combinations of any of EGFR-inhibitors **1.1** to **1.101,** especially those characterized as preferred hereinbefore, and salmeterol **2a.2** according to the invention contain a quantity of the actives such that the total dosage per single dose is about 100µg, 150µg, 200µg, 250µg, etc. (add stepwise 50µg) up to 50000µg.

It is clear to anyone skilled in the art that the suggested dosages per single dose specified above are not to be regarded as being limited to the numerical values actually stated. Fluctuations of about ± 2.5µg, particularly in the decimal range, are also included, as will be apparent to the skilled man. In these dosage ranges, the active substances **1** and **2a.2** may be present in the weight ratios given above.

For example, without restricting the scope of the invention thereto, the combinations in which any of the preferred EGFR inhibitors **1.1, 1.4, 1.6, 1.8, 1.9, 1.14, 1.17, 1.19, 1.21, 1.23, 1.24, 1.27, 1.28, 1.30, 1.34, 1.35, 1.37, 1.38, 1.40, 1.42, 1.43, 1.44, 1.48, 1.52, 1.55, 1.57, 1.59, 1.60, 1.63, 1.64, 1.66, 1.67, 1.69, 1.70, 1.71, 1.72, 1.78, 1.82, 1.83, 1.84, 1.88, 1.90, 1.91, 1.94** and **1.95** is used and in which **2a** denotes salmeterol xinafoate, the pharmaceutical compositions according to the invention may contain for instance the following quantities for each single dose: 100µg of **1** and 18.2µg of **2a,** 100µg of **1** and 36.3µg of **2a,** 100µg of **1** and 72.6µg of **2a,** 100µg of **1** and 108.9µg of **2a,** 100µg of **1** and 145.2µg of **2a,** 100µg of **1** and 290.4µg of **2a,** 200µg of **1** and 18.2µg of **2a,** 200µg of **1** and 36.3µg of **2a,** 200µg of **1** and 72.6µg of **2a,** 200µg of **1** and 108.9µg of **2a,** 200µg of **1** and 145.2µg of **2a,** 200µg of **1** and 290.4µg of **2a,** 500µg of **1** and 18.2µg of **2a,** 500µg of **1** and 36.3µg of **2a,** 500µg of **1** and 72.6µg of **2a,** 500µg of **1** and 108.9µg of **2a,** 500µg of **1** and 145.2µg of **2a,** 500µg of **1** and 290.4µg of **2a,** 1000µg of **1** and 18.2µg of **2a,** 1000µg of **1** and 36.3µg of **2a,** 1000µg of **1** and 72.6µg of **2a,** 1000µg of **1** and 108.9µg of **2a,** 1000µg of **1** and 145.2µg of **2a,** 1000µg of **1** and 290.4µg of **2a,** 10000µg of **1** and 18.2µg of **2a,** 10000µg of **1** and 36.3µg of **2a,** 10000µg of **1** and 72.6µg of **2a,** 10000µg of **1** and 108.9µg of **2a,** 10000µg of **1** and 145.2µg of **2a,** 10000µg of **1** and 290.4µg of **2a,** 25000µg of **1** and 18.2µg of **2a,** 25000µg of **1** and 36.3µg of **2a,** 25000µg of **1** and 72.6µg of **2a,** 25000µg of **1** and 108.9µg of **2a,** 25000µg of **1** and 145.2µg of **2a,** 25000µg of **1** and 290.4µg of **2a,** 50000µg of **1** and 18.2µg of **2a,** 50000µg of **1** and 36.3µg of **2a,** 50000µg of **1** and 72.6µg of **2a,** 50000µg of **1** and 108.9µg of **2a,** 50000µg of **1** and 145.2µg of **2a,** 50000µg of **1** and 290.4µg of **2a.**

### Pharmaceutical compositions comprising an EGFR kinase inhibitor 1 and a steroid 2b:

One embodiment of the invention is a pharmaceutical composition comprising an EGFR kinase inhibitor **1** and a steroid **2b.** Binary compositions containing only one active **1** and one active **2b,** optionally together with one or more pharmaceutically acceptable excipients or carriers, are preferred. In the pharmaceutical combinations according to the invention preferred steroids **2b,** which are optionally also referred to as corticosteroids, are selected from the group consisting of methyl prednisolone, prednisone, butixocort propionate, RPR-106541, flunisolide, beclomethasone, triamcinolone, budesonide, fluticasone, mometasone, ciclesonide, rofleponide, ST-126, dexamethasone, 6α,9α-difluoro-17α-[(2-furanylcarbonyl)oxy]-11β-hydroxy-16α-methyl-3-oxo-androsta-1,4-diene-17β-carbothioic acid (S)-fluoromethyl ester, and 6α,9α-difluoro-11β-hydroxy-16α-methyl-3-oxo-17α-propionyloxy-androsta-1,4-diene-17β-carbothioic acid (S)-(2-oxo-tetrahydro-furan-3S-yl) ester.

Preferably, the compound **2b** is selected from among flunisolide, beclomethasone, triamcinolone, budesonide, fluticasone, mometasone, ciclesonide, rofleponide, ST-126, dexamethasone, 6α,9α-difluoro-17α-[(2-furanylcarbonyl)oxy]-11β-hydroxy-16α-methyl-3-oxo-androsta-1,4-diene-17β-carbothioic acid (S)-fluoromethyl ester, and 6α,9α-difluoro-11β-hydroxy-16α-methyl-3-oxo-17α-propionyloxy-androsta-1,4-diene-17β-carbothioic acid (S)-(2-oxo-tetrahydro-furan-3S-yl) ester.

More preferably, the compound **2b** is selected from among budesonide **2b.1**, fluticasone **2b.2** (in particular fluticasone propionate ester), mometasone **2b.3** (for instance as the furoate ester), ciclesonide **2b.4,** 6α,9α-difluoro-17α-[(2-furanylcarbonyl)oxy]-11β-hydroxy-16α-methyl-3-oxo-androsta-1,4-diene-17β-carbothioic acid (S)-fluoromethyl ester **2b.5,** and 6α,9α-difluoro-11β-hydroxy-16α-methyl-3-oxo-17α-propionyloxy-androsta-1,4-diene-17β-carbothioic acid (S)-(2-oxo-tetrahydrofuran-3S-yl) ester **2b.6.**

Especially preferred pharmaceutical compositions according to the invention comprise the following specific combinations of EGFR kinase inhibitors **1** and steroids **2b,** either as free bases or pharmacologically acceptable acid addition salts, wherein in case of fluticasone **2b.2** the propionate ester and in case of mometasone **2b.3** the furoate ester are particularly preferred:
**1.1** and **2b.1, 1.4** and **2b.1, 1.6** and **2b.1, 1.8** and **2b.1, 1.9** and **2b.1, 1.14** and **2b.1, 1.17** and **2b.1, 1.19** and **2b.1, 1.21** and **2b.1, 1.23** and **2b.1, 1.24** and **2b.1**, **1.27** and **2b.1, 1.28** and **2b.1, 1.30** and **2b.1**, **1.34** and **2b.1, 1.35** and **2b.1, 1.37** and **2b.1, 1.38** and **2b.1, 1.40** and **2b.1, 1.42** and **2b.1, 1.43** and **2b.1, 1.44** and **2b.1, 1.48** and **2b.1, 1.52** and **2b.1, 1.55** and **2b.1, 1.57** and **2b.1, 1.59** and **2b.1, 1.60** and **2b.1, 1.63** and **2b.1, 1.64** and **2b.1, 1.66** and **2b.1, 1.67** and **2b.1, 1.69** and **2b.1, 1.70** and **2b.1**, **1.71** and **2b.1, 1.72** and **2b.1, 1.78** and **2b.1, 1.82** and **2b.1, 1.83** and **2b.1, 1.84** and **2b.1, 1.88** and **2b.1, 1.90** and **2b.1, 1.91** and **2b.1, 1.94** and **2b.1, 1.95** and **2b.1;**
**1.1** and **2b.2, 1.4** and **2b.2, 1.6** and **2b.2, 1.8** and **2b.2, 1.9** and **2b.2, 1.14** and **2b.2, 1.17** and **2b.2, 1.19** and **2b.2, 1.21** and **2b.2, 1.23** and **2b.2, 1.24** and **2b.2, 1.27** and **2b.2, 1.28** and **2b.2, 1.30** and **2b.2, 1.34** and **2b.2, 1.35** and **2b.2, 1.37** and **2b.2, 1.38** and **2b.2, 1.40** and **2b.2, 1.42** and **2b.2, 1.43** and **2b.2, 1.44** and **2b.2, 1.48** and **2b.2, 1.52** and **2b.2, 1.55** and **2b.2, 1.57** and **2b.2, 1.59** and **2b.2, 1.60** and **2b.2, 1.63** and **2b.2, 1.64** and **2b.2, 1.66** and **2b.2, 1.67** and **2b.2, 1.69** and **2b.2, 1.70** and **2b.2, 1.71** and **2b.2, 1.72** and **2b.2, 1.78** and **2b.2, 1.82** and **2b.2, 1.83** and **2b.2, 1.84** and **2b.2, 1.88** and **2b.2, 1.90** and **2b.2, 1.91** and **2b.2, 1.94** and **2b.2, 1.95** and **2b.2;**
**1.1** and **2b.3, 1.4** and **2b.3, 1.6** and **2b.3, 1.8** and **2b.3, 1.9** and **2b.3, 1.14** and **2b.3, 1.17** and **2b.3, 1.19** and **2b.3, 1.21** and **2b.3, 1.23** and **2b.3, 1.24** and **2b.3, 1.27** and **2b.3, 1.28** and **2b.3, 1.30** and **2b.3, 1.34** and **2b.3, 1.35** and **2b.3, 1.37** and **2b.3, 1.38** and **2b.3, 1.40** and **2b.3, 1.42** and **2b.3, 1.43** and **2b.3, 1.44** and **2b.3, 1.48** and **2b.3, 1.52** and **2b.3, 1.55** and **2b.3, 1.57** and **2b.3, 1.59** and **2b.3, 1.60** and **2b.3, 1.63** and **2b.3, 1.64** and **2b.3, 1.66** and **2b.3, 1.67** and **2b.3, 1.69** and **2b.3, 1.70** and **2b.3, 1.71** and **2b.3, 1.72** and **2b.3, 1.78** and **2b.3, 1.82** and **2b.3, 1.83** and **2b.3, 1.84** and **2b.3, 1.88** and **2b.3, 1.90** and **2b.3, 1.91** and **2b.3, 1.94** and **2b.3, 1.95** and **2b.3;**
**1.1** and **2b.4, 1.4** and **2b.4, 1.6** and **2b.4, 1.8** and **2b.4, 1.9** and **2b.4, 1.14** and **2b.4, 1.17** and **2b.4, 1.19** and **2b.4, 1.21** and **2b.4, 1.23** and **2b.4, 1.24** and **2b.4, 1.27** and **2b.4, 1.28** and **2b.4, 1.30** and **2b.4, 1.34** and **2b.4, 1.35** and **2b.4, 1.37** and **2b.4, 1.38** and **2b.4, 1.40** and **2b.4, 1.42** and **2b.4, 1.43** and **2b.4, 1.44** and **2b.4, 1.48** and **2b.4, 1.52** and **2b.4, 1.55** and **2b.4, 1.57** and **2b.4, 1.59** and **2b.4, 1.60** and **2b.4, 1.63** and **2b.4, 1.64** and **2b.4, 1.66** and **2b.4, 1.67** and **2b.4, 1.69** and **2b.4, 1.70** and **2b.4, 1.71** and **2b.4, 1.72** and **2b.4, 1.78** and **2b.4, 1.82** and **2b.4, 1.83** and **2b.4, 1.84** and **2b.4, 1.88** and **2b.4, 1.90** and **2b.4, 1.91** and **2b.4, 1.94** and **2b.4, 1.95** and **2b.4;**
**1.1** and **2b.5, 1.4** and **2b.5, 1.6** and **2b.5, 1.8** and **2b.5, 1.9** and **2b.5, 1.14** and **2b.5, 1.17** and **2b.5, 1.19** and **2b.5, 1.21** and **2b.5, 1.23** and **2b.5, 1.24** and **2b.5, 1.27** and **2b.5, 1.28** and **2b.5, 1.30** and **2b.5, 1.34** and **2b.5, 1.35** and **2b.5, 1.37** and **2b.5, 1.38** and **2b.5, 1.40** and **2b.5, 1.42** and **2b.5, 1.43** and **2b.5, 1.44** and **2b.5, 1.48** and **2b.5, 1.52** and **2b.5, 1.55** and **2b.5, 1.57** and **2b.5, 1.59** and **2b.5, 1.60** and **2b.5, 1.63** and **2b.5, 1.64** and **2b.5, 1.66** and **2b.5, 1.67** and **2b.5, 1.69** and **2b.5, 1.70** and **2b.5, 1.71** and **2b.5, 1.72** and **2b.5, 1.78** and **2b.5, 1.82** and **2b.5, 1.83** and **2b.5, 1.84** and **2b.5, 1.88** and **2b.5, 1.90** and **2b.5, 1.91** and **2b.5, 1.94** and **2b.5, 1.95** and **2b.5;**
**1.1** and **2b.6, 1.4** and **2b.6, 1.6** and **2b.6, 1.8** and **2b.6, 1.9** and **2b.6, 1.14** and **2b.6, 1.17** and **2b.6, 1.19** and **2b.6, 1.21** and **2b.6, 1.23** and **2b.6, 1.24** and **2b.6, 1.27** and **2b.6, 1.28** and **2b.6, 1.30** and **2b.6, 1.34** and **2b.6, 1.35** and **2b.6, 1.37** and **2b.6, 1.38** and **2b.6, 1.40** and **2b.6, 1.42** and **2b.6, 1.43** and **2b.6, 1.44** and **2b.6, 1.48** and **2b.6, 1.52** and **2b.6, 1.55** and **2b.6, 1.57** and **2b.6, 1.59** and **2b.6, 1.60** and **2b.6, 1.63** and **2b.6, 1.64** and **2b.6, 1.66** and **2b.6, 1.67** and **2b.6, 1.69** and **2b.6, 1.70** and **2b.6, 1.71** and **2b.6, 1.72** and **2b.6, 1.78** and **2b.6, 1.82** and **2b.6, 1.83** and **2b.6, 1.84** and **2b.6, 1.88** and **2b.6, 1.90** and **2b.6, 1.91** and **2b.6, 1.94** and **2b.6, 1.95** and **2b.6;**

Any reference to steroids **2b** within the scope of the present invention includes a reference to the salts or derivatives which may be formed from the steroids. Examples of possible salts or derivatives include: sodium salts, sulphobenzoates, phosphates, isonicotinates, acetates, propionates, dihydrogen phosphates, palmitates, pivalates or furoates. In some cases the compounds of formula **2b** may also occur in the form of their hydrates. Any reference to steroids **2b** within the scope of the present invention also includes a reference to the compounds **2b** in the form of their diastereomers, mixtures of diastereomers or in the form of the racemates.

The proportions in which the active substances **1** and **2b** may be used in the active substance combinations according to the invention are variable. Active substances **1** and **2b** may possibly be present in the form of their solvates or hydrates. Depending on the choice of the compounds **1** and **2b,** the weight ratios which may be used within the scope of the present invention vary on the basis of the different molecular weights of the various compounds and their different potencies.

As a rule, the pharmaceutical combinations according to the invention may contain the EGFR-inhibitor **1** and the steroid **2b** in ratios by weight ranging from 5000:1 to 1:250, preferably from 2500:1 to 1:150, more preferably 1000:1 to 1:100, most preferred from 250:1 to 1:25.

In the particularly preferred pharmaceutical combinations which contain in addition to **1** a compound selected for instance from among the group consisting of budesonide **2b.1,** fluticasone **2b.2,** mometasone **2b.3,** and ciclesonide **2b.4** as the steroid **2b,** the weight ratios of **1** to **2b** are preferably in a range from about 750:1 to 1:50, more preferably from 500:1 to 1:50.

For example, without restricting the scope of the invention thereto, preferred combinations according to the invention may contain an EGF kinase inhibitor **1** and one of the most preferred steroids selected from **2b.1, 2b.2, 2b.3** and **2b.4,** for example in the following ratios by weight (all based on free base): 500:1, 450:1, 400:1, 350:1, 300:1, 250:1, 200:1, 150:1, 100:1, 50:1, 40:1, 30:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20: 1:25, 1:30, 1:35, 1:40, 1:45, 1:50.

The pharmaceutical compositions according to the invention containing the combinations of **1** together with one of the most preferred steroids selected from **2b.1, 2b.2, 2b.3** and **2b.4** preferably are administered so that **1** and the steroid **2b** (values based on free base) are present together in dosages of 100µg to 50000µg, preferably from 500µg to 25000µg, more preferably from 2000µg to 12000µg per single dose.

For example, combinations of **1** and **2b** according to the invention contain an amount of **1** and **2b** (values based on free base) such that the total dosage per single dose is about 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 300µg, 400µg, 500µg, 600µg, 700µg, 800µg, 900µg, 1000µg, 1100µg, 1200µg, etc. (add stepwise 1000µg) up to 50000µg, or similar. It is clear to anyone skilled in the art that the suggested dosages per single dose specified above are not to be regarded as being limited to the numerical values actually stated. Fluctuations of about ± 2.5 µg, particularly in the decimal range, are also included, as will be apparent to the skilled man. In these dosage ranges, the active substances **1** and **2b** may be present in the weight ratios given above.

For example, without restricting the scope of the invention thereto, the combinations of **1** and one of the most preferred steroids **2b** selected from **2b.1, 2b.2, 2b.3** and **2b.4** may in particular contain a quantity of **1** and steroid **2b** (values based on free base) such that, for each single dose,
100µg of **1** and 25µg of **2b,** 100µg of **1** and 50µg of **2b,** 100µg of **1** and 75µg of **2b,** 100µg of **1** and 100µg of **2b,** 100µg of **1** and 125µg of **2b,** 100µg of **1** and 150µg of **2b,** 100µg of **1** and 200µg of **2b,** 100µg of **1** and 250µg of **2b,**
200µg of **1** and 25µg of **2b,** 200µg of **1** and 50µg of **2b,** 200µg of **1** and 75µg of **2b,** 200µg of **1** and 100µg of **2b,** 200µg of **1** and 125µg of **2b,** 200µg of **1** and 150µg of **2b,** 200µg of **1** and 200µg of **2b,** 200µg of **1** and 250µg of **2b,**
500µg of **1** and 25µg of **2b,** 500µg of **1** and 50µg of **2b,** 500µg of **1** and 75µg of **2b,** 500µg of **1** and 100µg of **2b,** 500µg of **1** and 125µg of **2b,** 500µg of **1** and 150µg of **2b,** 500µg of **1** and 200µg of **2b,** 500µg of **1** and 250µg of **2b,**
1000µg of **1** and 25µg of **2b,** 1000µg of **1** and 50µg of **2b,** 1000µg of **1** and 75µg of **2b**, 1000µg of **1** and 100µg of **2b,** 1000µg of **1** and 125µg of **2b,** 1000µg of **1** and 150µg of **2b,** 1000µg of **1** and 200µg of **2b,** 1000µg of **1** and 250µg of **2b,**
5000µg of **1** and 25µg of **2b,** 5000µg of **1** and 50µg of **2b,** 5000µg of **1** and 75µg of **2b,** 5000µg of **1** and 100µg of **2b,** 5000µg of **1** and 125µg of **2b,** 5000µg of **1** and 150µg of **2b,** 5000µg of **1** and 200µg of **2b,** 5000µg of **1** and 250µg of **2b,**
10000µg of **1** and 25µg of **2b,** 10000µg of **1** and 50µg of **2b,** 10000µg of **1** and 75µg of **2b,** 10000µg of **1** and 10000µg of **2b,** 10000µg of **1** and 125µg of **2b,** 10000µg of **1** and 150µg of **2b,** 10000µg of **1** and 200µg of **2b,** 10000µg of **1** and 250µg of **2b,**
25000µg of **1** and 25µg of **2b,** 25000µg of **1** and 50µg of **2b,** 25000µg of **1** and 75µg of **2b,** 25000µg of **1** and 100µg of **2b,** 25000µg of **1** and 125µg of **2b,** 25000µg of **1** and 150µg of **2b,** 25000µg of **1** and 200µg of **2b,** 25000µg of **1** and 250µg of **2b,**
50000µg of **1** and 25µg of **2b,** 50000µg of **1** and 50µg of **2b,** 50000µg of **1** and 75µg of **2b,** 50000µg of **1** and 100µg of **2b,** 50000µg of **1** and 125µg of **2b,** 50000µg of **1** and 150µg of **2b,** 50000µg of **1** and 200µg of **2b,** 50000µg of **1** and 250µg of **2b.**

From the aforementioned examples for suitable doses of the **1** and **2b** containing combinations according to the invention, the corresponding amounts of the preferably used acid addition salts of **1** and **2b** are readily calculable.

### Pharmaceutical compositions comprising an EGFR kinase inhibitor 1, a beta-2 mimetic 2a and a steroid 2b:

One embodiment of the invention is a pharmaceutical composition comprising an EGFR kinase inhibitor **1**, a beta-2 mimetic **2a** and a steroid **2b.** Ternary compositions containing one active **1**, one active **2a** and one active **2b,** optionally together with one or more pharmaceutically acceptable excipients or carriers, are preferred. Furthermore, specific components **1, 2a** and **2b** are the same as mentioned hereinbefore with regard to pharmaceutical compositions comprising only two of these classes of actives. Analogously, derivatives or racemates, enantiomers, diastereomers, pharmacologically acceptable acid addition salts and hydrates as well as preferred specific components **1**, **2a** and **2b** are the same as mentioned hereinbefore.

Especially preferred ternary pharmaceutical compositions according to the invention comprise the following specific combinations of EGFR kinase inhibitors **1,** beta-2 mimetics **2a** and steroids **2b,** either as free bases or pharmacologically acceptable acid addition salts, wherein in case of formoterol **2a.1** the fumarate, in case of salmeterol **2a.2** the xinafoate, in case of fluticasone **2b.2** the propionate ester and in case of mometasone **2b.3** the furoate ester are particularly preferred:
[Ter-1]: **1.1** and **2a.1** and **2b.1, 1.4** and **2a.1** and **2b.1, 1.6** and **2a.1** and **2b.1, 1.8** and **2a.1** and **2b.1, 1.9** and **2a.1** and **2b.1, 1.14** and **2a.1** and **2b.1, 1.17** and **2a.1** and **2b.1, 1.19** and **2a.1** and **2b.1, 1.21** and **2a.1** and **2b.1, 1.23** and **2a.1** and **2b.1, 1.24** and **2a.1** and **2b.1, 1.27** and **2a.1** and **2b.1, 1.28** and **2a.1** and **2b.1, 1.30** and **2a.1** and **2b.1, 1.34** and **2a.1** and **2b.1, 1.35** and **2a.1** and **2b.1, 1.37** and **2a.1** and **2b.1, 1.38** and **2a.1** and **2b.1, 1.40** and **2a.1** and **2b.1, 1.42** and **2a.1** and **2b.1, 1.43** and **2a.1** and **2b.1, 1.44** and **2a.1** and **2b.1, 1.48** and **2a.1** and **2b.1, 1.52** and **2a.1** and **2b.1, 1.55** and **2a.1** and **2b.1, 1.57** and **2a.1** and **2b.1, 1.59** and **2a.1** and **2b.1, 1.60** and **2a.1** and **2b.1, 1.63** and **2a.1** and **2b.1, 1.64** and **2a.1** and **2b.1, 1.66** and **2a.1** and **2b.1, 1.67** and **2a.1** and **2b.1, 1.69** and **2a.1** and **2b.1, 1.70** and **2a.1** and **2b.1, 1.71** and **2a.1** and **2b.1, 1.72** and **2a.1** and **2b.1, 1.78** and **2a.1** and **2b.1, 1.82** and **2a.1** and **2b.1, 1.83** and **2a.1** and **2b.1, 1.84** and **2a.1** and **2b.1, 1.88** and **2a.1** and **2b.1, 1.90** and **2a.1** and **2b.1, 1.91** and **2a.1** and **2b.1, 1.94** and **2a.1** and **2b.1, 1.95** and **2a.1** and **2b.1;**
[Ter-2]: **1.1** and **2a.2** and **2b.1, 1.4** and **2a.2** and **2b.1, 1.6** and **2a.2** and **2b.1, 1.8** and **2a.2** and **2b.1, 1.9** and **2a.2** and **2b.1, 1.14** and **2a.2** and **2b.1, 1.17** and **2a.2** and **2b.1, 1.19** and **2a.2** and **2b.1, 1.21** and **2a.2** and **2b.1, 1.23** and **2a.2** and **2b.1, 1.24** and **2a.2** and **2b.1, 1.27** and **2a.2** and **2b.1, 1.28** and **2a.2** and **2b.1, 1.30** and **2a.2** and **2b.1, 1.34** and **2a.2** and **2b.1, 1.35** and **2a.2** and **2b.1, 1.37** and **2a.2** and **2b.1, 1.38** and **2a.2** and **2b.1, 1.40** and **2a.2** and **2b.1, 1.42** and **2a.2** and **2b.1, 1.43** and **2a.2** and **2b.1, 1.44** and **2a.2** and **2b.1, 1.48** and **2a.2** and **2b.1, 1.52** and **2a.2** and **2b.1, 1.55** and **2a.2** and **2b.1, 1.57** and **2a.2** and **2b.1, 1.59** and **2a.2** and **2b.1, 1.60** and **2a.2** and **2b.1, 1.63** and **2a.2** and **2b.1, 1.64** and **2a.2** and **2b.1, 1.66** and **2a.2** and **2b.1, 1.67** and **2a.2** and **2b.1, 1.69** and **2a.2** and **2b.1, 1.70** and **2a.2** and **2b.1, 1.71** and **2a.2** and **2b.1, 1.72** and **2a.2** and **2b.1, 1.78** and **2a.2** and **2b.1, 1.82** and **2a.2** and **2b.1, 1.83** and **2a.2** and **2b.1, 1.84** and **2a.2** and **2b.1, 1.88** and **2a.2** and **2b.1, 1.90** and **2a.2** and **2b.1, 1.91** and **2a.2** and **2b.1, 1.94** and **2a.2** and **2b.1, 1.95** and **2a.2** and **2b.1;**
[Ter-3]: **1.1** and **2a.3** and **2b.1, 1.4** and **2a.3** and **2b.1, 1.6** and **2a.3** and **2b.1, 1.8** and **2a.3** and **2b.1, 1.9** and **2a.3** and **2b.1, 1.14** and **2a.3** and **2b.1, 1.17** and **2a.3** and **2b.1, 1.19** and **2a.3** and **2b.1, 1.21** and **2a.3** and **2b.1, 1.23** and **2a.3** and **2b.1, 1.24** and **2a.3** and **2b.1, 1.27** and **2a.3** and **2b.1, 1.28** and **2a.3** and **2b.1, 1.30** and **2a.3** and **2b.1, 1.34** and **2a.3** and **2b.1, 1.35** and **2a.3** and **2b.1, 1.37** and **2a.3** and **2b.1, 1.38** and **2a.3** and **2b.1, 1.40** and **2a.3** and **2b.1, 1.42** and **2a.3** and **2b.1, 1.43** and **2a.3** and **2b.1, 1.44** and **2a.3** and **2b.1, 1.48** and **2a.3** and **2b.1, 1.52** and **2a.3** and **2b.1, 1.55** and **2a.3** and **2b.1, 1.57** and **2a.3** and **2b.1, 1.59** and **2a.3** and **2b.1, 1.60** and **2a.3** and **2b.1, 1.63** and **2a.3** and **2b.1, 1.64** and **2a.3** and **2b.1, 1.66** and **2a.3** and **2b.1, 1.67** and **2a.3** and **2b.1, 1.69** and **2a.3** and **2b.1, 1.70** and **2a.3** and **2b.1, 1.71** and **2a.3** and **2b.1, 1.72** and **2a.3** and **2b.1, 1.78** and **2a.3** and **2b.1, 1.82** and **2a.3** and **2b.1, 1.83** and **2a.3** and **2b.1, 1.84** and **2a.3** and **2b.1, 1.88** and **2a.3** and **2b.1, 1.90** and **2a.3** and **2b.1, 1.91** and **2a.3** and **2b.1, 1.94** and **2a.3** and **2b.1, 1.95** and **2a.3** and **2b.1;**
[Ter-4]: **1.1** and **2a.4** and **2b.1, 1.4** and **2a.4** and **2b.1, 1.6** and **2a.4** and **2b.1, 1.8** and **2a.4** and **2b.1, 1.9** and **2a.4** and **2b.1, 1.14** and **2a.4** and **2b.1, 1.17** and **2a.4** and **2b.1, 1.19** and **2a.4** and **2b.1, 1.21** and **2a.4** and **2b.1, 1.23** and **2a.4** and **2b.1, 1.24** and **2a.4** and **2b.1, 1.27** and **2a.4** and **2b.1, 1.28** and **2a.4** and **2b.1, 1.30** and **2a.4** and **2b.1, 1.34** and **2a.4** and **2b.1, 1.35** and **2a.4** and **2b.1, 1.37** and **2a.4** and **2b.1, 1.38** and **2a.4** and **2b.1, 1.40** and **2a.4** and **2b.1, 1.42** and **2a.4** and **2b.1, 1.43** and **2a.4** and **2b.1, 1.44** and **2a.4** and **2b.1, 1.48** and **2a.4** and **2b.1, 1.52** and **2a.4** and **2b.1, 1.55** and **2a.4** and **2b.1, 1.57** and **2a.4** and **2b.1, 1.59** and **2a.4** and **2b.1, 1.60** and **2a.4** and **2b.1, 1.63** and **2a.4** and **2b.1, 1.64** and **2a.4** and **2b.1, 1.66** and **2a.4** and **2b.1, 1.67** and **2a.4** and **2b.1, 1.69** and **2a.4** and **2b.1, 1.70** and **2a.4** and **2b.1, 1.71** and **2a.4** and **2b.1, 1.72** and **2a.4** and **2b.1, 1.78** and **2a.4** and **2b.1, 1.82** and **2a.4** and **2b.1, 1.83** and **2a.4** and **2b.1, 1.84** and **2a.4** and **2b.1, 1.88** and **2a.4** and **2b.1, 1.90** and **2a.4** and **2b.1, 1.91** and **2a.4** and **2b.1, 1.94** and **2a.4** and **2b.1, 1.95** and **2a.4** and **2b.1;**

Further specific ternary combinations are those wherein the combinations listed under [Ter-1] to [Ter-4] the component **2b.1** is replaced by any of **2b.2, 2b.3, 2b.4, 2b.5** and **2b.6.**

The proportions in which the active substances **1, 2a** and **2b** may be used in the active substance combinations according to the invention are variable. Active substances **1, 2a** and **2b** may possibly be present in the form of their solvates or hydrates. Depending on the choice of the compounds **1, 2a** and **2b,** the weight ratios which may be used within the scope of the present invention vary on the basis of the different molecular weights of the various compounds and their different potencies. As a rule, the pharmaceutical combinations according to the invention may contain compounds **1** and **2a** in ratios by weight ranging from 15000:1 to 1:1, preferably from 6000:1 to 200:1, more preferably from 3000:1 to 500:1. At the same time the ratio of **1** to **2b** may be 250:1 to 1:250, preferably from 150:1 to 1:150, more preferably from 30:1 to 1:70, most preferably from 15:1 to 1:35.

The weight ratios specified hereinbefore and below are based on the free bases of the actives.

The ternary pharmaceutical combinations according to the invention may contain **1** and formoterol **2a.1** for example, in ratios by weight ranging from 15 000 : 1 to 200 : 1, preferably from 10 000 : 1 to 500 : 1, more preferably 5000 : 1 to 1 000 : 1.

For example, without restricting the scope of the invention thereto, preferred ternary combinations according to the invention may contain an EGF kinase inhibitor **1,** together with formoterol **2a.1** or salmeterol **2a.2** as component **2a** and budesonide or fluticasone as component **2b** in the following proportions **1 : 2a : 2b** by weight:
15000:1:60, 14500:1:60, 14000:1:60, 13500:1:60, 13000:1:60, 12500:1:60, 12000:1:60, 11500:1:60, 11000:1:60, 10500:1:60, 10000:1:60, 9500:1:60, 9000:1:60, 8500:1:60, 8000:1:60, 7500:1:60, 7000:1:60, 6500:1:60, 6000:1:60, 5500:1:60, 5000:1:60, 4500:1:60, 4000:1:60, 3500:1:60, 3000:1:60, 2500:1:60, 2000:1:60, 1500:1:60, 1000:1:60, 900:1:60, 800:1:60, 700:1:60, 600:1:60, 500:1:60, 400:1:60, 300:1:60, 200:1:60,
15000:1:100, 14500:1:200, 14000:1:500, 13500:1:1000, 13000:1:2000, 12500:1:3000, 12000:1:4000, 11500:1:5000, 11000:1:6000, 10500:1:7000, 10000:1:8000, 9500:1:9000, 9000:1:10000, 9000:1:11000, 9000:1:12000, 9000:1:13000, 9000:1:14000, 9000:1:15000, 8500:1:15000, 8000:1:16000, 7500:1:17000, 7000:1:18000, 6500:1:19000, 6000:1:20000, 5500:1:21000, 5000:1:22000, 4500:1:23000, 4000:1:24000, 3500:1:25000, 3000:1:26000, 2500:1:27000, 2000:1:28000, 1500:1:29000, 1000:1:30000, 900:1:31000, 800:1:32000, 700:1:33000, 600:1:34000, 500:1:35000, 400:1:40500, 300:1:45000, 200:1:50000,
200:1:100, 200:1:200, 200:1:500, 200:1:750, 200:1:1000, 200:1:1500, 200:1:2000, 200:1:3000, 200:1:4000, 200:1:5000, 200:1:6000, 200:1:7000, 200:1:8000, 200:1:9000, 200:1:10000, 200:1:12500, 200:1:15000, 200:1:17500, 200:1:20000, 200:1:22500, 200:1:25000, 200:1:30000, 200:1:35000, 200:1:40000, 200:1:45000, 200:1:50000.

The pharmaceutical compositions according to the invention containing the combinations of **1, 2a** and **2b** are normally administered so that **1, 2a** and **2b** are present together in doses of 10 to 50000µg, preferably from 50 to 40000µg, more preferably from 60 to 10000µg, even more preferably from 70 to 5000µg, preferably according to the invention from 100 to 1000µg, preferably from 150 to 750µg per single dose. For example, combinations of **1**, **2a** and **2b** according to the invention contain a quantity of one EGFR kinase inhibitor selected from compounds of **1.1** to **1.105,** formoterol **2a.1** or salmeterol **2a.2,** and budesonide **2b.1** or fluticasone **2b.2,** such that the total dosage per single dose is about 50µg, 60µg, 70µg, 80µg, etc. (add stepwise 10µg) up to 50000µg. The amounts specified hereinbefore and below are based on the free bases of the actives.

It is clear to anyone skilled in the art that the suggested dosages per single dose specified above are not to be regarded as being limited to the numerical values actually stated. Fluctuations of about ± 2.5 µg, particularly in the decimal range, are also included, as will be apparent to the skilled man. In these dosage ranges, the active substances **1** and **2a** and **2b** may be present in the weight ratios given above.

For example and without restricting the scope of the invention thereto, the combinations of **1, 2a** and **2b** according to the invention may contain an amount of an EGFR kinase inhibitor **1** selected from compounds of **1.1** to **1.101,** a steroid **2b** selected from budesonide **2b.1** and fluticasone **2b.2,** and a beta₂ agonist **2a** selected from formoterol **2a.1** and salmeterol **2a.2,** such that in each single dose 100µg of **1** and 25µg of **2b** and 25µg of **2a,** 100µg of **1** and 50µg of **2b** and 25µg of **2a,** 100µg of **1** and 100µg of **2b** and 25µg of **2a,** 100µg of **1** and 125µg of **2b** and 25µg of **2a,** 100µg of **1** and 200µg of **2b** and 25µg of **2a,** 100µg of **1** and 250µg of **2b** and 25µg of **2a,** 1000µg of **1** and 25µg of **2b** and 25µg of **2a,** 1000µg of **1** and 50µg of **2b** and 25µg of **2a,** 1000µg of **1** and 100µg of **2b** and 25µg of **2a,** 1000µg of **1** and 125µg of **2b** and 25µg of **2a,** 1000µg of **1** and 200µg of **2b** and 25µg of **2a,** 1000µg of **1** and 250µg of **2b** and 25µg of **2a,** 10000µg of **1** and 25µg of **2b** and 25µg of **2a,** 10000µg of **1** and 50µg of **2b** and 25µg of **2a,** 10000µg of **1** and 100µg of **2b** and 25µg of **2a,** 10000µg of **1** and 125µg of **2b** and 25µg of **2a,** 10000µg of **1** and 200µg of **2b** and 25µg of **2a,** 10000µg of **1** and 250µg of **2b** and 25µg of **2a,** 25000µg of **1** and 25µg of **2b** and 25µg of **2a,** 25000µg of **1** and 50µg of **2b** and 25µg of **2a,** 25000µg of **1** and 100µg of **2b** and 25µg of **2a,** 25000µg of **1** and 125µg of **2b** and 25µg of **2a,** 25000µg of **1** and 200µg of **2b** and 25µg of **2a,** 25000µg of **1** and 250µg of **2b** and 25µg of **2a,** 50000µg of **1** and 25µg of **2b** and 25µg of **2a,** 50000µg of **1** and 50µg of **2b** and 25µg of **2a,** 50000µg of **1** and 100µg of **2b** and 25µg of **2a,** 50000µg of **1** and 125µg of **2b** and 25µg of **2a,** 50000µg of **1** and 200µg of **2b** and 25µg of **2a,** 50000µg of **1** and 250µg of **2b** and 25µg of **2a,** 75000µg of **1** and 25µg of **2b** and 25µg of **2a,** 75000µg of **1** and 50µg of **2b** and 25µg of **2a,** 75000µg of **1** and 100µg of **2b** and 25µg of **2a,** 75000µg of **1** and 125µg of **2b** and 25µg of **2a,** 75000µg of **1** and 200µg of **2b** and 25µg of **2a,** 100µg of **1** and 25µg of **2b** and 50µg of **2a,** 100µg of **1** and 50µg of **2b** and 50µg of **2a,** 100µg of **1** and 100µg of **2b** and 50µg of **2a,** 100µg of **1** and 125µg of **2b** and 50µg of **2a,** 100µg of **1** and 200µg of **2b** and 50µg of **2a,** 100µg of **1** and 250µg of **2b** and 50µg of **2a,** 1000µg of **1** and 25µg of **2b** and 50µg of **2a,** 1000µg of **1** and 50µg of **2b** and 50µg of **2a,** 1000µg of **1** and 100µg of **2b** and 50µg of **2a,** 1000µg of **1** and 125µg of **2b** and 50µg of **2a,** 1000µg of **1** and 200µg of **2b** and 50µg of **2a,** 1000µg of **1'** and 250µg of **2b** and 50µg of **2a,** 10000µg of **1** and 25µg of **2b** and 50µg of **2a,** 10000µg of **1** and 50µg of **2b** and 50µg of **2a,** 10000µg of **1** and 100µg of **2b** and 50µg of **2a,** 10000µg of **1** and 125µg of **2b** and 50µg of **2a,** 10000µg of **1** and 200µg of **2b** and 50µg of **2a,** 10000µg of **1** and 250µg of **2b** and 50µg of **2a,** 25000µg of **1** and 25µg of **2b** and 50µg of **2a,** 25000µg of **1** and 50µg of **2b** and 50µg of **2a,** 25000µg of **1** and 100µg of **2b** and 50µg of **2a,** 25000µg of **1** and 125µg of **2b** and 50µg of **2a,** 25000µg of **1** and 200µg of **2b** and 50µg of **2a,** 25000µg of **1** and 250µg of **2b** and 50µg of **2a,** 50000µg of **1** and 25µg of **2b** and 50µg of **2a,** 75000µg of **1** and 50µg of **2b** and 50µg of **2a,** 50000µg of **1** and 100µg of **2b** and 50µg of **2a,** 50000µg of **1** and 125µg of **2b** and 50µg of **2a,** 50000µg of **1** and 200µg of **2b** and 50µg of **2a,** 50000µg of **1** and 250µg of **2b** and 50µg of **2a,** 75000µg of **1** and 25µg of **2b** and 50µg of **2a,** 75000µg of **1** and 50µg of **2b** and 50µg of **2a,** 75000µg of **1** and 100µg of **2b** and 50µg of **2a,** 75000µg of **1** and 125µg of **2b** and 50µg of **2a,** 75000µg of **1** and 200µg of **2b** and 50µg of **2a,** 75000µg of **1** and 250µg of **2b** and 50µg of **2a,** 100000µg of **1** and 25µg of **2b** and 50µg of **2a,** 100000µg of **1** and 50µg of **2b** and 50µg of **2a,** 100000µg of **1** and 100µg of **2b** and 50µg of **2a,** 100000µg of **1** and 125µg of **2b** and 50µg of **2a,** 100000µg of **1** and 200µg of **2b** and 50µg of **2a,** 100000µg of **1** and 250µg of **2b** and 50µg of **2a,** 100µg of **1** and 25µg of **2b** and 50µg of **2a,** 100µg of **1** and 50µg of **2b** and 50µg of **2a,** 100µg of **1** and 100µg of **2b** and 50µg of **2a,** 100µg of **1** and 125µg of **2b** and 50µg of **2a,** 100µg of **1** and 200µg of **2b** and 50µg of **2a,** 100µg of **1** and 250µg of **2b** and 50µg of **2a,** 100µg of **1 and** 25µg of **2b** and 100µg of **2a,** 100µg of **1** and 50µg of **2b** and 100µg of **2a,** 100µg of **1** and 100µg of **2b** and 100µg of **2a,** 100µg of **1** and 125µg of **2b** and 100µg of **2a,** 100µg of **1** and 200µg of **2b** and 100µg of **2a,** 100µg of **1** and 250µg of **2b** and 100µg of **2a,** 1000µg of **1** and 25µg of **2b** and 100µg of **2a,** 1000µg of **1** and 50µg of **2b** and 100µg of **2a,** 1000µg of **1** and 100µg of **2b** and 100µg of **2a,** 1000µg of **1** and 125µg of **2b** and 100µg of **2a,** 1000µg of **1** of **1'** and 200µg of **2b** and 100µg of **2a,** 1000µg of **1** and 250µg of **2b** and 100µg of **2a,** 10000µg of **1** and 25µg of **2b** and 100µg of **2a,** 10000µg of **1** and 50µg of **2b** and 100µg of **2a,** 10000µg of **1** and 100µg of **2b** and 100µg of **2a,** 10000µg of **1** and 125µg of **2b** and 100µg of **2a,** 10000µg of **1** and 200µg of **2b** and 100µg of **2a,** 10000µg of **1** and 250µg of **2b** and 100µg of **2a,** 25000µg of **1** and 25µg of **2b** and 100µg of **2a,** 25000µg of **1** and 50µg of **2b** and 100µg of **2a,** 25000µg of **1** and 100µg of **2b** and 100µg of **2a,** 25000µg of **1** and 125µg of **2b** and 100µg of **2a,** 25000µg of **1** and 200µg of **2b** and 100µg of **2a,** 25000µg of **1** and 250µg of **2b** and 100µg of **2a,** are administered.

Particularly preferred pharmaceutical combinations according to the invention contain 100-5000µg of one EGFR kinase inhibitor selected from compounds of **1.1** to **1.101,** 125-250 µg of budesonide **2b.1** or fluticasone **2b.2** and 10 to 40µg of formoterol **2a.1** or salmeterol **2a.2.**

The active substance combinations of **1, 2a** and **2b** according to the invention are preferably administered by inhalation.

### Pharmaceutical compositions comprising an EGFR kinase inhibitor 1 and a PDE-IV inhibitor 2c:

One embodiment of the invention is a pharmaceutical composition comprising an EGFR kinase inhibitor **1** and a PDE IV inhibitor **2c.** Binary compositions containing only one active **1** and one active **2c,** optionally together with one or more pharmaceutically acceptable excipients or carriers, are preferred. In the pharmaceutical combinations according to the invention preferred PDE IV inhibitors **2c** are selected from the group consisting of enprofylline, theophylline, roflumilast, ariflo (cilomilast), CP-325,366, BY343, D-4396 (Sch-351591), AWD-12-281 (GW-842470), N-(3,5-dichloro-1-oxo-pyridin-4-yl)-4-difluoromethoxy-3-cyclopropylmethoxybenzamide, NCS-613, pumafentine, (-)p-[(4*aR**,10*b*S*)-9-ethoxy-1,2,3,4,4a,10b-hexahydro-8-methoxy-2methylbenzo[s][1,6]naphthyridin-6-yl]-N,N-diisopropylbenzamide, (R)-(+)-1-(4-bromobenzyl)-4-[(3-cyclopentyloxy)-4-methoxyphenyl]-2-pyrrolidone, 3-(cyclopentyloxy-4-methoxyphenyl)-1-(4-N'-[N-2-cyano-S-methyl-isothioureido]benzyl)-2-pyrrolidone, cis[4-cyano-4-(3-cyclopentyloxy-4-methoxyphenyl)cyclohexan-1-carboxylic acid], 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], (R)-(+)-ethyl[4-(3-cyclopentyloxy-4-methoxyphenyl)pyrrolidine-2-ylidene]acetate, (S)-(-)-ethyl[4-(3-cyclopentyloxy-4-methoxyphenyl)-pyrrolidine-2-ylidene]acetate, CDP840, Bay-198004, D-4418, PD-168787, T-440, T-2585, arofylline, atizoram, V-11294A, CI-1018, CDC-801, CDC-3052, D-22888, YM-58997, Z-15370, 9-cyclopentyl-5,6-dihydro-7-ethyl-3-(2-thienyl)-9H-pyrazolo[3,4-c]-1,2,4-triazolo[4,3-a]pyridine, and 9-cyclopentyl-5,6-dihydro-7-ethyl-3-(*tert*-butyl)-9*H-*pyrazolo[3,4-c]-1,2,4-triazolo[4,3-a]pyridine, optionally in the form of the racemates, the enantiomers, the diastereomers and optionally the pharmacologically acceptable acid addition salts thereof, and the hydrates thereof.

In a preferred embodiment according to the invention the PDE IV inhibitors **2c** are selected from the group consisting of enprofylline, roflumilast, ariflo (cilomilast), AWD-12-281 (GW-842470), N-(3,5-dichloro-1-oxo-pyridin-4-yl)-4-difluoromethoxy-3-cyclopropylmethoxybenzamide, T-440, T-2585, arofylline, cis[4-cyano-4-(3-cyclopentyloxy-4-methoxyphenyl)cyclohexan-1-carboxylic acid], 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], PD-168787, atizoram, V-11294A, Cl-1018, CDC-801, D-22888, YM-58997, Z-15370, 9-cyclopentyl-5,6-dihydro-7-ethyl-3-(2-thienyl)-9H-pyrazolo[3,4-c]-1,2,4-triazolo[4,3-a]pyridine, and 9-cyclopentyl-5,6-dihydro-7-ethyl-3-(*tert*-butyl)-9*H*-pyrazolo[3,4-c]-1,2,4-triazolo[4,3-a]pyridine, optionally in the form of the racemates, the enantiomers, the diastereomers and optionally the pharmacologically acceptable acid addition salts thereof, and the hydrates thereof.

In another preferred embodiment according to the invention the PDE IV inhibitors **2c** are selected from the group consisting of ariflo (cilomilast) **2c.1,** roflumilast **2c.2,** AWD-12-281 (GW-842470) **2c.3,** arofylline, Z-15370, 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], atizoram, 9-cyclopentyl-5,6-dihydro-7-ethyl-3-(2-thienyl)-9H-pyrazolo[3,4-c]-1,2,4-triazolo[4,3-a]pyridine, and 9-cyclopentyl-5,6-dihydro-7-ethyl-3-(*tert*-butyl)-9*H*-pyrazolo[3,4-c]-1,2,4-triazolo[4,3-a]pyridine, while roflumilast, Z-15370, and AWD-12-281 are particularly preferred as compound **2c** according to the invention.

In a yet another preferred embodiment according to the invention the PDE IV inhibitors **2c** are selected from the group consisting of 2-(4-fluoro-phenoxy)-N-{4-[(6-fluoro-2-hydroxy-benzoylamino)-methyl]-benzyl} nicotinamide, 2-(4-fluoro-phenoxy)-N-{4-[(5-fluoro-2-hydroxy-benzoylamino)-methyl]-benzyl} nicotinamide, 2-(4-fluorophenoxy)-N-(4-[(3-hydroxy-4-methyl-benzoylamino)methyl]benzyl) nicotinamide, 2-(4-fluoro-phenoxy)-N-{4-[(3hydroxy-benzoylamino)-methyl]-benzyl} nicotinamide, 2-(4-fluoro-phenoxy)-N-{4-[(2-hydroxy-benzoylamino)methyl]-benzyl} nicotinamide, 2-(4-fluoro-phenoxy)-N-{4-[(4-hydroxy-benzoylamino)methyl]-benzyl} nicotinamide, 2-(4-fluoro-phenoxy)-N-{4-[(2-hydroxy-4-methyl-benzoylamino)methyl]-benzyl} nicotinamide, 2-(4-fluoro-phenoxy)-N-{4-[(3-hydroxy-2-methyl-benzoylamino)methyl]-benzyl} nicotinamide, 2-(4-fluoro-phenoxy)-N-{4-[(2-hydroxy-5-methyl-benzoylamino)methyl]-benzyl} nicotinamide, 5-fluoro-2-(4-fluoro-phenoxy)-N-{4-[(2-hydroxy-benzoylamino)-methyl]-benzyl} nicotinamide, 5-fluoro-2-(4-fluoro-phenoxy)-N-{4-[(2-hydroxy-acetylamino)methyl]-benzyl} nicotinamide, 5-fluoro-2-(4-fluoro-phenoxy)-N-{4-[(4-hydroxybenzoylamino)methyl]-benzyl} nicotinamide, 3-(3-{4-[(3-hydroxy-benzoylamino)methyl]-benzylcarbamoyl}-pyridin-2-yloxy)benzoic acid ethylester, 3-(3-{4-[(2-hydroxy-phenacetylamino)methyl]-benzylcarbamoyl}-pyridin-2-yloxy)benzoic acid ethylester, 3-(3-{4-[(3-hydroxy-phenacetylamino)methyl]-benzylcarbamoyl}-pyridin-2-yloxy)benzoic acid ethylester, 3-(3-{4-[(4-hydroxy-phenacetylamino)methyl]-benzylcarbamoyl}-pyridin-2-yloxy)benzoic acid ethylester, compound **2c.4** 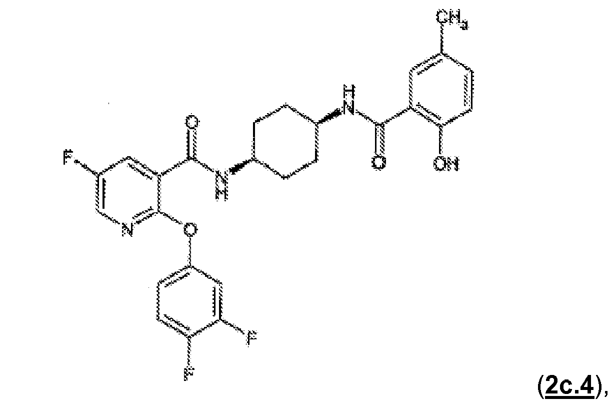 and
compound **2c.5** 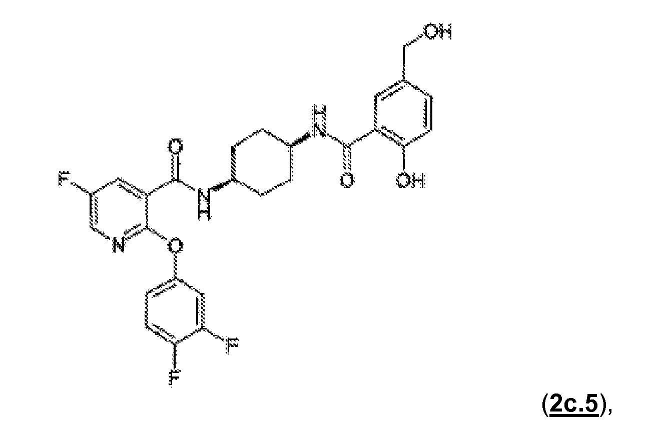 optionally in the form of the racemates, the enantiomers, the diastereomers and optionally the pharmacologically acceptable acid addition salts thereof, and the hydrates thereof.

Pharmaceutically acceptable salt forms of the active compounds within the pharmaceutical composition of the present invention are prepared for the most part by conventional means. Where the component compound contains a carboxylic acid group, a suitable salt thereof may be formed by reacting the compound with an appropriate base to provide the corresponding base addition salt. Examples of such bases are alkali metal hydroxides including potassium hydroxide, sodium hydroxide, and lithium hydroxide; alkaline earth metal hydroxides such as barium hydroxide and calcium hydroxide; alkali metal alkoxides, *e*.*g*., potassium ethanolate and sodium propanolate; and various organic bases such as piperidine, diethanolamine, and N-methylglutamine. Also included are the aluminum salts of the component compounds of the present invention.

For certain component compounds acid addition salts may be formed by treating said compounds with pharmaceutically acceptable organic and inorganic acids, e.g., hydrohalides such as hydrochloride, hydrobromide, hydroiodide; other mineral acids and their corresponding salts such as sulfate, nitrate, phosphate, etc.; and alkyl- and mono-arylsulfonates such as ethanesulfonate, toluenesulfonate, and benzenesulfonate; and other organic acids and their corresponding salts such as acetate, tartrate, maleate, succinate, citrate, benzoate, salicylate, ascorbate, etc.

Accordingly, the pharmaceutically acceptable acid addition salts of the component compounds of the present invention include, but are not limited to: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate, phthalate.

Particularly preferred examples of pharmacologically acceptable acid addition salts of the compounds **2c** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid or maleic acid. If desired, mixtures of the abovementioned acids may also be used to prepare the salts **2c.**

In the pharmaceutical compositions according to the invention, the compounds **2c** may be present in the form of their racemates, enantiomers or mixtures thereof. The separation of the enantiomers from the racemates may be carried out using methods known in the art (e.g. by chromatography on chiral phases, etc.).

Especially preferred pharmaceutical compositions according to the invention comprise the following specific combinations of EGFR kinase inhibitors **1** and PDE IV inhibitors **2c,** either as free bases or pharmacologically acceptable acid addition salts:
**1.1** and **2c.1, 1.4** and **2c.1, 1.6** and **2c.1, 1.8** and **2c.1, 1.9** and **2c.1, 1.14** and **2c.1, 1.17** and **2c.1, 1.19** and **2c.1, 1.21** and **2c.1, 1.23** and **2c.1, 1.24** and **2c.1, 1.27** and **2c.1, 1.28** and **2c.1, 1.30** and **2c.1, 1.34** and **2c.1, 1.35** and **2c.1, 1.37** and **2c.1, 1.38** and **2c.1, 1.40** and **2c.1, 1.42** and **2c.1, 1.43** and **2c.1, 1.44** and **2c.1, 1.48** and **2c.1, 1.52** and **2c.1, 1.55** and **2c.1, 1.57** and **2c.1, 1.59** and **2c.1, 1.60** and **2c.1, 1.63** and **2c.1, 1.64** and **2c.1, 1.66** and **2c.1, 1.67** and **2c.1, 1.69** and **2c.1, 1.70** and **2c.1, 1.71** and **2c.1, 1.72** and **2c.1, 1.78** and **2c.1, 1.82** and **2c.1, 1.83** and **2c.1, 1.84** and **2c.1, 1.88** and **2c.1, 1.90** and **2c.1, 1.91** and **2c.1, 1.94** and **2c.1, 1.95** and **2c.1;**
**1.1** and **2c.2, 1.4** and **2c.2, 1.6** and **2c.2, 1.8** and **2c.2, 1.9** and **2c.2, 1.14** and **2c.2, 1.17** and **2c.2, 1.19** and **2c.2, 1.21** and **2c.2, 1.23** and **2c.2, 1.24** and **2c.2, 1.27** and **2c.2, 1.28** and **2c.2, 1.30** and **2c.2, 1.34** and **2c.2, 1.35** and **2c.2, 1.37** and **2c.2, 1.38** and **2c.2, 1.40** and **2c.2, 1.42** and **2c.2, 1.43** and **2c.2, 1.44** and **2c.2, 1.48** and **2c.2, 1.52** and **2c.2, 1.55** and **2c.2, 1.57** and **2c.2, 1.59** and **2c.2, 1.60** and **2c.2, 1.63** and **2c.2, 1.64** and **2c.2, 1.66** and **2c.2, 1.67** and **2c.2, 1.69** and **2c.2, 1.70** and **2c.2, 1.71** and **2c.2, 1.72** and **2c.2, 1.78** and **2c.2, 1.82** and **2c.2, 1.83** and **2c.2, 1.84** and **2c.2, 1.88** and **2c.2, 1.90** and **2c.2, 1.91** and **2c.2, 1.94** and **2c.2, 1.95** and **2c.2;**
**1.1** and **2c.3, 1.4** and **2c.3, 1.6** and **2c.3, 1.8** and **2c.3, 1.9** and **2c.3, 1.14** and **2c.3, 1.17** and **2c.3, 1.19** and **2c.3, 1.21** and **2c.3, 1.23** and **2c.3, 1.24** and **2c.3, 1.27** and **2c.3, 1.28** and **2c.3, 1.30** and **2c.3, 1.34** and **2c.3, 1.35** and **2c.3, 1.37** and **2c.3, 1.38** and **2c.3, 1.40** and **2c.3, 1.42** and **2c.3, 1.43** and **2c.3, 1.44** and **2c.3, 1.48** and **2c.3, 1.52** and **2c.3, 1.55** and **2c.3, 1.57** and **2c.3, 1.59** and **2c.3, 1.60** and **2c.3, 1.63** and **2c.3, 1.64** and **2c.3, 1.66** and **2c.3, 1.67** and **2c.3, 1.69** and **2c.3, 1.70** and **2c.3, 1.71** and **2c.3, 1.72** and **2c.3, 1.78** and **2c.3, 1.82** and **2c.3, 1.83** and **2c.3, 1.84** and **2c.3, 1.88** and **2c.3, 1.90** and **2c.3, 1.91** and **2c.3, 1.94** and **2c.3, 1.95** and **2c.3;**
**1.1** and **2c.4, 1.4** and **2c.4, 1.6** and **2c.4, 1.8** and **2c.4, 1.9** and **2c.4, 1.14** and **2c.4, 1.17** and **2c.4, 1.19** and **2c.4, 1.21** and **2c.4, 1.23** and **2c.4, 1.24** and **2c.4, 1.27** and **2c.4, 1.28** and **2c.4, 1.30** and **2c.4, 1.34** and **2c.4, 1.35** and **2c.4, 1.37** and **2c.4, 1.38** and **2c.4, 1.40** and **2c.4, 1.42** and **2c.4, 1.43** and **2c.4, 1.44** and **2c.4, 1.48** and **2c.4, 1.52** and **2c.4, 1.55** and **2c.4, 1.57** and **2c.4, 1.59** and **2c.4, 1.60** and **2c.4, 1.63** and **2c.4, 1.64** and **2c.4, 1.66** and **2c.4, 1.67** and **2c.4, 1.69** and **2c.4, 1.70** and **2c.4, 1.71** and **2c.4, 1.72** and **2c.4, 1.78** and **2c.4, 1.82** and **2c.4, 1.83** and **2c.4, 1.84** and **2c.4, 1.88** and **2c.4, 1.90** and **2c.4, 1.91** and **2c.4, 1.94** and **2c.4, 1.95** and **2c.4;**
**1.1** and **2c.5, 1.4** and **2c.5, 1.6** and **2c.5, 1.8** and **2c.5, 1.9** and **2c.5, 1.14** and **2c.5, 1.17** and **2c.5, 1.19** and **2c.5, 1.21** and **2c.5, 1.23** and **2c.5, 1.24** and **2c.5, 1.27** and **2c.5, 1.28** and **2c.5, 1.30** and **2c.5, 1.34** and **2c.5, 1.35** and **2c.5, 1.37** and **2c.5, 1.38** and **2c.5, 1.40** and **2c.5, 1.42** and **2c.5, 1.43** and **2c.5, 1.44** and **2c.5, 1.48** and **2c.5, 1.52** and **2c.5, 1.55** and **2c.5, 1.57** and **2c.5, 1.59** and **2c.5, 1.60** and **2c.5, 1.63** and **2c.5, 1.64** and **2c.5, 1.66** and **2c.5, 1.67** and **2c.5, 1.69** and **2c.5, 1.70** and **2c.5, 1.71** and **2c.5, 1.72** and **2c.5, 1.78** and **2c.5, 1.82** and **2c.5, 1.83** and **2c.5, 1.84** and **2c.5, 1.88** and **2c.5, 1.90** and **2c.5, 1.91** and **2c.5, 1.94** and **2c.5, 1.95** and **2c.5;**
**1.1** and **2c.6, 1.4** and **2c.6, 1.6** and **2c.6, 1.8** and **2c.6, 1.9** and **2c.6, 1.14** and **2c.6, 1.17** and **2c.6, 1.19** and **2c.6, 1.21** and **2c.6, 1.23** and **2c.6, 1.24** and **2c.6, 1.27** and **2c.6, 1.28** and **2c.6, 1.30** and **2c.6, 1.34** and **2c.6, 1.35** and **2c.6, 1.37** and **2c.6, 1.38** and **2c.6, 1.40** and **2c.6, 1.42** and **2c.6, 1.43** and **2c.6, 1.44** and **2c.6, 1.48** and **2c.6, 1.52** and **2c.6, 1.55** and **2c.6, 1.57** and **2c.6, 1.59** and **2c.6, 1.60** and **2c.6, 1.63** and **2c.6, 1.64** and **2c.6, 1.66** and **2c.6, 1.67** and **2c.6, 1.69** and **2c.6, 1.70** and **2c.6, 1.71** and **2c.6, 1.72** and **2c.6, 1.78** and **2c.6, 1.82** and **2c.6, 1.83** and **2c.6, 1.84** and **2c.6, 1.88** and **2c.6, 1.90** and **2c.6, 1.91** and **2c.6, 1.94** and **2c.6, 1.95** and **2c.6.**

The proportions in which the active substances **1** and **2c** may be used in the active substance combinations according to the invention are variable. Active substances **1** and **2c** may possibly be present in the form of their solvates or hydrates. Depending on the choice of the compounds **1** and **2c,** the weight ratios which may be used within the scope of the present invention vary on the basis of the different molecular weights of the various salt forms.

As a rule, the pharmaceutical combinations according to the invention may contain compounds **1** and **2c** in ratios by weight ranging from 10000:1 to 1:500, preferably from 4000:1 to 1:100, more preferred from 2000:1 to 1:50, most preferred 1000:1 to 1:20. In particularly preferred pharmaceutical combinations that contain as component **2** one of the most preferred compounds ariflo **2c.1,** roflumilast **2c.2,** or AWD-12-281 **2c.3,** the weight ratios of **1** to **2c** are most preferably in a range in which **1** and **2c** are present in proportions of 4000:1 to 20:1, more preferably from 2000:1 to 100:1.
For example, without restricting the scope of the invention thereto, preferred combinations may contain **1** and PDE-IV inhibitor **2c** (as for instance ariflo, roflumilast or AWD-12-281) in the following weight ratios:
4000:1, 3900:1, 3800:1, 3700:1, 3600:1, 3500:1, 3400:1, 3300:1, 3200:1, 3100:1, 3000:1, 2900:1, 2800:1 2700:1, 2600:1, 2500:1, 2400:1, 2300:1, 2200:1 2100:1, 2000:1, 1900:1, 1800:1, 1700:1, 1600:1, 1500:1, 1400:1, 1300:1, 1200:1, 1100:1, 1000:1, 900:1, 800:1, 700:1, 600:1, 500:1, 400:1, 300:1, 200:1, 100:1, 90:1, 80:1, 70:1, 60:1, 50:1, 40:1, 35:1, 30:1, 25:1, 20:1.

The pharmaceutical compositions according to the invention containing the combinations of **1** and **2c** are normally administered so that **1** and **2c** are present together in doses of 1 to 100000µg, preferably from 10 to 50000µg, more preferably from 100 to 25000µg, better still from 500 to 10000µg per single dose. For example, combinations of **1** and **2c** according to the invention contain a quantity of **1** and PDE-IV inhibitor **2c** (as for instance **2c.1, 2c.2** or **2c.3)** such that the total dosage per single dose is about 5µg, 10µg, 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 125µg, 150µg, 175µg, 200µg, 300µg, 400µg, 500µg, 600µg, etc. (add stepwise 100µg) up to 50000µg, or similar.

The suggested dosages per single dose specified above are not to be regarded as being limited to the numerical values actually stated, but are intended as dosages which are disclosed by way of example. Of course, dosages which may fluctuate about the abovementioned numerical values within a range of about +/- 2.5 µg are also included in the values given above by way of example. In these dosage ranges, the active substances **1** and **2c** may be present in the weight ratios given above.

For example, without restricting the scope of the invention thereto, the combinations of **1** and **2c** according to the invention may contain a quantity of **1** and PDE-IV inhibitor **2** (as for instance **2c.1**, **2c.2** or **2c.3)** in such an amount that the following quantities of the active substances are administered per single dose:
100µg of **1** and 25µg of **2c,** 100µg of **1** and 50µg of **2c,** 100µg of **1** and 100µg of **2c,** 100µg of **1** and 200µg of **2c,** 100µg of **1** and 300µg of **2c,** 100µg of **1** and 400µg of **2c,** 100µg of **1** and 500µg of **2c,** 100µg of **1** and 600µg of **2c,** 100µg of **1** and 700µg of **2c,** 100µg of **1** and 800µg of **2c,** 100µg of **1** and 900µg of **2c,** 100µg of **1** and 1000µg of **2c,** 00µg of **1** and 1250µg of **2c,** 100µg of **1** and 1500µg of **2c,** 100µg of **1** and 1750µg of **2c,** 100µg of **1** and 2000µg of **2c,**
200µg of **1** and 25µg of **2c,** 200µg of **1** and 50µg of **2c,** 200µg of **1** and 100µg of **2c,** 200µg of **1** and 200µg of **2c,** 200µg of **1** and 300µg of **2c,** 200µg of **1** and 400µg of **2c,** 200µg of **1** and 500µg of **2c,** 200µg of **1** and 600µg of **2c,** 200µg of **1** and 700µg of **2c,** 200µg of **1** and 800µg of **2c,** 200µg of **1** and 900µg of **2c,** 200µg of **1** and 1000µg of **2c,** 200µg of **1** and 1250µg of **2c,** 200µg of **1** and 1500µg of **2c,** 200µg of **1** and 1750µg of **2c,** 200µg of **1** and 2000µg of **2c,**
500µg of **1** and 25µg of **2c,** 500µg of **1** and 50µg of **2c,** 500µg of **1** and 100µg of **2c,** 500µg of **1** and 200µg of **2c,** 500µg of **1** and 300µg of **2c,** 500µg of **1** and 400µg of **2c,** 500µg of **1** and 500µg of **2c,** 500µg of **1** and 600µg of **2c,** 500µg of **1** and 700µg of **2c,** 500µg of **1** and 800µg of **2c,** 500µg of **1** and 900µg of **2c,** 500µg of **1** and 1000µg of **2c,** 500µg of **1** and 1250µg of **2c,** 500µg of **1** and 1500µg of **2c,** 500µg of **1** and 1750µg of **2c,** 500µg of **1** and 2000µg of **2c,**
1000µg of **1** and 25µg of **2c,** 1000µg of **1** and 50µg of **2c,** 1000µg of **1** and 100µg of **2c,** 1000µg of **1** and 200µg of **2c,** 1000µg of **1** and 300µg of **2c,** 1000µg of **1** and 400µg of **2c,** 1000µg of **1** and 500µg of **2c,** 1000µg of **1** and 600µg of **2c,** 1000µg of **1** and 700µg of **2c,** 1000µg of **1** and 800µg of **2c,** 1000µg of **1** and 900µg of **2c,** 1000µg of **1** and 1000µg of **2c,** 1000µg of **1** and 1250µg of **2c,** 1000µg of **1** and 1500µg of **2c,** 1000µg of **1** and 1750µg of **2c,** 1000µg of **1** and 2000µg of **2c,**
5000µg of **1** and 25µg of **2c,** 5000µg of **1** and 50µg of **2c,** 5000µg of **1** and 100µg of **2c,** 5000µg of **1** and 200µg of **2c,** 5000µg of **1** and 300µg of **2c,** 5000µg of **1** and 400µg of **2c,** 5000µg of **1** and 500µg of **2c,** 5000µg of **1** and 600µg of **2c,** 5000µg of **1** and 700µg of **2c,** 5000µg of **1** and 800µg of **2c,** 5000µg of **1** and 900µg of **2c,** 5000µg of **1** and 1000µg of **2c,** 5000µg of **1** and 1250µg of **2c,** 5000µg of **1** and 1500µg of **2c,** 5000µg of **1** and 1750µg of **2c,** 5000µg of **1** and 2000µg of **2c,**
10000µg of **1** and 25µg of **2c,** 10000µg of **1** and 50µg of **2c,** 10000µg of **1** and 100µg of **2c,** 10000µg of **1** and 200µg of **2c,** 10000µg of **1** and 300µg of **2c,** 10000µg of **1** and 400µg of **2c,** 10000µg of **1** and 500µg of **2c,** 10000µg of **1** and 600µg of **2c,** 10000µg of **1** and 700µg of **2c,** 10000µg of **1** and 800µg of **2c,** 10000µg of **1** and 900µg of **2c,** 10000µg of **1** and 1000µg of **2c,** 10000µg of **1** and 1250µg of **2c,** 10000µg of **1** and 1500µg of **2c,** 10000µg of **1** and 1750µg of **2c,** 10000µg of **1** and 2000µg of **2c,**
25000µg of **1** and 25µg of **2c,** 25000µg of **1** and 50µg of **2c,** 25000µg of **1** and 100µg of **2c,** 25000µg of **1** and 200µg of **2c,** 25000µg of **1** and 300µg of **2c,** 25000µg of **1** and 400µg of **2c,** 25000µg of **1** and 500µg of **2c,** 25000µg of **1** and 600µg of **2c,** 25000µg of **1** and 700µg of **2c,** 25000µg of **1** and 800µg of **2c,** 25000µg of **1** and 900µg of **2c,** 25000µg of **1** and 1000µg of **2c,** 25000µg of **1** and 1250µg of **2c,** 25000µg of **1** and 1500µg of **2c,** 25000µg of **1** and 1750µg of **2c,** 25000µg of **1** and 2000µg of **2c,**
50000µg of **1** and 25µg of **2c,** 50000µg of **1** and 50µg of **2c,** 50000µg of **1** and 100µg of **2c,** 50000µg of **1** and 200µg of **2c,** 50000µg of **1** and 300µg of **2c,** 50000µg of **1** and 400µg of **2c,** 50000µg of **1** and 500µg of **2c,** 50000µg of **1** and 600µg of **2c,** 50000µg of **1** and 700µg of **2c,** 50000µg of **1** and 800µg of **2c,** 50000µg of **1** and 900µg of **2c,** 50000µg of **1** and 1000µg of **2c,** 50000µg of **1** and 1250µg of **2c,** 50000µg of **1** and 1500µg of **2c,** 50000µg of **1** and 1750µg of **2c,** 50000µg of **1** and 2000µg of **2c.**

### Pharmaceutical compositions comprising an EGFR kinase inhibitor 1 and a p38 MAP kinase inhibitor 2d:

One embodiment of the invention is a pharmaceutical composition comprising an EGFR kinase inhibitor **1** and a p38 MAP kinase inhibitor **2d.** Binary compositions containing only one active **1** and one active **2d**, optionally together with one or more pharmaceutically acceptable excipients or carriers, are preferred.

p38 Kinase inhibitors applicable within the scope of the invention are known in the art. Within the scope of the present invention the term p38 kinase inhibitors **2d** denotes compounds selected from the compounds that are disclosed for instance in US Patents 5,716,972, US 5,686,455, US 5,656,644, US 5,593,992, US 5,593,991, US 5,663,334, US 5,670,527, US 5,559,137, 5,658,903, US 5,739,143, US 5,756,499, US 6,277,989, US 6,340,685, and US 5,716,955 and PCT applications WO 92/12154, WO 94/19350, WO 95/09853, WO 95/09851, WO 95/09847, WO 95/09852, WO 97/25048, WO 97/25047, WO 97/33883, WO 97/35856, WO 97/35855, WO 97/36587, WO 97/47618, WO 97/16442, WO 97/16441, WO 97/12876, WO 98/25619, WO 98/06715, WO 98/07425, WO 98/28292, WO 98/56377, WO 98/07966, WO 98/56377, WO 98/22109, WO 98/24782, WO 98/24780, WO 98/22457, WO 98/52558, WO 98/52559, WO 98/52941, WO 98/52937, WO 98/52940, WO 98/56788, WO 98/27098, WO 98/47892, WO 98/47899, WO 98/50356, WO 98/32733, WO 99/58523, WO 99/01452, WO 99/01131, WO 99/01130, WO 99/01136, WO 99/17776, WO 99/32121, WO 99/58502, WO 99/58523, WO 99/57101, WO 99/61426, WO 99/59960, WO 99/59959, WO 99/00357, WO 99/03837, WO 99/01441, WO 99/01449, WO 99/03484, WO 99/15164, WO 99/32110, WO 99/32111, WO 99/32463, WO 99/64400, WO 99/43680, WO 99/17204, WO 99/25717, WO 99/50238, WO 99/61437, WO 99/61440, WO 00/26209, WO 00/18738, WO 00/17175, WO 00/20402, WO 00/01688, WO 00/07980, WO 00/07991, WO 00/06563, WO 00/12074, WO 00/12497, WO 00/31072, WO 00/31063, WO 00/23072, WO 00/31065, WO 00/35911, WO 00/39116, WO 00/43384, WO 00/41698, WO 00/69848, WO 00/26209, WO 00/63204, WO 00/07985, WO 00/59904, WO 00/71535, WO 00/10563, WO 00/25791, WO 00/55152, WO 00/55139, WO 00/17204, WO 00/36096, WO 00/55120, WO 00/55153, WO 00/56738, WO 01/21591, WO 01/29041, WO 01/29042, WO 01/62731, WO 01/05744, WO 01/05745, WO 01/05746, WO 01/05749, WO 01/05751, WO 01/27315, WO 01/42189, WO 01/00208, WO 01/42241, WO 01/34605, WO 01/47897, WO 01/64676, WO 01/37837, WO 01/38312, WO 01/38313, WO 01/36403, WO 01/38314, WO 01/47921, WO 01/27089, DE 19842833, and JP 2000 86657 whose disclosures are all incorporated herein by reference in their entirety.

Of particular interest for the pharmaceutical compositions according to the invention are those p38 inhibitors **2d** disclosed in US 6,277,989, US 6,340,685, WO 00/12074, WO 00/12497, WO 00/59904, WO 00/71535, WO 01/64676, WO 99/61426, WO 00/10563, WO 00/25791, WO 01/37837, WO 01/38312, WO 01/38313, WO 01/38314, WO 01/47921, WO 99/61437, WO 99/61440, WO 00/17175, WO 00/17204, WO 00/36096, WO 98/27098, WO 99/00357, WO 99/58502, WO 99/64400, WO 99/01131, WO 00/43384, WO 00/55152, WO 00/55139, and WO 01/36403.

In a preferred embodiment the invention relates to pharmaceutical compositions comprising **1** and **2d**, **characterized in that** the p38 kinase inhibitor **2d** is selected from the compounds of formula **(I)** as disclosed in WO 99/01131 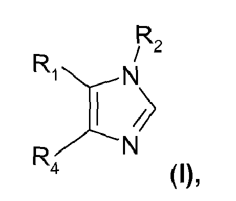 wherein
- R₁: is 4-pyridyl, pyrimidinyl, 4-pyridazinyl, 1,2,4-triazin-5-yl, quinolyl, isoquinolinyl, or quinazolin-4-yl ring, which ring is substituted with Y-Rₐ and optionally with an additional independent substituent selected from C₁₋₄ alkyl, halogen, hydroxyl, C₁₋₄ alkoxy, C₁₋₄ akylthio, C₁₋₄ aklylsulfinyl, CH₂OR₁₂, amino, mono and di- C₁₋₆ alkyl substituted amino, an N-heterocyclyl ring which ring has from 5 to 7 members and optionally contains an additional heteroatom selected from oxygen, sulfur or NR₁₅, N(R₁₀)C(O)R_{b} or NHRₐ;
- Y: is oxygen or sulfur;
- R₄: is phenyl, naphth-1-yl or naphthyl, or a heteroaryl, which is optionally substituted by one or two substituents, each of which is independently selected, and which, for a 4-phenyl, 4naphth-1-yl, 5-naphth-2-yl or 6-naphth-2- yl substituent, is halogen, cyano, nitro, C(Z)NR₇Rₗ₇, C(Z)OR₁₆, (CR₁₀R₂₀)ᵥCOR₁₂, SR₅, SOR₅, OR₁₂, halo-substituted-C₁₋₄ alkyl, C₁₋₄ alkyl, ZC(Z)R₁₂, NR₁₀C(Z)R₁₆, or (CR₁₀R₂₀)ᵥNR₁₀R₂₀ and which, for other positions of substitution, is halogen, cyano, C(Z)NR₁₃R₁₄, C(Z)OR₃, (CR₁₀R₂₀)_{m"}COR₃, S(O)ₘR₃, OR₃, halo-substituted-C₁₋₄ alkyl, C₁₋₄ alkyl, (CR₁₀R₂₀)_{m"}R₁₀C(Z)R₃, NR₁₀S(O)_{m'}R₈, NR₁₀S(O)_{m'}NR₇R₁₇, ZC(Z)R₃ or (CR₁₀R₂₀)_{m"}NR₁₃R₁₄;
- Z: is oxygen or sulfur;
- n: is an integer having a value of 1 to 10;
- m: is 0, or integer 1 or 2;
- m': is an integer having a value of 1 or 2;
- m": is 0, or an integer having a value of 1 to 5;
- v: is 0, or an integer having a value of 1 to 2;
- R₂: is -C(H) (A) (R₂₂);
- A: is optionally substituted aryl, heterocyclyl, or heteroaryl ring, or A is substituted C₁₋₁₀ alkyl;
- R₂₂: is an optionally substituted C₁₋₁₀ alkyl;
- Rₐ: is aryl, arylC₁₋₆ alkyl, heterocyclic, heterocyclylC₁₋₆ alkyl, heteroaryl, heteroarylC₁₋₆alkyl, wherein each of these moieties may be optionally substituted;
- R_{b}: is hydrogen, C₁₋₆ alkyl, C₃₋₇ cycloalkyl, aryl, aryl C₁₋₄ alkyl, heteroaryl, heteroarylC₁₋₄ alkyl, heterocyclyl, or heterocyclylC₁₋₄ alkyl, wherein each of these moieties may be optionally substituted;
- R₃: is heterocyclyl, heterocyclyl C₁₋₁₀ alkyl or R₈;
- R₅: is hydrogen, C₁₋₄ alkyl, C₂₋₄ alkenyl, C₂₋₄ alkynyl or NR₇R₁₇, excluding the moieties SR₅ being SNR₇R₁₇and SOR₅ being SOH;
- R₆: is hydrogen, a pharmaceutically acceptable cation, C₁₋₁₀ alkyl, C₃₋₇ cycloalkyl, aryl, aryl C₁₋₄ alkyl, heteroaryl, heteroaryl C₁₋₄ alkyl, heterocyclyl, aryl, or C₁₋₁₀ alkanoyl;
- R₇: and R₁₇ is each independently selected from hydrogen or C₁₋₄ alkyl or R₇ and R₁₇ together with the nitrogen to which they are attached form a heterocyclic ring of 5 to 7 members which ring optionally contains an additional heteroatom selected from oxygen, sulfur or NR₁₅;
- R₈: is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, aryl C₁₋₁₀ alkyl, heteroaryl, heteroaryl C₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄; wherein the aryl, arylalkyl, heteroaryl, heteroaryl alkyl may be optionally substituted;
- R₉: is hydrogen, C(Z) R₁₁ or optionally substituted C₁₋₁₀ alkyl, S(O)₂R₁₈, optionally substituted aryl or optionally substituted aryl C₁₋₄ alkyl;
- R₁₀ and R₂₀: is each independently selected from hydrogen or C₁₋₄ alkyl;
- R₁₁: is hydrogen, C₁₋₁₀ alkyl, C₃₋₇ cycloalkyl, heterocyclyl, heterocyclyl C₁₋₁₀ alkyl, aryl, arylC₁₋₁₀ alkyl, heteroaryl or heteroaryl C₁₋₁₀ alkyl, wherein these moieties may be optionally substituted;
- R₁₂: is hydrogen or R_{16;}
- R₁₃ an R₁₄: is each independently selected from hydrogen or optionally substituted C₁₋₄ alkyl, optionally substituted aryl or optionally substituted arylC₁₋₄ alkyl, or together with the nitrogen which they are attached form a heterocyclic ring of 5 to 7 members which ring optionally contains an additional heteroatom selected from oxygen, sulfur or NR₉;
- R₁₅: is R₁₀ or C(Z)-C₁₋₄ alkyl;
- R₁₆: is C₁₋₄ alkyl, halo-substituted-C₁₋₄ alkyl, or C₃₋₇cycloalkyl;
- R₁₈: is C₁₋₁₀ alkyl, C₃₋₇ cycloalkyl, heterocyclyl, aryl, aryl₁₋₁₀ alkyl, heterocyclyl, heterocyclyl- C₁₋₁₀alkyl, heteroaryl or heteroaryl₁₋₁₀ alkyl;
or a pharmaceutically acceptable salt thereof.

In the aforementioned compounds of formula **(I)** R₂ is a substituted alkyl derivative. It is recognised that the first methylene carbon in this chain is a tertiary carbon, and it will contain one hydrogen moiety. This ethylene group has two additional substituents, an R₂₂ moiety and an A moiety, -C(H)(A)( R₂₂). Both A and R₂₂ may not be unsubstituted C₁₋₁₀ alkyl moiety.

In a preferred embodiment, R₂ is a -C(AA₁)(A) moiety, wherein AA₁ is the R₂₂ moiety, but is specifically the side chain residue (R) of an amino acid, as is further described herein.

Suitably, A is an optionally substituted C₁₃₋₇ cycloalkyl, aryl, heteroaryl, or heterocyclic ring, or A is a substituted C₁₋₁₀ alkyl moiety.

When A is an aryl, heteroaryl and heterocyclic ring, the ring may be substituted independently one or more times, preferably, 1 to 3 times by C₁₋₁₀ alkyl; halogen; halo substituted C₁₋₁₀ alkyl such as CF₃; (CR₁₀R₂₀)ₜOR₁₁; (CR₁₀R₂₀)ₜNR₁₂R₁₄, especially amino or mono-or di-C₁₋₄ alkylamino; (CR₁₀R₂₀)ₜS(O)m R₁₈, wherein m is 0, 1 or 2; SH; NR₁₀C(Z)R₃ (such NHCO(C₁₋₁₀ alkyl)); or NR₁₀S(O)m R₈ (such as NHSO₂(C₁₋₁₀ alkyl)).

Suitably, t is 0, or an integer of 1 to 4.

When A is an optionally substituted cycloalkyl it is as defined below with the R₂₂ substitution.

When A is an optionally substituted heterocyclyl ring, the ring is preferably a morpholino, pyrrolidinyl, piperazinyl or a piperidinyl ring.

When A is an optionally substituted aryl moiety, it is preferably a phenyl ring.

When A is an optionally substituted heteroaryl ring, it is as defined below in the definition section.

When A is a substituted C₁₋₁₀alkyl moiety, the alkyl chain may be straight or branched. The chain is substituted independently 1 or more times, preferably 1 to 3 times by halogen, such as fluorine, chlorine, bromine or iodine; halosubstituted C₁₋₁₀ alkyl, such as CF₃; C₃₋₇ cycloaklyl, C₁₋₁₀ alkloxy, such as methoxy or ethoxy;

hydroxy substituted C₁₋₁₀ alkoxy; halosubstituted C₁₋₁₀ alkoxy, such as OCF₂CF₂H; OR₁₁; S(O)ₘR₁₈ (wherein m is 0, 1 or 2); NR₁₃R₁₄; C(Z)NR₁₃R₁₄; S(O)ₘNR₁₃R₁₄; NR₂₃C(Z)R₁₁; NHS(O)₂R₁₈; C(Z)R₁₁; OC(Z)R₁₁; C(Z)OR₁₁; C(Z)R₁₁OR₉; N(OR₆)C(Z)NR₁₃R₁₄; N(OR₆)C(Z)R₁₁; C(=NOR₆)R₁₁; NR₂₃C(=NR₁₉)NR₁₃R₁₄; OC(Z)NR₁₃R₁₄; NR₂₃C(Z)NR₁₃R₁₄; or NR₂₃C(Z)OR₁₀.

Preferably A is a C₃₋₇ cycloalkyl, or a C₁₋₆ alkyl, more preferably a C₁₋₂ alkyl, i.e. a methylene or ethylene moiety, more preferably a methylene moiety which is substituted by one of the above noted groups.

Preferably, when A is a C₁₋₁₀ alkyl, it is substituted by OR₁₁ where R₁₁ is preferably hydrogen, aryl or arylalkyl; NR₁₃R₁₄; OC(Z)R₁₁; C(Z)OR₁₁.

More preferably, A is substituted by OR₁₁ where R₁₁ is hydrogen.

Suitably, R₂₂ is a C₁₋₁₀ alkyl chain, which chain may be straight or branched and which may be optionally substituted independently, one or more times, preferably 1 to 3 times, by halogen, such as fluorine, chlorine or iodine; halo substituted C₁₋₁₀alkyl; C₁₋₁₀alkoxy, such as methoxy or ethoxy; hydroxy substituted C₁₋₁₀alkoxy; halosubstituted C₁₋₁₀alkoxy, such as OCF₂CF₂H; OR₁₁; S(O)ₘR₁₈; NR₁₃R₁₄; C(Z)NR₁₃R₁₄; S(O)_{m'}NR₁₃R₁₄; NR₂₃C(Z)R₁₁; NHS(O)₂R₁₈; C(Z)R₁₁; OC(Z)OR₁₁; C(Z)OR₁₁; C(Z)NR₁₁OR₉; N(OR₆)C(Z)NR₁₃R₁₄; N(OR₆)C(Z)R₁₁; C(=NOR₆)R₁₁; NR₂₃C(=NR₁₉)NR₁₃R₁₄; OC(Z)NR₁₃R₁₄; NR₂₃C(Z)NR₁₃R₁₄; NR₂₃C(Z)OR₁₀; optionally substituted C₃₋₇cycloalkyl; optionally substituted aryl, such as phenyl; optionally substituted heteroaryl; or an optionally substituted heterocyclic. The optional substituents on these cycloalkyl, aryl, heteroaryl, and heterocyclic moieties are as defined herein below.

It is noted that those R₂₂ substituent groups which contain carbon as the first connecting group, i.e. C(Z)OR₁₁; C(Z)NR₁₁OR₉, C(Z)R₁₁, C(Z)NR₁₃R₁₄, and C(=NOR₆)R₁₁, may be the sole carbon in alkyl chain. Therefore, the R₂₂ group may, for instance, be a carboxy, an aldehyde, or an amide, as well as being a substituent of a methylene unit, such as carbamoylmethyl, or acetamidomethyl.

Preferably R₂₂ is a C₁₋₆ unsubstituted or substituted alkyl group, such as a C₁₋₃ alkylene such as methyl, ethyl or isopropyl, or a methylene or ethylene moiety substituted by one of the above noted moieties, or as noted above those substituent groups which contain a carbon may substituent for the first methylene unit of the alkyl chain, such as carboxy, C(O)OR₁₁; C(O)NR₁₃R₁₄ or R₂₂ is an optionally substituted aryl group, such as a benzyl or phenethyl. In other words, R₂₂ can be an optionally substituted alkyl group, or R₂₂ can be C(Z)OR₁₁; C(Z)NR₁₁OR₉, C(Z)R₁₁, C(Z)NR₁₃R₁₄, or C(=NOR₆)R₁₁.

Preferably R₂₂ is C₁₋₆ unsubstituted or substituted alkyl group, more

preferably a C₁₋₂ alkylene chain, such as a methylene or ethylene moiety, more preferably methylene.

Preferably the alkyl chain is substituted by OR₁₁, where R₁₁ is preferably

hydrogen, aryl or arylalkyl; S(O)ₘR₁₈, where m is 0 and R₁₈ is a C₁₋₆ alkyl; or an optionally substituted aryl, i.e. a benzyl or phenethyl moiety.

More preferably, R₂₂ is phenyl, benzyl, CH₂OH, or CH₂-O-aryl.

Preferably, one or both of A and R₂₂ contain hydroxy moieties, such as in C₁₋₆ alkyl OR₁₁, wherein R₁₁ is hydrogen, i.e. CH₂CH₂OH.

Suitably, when AA₁ is the (R) side chain residue of an amino acid, it is a C₁₋₆ alkyl group, which may be straight or branched. This means the R group of the core amino acid of the structure R-C(H)(COOH)(NH₂). The R residue term is for example, CH₃ for alanine, (CH₃)₂CH- for valine, (CH₃)₂CH-CH₂-f or leucine, phenyl-CH₂- for phenylalanine, CH₃-S-CH₂-CH₂- for methionine, etc. All generally recognised primary amino acids are included in this groups, such as but not limited to, alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine, hydroxylysine, methylhistidine, and other naturally accurring amino acids not found in proteins, such as β-alanine, γ-aminobutyric acid, homocysteine, homoserine, citrulline, ornithine, canavanine, djenkolic acid, and β-cyanoalanine, or other naturally occurring non-mammalian amino acids.

Preferably AA₁ is the residue of phenylalanine, or alanine.

Preferably A is a hydroxy substituted C₁₋₁₀ alkyl and R₂₂ is a C₁₋₁₀ alkyl or a hydroxy substituted C₁₋₁₀ alkyl.

In a further preferred embodiment the invention relates to pharmaceutical compositions comprising **1** and **2d, characterized in that** the p38 kinase inhibitor **2d** is selected from the following compounds disclosed in WO 99/01131:
1-(1,3-Dihydroxyprop-2-yl)-(4-fluorophenyl)-5-(2-phenoxypyrimidin-4-yl)imidazole; *trans*-1-(4-Hydroxycyclohexyl)-4-(4-fluorophenyl)5-[(2-methoxy)pyrimidin-4-yl]imidazole;
1-(4-Piperidinyl)-4-(4-fluorophenyl)-5-(2-methoxy-4-pyrimidinyl)imidazole; (4-Fluorophenyl)-2-(4-methylsulfinylphenyl)-5-(4-pyridyl)-imidazole;

In yet another preferred embodiment the invention relates to pharmaceutical compositions comprising **1** and **2d, characterized in that** the p38 kinase inhibitor **2d** is selected from the compounds of formula **(II)** as disclosed in US 6,277,989 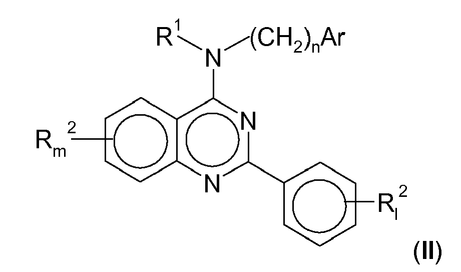 and the pharmaceutically acceptable salts thereof,
wherein
- R¹: is H, alkyl(1-6C) or arylalkyl optionally substituted on the aryl group with 1-3 substituents independently selected from alkyl (1-6C), halo, OR, NR₂, SR, -OOCR, -NROCR, RCO, -COOR, -CONR₂, -SO₂NR₂, CN, CF₃, and NO₂, wherein each R is independently H or lower alkyl (1-4C);
- each R²: is independently alkyl (1-6C), halo, OR, SR, OOCR, NROCR, COOR, RCO, CONR₂, SO₂NR₂, CN, CF₃ or NO₂, wherein each R is independently H or lower alkyl (1-4C);
- each of I, m, and n: is independently 0, 1 or 2; and
- Ar: is phenyl, 2-, 3- or 4-pyridyl, indolyl, 2- or 4-pyrimidyl, or benzimidazolyl, each optionally substituted with optionally substituted alkyl, alkenyl, alkynyl, aryl, N- aryl, NH-aroyl, halo, OR, NR₂, SR, -OOCR, -NROCR, RCO, -COOR, - CONR₂, SO₂NR₂, CN, CF₃, or NO₂, wherein each R is independently H or alkyl (1-4C);

Preferably the invention relates to pharmaceutical compositions comprising **1** and **2d**, **characterized in that** the p38 kinase inhibitor **2d** is selected from the compounds of formula **(II)** as disclosed in US 6,277,989, wherein
- R¹: is H;
- R²: is halo, m is 0, 1, or 2, and I is 1 or 2;
- Ar: is 4-pyridyl.

In a particularily preferred embodiment the invention relates to pharmaceutical compositions comprising **1** and **2d**, **characterized in that** the p38 kinase inhibitor **2d** is selected from the following compounds disclosed US 6,277,989:
2-phenyl-4-(4-pyridylamino)-quinazoline;
2-(2-bromophenyl)-4-(4-pyridylamino)-quinazoline;
2-(2-chlorophenyl)-4-(4-pyridylamino)-quinazoline;
2-(2-fluorophenyl)-4-(4-pyridylamino)-quinazoline;
2-(2-methylphenyl)-4-(4-pyridylamino)-quinazoline;
2-(4-fluorophenyl)-4-(4-pyridylamino)-quinazoline;
2-(3-methoxyanilyl)-4-(4-pyridylamino)-quinazoline;
2-(2,6-dichlorophenyl)-4-(4-pyridylamino)-quinazoline;
2-(2,6-dibromophenyl)-4-(4-pyridylamino)-quinazoline;
2-(2,6-difluorophenyl)-4-(4-pyridylamino)-quinazoline;
2-(2-fluorophenyl)-4-(4-pyridylamino)-6,7-dimethoxyquinazoline;
2-(4-fluorophenyl)-4-(4-pyridylamino)-6,7-dimethoxyquinazoline;
2-(2-fluorophenyl)-4-(4-pyridylamino)-6-nitroquinazoline;
2-(2-fluorophenyl)-4-(4-pyridylamino -6-aminoquinazoline;
2-(2-fluorophenyl)-4-(4-pyridylamino)-7-aminoquinazoline;
2-(2-fluorophenyl)-4-(4-pyridylamino)-6-(3-methoxybenzylamino)-quinazoline;
2-(2-fluorophenyl)-4-(4-pyridylamino)-6-(4-methoxybenzylamino)-quinazoline;
2-(2-fluorophenyl)-4-(4-pyridylamino)-6-(2-isobutylamino)-quinazoline; and
2-(2-fluorophenyl)-4-(4-pyridylamino)-6-(4-methylmercaptobenzylamino)-quina zoline; and the pharmaceutically acceptable salts thereof.

In yet another preferred embodiment the invention relates to pharmaceutical compositions comprising **1** and **2d, characterized in that** the p38 kinase inhibitor **2d** is selected from the compounds of formula **(IIIa), (IIIb), (IIIc),** or **(IIId)** as disclosed in US 6,340,685 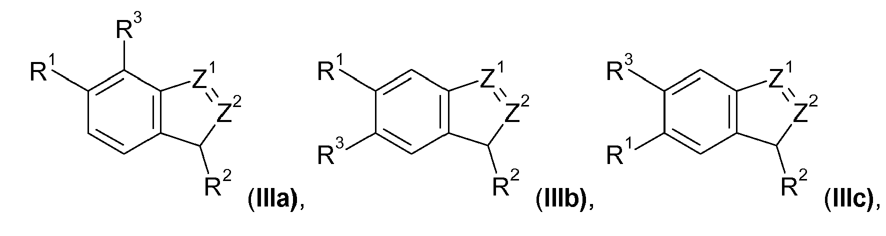 or 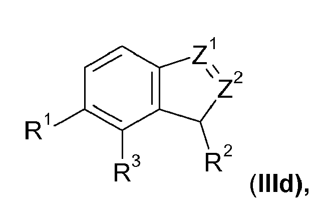 and the pharmaceutically acceptable salts thereof,
wherein each of Z¹ and Z² is independently CR⁴ or N;
where each R⁴ is independently selected from H and alkyl(1-6C);
wherein said alkyl optionally includes one or more heteroatoms selected from O, S and N, and wherein said alkyl is optionally substituted by one or more substituents selected from halo, OR, SR, NR₂, RCO, COOR, CONR₂, OOCR, NROCR, CN, =O, a 5 or 6 membered saturated carbocyclic ring or heterocyclic ring containing 1-2 N, and a 6-membered aromatic ring optionally containing 1-2 N heteroatoms, wherein R in the foregoing optional substituents is H or alkyl (1-6C);
- R¹: is 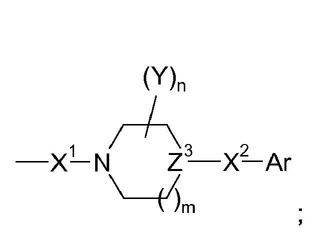 wherein X¹ is CO, SO, CHOH or SO₂ ;
- m: is 1;
- Y: is optionally substituted alkyl, optionally substituted aryl, or optionally substituted arylalkyl;
- n: is 0, 1 or 2;
- Z³: is N;
- X²: is CH or CH₂ ; and
- Ar: consists of one or two phenyl moieties directly coupled to X², said one or two phenyl moieties being optionally substituted by a substituent selected from halo, nitro, alkyl (1-6C), alkenyl (1-6C), CN, CF₃, RCO, COOR, CONR₂, NR₂, OR, SR, OOCR, NROCR, (wherein R in the foregoing is H or 1-6C alkyl), and phenyl, itself optionally substituted by the foregoing substituents;
- R²: is selected from H, and alkyl (1-6C); wherein said alkyl optionally includes one or more heteroatoms which are selected from O, S and N, and wherein said alkyl is optionally substituted by one or more substituents selected from halo, OR, SR, NR₂, RCO, COOR, CONR₂, OOCR, NROCR, (where R in the foregoing is H or 1-6C alkyl) CN, =O, a 5 or 6 membered saturated carbocyclic ring or heterocyclic ring containing 1-2 N, and a 6-membered aromatic ring optionally containing 1-2 N heteroatoms;
- R³: is H, halo, NO₂, alkyl (1-6C), alkenyl (1-6C), CN, OR, SR, NR₂, RCO, COOR, CONR₂, OOCR, or NROCR where R is H or alkyl (1-6C).

In a particularily preferred embodiment the invention relates to pharmaceutical compositions comprising **1** and **2d**, **characterized in that** the p38 kinase inhibitor **2d** is selected from the following compounds disclosed US 6,340,685:
4-(2,6-difluorobenzyl)-piperazinyl-benzimidazole-5-carboxamide;
4-(2,3-difluorobenzyl)-piperazinyl-benzimidazole-5-carboxamide;
4-(3,5-difluorobenzyl)-piperazinyl-benzimidazole-5-carboxamide;
4-(3-chlorobenzyl)-piperazinyl-benzimidazole-5-carboxamide;
4-(4-carboxymethyl benzyl)-piperazinyl-benzimidazole-5-carboxamide;
4-(4-methoxybenzyl)-piperazinyl-benzimidazole-5-carboxamide;
4-(4-trifluoromethoxybenzyl)-piperazinyl-benzimidazole-5-carboxamide;
4-(4-methylbenzyl)-piperazinyl-benzimidazole-5arboxamide;
4-(2,4-dichlorobenzoyl)-piperazinyl-benzimidazole-5-carboxamide;
4-(3,4-dichlorobenzoylm)piperazinyl-benzinidazole-5-carboxamnide;
4-[trans-3-(trifluoromethyl)-cinnamoyl]-piperazinyl-benzimidazole-5-carboxm ide;
4-(4-chlorobenzoyl)-piperazinyl-benzimidazole-5-carboxamide;
4-benzomethylbenzoylpiperazyl-benzimidazole-5-carboxamide;
4-(2-trifluoromethylbenzoyl)-piperazinyl-benzimidazole-5-carboxamide;
4-(4-methxybenzoyl)-piperazinyl-benzimidazole-5-carboxamide;
4-(3,4-dichlorophenyl)-piperazinyl-benzimnidazole-5-carboxamide;
4-(4-chlorobenzhydryl)-piperazinyl-benzimidazole-5-carboxamide;
4-trans-1-cinnamyl piperazinyl-benzimidazole-5-carboxamide;
4-(4-chlorophenyl)-piperazinyl-benzimidazole-5-carboxamide;
4-[bis(4-fluorophenyl)-methyl]-piperazinyl-benzimidazole-5-carboxamide;
4-(4-chlorobenzyl)-piperazinyl-benzimidazole-5-carboxamide;
4-(2-chlorobenzyl)-piperazinyl-benzimidazole-5-carboxamide;
4-benzylpiperazinyl-benzinudazole-5-carboxamnide;
4-(4-methylthiobenzyl)-piperazinyl-benzimidazole-5-carboxamide;
4-(3,4,5-trimethoxybenzyl)-piperazinyl-benzimidazole-5-carboxamide;
4-(2-naphthylmethyl)-piperazinyl-benzimidazole-5-carboxamide;
4-(4-diethylaminobenzyl)-piperazinyl-benzimidazole-5-carboxamide;
4-(biphenylmethyl)-piperazinyl-benzimidazole-5-carboxamide;
4-(4-phenoxybenzyl)-piperazinyl-benzimidazole-5-carboxamide;
4-(4-quinolinylmethyl)-piperazinyl-benzimidazole-5-carboxamide;
4-(4-chlorobenzyl)-piperazinyl-1-(2-propyl)-indole-5-carboxamide;
4-(3-chlorobenzyl)-piperazinyl-benzimidazole-5-carboxamide;
4-(3-chlorobenzyl)-piperazinyl-N-(2-propyl)-benzimidazole-5-carboxamide;
4-(3-chlorobenzyl)-piperazinyl-N-(2-propyl)-benzimidazole-6-carboxamide;
4-(3-chlorobenzyl)-piperazinyl-N-methyl-benzimidazole-5-carboxamide;
4-(3-chlorobenzyl)-piperazinyl-N-methyl-benzimidazole-6-carboxamide;
4-(3-chlorobenzyl)-piperazinyl-N-ethyl-benzimidazole-5-carboxamide; and
4-(3-chlorobenzyl)-piperazinyl-N-ethyl-benzimidazole-6-carboxamide.

In another preferred embodiment the invention relates to pharmaceutical compositions comprising **1** and **2d**, **characterized in that** the p38 kinase inhibitor **2d** is selected from the compounds of formula **(IV)** as disclosed in WO 00/43384 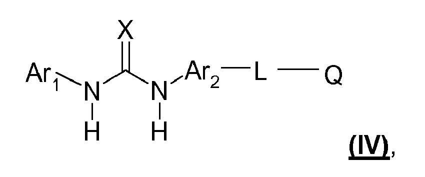 wherein
- Ar₁: is a heterocyclic group selected from the group consisting of pyrrole, pyrrolidine, pyrazole, imidazole, oxazole, thiazole, furan and thiophene; and wherein Ar₁ may be substituted by one or more R₁,R₂ or R₃;
- Ar₂: is phenyl, naphthyl, quinoline, isoquinoline, tetrahydronaphthyl, tetrahydroquinoline, tetrahydroisoquinoline, benzimidazole, benzofuran, indanyl, indenyl or indole each being optionally substituted with one to three R₂ groups;
- L,: a linking group, is a
C₁₋₁₀ saturated or unsaturated branched or unbranched carbon chain; wherein one or more methylene groups are optionally independently replaced by O,N or S; and
wherein said linking group is optionally substituted with 0-2 oxo groups and one or more C₁₋₄ branched or unbranched alkyl which may be substituted by one or more halogen atoms;
- Q: is selected from the group consisting of:
a) phenyl, naphthyl, pyridine, pyrimidine, pyridazine, imidazole, benzimidazole, furan, thiophene, pyran, naphthyridine, oxazo[4,5- *b*]pyridine and imidazo[4,5-*b*]pyridine, which are optionally substituted with one to three groups selected from the group consisting of halogen, C₁₋₆ alkyl, C₁₋₆ alkoxy, hydroxy, mono- or di-(C₁₋₃ alkyl)amino, C₁₋₆ alkyl-S(O)ₘ and phenylamino wherein the phenyl ring is optionally substituted with one to two groups consisting of halogen, C₁₋₆ alkyl and C_{1- 6} alkoxy;
b) tetrahydropyran, tetrahydrofuran, 1,3-dioxolanone, 1,3-dioxanone, 1,4- dioxane, morpholine, thiomorpholine, thiomorpholine sulfoxide, thiomorpholine sulfone, piperidine, piperidinone, tetrahydropyrimidone, cyclohexanone, cyclohexanol, pentamethylene sulfide, pentamethylene sulfoxide, pentamethylene sulfone, tetramethylene sulfide, tetramethylene sulfoxide and tetramethylene sulfone which are optionally substituted with one to three groups selected from the group consisting of C₁₋₆ alkyl, C₁₋₆ alkoxy, hydroxy, mono- or di-(C₁₋₃ alkyl)amino-C₁₋₃ alkyl, phenylamino-C₁₋₃ alkyl and C₁₋₃ alkoxy-C₁₋₃ alkyl;
c) C₁₋₆ alkoxy, secondary or tertiary amine wherein the amino nitrogen is covalently bonded to groups selected from the group consisting of C₁₋₃ alkyl and C₁₋₅ alkoxyalkyl and phenyl wherein the phenyl ring is optionally substituted with one to two groups consisting of halogen, C₁₋₆ alkoxy, hydroxy or mono- or di-(C₁₋₃ alkyl)amino, C₁₋₆ alkyl-S(O)ᵣ, phenyl-S(O)ₜ, wherein the phenyl ring is optionally substituted with one to two groups consisting of halogen, C₁₋₆ alkoxy, hydroxy or mono- or di-(C₁₋₃ alkyl)amino;
- R₁: is selected from the group consisting of:
(a) C₃₋₁₀ branched or unbranched alkyl, which may optionally be partially or fully halogenated, and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle selected from the group hereinabove described, being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, cyano, C₁₋₃ alkyloxy which is optionally partially or fully halogenated, NH₂C(O) and di(C₁₋₃)alkylaminocarbonyl;
(b) C₃₋₇ cycloalkyl selected from the group consisting of cyclopropyl, cyclobutyl, cyclopentanyl, cyclohexanyl, cycloheptanyl, bicyclopentanyl, bicyclohexanyl and bicycloheptanyl, which may optionally be partially or fully halogenated and which may optionally be substituted with one to three C₁₋₃ alkyl groups, or an analog of such cycloalkyl group wherein one to three ring methylene groups are replaced by groups independently selected from O, S, CHOH, >C=O, >C=S and NH;
(c) C₃₋₁₀ branched alkenyl which may optionally be partially or fully halogenated, and which is optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl and bicycloheptanyl, hydroxy, cyano, C₁₋₃ alkyloxy which is optionally partially or fully halogenated, NH₂C(O), mono- or di(C₁₋₃)alkylaminocarbonyl;
(d) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group may optionally be substituted with one to three C₁₋₃ alkyl groups;
(e) cyano; and, (f) methoxycarbonyl, ethoxycarbonyl and propoxycarbonyl;
- R₂: is selected from the group consisting of: a C₁₋₆ branched or unbranched alkyl which may optionally be partially or fully halogenated, acetyl, aroyl, C₁₋₄ branched or unbranched alkoxy, which may optionally be partially or fully halogenated, halogen, methoxycarbonyl and phenylsulfonyl;
- R₃: is selected from the group consisting of:
a) a phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl; wherein such phenyl, naphthyl or heterocyclic group is optionally substituted with one to five groups selected from the group consisting of a C₁₋₆ branched or unbranched alkyl, phenyl, naphthyl, heterocycle selected from the group hereinabove described, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halo, hydroxy, cyano, C₁₋₃ alkyloxy which may optionally be partially or fully halogenated, phenyloxy, naphthyloxy, heteraryloxy wherein the heterocyclic moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di-(C₁₋₃)alkyl aminocarbonyl, C₁₋₅ alkyl-C(O)-C₁₋₄ alkyl, amino- C₁₋₅ alkyl, mono- or di-(C₁₋₃)alkylamino-C₁₋₅ alkyl, amino-S(O)₂, di- (C₁₋₃)alkylamino-S(O)₂, R₄ -C₁₋₅ alkyl, R₅ -C₁₋₅ alkoxy, R₆ -C(O)-C₁₋₅ alkyl and R₇ -C₁₋₅ alkyl(R₈)N;
b) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopenta- nopyrimidine, cyclohexanopyrimidine, cyclopentanopyrazine, cyclohexano- pyrazine, cyclopentanopyridazine, cyclohexanopyridazine, cyclopentano- quinoline, cyclohexanoquinoline, cyclopentanoisoquinoline, cyclohexano- isoquinoline, cyclopentanoindole, cyclohexanoindole, cyclopentanobenz- imidazole, cyclohexanobenzimidazole, cyclopentanobenzoxazole, cyclo- hexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halo, cyano, C₁₋₃ alkyloxy which is optionally partially or fully halogenated, phenyloxy, naphthyloxy, heterocyclyloxy wherein the heterocyclyl moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di-(C₁₋₃)alkyl aminocarbonyl, C₁₋₄ alkyl-OC(O),
   C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉-C₁₋₅ alkyl, R₁₀-C₁₋₅ alkoxy, R₁₁-C(O)-C₁₋₅ alkyl, and R₁₂-C₁₋₅ alkyl(R₁₃)N;
c) cycloalkyl selected from the group consisting of cyclopentanyl, cyclohexanyl, cycloheptanyl, bicyclopentanyl, bicyclohexanyl and bicycloheptanyl, which the cycloalkyl may optionally be partially or fully halogenated and which may optionally be substituted with one to three C₁₋₃ alkyl groups;
d) C₅₋₇ cycloalkenyl, selected from the group consisting of cyclopentenyl, cyclohexenyl, cyclohexadienyl, cycloheptenyl, cycloheptadienyl, bicyclohexenyl and bicycloheptenyl, wherein such cycloalkenyl group may optionally be substituted with one to three C₁₋₃ alkyl groups; and
e) acetyl, aroyl, alkoxycarbonylalkyl or phenylsulfonyl;
f) C₁₋₆ branched or unbranched alkyl which may optionally be partially or fully halogenated;
or R₁ and R₂ taken together may optionally form a fused phenyl or pyridinyl ring,
and wherein each R₈, R₁₃ is independently selected from the group consisting of:
hydrogen and C₁₋₄ branched or unbranched alkyl which may optionally be partially or fully halogenated;
each R₄, R₅, R₆, R₇, R₉, R₁₀, R₁₁ and R₁₂ is independently selected from the group consisting of:
   morpholine, piperidine, piperazine, imidazole and tetrazole;
   m = 0, 1, 2;
   r = 0, 1, 2;
   t = 0, 1, 2;
   X = O or S and physiologically acceptable acids or salts thereof.

In a preferred embodiment the invention relates to pharmaceutical compositions comprising **1** and **2d**, **characterized in that** the p38 kinase inhibitor **2d** is selected from the compounds of formula **(IV)** as disclosed in WO 00/43384 wherein Ar₂ is naphthyl, tetrahydronaphthyl, indanyl or indenyl.

A more preferred subgeneric aspect of the invention relates to pharmaceutical compositions comprising **1** and **2d**, **characterized in that** the p38 kinase inhibitor **2d** is a compound of the formula **(IV)** wherein Ar₂ is naphthyl.

A yet more preferred subgeneric aspect of the invention relates to pharmaceutical compositions comprising **1** and **2d, characterized in that** the p38 kinase inhibitor **2d** is selected from compounds of the formula **(IV)**, as described in the immediate previous paragraph, wherein:
- Ar₁: is thiophene or pyrazole;
- Ar₂: is 1-naphthyl;
- L: is C₁₋₆ saturated or unsaturated branched or unbranched carbon chain wherein one or more methylene groups are optionally independently replaced by O,N or S; and wherein said linking group is optionally substituted with 0-2 oxo groups and one or more C₁₋₄ branched or unbranched alkyl which may be substituted by one or more halogen atoms;
- R₁: is selected from the group consisting of C₁₋₄alkyl branched or unbranched, cyclopropyl and cyclohexyl which may optionally be partially or fully halogenated and which may optionally be substituted with one to three C₁₋₃ alkyl groups;
- R₃: is selected from the group consisting of C₁₋₄alkyl branched or unbranched, cyclopropyl, phenyl, pyridinyl each being optionally substituted as described above, alkoxycarbonylalkyl; C₁₋₆alkyl branched or unbranched; cyclopropyl or cyclopentyl optionally substituted as described above.

A yet further preferred subgeneric aspect of the invention relates to pharmaceutical compositions comprising **1** and **2d, characterized in that** the p38 kinase inhibitor **2d** is selected from compounds of the formula **(IV)**, as described in the immediate previous paragraph, wherein Ar₁ is pyrazole.

A still yet further preferred subgeneric aspect of previous the invention relates to pharmaceutical compositions comprising **1** and **2d**, **characterized in that** the p38 kinase inhibitor **2d** is selected from compounds of the formula **(IV)**, as described in the immediate paragraph, wherein L is C₁₋₅ saturated carbon chain wherein one or more methylene groups are optionally independently replaced by O,N or S; and wherein said linking group is optionally substituted with 0-2 oxo groups and one or more C₁₋₄ branched or unbranched alkyl which may be substituted by one or more halogen atoms;

Particularly preferred embodiments of L are propoxy, ethoxy, methoxy, methyl, propyl, C₃₋₅ acetylene or methylamino each being optionally substituted are described herein.

A more particularly preferred embodiment of L is ethoxy optionally substituted.

The following compounds are representative of the compounds of formula **(IV)** and are of particular interest as component **2d** in the compositions according to the invention:
1-[5-*tert*-butyl-2-*p*-tolyl-2H-pyrazol-3-yl]-3-[4-(2-morpholin-4-yl-ethoxy)naphthalen-1-yl]-urea;
1-[5-*tert*-butyl-2-*p*-tolyl-2H-pyrazol-3-yl]-3-[4-(2-(*cis*-2,6-dimethylmorpholin-4-yl)-ethoxy)naphthalen-1-yl]-urea;
1-[5-*tert*-butyl-2-*p*-tolyl-2H-pyrazol-3-yl]-3-[4-(2-(*trans*-2,6-dimethylmorpholin-4-yl)-ethoxy)naphthalen-1-yl]-urea;
1-[5-*tert*-butyl-2-*p*-tolyl-2H-pyrazol-3-yl]-3-[4-(2-(2-(methoxymethyl)morpholin-4-yl)-ethoxy)naphthalen-1-yl]-urea;
1-[5-*tert*-butyl-2-*p*-tolyl-2H-pyrazol-3-yl]-3-[4-(2-(morpholin-4-yl)-2-oxoethoxy)naphthalen-1-yl]-urea;
1-[5-*tert*-butyl-2-*p*-tolyl-2H-pyrazol-3-yl]-3-[4-(2-(morpholin-4-yl)-2-methylethoxy)-naphthalen-1-yl]-urea;
1-[5-*tert*-butyl-2-*p*-tolyl-2H-pyrazol-3-yl]-3-[4-(2-(morpholin-4-yl)-1-methylethoxy)-naphthalen-1-yl]-urea;
1-[5-*tert*-butyl-2-*p*-tolyl-2H-pyrazol-3-yl]-3-[4-(2-thiomorpholin-4-yl-ethoxy)naphthalen-1-yl]-urea;
1-[5-*tert*-butyl-2-*p*-tolyl-2H-pyrazol-3-yl]-3-[4-(2-(1-oxothiomorpholin-4-yl)ethoxy)-naphthalen-1-yl]-urea;
1-[5-*tert*-butyl-2-*p*-tolyl-2H-pyrazol-3-yl]-3-[4-(2-morpholin-4-yl-ethoxy)-3-methylnaphthalen-1-yl]-urea;
1-[5-*tert*-butyl-2-*p*-tolyl-2H-pyrazol-3-yl]-3-[4-(2-piperidin-4-yl-ethoxy)naphthalen-1-yl]-urea;
1-[5-*tert*-butyl-2-*p*-tolyl-2H-pyrazol-3-yl]-3-[4-(2-(1-acetylpiperidin-4-yl)ethoxy)naphthalen-1-yl]-urea;
1-[5-*tert*-butyl-2-*p*-tolyl-2H-pyrazol-3-yl]-3-[4-(2-thiazolidin-3-yl-ethoxy)naphthalen-1-yl]-urea;
1-[5-*tert*-butyl-2-*p*-tolyl-2H-pyrazol-3-yl]-3-[4-(2-(morpholin-4-yl-carbonyloxo)-ethoxy)naphthalen-1-yl]-urea;
1-[5-*tert*-butyl-2-*p*-tolyl-2H-pyrazol-3-yl]-3-[4-(2-(tetrahydropyran-4-yl)ethoxy)-naphthalen-1-yl]-urea;
1-[5-*tert*-butyl-2-*p*-tolyl-2H-pyrazol-3-yl]-3-[4-(2-(N-methyl-2-methoxyethylamino)-ethoxy)naphthalen-1-yl]-urea;
1-[5-*tert*-butyl-2-*p*-tolyl-2H-pyrazol-3-yl]-3-[4-(2-(1-oxo-tetrahydrothiophen-3-yl)ethoxy)naphthalen-1-yl]-urea;
1-[5-*tert*-butyl-2-*p*-tolyl-2H-pyrazol-3-yl]-3-[4-(3-morpholin-4-yl-propyl)naphthalen-1-yl]-urea;
1-[5-*tert*-butyl-2-*p*-tolyl-2H-pyrazol-3-yl]-3-[4-(morpholin-4-yl-methyl)naphthalen-1-yl]-urea;
1-[5-*tert*-butyl-2-*p*-tolyl-2H-pyrazol-3-yl]-3-[4-(3-thiazolidin-3-yl-propyl)naphthalen-1-yl]-urea;
1-[5-*tert*-butyl-2-*p*-tolyl-2H-pyrazol-3-yl]-3-[4-(3-(tetrahydopyran-2-yl-oxy)propyl)-naphthalen-1-yl]-urea;
1-[5-*tert*-butyl-2-*p*-tolyl-2H-pyrazol-3-yl]-3-[4-(2-pyridin-4-yl-ethyl)naphthalen-1-yl]-urea;
1-[5-*tert*-butyl-2-*p*-tolyl-2H-pyrazol-3-yl]-3-[4-(2-pyridin-4-yl-ethenyl)naphthalen-1-yl]-urea;
1-[5-*tert*-butyl-2-*p*-tolyl-2H-pyrazol-3-yl]-3-[4-(3-(morpholin-4-yl)propyn-1-yl)naphthalen-1-yl]-urea;
1-[5-*tert*-butyl-2-*p*-tolyl-2H-pyrazol-3-yl]-3-[4-(3-(tetrahydropyran-2-yl-oxy)propyn-1-yl)naphthalen-1-yl]-urea;
1-[5-*tert*-butyl-2-*p*-tolyl-2H-pyrazol-3-yl]-3-[4-(3-(methoxymethyloxy)propyn-1-yl)naphthalen-1-yl]-urea;
1-[5-*tert*-butyl-2-*p*-tolyl-2H-pyrazol-3-yl]-3-[4-(3-(morpholin-4-yl)-3-methylpropyn-1-yl)naphthalen-1-yl]-urea;
1-[5-*tert*-butyl-2-*p*-tolyl-2H-pyrazol-3-yl]-3-[4-(3-(morpholin-4-yl)-3,3-dimethylpropyn-1-yl)naphthalen-1-yl]-urea;
1-[5-*tert*-butyl-2-*p*-tolyl-2H-pyrazol-3-yl]-3-[4-(3-(tetrahydropyran-2-yl-oxy)butyn-1-yl)naphthalen-1-yl]-urea;
1-[5-*tert*-butyl-2-*p*-tolyl-2H-pyrazol-3-yl]-3-[4-(3-(furan-2-ylcarbonyloxy)propyn-1-yl)naphthalen-1-yl]-urea;
1-[5-*tert*-butyl-2-*p*-tolyl-2H-pyrazol-3-yl]-3-[4-(3-(piperdin-1-yl)propyn-1-yl)naphthalen-1-yl]-urea;
1-[5-*tert*-butyl-2-*p*-tolyl-2H-pyrazol-3-yl]-3-[4-(3-(2-methoxymethylmorpholin-4-yl)propyn-1-yl)naphthalen-1-yl]-urea;
1-[5-*tert*-butyl-2-*p*-tolyl-2H-pyrazol-3-yl]-3-[4-(pyridin-4-yl-methoxy)naphthalen-1-yl]-urea;
1-[5-*tert*-butyl-2-*p*-tolyl-2H-pyrazol-3-yl]-3-[4-(2-pyridin-4-yl-ethoxy)naphthalen-1-yl]-urea;
1-[5-*tert*-butyl-2-*p*-tolyl-2H-pyrazol-3-yl]-3-[4-(3-pyridin-4-yl-propoxy)naphthalen-1-yl]-urea;
1-[5-*tert*-butyl-2-*p*-tolyl-2H-pyrazol-3-yl]-3-[4-(2-imidazol-1-yl-ethoxy)naphthalen-1-yl]-urea;
1-[5-*tert*-butyl-2-*p*-tolyl-2H-pyrazol-3-yl]-3-[4-(2-benzimidazol-1-yl-ethoxy)naphthalen-1-yl]-urea;
1-[5-*tert*-butyl-2-*p*-tolyl-2H-pyrazol-3-yl]-3-[4-(2-(3,4-dimethoxyphenyl)-ethoxy)naphthalen-1-yl]-urea;
1-[5-*tert*-butyl-2-*p*-tolyl-2H-pyrazol-3-yl]-3-[4-(pyridin-4-yl-methylamino)naphthalen-1-yl]-urea;
1-[5-*tert*-butyl-2-*p*-tolyl-2H-pyrazol-3-yl]-3-[4-(pyridin-4-yl-carbonylamino)naphthalen-1-yl]-urea;
1-[5-*tert*-butyl-2-*p*-tolyl-2H-pyrazol-3-yl]-3-[4-(morpholin-4-yl-acetamido)naphthalen-1-yl]-urea;
1-[5-*tert*-butyl-2-*p*-tolyl-2H-pyrazol-3-yl]-3-[4-(pyridin-3-yl-methylamino)naphthalen-1-yl]-urea;
1-[5-*tert*-butyl-2-*p*-tolyl-2H-pyrazol-3-yl]-3-[4-(pyridin-3-yl-carbonylamino)naphthalen-1-yl]-urea;
1-[5-*iso*-propyl-2-phenyl-2H-pyrazol-3-yl]-3-[4-(2-morpholin-4-yl-ethoxy)naphthalen-1-yl]-urea;
1-[5-(tetrahydropyran-3-yl)-2-phenyl-2H-pyrazol-3-yl]-3-[4-(2-morpholin-4-yl-ethoxy)-naphthalen-1-yl]-urea;
1-[5-cyclohexyl-2-phenyl-2H-pyrazol-3-yl]-3-[4-(2-morpholin-4-yl-ethoxy)naphthalen-1-yl]-urea;
1-[5-(2,2,2-trifluoroethyl)-2-phenyl-2H-pyrazol-3-yl]-3-[4-(2-morpholin-4-yl-ethoxy)-naphthalen-1-yl]-urea;
1-[5-(1-methylcycloprop-1-yl)-2-phenyl-2H-pyrazol-3-yl]-3-[4-(2-morpholin-4-yl-ethoxy)naphthalen-1-yl]-urea;
1-[5-ethoxycarbonyl-2-phenyl-2H-pyrazol-3-yl]-3-[4-(2-morpholin-4-yl-ethoxy)naphthalen-1-yl]-urea;
1-[5-(1-methylcyclohex-1-yl)-2-phenyl-2H-pyrazol-3-yl]-3-[4-(2-morpholin-4-yl-ethoxy)naphthalen-1-yl]-urea;
1-[5-*tert*-butyl-2-methyl-2H-pyrazol-3-yl]-3-[4-(2-morpholin-4-yl-ethoxy)naphthalen-1-yl]-urea;
1-[5-*tert*-butyl-2-benzyl-2H-pyrazol-3-yl]-3-[4-(2-morpholin-4-yl-ethoxy)naphthalen-1-yl]-urea;
1-[5-*tert*-butyl-2-(4-chlorophenyl)-2H-pyrazol-3-yl]-3-[4-(2-morpholin-4-yl-ethoxy)-naphthalen-1-yl]-urea;
1-[5-*tert*-butyl-2-butyl-2H-pyrazol-3-yl]-3-[4-(2-morpholin-4-yl-ethoxy)naphthalen-1-yl]-urea;
1-[5-*tert*-butyl-2-(ethoxycarbonylmethyl)-2H-pyrazol-3-yl]-3-[4-(2-morpholin-4-yl-ethoxy)naphthalen-1-yl]-urea;
1-[5-*tert*-butyl-2-(4-methyl-3-carbamylphenyl)-2H-pyrazol-3-yl]-3-[4-(2-morpholin-4-yl-ethoxy)naphthalen-1-yl]-urea;
1-[5-*tert*-butyl-2-(4-methyl-3-(2-ethoxycarbonylvinyl)phenyl)-2H-pyrazol-3-yl]-3-[4-(2-morpholin-4-yl-ethoxy)naphthalen-1-yl]-urea;
1-[5-*tert*-butyl-2-(4-methyl-3-(morpholin-4-yl)methylphenyl)-2H-pyrazol-3-yl]-3-[4-(2-morpholin-4-yl-ethoxy)naphthalen-1-yl]-urea;
1-[5-*tert*-butyl-2-(4-methyl-3-dimethylaminomethylphenyl)-2H-pyrazol-3-yl]-3-[4-(2-morpholin-4-yl-ethoxy)naphthalen-1-yl]-urea;
1-[5-*tert*-butyl-2-(3-(2-morpholin-4-yl-ethyl)phenyl)-2H-pyrazol-3-yl]-3-[4-(2-morpholin-4-yl-ethoxy)naphthalen-1-yl]-urea;
1-[5-*tert*-butyl-2-(3-(tetrahydropyran-4-ylamino)phenyl)-2H-pyrazol-3-yl]-3-[4-(2-morpholin-4-yl-ethoxy)naphthalen-1-yl]-urea;
1-[5-*tert*-butyl-2-(3-dimethylaminomethylphenyl)-2H-pyrazol-3-yl]-3-[4-(2-morpholin-4-yl-ethoxy)naphthalen-1-yl]-urea;
1-[5-*tert*-butyl-2-(4-(tetrahydropyran-4-ylamino)phenyl)-2H-pyrazol-3-yl]-3-[4-(2-morpholin-4-yl-ethoxy)naphthalen-1-yl]-urea;
1-[5-*tert*-butyl-2-(4-(3-benzylureido)phenyl)-2H-pyrazol-3-yl]-3-[4-(2-morpholin-4-yl-ethoxy)naphthalen-1-yl]-urea;
1-[5-*tert*-butyl-2-(2-chloropyridin-5-yl)-2H-pyrazol-3-yl]-3-[4-(2-morpholin-4-yl-ethoxy)-naphthalen-1-yl]-urea;
1-[5-*tert*-butyl-2-(2-methylpyridin-5-yl)-2H-pyrazol-3-yl]-3-[4-(2-morpholin-4-yl-ethoxy)naphthalen-1-yl]-urea;
1-[5-*tert*-butyl-2-(2-methoxypyridin-5-yl)-2H-pyrazol-3-yl]-3-[4-(2-morpholin-4-yl-ethoxy)naphthalen-1-yl]-urea;
1-[5-*tert*-butyl-2-(pyridin-3-yl)-2H-pyrazol-3-yl]-3-[4-(2-morpholin-4-yl-ethoxy)naphthalen-1-yl]-urea;
1-[5-*tert*-butyl-2-(2-methylpyridin-5-yl)-2H-pyrazol-3-yl]-3-[4-(2-pyridin-4-yl-ethoxy)-naphthalen-1-yl]-urea;
1-[5-*tert*-butyl-2-(2-methylpyridin-5-yl)-2H-pyrazol-3-yl]-3-[4-(2-(*trans*-2,6-dimethyl-morphol in-4-yl)ethoxy)naphthalen-1-yl]-urea;
1-[5-*tert*-butyl-2-(2-methylpyridin-5-yl)-2H-pyrazol-3-yl]-3-[4-(3-morpholin-4-yl-propyn-1-yl)naphthalen-1-yl]-urea;
1-[5-*tert*-butyl-2-*p*-tolyl-2H-pyrazol-3-yl]-3-[4-(2-(2-dimethylaminomethylmorpholin-4-yl)ethoxy)naphthalen-1-yl]-urea;
1-[5-*tert*-butyl-2-*iso*-propyl-2H-pyrazol-3-yl]-3-[4-(2-morpholin-4-yl-ethoxy)naphthalen-1-yl]-urea;
1-[5-*tert*-butyl-2-cyclopropyl-2H-pyrazol-3-yl]-3-[4-(2-morpholin-4-yl-ethoxy)naphthalen-1-yl]-urea;
1-[5-*tert*-butyl-2-(thiophen-3-yl)-2H-pyrazol-3-yl]-3-[4-(2-morpholin-4-yl-ethoxy)naphthalen-1-yl]-urea;
1-[5-*tert*-butyl-2-cyclopentyl-2H-pyrazol-3-yl]-3-[4-(2-morpholin-4-yl-ethoxy)naphthalen-1-yl]-urea;
1-[5-*tert*-butyl-2-*iso*-propyl-2H-pyrazol-3-yl]-3-[4-(tetrahyropyran-4-yl-ethoxy)naphthalen-1-yl]-urea;
1-[5-*tert*-butyl-2-cyclopropyl-2H-pyrazol-3-yl]-3-[4-(1-oxo-tetrahydrothiophen-3-yl-ethoxy)naphthalen-1-yl]-urea;
1-[5-*tert*-butyl-2-(thiophen-3-yl)-2H-pyrazol-3-yl]-3-[4-(2-pyridinyl-4-yl-ethoxy)-naphthalen-1-yl]-urea;
1-[5-*tert*-butyl-2-cyclopentyl-2H-pyrazol-3-yl]-3-[4-(pyridin-4-yl-methoxy)naphthalen-1-yl]-urea;
1-[5-*tert*-butyl-2-*p*-tolyl-2H-pyrazol-3-yl]-3-[4-(3-(pyridin-4-yl)propyn-1-yl)naphthalen-1-yl]-urea;
1-[5-*tert*-butyl-2-*p*-tolyl-2H-pyrazol-3-yl]-3-[4-(3-(2-methylaminopyridin-4-yl)propyn-1-yl)naphthalen-1-yl]-urea;
1-[5-*tert*-butyl-2-*p*-tolyl-2H-pyrazol-3-yl]-3-[4-(3-(1-oxo-tetrahydothiophen-3-yl)propyn-1-yl)naphthalen-1-yl]-urea;
1-[5-*tert*-butyl-2-*p*-tolyl-2H-pyrazol-3-yl]-3-[4-(3-(thiazolidin-3-yl)propyn-1-yl)naphthalen-1-yl]-urea;
1-[5-*tert*-butyl-2-*p*-tolyl-2H-pyrazol-3-yl]-3-[4-(3-(tetrahydropyran-4-yl)propyn-1-yl)-naphthalen-1-yl]-urea;
1-[5-*tert*-butyl-2-*p*-tolyl-2H-pyrazol-3-yl]-3-[4-(2-methylaminopyrimidin-4-yl-methoxy)-naphthalen-1-yl]-urea;
1-[5-*tert*-butyl-2-*p*-tolyl-2H-pyrazol-3-yl]-3-[4-(2-(2-methylaminopyrimidin-4-yl)ethoxy)-naphthalen-1-yl]-urea;
1-[5-*tert*-butyl-2-*p*-tolyl-2H-pyrazol-3-yl]-3-[4-(2-(4-methoxybenzimidazol-1-yl)ethoxy)-naphthalen-1-yl]-urea;
1-[5-*tert*-butyl-2-*p*-tolyl-2H-pyrazol-3-yl]-3-[4-(2-(4-methylaminobenzimidazol-1-yl)ethoxy)naphthalen-1-yl]-urea;
1-[5-*tert*-butyl-2-*p*-tolyl-2H-pyrazol-3-yl]-3-[4-(2-(2-imidazo[4,5-b]pyridin-1-yl)ethoxy)-naphthalen-1-yl]-urea;
1-[5-*tert*-butyl-2-*p*-tolyl-2H-pyrazol-3-yl]-3-[4-(2-[1,8]naphthyridin-4-yl)ethoxy)naphthalen-1-yl]-urea;
1-[5-*tert*-butyl-2-*p*-tolyl-2H-pyrazol-3-yl]-3-[4-(2-(3,4-dihydro-2H-pyrano[2,3-b]pyridin-5-yl)ethoxy)naphthalen-1-yl]-urea;
1-[5-*tert*-butyl-2-pyridin-3-yl-2H-pyrazol-3-yl]-3-[4-(2-methylaminopyrimidin-4-yl-methoxy)naphthalen-1-yl]-urea;
1-[5-*tert*-butyl-2-(2-methylpyridin-5-yl)-2H-pyrazol-3-yl]-3-[4-(2-(2-methylaminopyri-midin-4-yl)ethoxy)naphthalen-1-yl]-urea;
1-[5-*tert*-butyl-2-(2-methylpyridin-5-yl)-2H-pyrazol-3-yl]-3-[4-(2-(4-methoxybenzimidazol-1-yl)ethoxy)naphthalen-1-yl]-urea;
1-[5-*tert*-butyl-2-(2-methylpyridin-5-yl)-2H-pyrazol-3-yl]-3-[4-(2-(4-methylaminobenzimidazol-1-yl)ethoxy)naphthalen-1-yl]-urea;
1-[5-*tert*-butyl-2-(2-methylpyridin-5-yl)-2H-pyrazol-3-yl]-3-[4-(2-(2-imidazo[4,5-b]pyridin-1-yl)ethoxy)naphthalen-1-yl]-urea;
1-[5-*tert*-butyl-2-(2-methylpyridin-5-yl)-2H-pyrazol-3-yl]-3-[4-(2-[1,8]naphthyridin-4-yl)ethoxy)naphthalen-1-yl]-urea;
1-[5-*tert*-butyl-2-(2-methylpyridin-5-yl)-2H-pyrazol-3-yl]-3-[4-(2-(3,4-dihydro-2H-pyrano[2,3-b]pyridin-5-yl)ethoxy)naphthalen-1-yl]-urea;
1-[5-*tert*-butyl-2-cyclopropyl-2H-pyrazol-3-yl]-3-[4-(2-methylaminopyrimidin-4-yl-methoxy)naphthalen-1-yl]-urea;
1-[5-*tert*-butyl-2-cyclopropyl-2H-pyrazol-3-yl]-3-[4-(2-(2-methylaminopyrimidin-4-yl)ethoxy)naphthalen-1-yl]-urea;
1-[5-*tert*-butyl-2-cyclopropyl-2H-pyrazol-3-yl]-3-[4-(2-(4-methoxybenzimidazol-1-yl)ethoxy)naphthalen-1-yl]-urea;
1-[5-*tert*-butyl-2-cyclopropyl-2H-pyrazol-3-yl]-3-[4-(2-(4-methylaminobenzimidazol-1-yl)ethoxy)naphthalen-1-yl]-urea;
1-[5-*tert*-butyl-2-methyl-2H-pyrazol-3-yl]-3-[4-(2-(2-imidazo[4,5-b]pyridin-1-yl)ethoxy)-naphthalen-1-yl]-urea;
1-[5-*tert*-butyl-2-methyl-2H-pyrazol-3-yl]-3-[4-(2-[1,8]naphthyridin-4-yl)ethoxy)naphthalen-1-yl]-urea;
1-[5-*tert*-butyl-2-methyl-2H-pyrazol-3-yl]-3-[4-(2-(3,4-dihydro-2H-pyrano[2,3-b]pyridin-5-yl)ethoxy)naphthalen-1-yl]-urea;
and their physiologically acceptable acids or salts thereof.

In a particularly preferred embodiment the invention relates to pharmaceutical compositions comprising **1** and **2d, characterized in that** the p38 kinase inhibitor **2d** is selected from the following compounds of formula **(IV)** as disclosed in WO 00/43384:
1-[5-*tert*-butyl-2-*p*-tolyl-2H-pyrazol-3-yl]-3-[4-(2-morpholin-4-yl-ethoxy)naphthalen-1-yl]-urea;
1-[5-*tert*-butyl-2-*p*-tolyl-2H-pyrazol-3-yl]-3-[4-(2-(*cis*-2,6-dimethylmorpholin-4-yl)ethoxy)naphthalen-1-yl]-urea;
1-[5-*tert*-butyl-2-p-tolyl-2H-pyrazol-3-yl]-3-[4-(2-(*trans*-2,6-dimethylmorpholin-4-yl)ethoxy)naphthalen-1-yl]-urea;
1-[5-*tert*-butyl-2-*p*-tolyl-2H-pyrazol-3-yl]-3-[4-(2-(2-(methoxymethyl)morpholin-4-yl)ethoxy)naphthalen-1-yl]-urea;
1-[5-*tert*-butyl-2-*p*-tolyl-2H-pyrazol-3-yl]-3-[4-(2-(morpholin-4-yl)-2-oxoethoxy)naphthalen-1-yl]-urea;
1-[5-*tert*-butyl-2-*p*-tolyl-2H-pyrazol-3-yl]-3-[4-(2-(morpholin-4-yl)-2-methylethoxy)-naphthalen-1-yl]-urea;
1-[5-*tert*-butyl-2-*p*-tolyl-2H-pyrazol-3-yl]-3-[4-(2-(morpholin-4-yl)-1-methyl-ethoxy)naphthalen-1-yl]-urea;
1-[5-*tert*-butyl-2-*p*-tolyl-2H-pyrazol-3-yl]-3-[4-(2-thiomorpholin-4-yl-ethoxy)naphthalen-1-yl]-urea;
1-[5-*tert*-butyl-2-*p*-tolyl-2H-pyrazol-3-yl]-3-[4-(2-(1-oxothiomorpholin-4-yl)ethoxy)naphthalen-1-yl]-urea;
1-[5-*tert*-butyl-2-*p*-tolyl-2H-pyrazol-3-yl]-3-[4-(2-morpholin-4-yl-ethoxy)-3-methylnaph-thalen-1-yl]-urea;
1-[5-*tert*-butyl-2-*p*-tolyl-2H-pyrazol-3-yl]-3-[4-(2-(morpholin-4-yl-carbonyloxo)ethoxy)-naphthalen-1-yl]-urea;
1-[5-*tert*-butyl-2-*p*-tolyl-2H-pyrazol-3-yl]-3-[4-(2-(tetrahydropyran-4-yl)ethoxy)naphthalen-1-yl]-urea;
1-[5-*tert*-butyl-2-*p*-tolyl-2H-pyrazol-3-yl]-3-[4-(2-(1-oxo-tetrahydrothiophen-3-yl)-ethoxy)naphthalen-1-yl]-urea;
1-[5-*tert*-butyl-2-*p*-tolyl-2H-pyrazol-3-yl]-3-[4-(3-morpholin-4-yl-propyl)naphthalen-1-yl]-urea;
1-[5-*tert*-butyl-2-*p*-tolyl-2H-pyrazol-3-yl]-3-[4-(morpholin-4-yl-methyl)naphthalen-1-yl]-urea;
1-[5-*tert*-butyl-2-*p*-tolyl-2H-pyrazol-3-yl]-3-[4-(2-pyridin-4-yl-ethyl)naphthalen-1-yl]-urea;
1-[5-*tert*-butyl-2-*p*-tolyl-2H-pyrazol-3-yl]-3-[4-(3-(morpholin-4-yl)propyn-1-yl)naphthalen-1-yl]-urea;
1-[5-*tert*-butyl-2-*p*-tolyl-2H-pyrazol-3-yl]-3-[4-(3-(tetrahydropyran-2-yl-oxy)propyn-1-yl)naphthalen-1-yl]-urea;
1-[5-*tert*-butyl-2-*p*-tolyl-2H-pyrazol-3-yl]-3-[4-(3-(tetrahydropyran-2-yl-oxy)butyn-1-yl)naphthalen-1-yl]-urea;
1-[5-*tert*-butyl-2-*p*-tolyl-2H-pyrazol-3-yl]-3-[4-(3-(piperdin-1-yl)propyn-1-yl)naphthalen-1-yl]-urea;
1-[5-*tert*-butyl-2-*p*-tolyl-2H-pyrazol-3-yl]-3-[4-(3-(2-methoxymethylmorpholin-4-yl)propyn-1-yl)naphthalen-1-yl]-urea;
1-[5-*tert*-butyl-2-*p*-tolyl-2H-pyrazol-3-yl]-3-[4-(pyridin-4-yl-methoxy)naphthalen-1-yl]-urea;
1-[5-*tert*-butyl-2-*p*-tolyl-2H-pyrazol-3-yl]-3-[4-(2-pyridin-4-yl-ethoxy)naphthalen-1-yl]-urea;
1-[5-*tert*-butyl-2-*p*-tolyl-2H-pyrazol-3-yl]-3-[4-(3-pyridin-4-yl-propoxy)naphthalen-1-yl]-urea;
1-[5-*tert*-butyl-2-*p*-tolyl-2H-pyrazol-3-yl]-3-[4-(2-imidazol-1-yl-ethoxy)naphthalen-1-yl]-urea;
1-[5-*tert*-butyl-2-*p*-tolyl-2H-pyrazol-3-yl]-3-[4-(2-(3,4-dimethoxyphenyl)-ethoxy)naphthalen-1-yl]-urea;
1-[5-*tert*-butyl-2-*p*-tolyl-2H-pyrazol-3-yl]-3-[4-(pyridin-4-yl-methylamino)naphthalen-1-yl]-urea;
1-[5-*iso*-propyl-2-phenyl-2H-pyrazol-3-yl]-3-[4-(2-morpholin-4-yl-ethoxy)naphthalen-1-yl]-urea;
1-[5-cyclohexyl-2-phenyl-2H-pyrazol-3-yl]-3-[4-(2-morpholin-4-yl-ethoxy)naphthalen-1-yl]-urea;
1-[5-(2,2,2-trifluoroethyl)-2-phenyl-2H-pyrazol-3-yl]-3-[4-(2-morpholin-4-yl-ethoxy)-naphthalen-1-yl]-urea;
1-[5-(1-methylcycloprop-1-yl)-2-phenyl-2H-pyrazol-3-yl]-3-[4-(2-morpholin-4-yl-ethoxy)naphthalen-1-yl]-urea;
1-[5-(1-methylcyclohex-1-yl)-2-phenyl-2H-pyrazol-3-yl]-3-[4-(2-morpholin-4-yl-ethoxy)naphthalen-1-yl]-urea;
1-[5-*tert*-butyl-2-methyl-2H-pyrazol-3-yl]-3-[4-(2-morpholin-4-yl-ethoxy)naphthalen-1-yl]-urea;
1-[5-*tert*-butyl-2-(4-chlorophenyl)-2H-pyrazol-3-yl]-3-[4-(2-morpholin-4-yl-ethoxy)-naphthalen-1-yl]-urea;
1-[5-*tert*-butyl-2-butyl-2H-pyrazol-3-yl]-3-[4-(2-morpholin-4-yl-ethoxy)naphthalen-1-yl]-urea;
1-[5-*tert*-butyl-2-(4-methyl-3-carbamylphenyl)-2H-pyrazol-3-yl]-3-[4-(2-morpholin-4-yl-ethoxy)naphthalen-1-yl]-urea;
1-[5-*tert*-butyl-2-(4-methyl-3-(morpholin-4-yl)methylphenyl)-2H-pyrazol-3-yl]-3-[4-(2-morpholin-4-yl-ethoxy)naphthalen-1-yl]-urea;
1-[5-*tert*-butyl-2-(4-methyl-3-dimethylaminomethylphenyl)-2H-pyrazol-3-yl]-3-[4-(2-morpholin-4-yl-ethoxy)naphthalen-1-yl]-urea;
1-[5-*tert*-butyl-2-(3-dimethylaminomethylphenyl)-2H-pyrazol-3-yl]-3-[4-(2-morpholin-4-yl-ethoxy)naphthalen-1-yl]-urea;
1-[5-*tert*-butyl-2-(2-chloropyridin-5-yl)-2H-pyrazol-3-yl]-3-[4-(2-morpholin-4-yl-ethoxy)-naphthalen-1-yl]-urea;
1-[5-*tert*-butyl-2-(2-methylpyridin-5-yl)-2H-pyrazol-3-yl]-3-[4-(2-morpholin-4-yl-ethoxy)naphthalen-1-yl]-urea;
1-[5-*tert*-butyl-2-(2-methoxypyridin-5-yl)-2H-pyrazol-3-yl]-3-[4-(2-morpholin-4-yl-ethoxy)naphthalen-1-yl]-urea;
1-[5-*tert*-butyl-2-(pyridin-3-yl)-2H-pyrazol-3-yl]-3-[4-(2-morpholin-4-yl-ethoxy)-naphthalen-1-yl]-urea;
1-[5-*tert*-butyl-2-(2-methylpyridin-5-yl)-2H-pyrazol-3-yl]-3-[4-(2-pyridin-4-yl-ethoxy)-naphthalen-1-yl]-urea;
1-[5-*tert*-butyl-2-(2-methylpyridin-5-yl)-2H-pyrazol-3-yl]-3-[4-(2-(*trans*-2,6-dimethyl-morphol in-4-yl)ethoxy)naphthalen-1-yl]-urea;
1-[5-*tert*-butyl-2-(2-methylpyridin-5-yl)-2H-pyrazol-3-yl]-3-[4-(3-morpholin-4-yl-propyn-1-yl)naphthalen-1-yl]-urea.

Particularly preferred p38 kinase inhibitors **2d** within the scope of the present invention are the following compounds of the formula **(IV):**
1-[5-*tert*-butyl-2-*p*-tolyl-2H-pyrazol-3-yl]-3-[4-(2-morpholin-4-yl-ethoxy)naphthalen-1-yl]-urea;
1-[5-*tert*-butyl-2-*p*-tolyl-2H-pyrazol-3-yl]-3-[4-(2-(1-oxothiomorpholin-4-yl)ethoxy)-naphthalen-1-yl]-urea;
1-[5-*tert*-butyl-2-(2-methylpyridin-5-yl)-2H-pyrazol-3-yl]-3-[4-(2-pyridin-4-yl-ethoxy)-naphthalen-1-yl]-urea;
1-[5-*tert*-butyl-2-(2-methoxypyridin-5-yl)-2H-pyrazol-3-yl]-3-[4-(2-morpholin-4-yl-ethoxy)naphthalen-1-yl]-urea or
1-[5-*tert*-butyl-2-methyl-2H-pyrazol-3-yl]-3-[4-(2-morpholin-4-yl-ethoxy)naphthalen-1-yl]-urea.

In another preferred embodiment the invention relates to pharmaceutical compositions comprising **1** and **2d**, **characterized in that** the p38 kinase inhibitor **2d** is selected from the compounds of formula **(V)** as disclosed in WO 00/55139 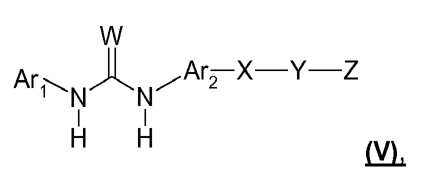 wherein:
- Ar₁: is selected from the group consisting of: pyrrole, pyrrolidine, pyrazole, imidazole, oxazole, thiazole, furan and thiophene; wherein Ar₁ may be substituted by one or more R₁, R₂ or R₃;
- Ar₂: is: phenyl, naphthyl, quinoline, isoquinoline, tetrahydronaphthyl, tetrahydroquinoline, tetrahydroisoquinoline, benzimidazole, benzofuran, indanyl, indenyl or indole each being optionally substituted with zero to three R₂ groups;
- X: is:
a) a C₅₋₈ cycloalkyl or cycloalkenyl optionally substituted with 0-2 oxo groups or 0-3 C₁₋₄ branched or unbranched alkyl, C₁₋₄ alkoxy or C₁₋₄ alkylamino chains;
b) phenyl, furan, thiophene, pyrrole, imidazolyl, pyridine, pyrimidine, pyridinone, dihydropyridinone, maleimide, dihydromaleimide, piperdine, piperazine or pyrazine each being optionally independently substituted with 0-3 C₁₋₄ branched or unbranched alkyl, C₁₋₄alkoxy, hydroxy, nitrile, mono- or di-(C₁₋₃ alkyl)amino, C₁₋₆ alkyl-S(O)ₘ, or halogen;
- Y: is: a bond or a C₁₋₄ saturated or unsaturated branched or unbranched carbon chain optionally partially or fully halogenated, wherein one or more methylene groups are optionally replaced by O, NH, S(O), S(O)₂ or S and wherein Y is optionally independently substituted with 0-2 oxo groups and one or more C₁₋₄ branched or unbranched alkyl which may be substituted by one or more halogen atoms;
- Z: is:
a) phenyl, pyridine, pyrimidine, pyridazine, imidazole, furan, thiophene, pyran, which are optionally substituted with one to three groups consisting of halogen, C₁₋₆ alkyl, C₁₋₆ alkoxy, hydroxy, mono- or di-(C₁₋₃ alkyl)amino, C₁₋₆ alkyl-S(O)ₘ , COOH and phenylamino wherein the phenyl ring is optionally substituted with one to two groups consisting of halogen, C₁₋₆ alkyl and C₁₋₆ alkoxy;
b) tetrahydropyran, tetrahydrofuran, 1,3-dioxolanone, 1,3-dioxanone, 1,4- dioxane, morpholine, thiomorpholine, thiomorpholine sulfoxide, piperidine, piperidinone, piperazine, tetrahydropyrimidone, cyclohexanone, cyclohexanol, pentamethylene sulfide, pentamethylene sulfoxide, pentamethylene sulfone, tetramethylene sulfide, tetramethylene sulfoxide or tetramethylene sulfone which are optionally substituted with one to three groups consisting of nitrile, C₁₋₆ alkyl, C₁₋₆ alkoxy, hydroxy, mono- or di-(C₁₋₃ alkyl)amino-C₁₋₃ alkyl, phenylamino-C₁₋₃ alkyl and C₁₋₃ alkoxy-C₁₋₃ alkyl;
c) C₁₋₆ alkoxy, secondary or tertiary amine wherein the amino nitrogen is covalently bonded to groups selected from the group consisting of C₁₋₃ alkyl, C₁₋₅ alkoxyalkyl, pyridinyl-C₁₋₃ alkyl, imidazolyl-C₁₋₃ alkyl, tetrahydrofuranyl-C₁₋₃ alkyl, phenylamino, wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
- R₁: is :
a) C₃₋₁₀ branched or unbranched alkyl optionally partially or fully halogenated and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle selected from the group hereinabove described in this paragraph, and being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, nitrile, C₁₋₃ alkyloxy which is optionally partially or fully halogenated, NH₂C(O) and di(C₁₋₃)alkylaminocarbonyl;
b) C₃₋₇ cycloalkyl selected from the group consisting of cyclopropyl, cyclobutyl, cyclopentanyl, cyclohexanyl, cycloheptanyl, bicyclopentanyl, bicyclohexanyl and bicycloheptanyl each being optionally be partially or fully halogenated and optionally substituted with one to three C₁₋₃ alkyl groups, or an analog of such cycloalkyl group wherein one to three ring methylene groups are replaced by groups independently selected from the group consisting of O, S, CHOH, >C=O, >C=S and NH;
c) C₃₋₁₀ branched alkenyl optionally partially or fully halogenated and optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from the group consisting of halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, hydroxy, nitrile, C₁₋₃ alkoxy which is optionally partially or fully halogenated, NH₂C(O) and mono- or di(C₁₋₃)alkylaminocarbonyl;
d) a C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) nitrile; or
f) C₁₋₆ branched or unbranched alkoxycarbonyl, C₁₋₆ branched or unbranched alkylaminocarbonyl, C₁₋₆ branched or unbranched alkylcarbonylamino-C₁₋₃-alkyl;
- R₂: is: a C₁₋₆ branched or unbranched alkyl optionally partially or fully halogenated, acetyl, aroyl, C₁₋₄ branched or unbranched alkoxy optionally partially or fully halogenated, halogen, methoxycarbonyl or phenylsulfonyl;
- R₃: is:
a) phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl, wherein such phenyl, naphthyl or heterocyclic group is optionally substituted with one to five groups selected from the group consisting of phenyl, naphthyl, heterocycle selected from the group hereinabove described in this paragraph, C₁₋₆ branched or unbranched alkyl which is optionally partially or fully halogenated, cyclopropyl, cyclobutyl, cyclopentyl, cyclohexyl, cycloheptyl, bicyclopentyl, bicyclohexyl, bicycloheptyl, phenyl C₁₋₅ alkyl, naphthyl C₁₋₅ alkyl, halogen, hydroxy, nitrile, C₁₋₃ alkyloxy which may optionally be partially or fully halogenated, phenyloxy, naphthyloxy, heteraryloxy wherein the heterocyclic moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di- (C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino- S(O)₂, R₄ -C₁₋₅ alkyl, R₅ -C₁₋₅ alkoxy, R₆ -C(O)-C₁₋₅ alkyl and R₇ -C₁₋₅ alkyl(R₈)N, carboxy-mono- or di-(C₁₋₅)-alkyl-amino;
b) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopentano- pyrimidine, cyclohexanopyrimidine, cyclopentanopyrazine, cyclohexano- pyrazine, cyclopentanopyridazine, cyclohexanopyridazine, cyclopentano- quinoline, cyclohexanoquinoline, cyclopentanoisoquinoline, cyclohexano- isoquinoline, cyclopentanoindole, cyclohexanoindole, cyclopentano- benzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from the group consisting of phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halogen, nitrile, C₁₋₃ alkoxy which is optionally partially or fully halogenated, phenyloxy, naphthyloxy, heterocyclyloxy wherein the heterocyclyl moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₄ alkyl- OC(O), C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉ -C₁₋₅ alkyl, R₁₀ -C₁₋₅ alkoxy, R₁₁ -C(O)-C₁₋₅ alkyl, and R₁₂ -C₁₋₅ alkyl(R₁₃)N;
c) cycloalkyl selected from the group consisting of cyclopentyl, cyclohexyl, cycloheptyl, bicyclopentyl, bicyclohexyl and bicycloheptyl, wherein the cycloalkyl is optionally partially or fully halogenated and optionally substituted with one to three C₁₋₃ alkyl groups;
d) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) acetyl, aroyl, alkoxycarbonylalkyl or phenylsulfonyl; or
f) C₁₋₆ branched or unbranched alkyl optionally partially or fully halogenated;
or R₁ and R₂ taken together may optionally form a fused phenyl or pyridinyl ring;
each R₈ and R₁₃ is independently selected from the group consisting of:
hydrogen and C₁₋₄ branched or unbranched alkyl optionally be partially or fully halogenated;
each R₄, R₅, R₆, R₇, R₉, R₁₀, R₁₁ and R₁₂ is independently selected from the group consisting of morpholine, piperidine, piperazine, imidazole and tetrazole;
m is 0, 1 or 2;
W is O or S and pharmaceutically acceptable derivatives thereof.

In another preferred embodiment the invention relates to pharmaceutical compositions comprising **1** and **2d**, **characterized in that** the p38 kinase inhibitor **2d** is selected from the compounds of formula **(V)** as disclosed in WO 00/55139
wherein:
- Ar₂: is naphthyl, tetrahydronaphthyl, indanyl or indenyl and W is O.

In another preferred embodiment the invention relates to pharmaceutical compositions comprising **1** and **2d**, **characterized in that** the p38 kinase inhibitor **2d** is selected from the compounds of formula **(V)** as disclosed in WO 00/55139
wherein:
- Ar₁: is selected from thiophene and pyrazole;
- X: is C₅₋₇ cycloalkyl or C₅₋₇cycloalkenyl optionally substituted with 0-2 oxo groups or 0-3 C₁₋₄ branched or unbranched alkyl, C₁₋₄ alkoxy or C₁₋₄ alkylamino; or X is phenyl, pyridine, tetrahydropyridine, pyrimidine, furan or thiophene each being optionally independently substituted with 0-3 C₁₋₄ branched or unbranched alkyl, C₁₋₄alkoxy, hydroxy, nitrile, mono- or di-(C₁₋₃ alkyl)amino, C₁₋₆ alkyl-S(O)ₘ or halogen;
- R₁: is C₁₋₄alkyl branched or unbranched, cyclopropyl or cyclohexyl optionally partially or fully halogenated and optionally substituted with one to three C₁₋₃ alkyl groups;
- R₃: is C₁₋₄alkyl branched or unbranched, phenyl, pyrimidinyl, pyrazolyl or pyridinyl each being optionally substituted as described hereinabove in the broadest generic aspect, alkoxycarbonylalkyl or cyclopropyl or cyclopentyl optionally substituted as described hereinabove in the broadest generic aspect.

In yet another preferred embodiment the invention relates to pharmaceutical compositions comprising **1** and **2d, characterized in that** the p38 kinase inhibitor **2d** is selected from the compounds of formula **(V)** as disclosed in WO 00/55139
wherein:
- Ar₁: is pyrazole;
- X: is cyclopentenyl, cyclohexenyl or cycloheptenyl, optionally substituted with an oxo group or 0-3 C₁₋₄ branched or unbranched alkyl, C₁₋₄alkoxy or C₁₋₄alkylamino; or X is phenyl, pyridine, furan or thiophene each being optionally independently substituted with 0-3 C₁₋₄ branched or unbranched alkyl, C₁₋₄alkoxy, hydroxy, nitrile, mono- or di-(C₁₋₃ alkyl)amino, C₁₋₆ alkyl- S(O)ₘ or halogen.

In yet still another preferred embodiment the invention relates to pharmaceutical compositions comprising **1** and **2d**, **characterized in that** the p38 kinase inhibitor **2d** is selected from the compounds of formula **(V)** as disclosed in WO 00/55139
wherein:
- Y: is -CH₂-, -CH₂CH₂-, -CH₂NH-, -CH₂CH₂NH- or a bond; and
- Z: is phenyl, imidazole, furan, piperazine, tetrahydropyran, morpholine, thiomorpholine, thiomorpholine sulfoxide, piperidine, pyridine, secondary or tertiary amine wherein the amino nitrogen is covalently bonded to groups selected from the group consisting of C₁₋₃ alkyl and C₁₋₅ alkoxyalkyl, phenylamino wherein the phenyl ring is optionally substituted with one to two halogen, C₁₋₆ alkoxy, hydroxy or mono- or di-(C₁₋₃ alkyl)amino, C₁₋₆ alkyl-S(O)ₘ and phenyl-S(O)ₘ wherein the phenyl ring is optionally substituted with one to two halogen, C₁₋₆ alkoxy, hydroxy or mono- or di-(C₁₋₃ alkyl)amino.

In a further embodiment the invention relates to pharmaceutical compositions comprising **1** and **2d**, **characterized in that** the p38 kinase inhibitor **2d** is selected from the compounds of formula **(V)** as disclosed in WO 00/55139 wherein:
- Ar₁: is 5-*tert*-butyl-pyrazol-3-yl; wherein the pyrazole ring may be substituted by R₃;
- R₃: is C₁₋₄alkyl branched or unbranched, phenyl, pyrimidinyl, pyrazolyl, pyridinyl each being optionally substituted as described hereinabove in the broadest generic aspect, alkoxycarbonylalkyl or cyclopropyl or cyclopentyl optionally substituted as described hereinabove in the broadest generic aspect.

In another preferred embodiment the invention relates to pharmaceutical compositions comprising **1** and **2d**, **characterized in that** the p38 kinase inhibitor **2d** is selected from the compounds of formula **(V)** as disclosed in WO 00/55139
wherein X is pyridinyl.

In another preferred embodiment the invention relates to pharmaceutical compositions comprising **1** and **2d**, **characterized in that** the p38 kinase inhibitor **2d** is selected from the compounds of formula **(V)** as disclosed in WO 00/55139
wherein the pyridinyl is attached to Ar₁ via the 3-pyridinyl position.

In another preferred embodiment the invention relates to pharmaceutical compositions comprising **1** and **2d**, **characterized in that** the p38 kinase inhibitor **2d** is selected from the compounds of formula **(V)** as disclosed in WO 00/55139 that are mentioned below:
1-[5-*tert*-butyl-2-*p*-tolyl-2H-pyrazol-3-yl]-3-[4-(4-(morpholin-4-yl)phenyl)naphthalen-1-yl]urea;
1-[5-*tert*-butyl-2-*p*-tolyl-2H-pyrazol-3-yl]-3-[4-(4-(morpholin-4-yl-methyl)phenyl)naphthalen-1-yl]urea;
1-[5-*tert*-butyl-2-*p*-tolyl-2H-pyrazol-3-yl]-3-[4-(4-(2-(morpholin-4-yl)ethyl)phenyl)naphthalen-1-yl]urea;
1-[5-*tert*-butyl-2-*p*-tolyl-2H-pyrazol-3-yl]-3-[4-(4-dimethylaminophenyl)naphthalen-1-yl]urea;
1-[5-*tert*-butyl-2-*p*-tolyl-2H-pyrazol-3-yl]-3-[4-(3-(morpholin-4-yl)phenyl)naphthalen-1-yl]urea;
1-[5-*tert*-butyl-2-*p*-tolyl-2H-pyrazol-3-yl]-3-[4-(3-(morpholin-4-yl-methyl)phenyl)naphthalen-1-yl]urea;
1-[5-*tert*-butyl-2-*p*-tolyl-2H-pyrazol-3-yl]-3-[4-(6-morpholin-4-ylmethyl-pyridin-3-yl)naphthalen-1-yl]urea;
1-[5-*tert*-butyl-2-*p*-tolyl-2H-pyrazol-3-yl]-3-[4-(5-morpholin-\4-ylmethyl-pyridin-2-yl)naphthalen-1-yl]urea;
1-[5-*tert*-butyl-2-*p*-tolyl-2H-pyrazol-3-yl]-3-[4-(5-morpholin-4-ylmethyl-fur-2-yl)naphthalen-1-yl]urea;
1-[5-*tert*-butyl-2-(6-methyl-pyridin-3-yl)-2H-pyrazol-3-yl]-3-[4-(6-morpholin-4-ylmethyl-pyridin-3-yl)naphthalen-1-yl]urea;
1-[5-*tert*-butyl-2-methyl-2H-pyrazol-3-yl]-3-[4-(6-morpholin-4-ylmethyl-pyridin-3-yl)naphthalen-1-yl]urea;
1-[5-*tert*-butyl-2-phenyl-2H-pyrazol-3-yl]-3-[4-(4-piperdin-1-ylmethyl-phenyl)naphthalen-1-yl]urea;
1-[5-*tert*-butyl-2-phenyl-2H-pyrazol-3-yl]-3-[4-(4-(4-methylpiperazin-1-yl)methylphenyl)naphthalen-1-yl]urea;
1-[5-*tert*-butyl-2-*p*-tolyl-2H-pyrazol-3-yl]-3-[4-(3,4-di(morpholin-4-yl-methyl)phenyl)-naphthalen-1-yl]urea;
1-[5-*tert*-butyl-2-(6-methyl-pyridin-3-yl)-2H-pyrazol-3-yl]-3-[4-(6-pyridin-4-ylmethyl-pyridin-3-yl)naphthalen-1-yl]urea;
1-[5-*tert*-butyl-2-(6-methyl-pyridin-3-yl)-2H-pyrazol-3-yl]-3-[4-(6-(1-oxo-thiomorpholin-4-ylmethyl)pyridin-3-yl)naphthalen-1-yl]urea;
1-[5-*tert*-butyl-2-*p*-tolyl-2H-pyrazol-3-yl]-3-[4-(6-(1-oxo-thiomorpholin-4-ylmethyl)pyridin-3-yl)naphthalen-1-yl]urea;
1-[5-*tert*-butyl-2-(6-methyl-pyridin-3-yl)-2H-pyrazol-3-yl]-3-[4-(6-tetrahydropyran-4-ylmethyl-pyridin-3-yl)naphthalen-1-yl]urea;
1-[5-*tert*-butyl-2-(6-methyl-pyridin-3-yl)-2H-pyrazol-3-yl]-3-[4-(6-(1-oxo-tetrahydrothiophen-3-ylmethyl)pyridin-3-yl)naphthalen-1-yl]urea;
1-[5-*tert*-butyl-2-(6-methyl-pyridin-3-yl)-2H-pyrazol-3-yl]-3-[4-(6-(imidazol-1-ylmethyl)pyridin-3-yl)naphthalen-1-yl]urea;
1-[2-(3-dimethylaminomethylphenyl)-5-(1-methyl-cyclohexyl)-2H-pyrazol-3-yl]-3-[4-(6-morpholin-4-ylmethyl-pyridin-3-yl)naphthalen-1-yl]urea;
1-[2-(5-(1-methyl-cyclohexyl)-2-(6-methyl-pyridin-3-yl)-2H-pyrazol-3-yl]-3-[4-(6-morpholin-4-ylmethyl-pyridin-3-yl)naphthalen-1-yl]urea;
1-[5-*tert*-butyl-2-*p*-tolyl-2H-pyrazol-3-yl]-3-[4-(2-morpholin-4-ylmethyl-pyrimidin-5-yl)naphthalen-1-yl]urea;
1-[5-*tert*-butyl-2-(6-methyl-pyridin-3-yl)-2H-pyrazol-3-yl]-3-[4-(3-methoxy-5-(2-morpholin-4-yl-ethoxy)phenyl)naphthalen-1-yl]urea;
1-[5-*tert*-butyl-2-(6-methyl-pyridin-3-yl)-2H-pyrazol-3-yl]-3-[4-(3-(2-morpholin-4-yl-ethoxy)phenyl)naphthalen-1-yl]urea;
1-[5-*tert*-butyl-2-(6-methyl-pyridin-3-yl)-2H-pyrazol-3-yl]-3-[4-3-(dimethylamino)phenyl)naphthalen-1-yl]urea;
1-[5-*tert*-butyl-2-(6-methyl-pyridin-3-yl)-2H-pyrazol-3-yl]-3-[4-3-(methylsulfonyl)phenyl)naphthalen-1-yl]urea;
5-*tert*-butyl-3-{3-[4-(6-morpholin-4-ylmethyl-pyridin-3-yl)naphthalen-1-yl]ureido}thiophene-2-carboxylic acid methyl ester;
5-*tert*-butyl-3-{3-[4-(6-morpholin-4-ylmethyl-pyridin-3-yl)naphthalen-1-yl]ureido}-thiophene-2-carboxylic acid methylamide;
5-*tert*-butyl-1-methyl-3-{3-[4-(6-morpholin-4-ylmethyl-pyridin-3-yl)naphthalen-1-yl]ureido}-1H-pyrrole-2-carboxylic acid methyl ester;
5-*tert*-butyl-1-methyl-3-{3-[4-(6-morpholin-4-ylmethyl-pyridin-3-yl)naphthalen-1-yl]ureido}-1H-pyrrole-2-carboxylic acid methylamide;
2-acetylamino N-(5-*tert*-butyl-3-{3-[4-(6-morpholin-4-ylmethyl-pyridin-3-yl)naphthalen-1-yl]ureido}thiophen-2-ylmethyl)acetamide;
1-[5-*tert*-butyl-2-*p*-tolyl-2H-pyrazol-3-yl]-3-[4-(3-morpholin-4-yl-cyclohex-1-enyl)naphthalen-1-yl]urea;
1-[5-*tert*-butyl-2-*p*-tolyl-2H-pyrazol-3-yl]-3-[4-(3-morpholin-4-yl-cylohept-1-enyl)naphthalen-1-yl]urea;
1-[5-*tert*-butyl-2-*p*-tolyl-2H-pyrazol-3-yl]-3-[4-(3-(2-morpholin-4-yl-ethylamino)cyclo-hex-1-enyl)naphthalen-1-yl]urea;
1-[5-*tert*-butyl-2-*p*-tolyl-2H-pyrazol-3-yl]-3-[4-(3-morpholin-4-yl-cyclohept-1-enyl)naphthalen-1-yl]urea;
1-[5-*tert*-butyl-2-(6-methyl-pyridin-3-yl)-2H-pyrazol-3-yl]-3-[4-(3-(pyridin-4-yl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(dimethylaminoethylamino)cyclohex-1-enyl)naphthalen-1-yl]urea;
1-[5-*tert*-butyl-2-(6-methyl-pyridin-3-yl)-2H-pyrazol-3-yl]-3-[4-(3-(pyridin-3-yl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(phenyl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-phenylethylamino)-cyclohex-1-enyl)naphthalen-1-yl]urea;
1-[5-*tert*-butyl-2-(6-methyl-pyridin-3-yl)-2H-pyrazol-3-yl]-3-[4-(3-(furan-2-yl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-pyridin-2-yl-ethylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-piperdin-1-yl-ethylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-imidazol-4-yl-ethylamino)cyclohex-1-enyl)naphthalen-1-yl]urea;
1-[5-*tert*-butyl-2-(6-methyl-pyridin-3-yl)-2H-pyrazol-3-yl]-3-[4-(3-(pyridin-2-yl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-(4-methoxyphenyl)-ethylamino)cyclohex-1-enyl)naphthalen-1-yl]urea;
1-[5-*tert*-butyl-2-*p*-tolyl-2H-pyrazol-3-yl]-3-[4-(4-morpholin-4-ylmethyl-3-oxo-cyclohex-1-enyl)naphthalen-1-yl]urea;
1-[5-*tert*-butyl-2-*p*-tolyl-2H-pyrazol-3-yl]-3-[4-(4-(1-oxo-tetrahydrothiophen-3-ylmethyl)-3-oxo-cyclohex-1-enyl)naphthalen-1-yl]urea;
1-[5-*tert*-butyl-2-*p*-tolyl-2H-pyrazol-3-yl]-3-[4-(4-(1-oxo-thiomorpholin-4-ylmethyl)-3-oxo-cyclohex-1-enyl)naphthalen-1-yl]urea;
1-[5-*tert*-butyl-2-*p*-tolyl-2H-pyrazol-3-yl]-3-[4-(4-methylpiperazin-1-ylmethyl)-3-oxo-cyclohex-1-enyl)naphthalen-1-yl]urea;
1-[5-*tert*-butyl-2-(6-methyl-pyridin-3-yl)-2H-pyrazol-3-yl]-3-[4-{6-oxo-1-(tetrahydropyran-4-ylmethyl)-1,2,3,6-tetrahydro-pyridin-4-yl}naphthalen-1-yl]urea;
1-[5-*tert*-butyl-2-(6-methyl-pyridin-3-yl)-2H-pyrazol-3-yl]-3-[4-(2-oxo-1-pyridin-4-ylmethyl-piperdin-4-yl)naphthalen-1-yl]urea;
1-[5-*tert*-butyl-2-*p*-tolyl-2H-pyrazol-3-yl]-3-[4-(6-oxo-1-pyridin-4-yl-1,2,3,6-tetrahydro-pyridin-4-yl)naphthalen-1-yl]urea;
1-[5-*tert*-butyl-2-(6-methyl-pyridin-3-yl)-2H-pyrazol-3-yl]-3-[4-(6-oxo-1-pyridin-4-yl-1,2,3,6-tetrahydro-pyridin-4-yl)naphthalen-1-yl]urea;
5-*tert*-butyl-3-{3-[4-(6-oxo-1-pyridin-4-yl-1,2,3,6-tetrahydro-pyridin-4-yl)naphthalen-1-yl]ureidolthiophene-2-carboxylic acid methyl ester;
5-*tert*-butyl-1-methyl-3-{3-[4-(6-oxo-1-pyridin-4-yl-1,2,3,6-tetrahydro-pyridin-4-yl)naphthalen-1-yl]ureido}pyrrole-2-carboxylic acid methyl ester;
5-*tert*-butyl-1-methyl-3-{3-[4-(6-oxo-1-pyridin-4-yl-1,2,3,6-tetrahydro-pyridin-4-yl)naphthalen-1-yl]ureido}pyrrole-2-carboxylic acid methyl amide;
5-*tert*-butyl-3-{3-[4-(3-morpholin-4-yl-cyclohex-1-enyl)naphthalen-1-yl]ureido}thiophene-2-carboxylic acid methyl ester;
5-*tert*-butyl-1-methyl-3-{3-[4-(3-morpholin-4-yl-cyclohex-1-enyl)naphthalen-1-yl]ureido}pyrrole-2-carboxylic acid methyl ester; and
5-*tert*-butyl-1-methyl-3-{3-[4-(3-morpholin-4-yl-cyclohex-1-enyl)naphthalen-1-yl]ureido}pyrrole-2-carboxylic acid methyl amide and
the pharmaceutically acceptable derivatives thereof.

Preferably the invention relates to pharmaceutical compositions comprising **1** and **2d, characterized in that** the p38 kinase inhibitor **2d** is selected from the following compounds of formula **(V):**
1-[5-*tert*-butyl-2-*p*-tolyl-2H-pyrazol-3-yl]-3-[4-(4-(morpholin-4-yl-methyl)phenyl)naphthalen-1-yl]urea;
1-[5-*tert*-butyl-2-*p*-tolyl-2H-pyrazol-3-yl]-3-[4-(4-(2-(morpholin-4-yl)ethyl)phenyl)naphthalen-1-yl]urea;
1-[5-*tert*-butyl-2-*p*-tolyl-2H-pyrazol-3-yl]-3-[4-(3-(morpholin-4-yl-methyl)phenyl)naphthalen-1-yl]urea;
1-[5-*tert*-butyl-2-*p*-tolyl-2H-pyrazol-3-yl]-3-[4-(6-morpholin-4-ylmethyl-pyridin-3-yl)-naphthalen-1-yl]urea;
1-[5-*tert*-butyl-2-*p*-tolyl-2H-pyrazol-3-yl]-3-[4-(5-morpholin-4-ylmethyl-pyridin-2-yl)-naphthalen-1-yl]urea;
1-[5-*tert*-butyl-2-*p*-tolyl-2H-pyrazol-3-yl]-3-[4-(5-morpholin-4-ylmethyl-fur-2-yl)naphthalen-1-yl]urea;
1-[5-*tert*-butyl-2-(6-methyl-pyridin-3-yl)-2H-pyrazol-3-yl]-3-[4-(6-morpholin-4-ylmethyl-pyridin-3-yl)naphthalen-1-yl]urea;
1-[5-*tert*-butyl-2-methyl-2H-pyrazol-3-yl]-3-[4-(6-morpholin-4-ylmethyl-pyridin-3-yl)naphthalen-1-yl]urea and
the pharmaceutically acceptable derivatives thereof.

In another embodiment the invention relates to pharmaceutical compositions comprising **1** and **2d, characterized in that** the p38 kinase inhibitor **2d** is selected from the compounds of formula **(Va)** as disclosed in WO 00/55139 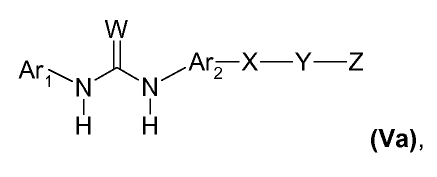 wherein:
- Ar₁: is: pyrrole, pyrrolidine, pyrazole, imidazole, oxazole, thiazole, furan and thiophene;
wherein Ar₁ is optionally substituted by one or more R₁, R₂ or R₃;
- Ar₂: is: phenyl, naphthyl, quinoline, isoquinoline, tetrahydronaphthyl, tetrahydroquinoline, tetrahydroisoquinoline, benzimidazole, benzofuran, indanyl, indenyl and indole each being optionally substituted with zero to three R₂ groups;
- X: is: a C₅₋₈ cycloalkyl or cycloalkenyl optionally substituted with one to two oxo groups or one to three C₁₋₄ alkyl, C₁₋₄ alkoxy or C₁₋₄ alkylamino chains each being branched or unbranched;
phenyl, furanyl, thienyl, pyrrolyl, pyrazolyl, imidazolyl, pyridinyl, tetrahydropyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl; each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y: is: a bond or a C₁₋₄ saturated or unsaturated branched or unbranched carbon chain optionally partially or fully halogenated, wherein one or more C atoms are optionally replaced by O, N, or S(O)ₘ and wherein Y is optionally independently substituted with one to two oxo groups, nitrile, phenyl, hydroxy or one or more C₁₋₄ alkyl optionally substituted by one or more halogen atoms;
- Z: is: aryl, indanyl, heteroaryl selected from benzimidazolyl, pyridinyl, pyrimidinyl, pyridazinyl, pyrazinyl, imidazolyl, pyrazolyl, triazolyl, tetrazolyl, furanyl, thienyl and pyranyl, heterocycle selected from piperazinyl, tetrahydropyrimidonyl, cyclohexanonyl, cyclohexanolyl, 2-oxa- or 2-thia-5-aza-bicyclo[2.2.1]heptanyl, pentamethylene sulfidyl, pentamethylene sulfoxidyl, pentamethylene sulfonyl, tetramethylene sulfidyl, tetramethylene sulfoxidyl or tetramethylene sulfonyl, tetrahydropyranyl, tetrahydrofuranyl, 1,3-dioxolanonyl, 1,3-dioxanonyl, 1,4- dioxanyl, morpholino, thiomorpholino, thiomorpholino sulfoxidyl, thiomorpholino sulfonyl, piperidinyl, piperidinonyl, pyrrolidinyl and dioxolanyl, each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, heteroaroyl, heterocycleC₁₋₃acyl wherein the heteroaryl and heterocycle are as defined hereinabove in this paragraph, C₁₋₃acyl, oxo, hydroxy, pyridinyl- C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino-S(O)ₘ, C₁₋₆ alkyl-S(O)ₘ or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino, aminocarbonyl or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group is optionally substituted with one to two halogen, C₁₋₆ alkyl, C₁₋₆ alkoxy, hydroxy or mono- or di-(C₁₋₃ alkyl)amino;
or Z is optionally substituted with one to three aryl, heterocycle or heteroaryl as hereinabove described in this paragraph each in turn is optionally substituted by halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by C₁₋₆alkyl, aminoC₁₋₆alkyl, arylC₀₋₃alkyl, C₁₋₅alkoxyC₁₋₃alkyl, C₁₋₅alkoxy, aroyl, C₁₋₃acyl, C₁₋₃alkyl-S(O)ₘ-, arylC₀₋₃alkyl-S(O)ₘ- , nitrileC₁₋₄alkyl or C₁₋₃alkoxyC₁₋₃alkyl, each of the aforementioned alkyl and aryl attached to the amino group is optionally substituted with one to two halogen, C₁₋₆ alkyl, C₁₋₆ alkoxy, hydroxy or mono- or di-(C₁₋₃ alkyl)amino, C₁₋₆ alkoxyheteroarylC₀₋₃alkyl, heteroarylC₀₋₃alkyl or heterocycyleC₀₋₃alkyl wherein the heteroaryl and heterocycle is hereinabove described in this paragraph,
or Z is C₁₋₆alkyl branched or unbranched, C₁₋₆alkoxy, C₁₋₃acylamino, nitrileC₁₋₄alkyl, C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃alkyl)amino;
- R₁: is :
a) C₁₋₁₀ branched or unbranched alkyl optionally partially or fully halogenated, and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle, selected from the group hereinabove described, being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, nitrile, C₁₋₃ alkyloxy which is optionally partially or fully halogenated, NH₂C(O) and di(C₁₋₃)alkylaminocarbonyl;
b) C₃₋₇ cycloalkyl selected from the group consisting of cyclopropyl, cyclobutyl, cyclopentyl, cyclohexyl, cycloheptyl, bicyclopentyl, bicyclohexyl and bicycloheptyl, each optionally partially or fully halogenated and optionally substituted with one to three C₁₋₃ alkyl groups, or an analog of such cycloalkyl group wherein one to three ring methylene groups are replaced by groups independently selected from the group consisting of O, S, CHOH, >C=O, >C=S and NH;
c) C₃₋₁₀ branched alkenyl optionally partially or fully halogenated and optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from the group consisting of halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, hydroxy, nitrile, C₁₋₃ alkoxy which is optionally partially or fully halogenated, NH₂C(O) and mono- or di(C₁₋₃)alkylaminocarbonyl;
d) a C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃alkyl groups;
e) nitrile; or
f) C₁₋₆ branched or unbranched alkoxycarbonyl, C₁₋₆ branched or unbranched alkylaminocarbonyl, C₁₋₆ branched or unbranched alkylcarbonylamino-C₁₋₃-alkyl;
- R₂: is: a C₁₋₆ branched or unbranched alkyl optionally partially or fully halogenated and optionally substituted with nitrile,
or R₂ is acetyl, aroyl, C₁₋₄ branched or unbranched alkoxy optionally partially or fully halogenated, halogen, methoxycarbonyl or phenylsulfonyl;
- R₃: is:
a) phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl, wherein such phenyl, naphthyl or heterocyclic group is optionally substituted with one to five groups selected from the group consisting of a phenyl, naphthyl, heterocycle selected from the group hereinabove described in this paragraph, C₁₋₆ branched or unbranched alkyl which is optionally partially or fully halogenated, cyclopropyl, cyclobutyl, cyclopentyl, cyclohexyl, cycloheptyl, bicyclopentyl, bicyclohexyl, bicycloheptyl, phenyl C₁₋₅ alkyl, naphthyl C₁₋₅ alkyl, halogen, hydroxy, oxo, nitrile, C₁₋₃ alkoxy optionally partially or fully halogenated, C₁₋₃ alkoxyC₁₋₅alkyl, C₁₋₃thioalkyl, C₁₋₃thioalkylC₁₋₅alkyl, phenyloxy, naphthyloxy, heteraryloxy wherein the heterocyclic moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino-S(O)₂, R₄ -C₁₋₅ alkyl, R₅ -C₁₋₅ alkoxy, R₆ -C(O)-C₁₋₅ alkyl and R₇ -C₁₋₅ alkyl(R₈)N, carboxy-mono- or di-(C₁₋₅ )-alkyl-amino;
b) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopentano- pyrimidine, cyclohexanopyrimidine, cyclopentanopyrazine, cyclohexano- pyrazine, cyclopentanopyridazine, cyclohexanopyridazine, cyclopentano- quinoline, cyclohexanoquinoline, cyclopentanoisoquinoline, cyclohexano- isoquinoline, cyclopentanoindole, cyclohexanoindole, cyclopentanobenz- imidazole, cyclohexanobenzimidazole, cyclopentanobenzoxazole, cyclo- hexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from the group consisting of phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halogen, nitrile, C₁₋₃ alkoxy which is optionally partially or fully halogenated, phenyloxy, naphthyloxy, heterocyclyloxy wherein the heterocyclyl moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di- (C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉ -C₁₋₅ alkyl, R₁₀ -C₁₋₅ alkoxy, R₁₁ -C(O)-C₁₋₅ alkyl and R₁₂ -C₁₋₅ alkyl(R₁₃)N;
c) cycloalkyl selected from the group consisting of cyclopropyl, cyclobutyl, cyclopentyl, cyclohexyl, cycloheptyl, bicyclopentyl, bicyclohexyl and bicycloheptyl, wherein the cycloalkyl is optionally partially or fully halogenated and optionally substituted with one to three C₁₋₃alkyl groups;
d) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃alkyl groups;
e) acetyl, aroyl, C₁₋₆alkoxycarbonylC₁₋₆alkyl or phenylsulfonyl; or
f) C₁₋₆ branched or unbranched alkyl optionally partially or fully halogenated;
or R₁ and R₂ taken together optionally form a fused phenyl or pyridinyl ring;
each R₈ and R₁₃ is independently selected from the group consisting of:
hydrogen and C₁₋₄ branched or unbranched alkyl optionally partially or fully halogenated;
each R₄, R₅, R₆, R₇, R₉, R_{10,} R₁₁ and R₁₂ is independently selected from the group consisting of morpholine, piperidine, piperazine, imidazole and tetrazole;
m is 0, 1 or 2;
W is O or S;
wherein X is directly attached to one or two -Y-Z, and
pharmaceutically acceptable derivatives thereof.

In another embodiment the invention relates to pharmaceutical compositions comprising **1** and **2d, characterized in that** the p38 kinase inhibitor **2d** is selected from the compounds of formula **(Va)** wherein:
- Ar₂: is naphthyl, tetrahydronaphthyl, indanyl or indenyl and
- W: is O.

In another embodiment the invention relates to pharmaceutical compositions comprising **1** and **2d, characterized in that** the p38 kinase inhibitor **2d** is selected from the compounds of formula **(Va)** wherein:
- Ar₁: is thiophene or pyrazole each substituted independently by one to three R₁, R₂ or R₃;
- X: is: a C₅₋₇ cycloalkyl or cycloalkenyl optionally substituted with one to two oxo groups or one to three C₁₋₄ alkyl, C₁₋₄ alkoxy or C₁₋₄ alkylamino chains each being branched or unbranched;
phenyl, indanyl, furanyl, thienyl, imidazolyl, pyridinyl, pyrazinyl, tetrahydrapyridinyl, pyrimidinyl, pyridinonyl, piperdinyl, benzimidazole or piperazinyl; each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y: is: a bond or a C₁₋₄ saturated or unsaturated branched or unbranched carbon chain optionally partially or fully halogenated, wherein one or more C atoms are optionally replaced by O or N, and wherein Y is optionally independently substituted with one to two oxo groups, nitrile, phenyl, hydroxy or one or more C₁₋₄ alkyl optionally substituted by one or more halogen atoms;
- Z: is: phenyl, heteroaryl selected from pyridinyl, imidazolyl, furanyl and thienyl, heterocycle selected from piperazinyl, 2-oxa-5-aza-bicyclo[2.2.1]heptanyl, pentamethylene sulfidyl, pentamethylene sulfoxidyl, pentamethylene sulfonyl, tetrahydrofuranyl, morpholino, thiomorpholino and piperidinyl, each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, morpholinocarbonyl, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino- S(O)ₘ, C₁₋₆ alkyl-S(O)ₘ or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino, aminocarbonyl or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group are optionally substituted with one to two halogen, C₁₋₆ alkyl or C₁₋₆ alkoxy;
or Z is optionally substituted with one to three aryl, heterocycle or heteroaryl as hereinabove described in this paragraph each in turn is optionally substituted by halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by aroyl, C₁₋₃acyl, C₁₋₆alkyl, C₁₋₅ alkoxyC₁₋₃ alkyl, pyridinylC₁₋₃alkyl, tetrahydrafuranylC₁₋₃alkyl, nitrileC_{1- 4}alkyl or phenyl wherein the phenyl ring is optionally substituted with one to two halogen, C₁₋₆ alkoxy, hydroxy or mono- or di-(C₁₋₃ alkyl)amino,
or Z is C₁₋₆alkyl branched or unbranched, C₁₋₆alkoxy or nitrileC₁₋₄alkyl;
- R₁: is: C₁₋₄ branched or unbranched alkyl optionally partially or fully halogenated;
cyclopropyl, cyclobutyl, cyclopentyl, cyclohexyl and cycloheptyl optionally partially or fully halogenated and optionally substituted with one to three C₁₋₃ alkyl groups, or an analog of such cycloalkyl group wherein one to three ring methylene groups are replaced by groups independently selected from the group consisting of O, S and NH;
C₃₋₁₀ branched alkenyl optionally partially or fully halogenated and optionally substituted with one to three C₁₋₅ branched or unbranched alkyl; cyclopentenyl and cyclohexenyl optionally substituted with one to three C₁₋₃ alkyl groups;
- R₂: is: a C₁₋₆ branched or unbranched alkyl optionally partially or fully halogenated and optionally substituted with nitrile;
- R₃: is: phenyl or heterocyclic group selected from the group consisting of pyridinyl, pyrimidinyl, pyrazinyl, pyridazinyl and pyrazolyl, wherein such phenyl or heterocyclic group is optionally substituted with one to five groups selected from the group consisting of a phenyl, heterocycle selected from the group hereinabove described in this paragraph, C₁₋₆ branched or unbranched alkyl which is optionally partially or fully halogenated, cyclopropyl, cyclobutyl, cyclopentyl, cyclohexyl, cycloheptyl, bicyclopentyl, bicyclohexyl, bicycloheptyl, phenyl C₁₋₅ alkyl, naphthyl C₁₋₅ alkyl, halogen, hydroxy, oxo, nitrile, C₁₋₃ alkoxy optionally be partially or fully halogenated, C₁₋₃ alkoxyC₁₋₅alkyl, C₁₋₃thioalkyl, C₁₋₃thioalkylC₁₋₅alkyl, phenyloxy, naphthyloxy, heteraryloxy wherein the heterocyclic moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino-S(O)₂,
R₄ -C₁₋₅ alkyl, R₅ -C₁₋₅ alkoxy, R₆ -C(O)-C₁₋₅ alkyl and R₇ -C₁₋₅ alkyl(R₈)N, carboxy-mono- or di-(C₁₋₅ )-alkyl-amino;
a fused aryl selected from the group consisting of benzocyclobutanyl, indanyl, indenyl; wherein the fused aryl is substituted with 0 to 3 groups independently selected from the group consisting of phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halogen, nitrile, C₁₋₃ alkoxy which is optionally partially or fully halogenated, phenyloxy, naphthyloxy, heterocyclyloxy wherein the heterocyclyl moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉-C₁₋₅alkyl, R₁₀-C₁₋₅alkoxy, R₁₁-C(O)-C₁₋₅alkyl and R₁₂-C₁₋₅alkyl(R₁₃)N;
cycloalkyl selected from the group consisting of cyclopropyl, cyclobutyl, cyclopentyl, cyclohexyl, cycloheptyl, wherein the cycloalkyl is optionally partially or fully halogenated and optionally substituted with one to three C₁₋₃ alkyl groups;
C₁₋6alkoxycarbonylC₁₋₆alkyl;
or R₁ and R₂ taken together optionally form a fused phenyl or pyridinyl ring;
each R₈ and R₁₃ is independently selected from the group consisting of:
hydrogen and C₁₋₄ branched or unbranched alkyl optionally partially or fully halogenated;
   and
each R₄, R₅, R₆, R₇, R₉, R₁₀, R₁₁ and R₁₂ is independently selected from the group consisting of morpholine, piperidine, piperazine, imidazole and tetrazole;
wherein X is directly attached to one -Y-Z.

In another embodiment the invention relates to pharmaceutical compositions comprising **1** and **2d, characterized in that** the p38 kinase inhibitor **2d** is selected from the compounds of formula **(Va)** wherein:
- Ar₁: is pyrazole;
- X: is: cyclopentenyl, cyclohexenyl, cycloheptenyl, optionally substituted with an oxo group or one to three C₁₋₄ alkyl, C₁₋₄ alkoxy or C₁₋₄ alkylamino chains each being branched or unbranched;
phenyl, furanyl, thienyl, pyridinyl, pyrazinyl piperidinyl or pyrimidinyl each being optionally independently substituted with one to three C₁₋₂ alkyl, C₁₋₂alkoxy, hydroxy or halogen;
- Z: is: phenyl, heteroaryl selected from pyridinyl, imidazolyl and furanyl, heterocycle selected from 2-oxa-5-aza-bicyclo[2.2.1]heptanyl, pentamethylene sulfidyl, pentamethylene sulfoxidyl, pentamethylene sulfonyl, tetrahydrofuranyl, tetrahydropyranyl, piperazinyl, morpholino, thiomorpholino, thiomorpholino sulfoxide and piperidinyl,
each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, morpholinocarbonyl, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino- S(O)ₘ, C₁₋₆ alkyl-S(O)ₘ, or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino, aminocarbonyl or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ-, pyridinylC₀₋₃alkyl, tetrahydrafuranylC₀₋₃alkyl, or arylC₀₋₃alkyl-S(O)ₘ- each of the aforementioned alkyl and aryl attached to the amino group is optionally substituted with one to two halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by C₁₋₆alkyl, pyridinylC₀₋₃alkyl,
or Z tetrahydrafuranylC₀₋₃alkyl, C₁₋₅ alkoxyC₁₋₃ alkyl, C₁₋₃acyl, nitrileC₁₋₄alkyl or phenyl wherein the phenyl ring is optionally substituted with one to two halogen, C₁₋₆ alkoxy, hydroxy or mono- or di-(C₁₋₃ alkyl)amino, is C₁₋₆alkyl branched or unbranched, C₁₋₆alkoxy or nitrileC₁₋₄alkyl;
- R₁: is: C₁₋₄ branched or unbranched alkyl optionally partially or fully halogenated;
cyclopropyl, cyclobutyl, cyclopentanyl, cyclohexanyl and cycloheptanyl optionally partially or fully halogenated and optionally substituted with one to three C₁₋₃ alkyl groups, or an analog of such cycloalkyl group wherein one to three ring methylene groups are replaced by groups independently selected from the group consisting of O, S and NH;
C₃₋₁₀ branched alkenyl optionally partially or fully halogenated and optionally substituted with one to three C₁₋₃ branched or unbranched alkyl;
cyclopentenyl and cyclohexenyl optionally substituted with one to three C₁₋₃ alkyl groups;
- R₂: is: a C₁₋₆ branched or unbranched alkyl optionally partially or fully halogenated and optionally substituted with nitrile;
- R₃: is: phenyl or heterocyclic group selected from the group consisting of pyridinyl, pyrimidinyl, pyridazinyl and pyrazolyl, wherein such phenyl or heterocyclic group is optionally substituted with one to five groups selected from the group consisting of a phenyl, heterocycle selected from the group hereinabove described in this paragraph, C₁₋₆ branched or unbranched alkyl which is optionally partially or fully halogenated, phenyl C₁₋₅ alkyl, halogen, hydroxy, oxo, nitrile, C₁₋₃ alkoxy optionally partially or fully halogenated, C₁₋₃thioalkyl, C₁₋₃thioalkylC₁₋₅alkyl, amino, mono- or di-(C₁₋₃)alkylamino, NH₂C(O) or a mono- or di-(C₁₋₃)alkyl aminocarbonyl,
C₁₋₆alkoxycarbonylC₁₋₆alkyl;
or R₃ is cyclopropyl or cyclopentyl each optionally partially or fully halogenated and optionally substituted with one to three C₁₋₃alkyl groups
or R₁ and R₂ taken together optionally form a fused phenyl or pyridinyl ring.

In another embodiment the invention relates to pharmaceutical compositions comprising **1** and **2d, characterized in that** the p38 kinase inhibitor **2d** is selected from the compounds of formula **(Va)** wherein:
- Y: is -CH₂-, -O-(CH₂)₀₋₃-, -CH₂CH₂-, -CH₂NH-, -CH₂CH₂-NH-, NH-CH₂CH₂-, -CH₂-NH-CH₂-, -NH-, -NH-C(O)-, -C(O)-, -CH(OH)-, -CH₂(CH₂CH₃)- or a bond;
- X: is: cyclohexenyl optionally substituted with an oxo group or one to three C₁₋₄ alkyl, C₁₋₄ alkoxy or C₁₋₄ alkylamino chains each being branched or unbranched;
phenyl, pyridinyl, pyrazinyl, piperidinyl or pyrimidinyl each being optionally independently substituted with one to three C₁₋₂ alkyl, C₁₋₂alkoxy, hydroxy or halogen;
- Z: is: phenyl, heteroaryl selected from pyridinyl, imidazolyl and furanyl, heterocycle selected from 2-oxa-5-aza-bicyclo[2.2.1]heptanyl, pentamethylene sulfidyl, pentamethylene sulfoxidyl, pentamethylene sulfonyl, tetrahydrofuranyl, tetrahydropyranyl, piperazinyl, morpholino, thiomorpholino, thiomorpholino sulfoxide and piperidinyl,
each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, morpholinocarbonyl, C₁₋₃acyl, oxo, hydroxy, pyridinyl-C₁₋₃ alkyl,
or imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino- S(O)ₘ, C₁₋₆ alkyl-S(O)ₘ, or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino or aminocarbonyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C_{1- 3}alkyl-S(O)ₘ- or arylC₀₋₃alkyl-S(O)ₘ- each of the aforementioned alkyl and aryl attached to the amino group is optionally substituted with one to two halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by C₁₋₃alkyl, pyridinylC₁₋₂alkyl, tetrahydrafuranylC₁₋₂alkyl, C₁₋₃ alkoxyC₁₋₃ alkyl, C₁₋₃acyl, nitrileC₁₋₄alkyl, phenyl wherein the phenyl ring is optionally substituted with one to two halogen, C₁₋₆ alkoxy, hydroxy or mono- or di-(C₁₋₃ alkyl)amino, Z is C₁₋₆alkyl branched or unbranched, C₁₋₆alkoxy or nitrileC₁₋₄alkyl;
- R₁: is: C₁₋₄ branched or unbranched alkyl optionally partially or fully halogenated;
- R₂: is: a C₁₋₃ branched or unbranched alkyl optionally partially or fully halogenated and optionally substituted with nitrile;
- R₃: is: phenyl or heterocyclic group selected from the group consisting of pyridinyl, pyrimidinyl, and pyrazolyl, wherein such phenyl or heterocyclic group is optionally substituted with one to five groups selected from the group consisting of C₁₋₃ branched or unbranched alkyl which is optionally partially or fully halogenated, C₁₋₃ alkoxy which optionally partially or fully halogenated, C₁₋₃thioalkyl, C₁₋₃thioalkylC₁₋₅alkyl, amino or NH₂C(O);
C₁₋₃alkoxycarbonyl;
or R₃ is cyclopropyl or cyclopentyl each optionally partially or fully halogenated and optionally substituted with one to three C₁₋₃ alkyl groups.

In a further embodiment the invention relates to pharmaceutical compositions comprising **1** and **2d, characterized in that** the p38 kinase inhibitor **2d** is selected from the compounds of formula **(Va)** wherein:
- Ar₁: is 5-tert-butyl-pyrazol-3-yl; wherein the pyrazole ring is substituted independently by one to two R₂ or R₃;
- X: is: cyclohexenyl;
phenyl, pyridinyl, pyrazinyl, piperidinyl or pyrimidinyl each being optionally independently substituted with C₁₋₂alkoxy or hydroxy;
- Z: is: phenyl, heteroaryl selected from pyridinyl and furanyl, heterocycle selected from 2-oxa-5-aza-bicyclo[2.2.1 ]heptanyl, pentamethylene sulfidyl, pentamethylene sulfoxidyl, tetrahydrofuranyl, piperazinyl, morpholino, thiomorpholino and piperidinyl,
each of the aforementioned Z are optionally substituted with one to three C₁₋₃ alkyl, C₁₋₃ alkoxy, oxo , hydroxy or NH₂C(O)-;
or Z is hydroxyC₁₋₃alkyl, amino wherein the N atom is optionally independently mono- or di-substituted by pyridinylmethyl, tetrahydrafuranylmethyl, C₁₋₃ alkoxyC₁₋₃alkyl, C₁₋₃acyl or nitrileC₁₋₄alkyl,
or Z is nitrileC₁₋₄alkyl;
- R₃: is: phenyl or heterocyclic group selected from the group consisting of pyridinyl, pyrimidinyl, and pyrazolyl, wherein such phenyl or heterocyclic group is optionally substituted with one to two groups selected from the group consisting of C₁₋₂ alkyl which is optionally partially or fully halogenated, C₁₋₂ alkoxy which optionally partially or fully halogenated, C₁₋₂thioalkyl, C_{1- 2}thioalkylC₁₋₃alkyl, amino or NH₂C(O);
C₁₋₃alkoxycarbonyl;
or R₃ is cyclopropyl or cyclopentyl each optionally partially or fully halogenated and optionally substituted with one to three C₁₋₃alkyl groups.

In a still further embodiment the invention relates to pharmaceutical compositions comprising **1** and **2d, characterized in that** the p38 kinase inhibitor **2d** is selected from the compounds of formula **(Va)** wherein X is pyridinyl.

In a yet still further embodiment the invention relates to pharmaceutical compositions comprising **1** and **2d, characterized in that** the p38 kinase inhibitor **2d** is selected from the compounds of formula **(Va)** wherein the pyridinyl is attached to Ar₁ via the 3-pyridinyl position.

Preferably the invention relates to pharmaceutical compositions comprising **1** and 2d, **characterized in that** the p38 kinase inhibitor **2d** is selected from the following compounds of formula **(Va):**
1-[5-tert-butyl-2-p-tolyl-2H-pyrazol-3-yl]-3-[4-(4-morpholin-4-yl-methylphenyl)-naphthalen-1-yl]-urea;
1-[5-tert-butyl-2-p-tolyl-2H-pyrazol-3-yl]-3-[3-(4-morpholin-4-yl-methylphenyl)-naphthalen-1-yl]-urea;
1-[5-tert-butyl-2-p-tolyl-2H-pyrazol-3-yl]-3-[4-(5-morpholin-4-yl-methylfuran-2-yl)-naphthalen-1-yl]-urea;
1-[5-tert-butyl-2-p-tolyl-2H-pyrazol-3-yl]-3-[4-(3-(morpholin-4-yl-methyl)cyclohexenyl)-naphthalen-1-yl]-urea;
1-[5-tert-butyl-2-p-tolyl-2H-pyrazol-3-yl]-3-[4-(2-(4-morpholin-4-yl)ethylphenyl)-naphthalen-1-yl]-urea;
1-[5-tert-butyl-2-p-tolyl-2H-pyrazol-3-yl]-3-[4-(4-dimethylaminomethylphenyl)-naphthalen-1-yl]-urea;
1-[5-tert-butyl-2-p-tolyl-2H-pyrazol-3-yl]-3-[4-(5-(morpholin-4-yl-methyl)pyridin-2-yl)-naphthalen-1-yl]-urea;
1-[5-tert-butyl-2-p-tolyl-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(morpholin-4-yl-methyl)pyridin-3-yl)-naphthalen-1-yl]-urea;
1-[5-tert-butyl-2-methyl-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(3-(2-(morpholin-4-yl)ethylamino)cyclohexenyl)-naphthalen-1-yl]-urea;
1-[5-tert-butyl-2-p-tolyl-2H-pyrazol-3-yl]-3-[4-(3,4-(morpholin-4-yl-methyl)phenyl)-naphthalen-1-yl]-urea;
1-[5-tert-butyl-2-p-tolyl-2H-pyrazol-3-yl]-3-[4-(4-methylpiperzin-1-yl-methyl)phenyl)-naphthalen-1-yl]-urea;
1-[5-tert-butyl-2-p-tolyl-2H-pyrazol-3-yl]-3-[4-(piperdin-1-yl-methyl)phenyl)-naphthalen-1-yl]-urea;
1-[5-tert-butyl-2-(6-methyl-pyridin-3-yl)-2H-pyrazol-3-yl]-3-[4-(3-(2-(pyridin-2-yl)ethylamino)cyclohexenyl)-naphthalen-1-yl]-urea;
1-[5-tert-butyl-2-p-tolyl-2H-pyrazol-3-yl]-3-[4-(4-(2-(pyridin-4-yl)ethylaminomethyl)-phenyl)naphthalen-1-yl]-urea;
1-[5-tert-butyl-2-p-tolyl-2H-pyrazol-3-yl]-3-[4-(4-(pyridin-3-yl-methylaminomethyl)-phenyl)naphthalen-1-yl]-urea;
1-[5-tert-butyl-2-(6-methyl-pyridin-3-yl)-2H-pyrazol-3-yl]-3-[4-(4-(3,4-dimethoxyphenyl-methyl)-3-hydroxyphenyl)naphthalen-1-yl]-urea;
1-[5-tert-butyl-2-p-tolyl-2H-pyrazol-3-yl]-3-[4-(6-oxo-1,6-dihydro-pyridin-3-yl)naphthalen-1-yl]-urea;
1-[5-tert-butyl-2-(6-methyl-pyridin-3-yl)-2H-pyrazol-3-yl]-3-[4-(4-(morpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(morpholin-4-yl-methyl)imidazol-1-yl)naphthalen-1-yl]-urea;
1-[5-tert-butyl-2-p-tolyl-2H-pyrazol-3-yl]-3-[4-(4-(morpholin-4-yl-methyl)imidazol-1-yl)naphthalen-1-yl]-urea;
1-[5-tert-butyl-2-(6-methyl-pyridin-3-yl)-2H-pyrazol-3-yl]-3-[4-(4-(furan-3-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(6-(4-hydroxybutyl-amino)pyridin-3-yl)-naphthalen-1-yl]-urea;
1-[5-tert-butyl-2-(6-methyl-pyridin-3-yl)-2H-pyrazol-3-yl]-3-[4-(4-(pyridin-3-yl-methyl)-3-hydroxyphenyl)naphthalen-1-yl]-urea;
1-[5-tert-butyl-2-(4-methyl-3-carbamylphenyl)-2H-pyrazol-3-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(4-(imidazol-2-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(3-hydroxymorpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(N-2-methoxyethy-N-methylaminomethyl)phenyl)naphthalen-1-yl]-urea;
1-[5-tert-butyl-2-(6-methyl-pyridin-3-yl)-2H-pyrazol-3-yl]-3-[4-(4-(4-hydroxymorpholin-4-yl-methyl)phenyl)naphthalen-1-yl]-urea;
1-[5-tert-butyl-2-(6-methyl-pyridin-3-yl)-2H-pyrazol-3-yl]-3-[4-(3-(morpholin-4-yl-methyl)cyclohexenyl)-naphthalen-1-yl]-urea;
1-[5-tert-butyl-2-(6-methyl-pyridin-3-yl)-2H-pyrazol-3-yl]-3-[4-(4-(tetrahydrofuran-3-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(N,N-di-(2-methoxyethyl)aminomethyl)phenyl)naphthalen-1-yl]-urea;
1-[5-tert-butyl-2-(6-methyl-pyridin-3-yl)-2H-pyrazol-3-yl]-3-[4-(6-(3-cyanopropoxy)-pyridin-3-yl)naphthalen-1-yl]-urea;
1-[5-tert-butyl-2-(6-methyl-pyridin-3-yl)-2H-pyrazol-3-yl]-3-[4-(4-morpholin-4-yl-methyl-piperdinyl)naphthalen-1-yl]-urea;
1-[5-tert-butyl-2-(6-methyl-pyridin-3-yl)-2H-pyrazol-3-yl]-3-[4-(4-(N,N-di-(2-cyanoethyl)aminomethyl)phenyl)naphthalen-1-yl]-urea;
1-[5-tert-butyl-2-p-tolyl-2H-pyrazol-3-yl]-3-[4-(1-morpholin-4-yl-indan-5-yl)-naphthalen-1-yl]-urea;
1-[5-tert-butyl-2-(6-methyl-pyridin-3-yl)-2H-pyrazol-3-yl]-3-[4-(4-(furan-2-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(thiomorpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(3-carboxamido-morphol in-4-yl-methyl)phenyl)naphthalen-1-yl]-urea;
1-[5-tert-butyl-2-(6-methyl-pyridin-3-yl)-2H-pyrazol-3-yl]-3-[4-(4-(2-methyl-3-oxo-piperzin-1-yl-methyl)phenyl)naphthalen-1-yl]-urea;
1-[5-tert-butyl-2-(2-methylpyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(6-(4-hydroxybutyloxy)-pyridin-3-yl)-naphthalen-1-yl]-urea;
1-[3-tert-butyl-1'H-[1,4']bipyrazol-5-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(4-(furan-2-yl-methyl)-3-methoxyphenyl)naphthalen-1-yl]-urea;
1-[5-tert-butyl-2-(6-methyl-pyridin-3-yl)-2H-pyrazol-3-yl]-3-[4-(5-(morpholin-4-carbonyl)pyrazin-2-yl)naphthalen-1-yl]-urea;
1-[5-tert-butyl-2-(6-methyl-pyridin-3-yl)-2H-pyrazol-3-yl]-3-[4-(6-(tetrahydrothiopyran-4-yl-amino)pyridin-3-yl)-naphthalen-1-yl]-urea;
1-[5-tert-butyl-2-(2-cyanoethyl)-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(2,6-dimethylmorpholin-4-yl-methyl)pyridin-3-yl)-naphthalen-1-yl]-urea;
1-[5-tert-butyl-2-(2-methoxypyridin-5-yl)-2H-pyrazol-3-yl]-3-[4-(6-(morpholin-4-yl-methyl)pyridin-3-yl)-naphthalen-1-yl]-urea;
1-[5-tert-butyl-2-(2-aminoypyridin-5-yl)-2H-pyrazol-3-yl]-3-[4-(6-(morpholin-4-yl-methyl)pyridin-3-yl)-naphthalen-1-yl]-urea;
1-[5-tert-butyl-2-(6-oxo-1,6-dihydropyridin-3-yl)-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(morpholin-4-yl-4-carbonyl)pyridin-3-yl)-naphthalen-1-yl]-urea;
1-[5-tert-butyl-2-(6-methyl-pyridin-3-yl)-2H-pyrazol-3-yl]-3-[4-(6-(2-oxa-5-aza-bicyclo-[2.2.1]hept-5-yl-methyl)pyridin-3-yl)-naphthalen-1-yl]-urea;
1-[5-tert-butyl-2-p-tolyl-2H-pyrazol-3-yl]-3-[4-(4-(3-carbamylphenyl)naphthalen-1-yl]-urea;
1-[5-tert-butyl-2-(6-methyl-pyridin-3-yl)-2H-pyrazol-3-yl]-3-[4-(4-(N-(2-cyanoethyl)-N-(pyridin-3-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(4-(N-(2-cyanoethyl)-N-(pyridin-2-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(4-(N-(2-cyanoethyl)-N-(tetrahydrofuran-2-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(6-(morpholin-4-yl-methyl)-4-methoxypyridin-3-yl)-naphthalen-1-yl]-urea;
1-[5-tert-butyl-2-(6-methyl-pyridin-3-yl)-2H-pyrazol-3-yl]-3-[4-(6-(1-morpholin-4-yl-propyl)pyridin-3-yl)-naphthalen-1-yl]-urea;
1-[5-tert-butyl-2-(6-methyl-pyridin-3-yl)-2H-pyrazol-3-yl]-3-[4-(6-(N-(3-methoxypropyl)amino)pyridin-3-yl)-naphthalen-1-yl]-urea;
1-[5-tert-butyl-2-(6-methyl-pyridin-3-yl)-2H-pyrazol-3-yl]-3-[4-(6-(N-(3-methoxypropyl)-N-methylamino)pyridin-3-yl)-naphthalen-1-yl]-urea;
1-[3-tert-butyl-1'-methyl-1'H-[1,4']bipyrazol-5-yl]-3-[4-(6-(morpholin-4-yl-methyl)pyridin-3-yl)naphthalen-1-yl]-urea;
1-[5-tert-butyl-2-benzyl-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(4-(N-N-di-(2-cyanoethyl)aminomethyl)phenyl)-naphthalen-1-yl]-urea;
1-[5-tert-butyl-2-p-tolyl-2H-pyrazol-3-yl]-3-[4-(4-(4-carbamylphenyl)naphthalen-1-yl]-urea;
1-[5-tert-butyl-2-(6-methyl-pyridin-3-yl)-2H-pyrazol-3-yl]-3-[4-(6-(1-oxo-tetrahydrothiopyran-4yl-amino)pyridin-3-yl)-naphthalen-1-yl]-urea;
1-[5-tert-butyl-2-(6-methyl-pyridin-3-yl)-2H-pyrazol-3-yl]-3-[4-(6-(tetrahydropyran-4yl-amino)pyridin-3-yl)-naphthalen-1-yl]-urea;
1-[3-tert-butyl-1'-(3-cyanopropyl)-1'H-[1,4']bipyrazol-5-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(3-methanesulfinylphenyl)naphthalen-1-yl]-urea;
1-[5-tert-butyl-2-p-tolyl-2H-pyrazol-3-yl]-3-[4-(3-methanesulfonylphenyl)naphthalen-1-yl]-urea;
1-[5-tert-butyl-2-p-tolyl-2H-pyrazol-3-yl]-3-[4-(3-sulfonamidophenyl)naphthalen-1-yl]-urea;
1-[5-tert-butyl-2-p-tolyl-2H-pyrazol-3-yl]-3-[4-(3-(morpholin-4-yl)carbonylphenyl)-naphthalen-1-yl]-urea;
1-[5-tert-butyl-2-(6-methyl-pyridin-3-yl)-2H-pyrazol-3-yl]-3-[4-(5-(tetrahydrothiopyran-4yl-amino)pyrazin-2-yl)-naphthalen-1-yl]-urea;
1-[5-tert-butyl-2-(6-methyl-pyridin-3-yl)-2H-pyrazol-3-yl]-3-[4-(6-(methylcarbonyl-amino)pyridin-3-yl)-naphthalen-1-yl]-urea;
1-[5-tert-butyl-2-p-tolyl-2H-pyrazol-3-yl]-3-[4-(6-(morpholin-4-yl-4-carbonyl)phenyl)-naphthalen-1-yl]-urea;
1-[3-tert-butyl-1'-(3-methylsulfanylpropyl)-1'H-[1,4']bipyrazol-5-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(5-(morpholin-4-yl-carbonyl)pyridin-3-yl)-naphthalen-1-yl]-urea;
1-[5-tert-butyl-2-(6-methyl-pyridin-3-yl)-2H-pyrazol-3-yl]-3-[4-(5-(morpholin-4-yl-methyl)pyrazin-2-yl)-naphthalen-1-yl]-urea;
1-[5-tert-butyl-2-(6-methyl-pyridin-3-yl)-2H-pyrazol-3-yl]-3-[4-(6-aminopyridin-3-yl)naphthalen-1-yl]-urea;
1-[5-tert-butyl-2-(6-methyl-pyridin-3-yl)-2H-pyrazol-3-yl]-3-[4-(6-(1-methylpiperdin-4-yl-amino)pyridin-3-yl)naphthalen-1-yl]-urea;
1-[5-tert-butyl-2-(2-methylpyrimidin-5-yl)-2H-pyrazol-3-yl]-3-[4-(6-(2-methyl-3-oxo-piperzin-1-yl-methyl)pyrid in-3-yl)naphthalen-1-yl]-urea;
1-[5-tert-butyl-2-(2-methylpyrimidin-5-yl)-2H-pyrazol-3-yl]-3-[4-(6-(morpholin-4-yl-carbonyl)pyridin-3-yl)naphthalen-1-yl]-urea;
1-[5-tert-butyl-2-(2-methylpyrimidin-5-yl)-2H-pyrazol-3-yl]-3-[4-(6-(N,N-di-(2-methoxyethyl)aminomethyl)pyridin-3-yl)naphthalen-1-yl]-urea;
1-[5-tert-butyl-2-(2-methylpyrimidin-5-yl)-2H-pyrazol-3-yl]-3-[4-(6-(1-oxo-thiomorpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(6-(tetrahydropyran-4-yl-amino)pyridin-3-yl)naphthalen-1-yl]-urea;
1-[5-tert-butyl-2-(2-methylpyrimidin-5-yl)-2H-pyrazol-3-yl]-3-[4-(5-(morpholin-4-yl-methyl)pyrazin-2-yl)naphthalen-1-yl]-urea;
1-[5-tert-butyl-2-(2-methylthiopyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(6-(2-methyl-3-oxo-piperzin-1-yl-methyl)-pyridin-3-yl)naphthalen-1-yl]-urea;
1-[5-tert-butyl-2-p-tolyl-2H-pyrazol-3-yl]-3-[4-(6-(pyridin-3-yl-oxy)pyridin-3-yl)naphthalen-1-yl]-urea
1-[5-tert-butyl-2-p-tolyl-2H-pyrazol-3-yl]-3-[4-(6-(pyridin-3-yl-amino)pyridin-3-yl)naphthalen-1-yl]-urea;
1-[5-tert-butyl-2-(2-methoxypyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(5-carbamylpyridin-3-yl)naphthalen-1-yl]-urea;
1-[5-tert-butyl-2-(2-aminopyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(4-(morpholin-4-yl-methyl)phenyl)naphthalen-1-yl]-urea;
1-[3-tert-butyl-1'-methyl-1'H-[1,4']bipyrazol-5-yl]-3-[4-(6-(morpholin-4-yl-methyl)phenyl)naphthalen-1-yl]-urea;
1-[5-tert-butyl-2-(2-cyclopropylpyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(2-(pyridin-3-yl-amino)pyrimidin-5-yl)naphthalen-1-yl]-urea;
1-[5-tert-butyl-2-p-tolyl-2H-pyrazol-3-yl]-3-[4-(6-(1-oxo-tetrahydrothiopyran-4-yl-amino)pyridin-3-yl)naphthalen-1-yl]-urea;
1-[5-tert-butyl-2-p-tolyl-2H-pyrazol-3-yl]-3-[4-(6-(thiomorpholin-4-yl-methyl)pyridin-3-yl)naphthalen-1-yl]-urea;
1-[5-tert-butyl-2-p-tolyl-2H-pyrazol-3-yl]-3-[4-(3-benzyl-3H-imidazo[4,5-b]pyridin-6-yl)naphthalen-1-yl]-urea;
1-[5-tert-butyl-2-(6-methyl-pyridin-3-yl)-2H-pyrazol-3-yl]-3-[4-(6-(pyridin-3-yl-methyl)-pyridin-3-yl)naphthalen-1-yl]-urea;
1-[5-tert-butyl-2-p-tolyl-2H-pyrazol-3-yl]-3-[4-(2-(morpholin-4-yl-carbonyl)pyrimidin-5-yl)naphthalen-1-yl]-urea;
1-[5-tert-butyl-2-p-tolyl-2H-pyrazol-3-yl]-3-[4-(2-(morpholin-4-yl-methyl)pyrimidin-5-yl)naphthalen-1-yl]-urea;
1-[5-tert-butyl-2-p-tolyl-2H-pyrazol-3-yl]-3-[4-(3-amino-4-carbamylphenyl)naphthalen-1-yl]-urea;
1-[5-tert-butyl-2-p-tolyl-2H-pyrazol-3-yl]-3-[4-(6-(1-oxo-thiomorpholin-4-yl-methyl)-pyridin-3-yl)naphthalen-1-yl]-urea;
1-[5-tert-butyl-2-p-tolyl-2H-pyrazol-3-yl]-3-[4-(6-(pyridin-3-yl-methyl)pyridin-3-yl)naphthalen-1-yl]-urea;
1-[5-tert-butyl-2-p-tolyl-2H-pyrazol-3-yl]-3-[4-(6-(hydroxy-pyridin-3-yl-methyl)pyridin-3-yl)naphthalen-1-yl]-urea;
1-[5-tert-butyl-2-(2-methylpyrimidin-5-yl)-2H-pyrazol-3-yl]-3-[4-(2-(morpholin-4-yl-methyl)pyrimidin-5-yl)naphthalen-1-yl]-urea;
and the pharmaceutically acceptable derivatives thereof.

In another embodiment the invention relates to pharmaceutical compositions comprising **1** and **2d, characterized in that** the p38 kinase inhibitor **2d** is selected from the following compounds of formula **(Va):**
1-[5-tert-butyl-2-p-tolyl-2H-pyrazol-3-yl]-3-[4-(5-(morpholin-4-yl-methyl)pyridin-2-yl)-naphthalen-1-yl]-urea;
1-[5-tert-butyl-2-p-tolyl-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(3-(2-(pyridin-2-yl)ethyl-amino)cyclohexenyl)-naphthalen-1-yl]-urea;
1-[5-tert-butyl-2-p-tolyl-2H-pyrazol-3-yl]-3-[4-(4-(pyridin-3-yl-methylaminomethyl)-phenyl)naphthalen-1-yl]-urea;
1-[5-tert-butyl-2-(6-methyl-pyridin-3-yl)-2H-pyrazol-3-yl]-3-[4-(4-(morpholin-4-yl-methyl)phenyl)naphthalen-1-yl]-urea;
1-[5-tert-butyl-2-(6-methyl-pyridin-3-yl)-2H-pyrazol-3-yl]-3-[4-(6-(4-hydroxybutyl-amino)pyridin-3-yl)-naphthalen-1-yl]-urea;
1-[5-tert-butyl-2-(4-methyl-3-carbamylphenyl)-2H-pyrazol-3-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(4-(3-hydroxypiperidin-1-yl-methyl)phenyl)naphthalen-1-yl]-urea;
1-[5-tert-butyl-2-(6-methyl-pyridin-3-yl)-2H-pyrazol-3-yl]-3-[4-(4-(4-hydroxymorpholin-4-yl-methyl)phenyl)naphthalen-1-yl]-urea;
1-[5-tert-butyl-2-(6-methyl-pyridin-3-yl)-2H-pyrazol-3-yl]-3-[4-(3-(morpholin-4-yl-methyl)cyclohexenyl)-naphthalen-1-yl]-urea;
1-[5-tert-butyl-2-(6-methyl-pyridin-3-yl)-2H-pyrazol-3-yl]-3-[4-(4-(tetrahydrofuran-3-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(N,N-di-(2-methoxyethyl)aminomethyl)phenyl)naphthalen-1-yl]-urea;
1-[5-tert-butyl-2-(6-methyl-pyridin-3-yl)-2H-pyrazol-3-yl]-3-[4-(6-(3-cyanopropoxy)py-ridin-3-yl)naphthalen-1-yl]-urea;
1-[5-tert-butyl-2-(6-methyl-pyridin-3-yl)-2H-pyrazol-3-yl]-3-[4-(4-morpholin-4-yl-methyl-piperdinyl)naphthalen-1-yl]-urea;
1-[5-tert-butyl-2-(6-methyl-pyridin-3-yl)-2H-pyrazol-3-yl]-3-[4-(4-(N,N-di-(2-cyanoethyl)aminomethyl)phenyl)naphthalen-1-yl]-urea;
1-[5-tert-butyl-2-(6-methyl-pyridin-3-yl)-2H-pyrazol-3-yl]-3-[4-(4-(furan-2-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(thiomorpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(3-carboxamido-piperidin-1-yl-methyl)phenyl)naphthalen-1-yl]-urea;
1-[5-tert-butyl-2-(6-methyl-pyridin-3-yl)-2H-pyrazol-3-yl]-3-[4-(4-(2-methyl-3-oxo-piperzin-1-yl-methyl)phenyl)naphthalen-1-yl]-urea;
1-[5-tert-butyl-2-(2-methylpyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(6-(4-hydroxybutyl-oxy)pyridin-3-yl)-naphthalen-1-yl]-urea;
1-[3-tert-butyl-1'H-[1,4']bipyrazol-5-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(6-(tetrahydrothiopyran-4-yl-amino)pyridin-3-yl)-naphthalen-1-yl]-urea;
1-[5-tert-butyl-2-(2-cyanoethyl)-2H-pyrazol-3-yl]-3-[4-(6-(morpholin-4-yl-methyl)-pyridin-3-yl)-naphthalen-1-yl]-urea;
1-[5-tert-butyl-2-(6-methyl-pyridin-3-yl)-2H-pyrazol-3-yl]-3-[4-(6-(2,6-dimethylmorpholin-4-yl-methyl)pyridin-3-yl)-naphthalen-1-yl]-urea;
1-[5-tert-butyl-2-(2-methoxypyridin-5-yl)-2H-pyrazol-3-yl]-3-[4-(6-(morpholin-4-yl-methyl)pyridin-3-yl)-naphthalen-1-yl]-urea;
1-[5-tert-butyl-2-(2-aminoypyridin-5-yl)-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(morpholin-4-yl-4-carbonyl)pyridin-3-yl)-naphthalen-1-yl]-urea;
1-[5-tert-butyl-2-(6-methyl-pyridin-3-yl)-2H-pyrazol-3-yl]-3-[4-(6-(2-oxa-5-azabicyclo[2.2.1]hept-5-yl-methyl)pyridin-3-yl)-naphthalen-1-yl]-urea;
1-[5-tert-butyl-2-(6-methyl-pyridin-3-yl)-2H-pyrazol-3-yl]-3-[4-(4-(N-(2-cyanoethyl)-N-(pyridin-3-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(4-(N-(2-cyanoethyl)-N-(tetrahydrofuran-2-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(6-(morpholin-4-yl-methyl)-4-methoxypyridin-3-yl)-naphthalen-1-yl]-urea;
1-[5-tert-butyl-2-(6-methyl-pyridin-3-yl)-2H-pyrazol-3-yl]-3-[4-(6-(1-morpholin-4-yl-propyl)pyridin-3-yl)-naphthalen-1-yl]-urea;
1-[3-tert-butyl-1'-methyl-1'H-[1,4']bipyrazol-5-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(6-(1-oxo-tetrahydrothiopyran-4yl-amino)pyridin-3-yl)-naphthalen-1-yl]-urea;
1-[5-tert-butyl-2-(6-methyl-pyridin-3-yl)-2H-pyrazol-3-yl]-3-[4-(6-(tetrahydropyran-4yl-amino)pyridin-3-yl)-naphthalen-1-yl]-urea;
1-[5-tert-butyl-2-(6-methyl-pyridin-3-yl)-2H-pyrazol-3-yl]-3-[4-(5-(tetrahydrothiopyran-4yl-amino)pyrazin-2-yl)-naphthalen-1-yl]-urea;
1-[5-tert-butyl-2-(6-methyl-pyridin-3-yl)-2H-pyrazol-3-yl]-3-[4-(6-(methylcarbonylamino)pyridin-3-yl)-naphthalen-1-yl]-urea;
1-[3-tert-butyl-1'-(3-methylsulfanylpropyl)-1'H-[1,4']bipyrazol-5-yl]-3-[4-(6-(morpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(6-(1-oxo-thiomorpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(6-(tetrahydropyran-4-yl-amino)pyridin-3-yl)naphthalen-1-yl]-urea;
1-[5-tert-butyl-2-(2-methylthiopyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-(2-aminopyrimidin-5-yl)-2H-pyrazol-3-yl]-3-[4-(6-(morpholin-4-yl-methyl)pyridin-3-yl)naphthalen-1-yl]-urea;
1-[3-tert-butyl-1'-methyl-1'H-[1,4']bipyrazol-5-yl]-3-[4-(6-(morpholin-4-yl-methyl)phenyl)naphthalen-1-yl]-urea;
1-[5-tert-butyl-2-p-tolyl-2H-pyrazol-3-yl]-3-[4-(6-(1-oxo-tetrahydrothiopyran-4-yl-amino)pyridin-3-yl)naphthalen-1-yl]-urea;
1-[5-tert-butyl-2-p-tolyl-2H-pyrazol-3-yl]-3-[4-(6-(thiomorphol in-4-yl-methyl)pyrid in-3-yl)naphthalen-1-yl]-urea;
1-[5-tert-butyl-2-p-tolyl-2H-pyrazol-3-yl]-3-[4-(2-(morpholin-4-yl-carbonyl)pyrimidin-5-yl)naphthalen-1-yl]-urea;
1-[5-tert-butyl-2-p-tolyl-2H-pyrazol-3-yl]-3-[4-(2-(morpholin-4-yl-methyl)pyrimidin-5-yl)naphthalen-1-yl]-urea;
1-[5-tert-butyl-2-p-tolyl-2H-pyrazol-3-yl]-3-[4-(6-(1-oxo-thiomorpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(2-(morpholin-4-yl-methyl)pyrimidin-5-yl)naphthalen-1-yl]-urea and
the pharmaceutically acceptable derivatives thereof.

In another preferred embodiment the invention relates to pharmaceutical compositions comprising **1** and **2d, characterized in that** the p38 kinase inhibitor **2d** is selected from the compounds of formula **(VI)** as disclosed in WO 00/55139 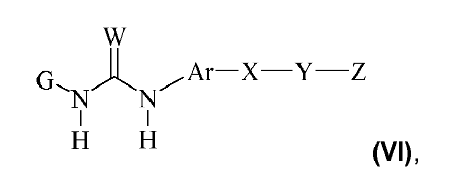 wherein:
- G: is : an aromatic C₆₋₁₀ carbocycle or a nonaromatic C₃₋₁₀ carbocycle saturated or unsaturated;
a 6-10 membered heteroaryl containing 1 or more heteroatoms chosen from O, N and S;
a 5-8 membered monocyclic heterocycle containing one or more heteroatoms chosen from O, N and S;
or
an 8-11 membered bicyclic heterocycle, containing one or more heteroatoms chosen from O, N and S;
wherein G is substituted by one or more R₁, R₂ or R₃;
- Ar: is: phenyl, naphthyl, quinolinyl, isoquinolinyl, tetrahydronaphthyl, tetrahydroquinolinyl, tetrahydroisoquinolinyl, benzimidazolyl, benzofuranyl, dihydrobenzofuranyl, indolinyl, benzothienyl, dihydrobenzothienyl, indanyl, indenyl or indolyl each being optionally substituted by one or more R₄ or R₅;
- X: is: a C₅₋₈ cycloalkyl or cycloalkenyl optionally substituted with one to two oxo groups or one to three C₁₋₄ alkyl, C₁₋₄ alkoxy or C₁₋₄ alkylamino chains;
phenyl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl;
- Y: is: a bond or a C₁₋₄ saturated or unsaturated branched or unbranched carbon chain optionally partially or fully halogenated, wherein one or more methylene groups are optionally replaced by O, N, or S(O)ₘ and wherein Y is optionally independently substituted with one to two oxo groups, phenyl or one or more C₁₋₄alkyl optionally substituted by one or more halogen atoms;
- Z: is: phenyl, pyridinyl, pyrimidinyl, pyridazinyl, pyrazinyl, imidazolyl, pyrazolyl, triazolyl, tetrazolyl, furanyl, thienyl, pyranyl each being optionally substituted with one to three halogen, C₁₋₆ alkyl, C₁₋₆ alkoxy, hydroxy, amino, mono- or di- (C₁₋₃ alkyl)amino, C₁₋₆ alkyl-S(O)ₘ, CN, CONH₂, COOH or phenylamino wherein the phenyl ring is optionally substituted with one to two halogen, C₁₋₆ alkyl or C₁₋₆ alkoxy;
tetrahydropyranyl, tetrahydrofuranyl, 1,3-dioxolanonyl, 1,3-dioxanonyl, 1,4- dioxanyl, morpholinyl, thiomorpholinyl, thiomorpholino sulfoxidyl, thiomorpholino sulfonyl, piperidinyl, piperidinonyl, piperazinyl, tetrahydropyrimidonyl, cyclohexanonyl, cyclohexanolyl, pentamethylene sulfidyl, pentamethylene sulfoxidyl, pentamethylene sulfonyl, tetramethylene sulfide, tetramethylene sulfoxidyl or tetramethylene sulfonyl each being optionally substituted with one to three nitrile, C₁₋₆ alkyl, C₁₋₆ alkoxy, hydroxy, amino, mono- or di-(C₁₋₃ alkyl)amino-C₁₋₃ alkyl, CONH₂, phenylamino-C₁₋₃ alkyl or C₁₋₃ alkoxy-C₁₋₃ alkyl;
halogen, C₁₋₄ alkyl, nitrile, amino, hydroxy, C₁₋₆ alkoxy, NH₂C(O), mono- or di(C₁₋₃alkyl) aminocarbonyl, mono- or di(C₁₋₆alkyl)amino, secondary or tertiary amine wherein the amino nitrogen is covalently bonded to C₁₋₃ alkyl or C₁₋₅ alkoxyalkyl, pyridinyl-C₁₋₃ alkyl, imidazolyl-C₁₋₃ alkyl, tetrahydrofuranyl-C₁₋₃ alkyl, nitrile-C₁₋₃ alkyl, carboxamide-C₁₋₃ alkyl, phenyl, wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, or phenyl-S(O)ₘ, wherein the phenyl ring is optionally substituted with one to two halogen, C₁₋₆ alkoxy, hydroxy, halogen or mono- or di-(C₁₋₃ alkyl)amino;
C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
- each R₁: is independently: C₁₋₁₀ alkyl optionally be partially or fully halogenated, and optionally substituted with one to three C₃₋₁₀ cycloalkanyl, hydroxy, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl; each of the aforementioned being optionally substituted with one to five groups selected from halogen, C₁₋₆ alkyl which is optionally partially or fully halogenated, C₃₋₈ cycloalkanyl, C₅₋₈ cycloalkenyl, hydroxy, nitrile, C₁₋₃ alkoxy which is optionally partially or fully halogenated or NH₂C(O), mono- or di(C₁₋₃alkyl)amino, and mono- or di(C₁₋₃alkyl)aminocarbonyl;
cyclopropyloxy, cyclobutyloxy, cyclopentyloxy, cyclohexyloxy, or cycloheptyloxy each being optionally partially or fully halogenated and optionally substituted with one to three C₁₋₃ alkyl groups optionally partially or fully halogenated, CN, hydroxyC₁₋₃alkyl or aryl; or an analog of such cycloalkyl group wherein one to three ring methylene groups are independently replaced by O, S(O)ₘ, CHOH, >C=O, >C=S or NH;
phenyloxy or benzyloxy each being optionally partially or fully halogenated and optionally substituted with one to three C₁₋₃ alkyl groups optionally partially or fully halogenated, CN, hydroxyC₁₋₃alkyl or aryl; or an analog of such cycloaryl group wherein one to two ring methyne groups are independently replaced by N;
cyclopropanyl, cyclobutanyl, cyclopentanyl, cyclohexanyl, cycloheptanyl, bicyclopentanyl, bicyclohexanyl or bicycloheptanyl, each being optionally partially or fully halogenated and optionally substituted with one to three C₁₋₃ alkyl groups optionally partially or fully halogenated, CN, hydroxyC₁₋₃alkyl or aryl; or an analog of such cycloalkyl group wherein one to three ring methylene groups are independently replaced by O, S(O)ₘ, CHOH, >C=O, >C=S or NH;
C₃₋₁₀ branched or unbranced alkenyl each being optionally partially or fully halogenated, and optionally be substituted with one to three C₁₋₅ branched or unbranched alkyl, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl, each of the aforementioned being substituted with zero to five halogen, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl and bicycloheptanyl, hydroxy, nitrile, C₁₋₃ alkyloxy which is optionally partially or fully halogenated, NH₂C(O), mono- or di(C₁₋₃alkyl)aminocarbonyl; the C₃₋₁₀ branched or unbranced alkenyl being optionally interrupted by one or more heteroatoms chosen from O, N and S(O)ₘ;
cyclopentenyl, cyclohexenyl, cyclohexadienyl, cycloheptenyl, cycloheptadienyl, bicyclohexenyl or bicycloheptenyl, wherein such cycloalkenyl group is optionally substituted with one to three C₁₋₃ alkyl groups;
nitrile, halogen;
methoxycarbonyl, ethoxycarbonyl and propoxycarbonyl;
silyl containing three C₁₋₄ alkyl groups optionally partially or fully halogenated;
C₃₋₆ alkynyl branched or unbranched carbon chain optionally partially or fully halogenated, wherein one or more methylene groups are optionally replaced by O, NH or S(O)ₘ and wherein said alkynyl group is optionally independently substituted with one to two oxo groups, pyrrolidinyl, pyrrolyl, one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
- each R₂, R₄, and R₅: is a C₁₋₆ branched or unbranched alkyl optionally partially or fully halogenated, acetyl, aroyl, C₁₋₄ branched or unbranched alkoxy, each being optionally partially or fully halogenated, halogen, nitrile, methoxycarbonyl, C₁₋₃ alkyl- S(O)ₘ optionally partially or fully halogenated, or phenylsulfonyl;
C₁₋₆ alkoxy, hydroxy, amino, or mono- or di-(C₁₋₄ alkyl)amino, nitrile, halogen;
OR₆;
nitro; or
mono- or di-(C₁₋₄ alkyl)amino-S(O)₂ optionally partially or fully halogenated, or H₂NSO₂;
- each R₃: is independently:
phenyl, naphthyl, morpholinyl, pyridinyl, pyrimidinyl, pyrazinyl, pyridazinyl, pyrrolyl, pyrrolidinyl, imidazolyl, pyrazolyl, thiazolyl, oxazoyl, triazolyl, tetra- zolyl, thienyl, furyl, tetrahydrofuryl, isoxazolyl, isothiazolyl, quinolinyl, iso- quinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthyl- pyridinyl, quinoxalinyl, quinazolinyl, purinyl or indazolyl, each of the aforemen- tioned is optionally substituted with one to three phenyl, naphthyl, heterocycle or heteroaryl as hereinabove described in this paragraph, C₁₋₆ branched or unbranched alkyl which is optionally partially or fully halogenated, cyclopro- panyl, cyclobutanyl, cyclopentanyl, cyclohexanyl, cycloheptanyl, bicyclopen- tanyl, bicyclohexanyl, bicycloheptanyl, phenyl C₁₋₅ alkyl, naphthyl C₁₋₅ alkyl, halogen, hydroxy, oxo, nitrile, C₁₋₃ alkyloxy optionally partially or fully halo- genated, phenyloxy, naphthyloxy, heteroaryloxy or heterocyclicoxy wherein the heterocyclic or heteroaryl moiety is as hereinabove described in this para- graph, nitro, amino, mono- or di-(C₁₋₃alkyl)amino, phenylamino, naphthyl- amino, heteroaryl or heterocyclic amino wherein the heteroaryl heterocyclic moiety is as hereinabove described in this paragraph, NH₂C(O), a mono- or di- (C₁₋₃alkyl) aminocarbonyl, C₁₋₅ alkyl-C(O)-C₁₋₄ alkyl, amino-C₁₋₅ alkyl, mono- or di-(C₁₋₃alkyl)amino-C₁₋₅ alkyl, amino-S(O)₂, di-(C₁₋₃alkyl)amino-S(O)₂, R₇-C₁₋₅ alkyl, R₈-C₁₋₅ alkoxy, R₉-C(O)-C₁₋₅ alkyl, R₁₀-C₁₋₅ alkyl(R₁₁)N, carboxy-mono- or di-(C₁₋₅alkyl)-amino;
a fused aryl selected from benzocyclobutanyl, indanyl, indenyl, dihydronaph- thyl, tetrahydronaphthyl, benzocycloheptanyl and benzocycloheptenyl, or a fused heteroaryl selected from cyclopentenopyridinyl, cyclohexanopyridinyl, cyclopentanopyrimidinyl, cyclohexanopyrimidinyl, cyclopentanopyrazinyl, cy- clohexanopyrazinyl, cyclopentanopyridazinyl, cyclohexanopyridazinyl, cyclo- pentanoquinolinyl, cyclohexanoquinolinyl, cyclopentanoisoquinolinyl, cyclo- hexanoisoquinolinyl, cyclopentanoindolyl, cyclohexanoindolyl, cyclopentano- benzimidazolyl, cyclohexanobenzimidazolyl, cyclopentanobenzoxazolyl, cyclohexanobenzoxazolyl, cyclopentanoimidazolyl, cyclohexanoimidazolyl, cyclopentanothienyl and cyclohexanothienyl; wherein the fused aryl or fused heteroaryl ring is independently substituted with zero to three phenyl, naphthyl, pyridinyl, pyrimidinyl, pyrazinyl, pyridazinyl, pyrrolyl, imidazolyl, pyrazolyl, thienyl, furyl, isoxazolyl, isothiazolyl, C₁₋₆ alkyl which is optionally partially or fully halogenated, halogen, nitrile, C₁₋₃ alkyloxy which is optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), mono- or di-(C₁₋₃alkyl)aminocarbonyl, C₁₋₄ alkyl-OC(O), C₁₋₅ alkyl-C(O)-C₁₋₄ alkyl, amino-C₁₋₅ alkyl, mono- or di-(C₁₋₃)alkylamino-C₁₋₅ alkyl, R₁₂-C₁₋₅ alkyl, R₁₃-C₁₋₅alkoxy, R₁₄-C(O)-C₁₋₅ alkyl or R₁₅-C₁₋₅ alkyl(R₁₆)N;
cyclopropanyl, cyclobutanyl, cyclopentanyl, cyclohexanyl, cycloheptanyl, bicyclopentanyl, bicyclohexanyl or bicycloheptanyl, each being optionally partially or fully halogenated and optionally substituted with one to three C₁₋₃ alkyl groups, or an analog of such cycloalkyl group wherein one to three ring methylene groups are independently replaced by O, S, CHOH, >C=O, >C=S or NH;
cyclopentenyl, cyclohexenyl, cyclohexadienyl, cycloheptenyl, cycloheptadienyl, bicyclohexenyl or bicycloheptenyl, each optionally substituted with one to three C₁₋₃ alkyl groups;
C₁₋₄ alkyl-phenyl-C(O)-C₁₋₄ alkyl-, C₁₋₄ alkyl-C(O)-C₁₋₄ alkyl- or C₁₋₄ alkyl- phenyl-S(O)ₘ-C₁₋₄ alkyl-;
C₁₋₆ alkyl or C₁₋₆ branched or unbranched alkoxy each of which is optionally partially or fully halogenated or optionally substituted with R₁₇;
OR₁₈ or C₁₋₆ alkyl optionally substituted with OR₁₈;
amino or mono- or di-(C₁₋₅alkyl)amino optionally substituted with R₁₉;
R₂₀C(O)N(R₂₁)-, R₂₂O- or R₂₃R₂₄NC(O)-; R₂₆(CH₂)ₘC(O)N(R₂₁)- or R₂₆C(O)(CH₂)ₘN(R₂₁)-;
C₂₋₆alkenyl substituted by R₂₃R₂₄NC(O)-;
C₂₋₆ alkynyl branched or unbranched carbon chain, optionally partially or fully halogenated, wherein one or more methylene groups are optionally replaced by O, NH, S(O)ₘ and wherein said alkynyl group is optionally independently substituted with one to two oxo groups, pyrroldinyl, pyrrolyl, morpholinyl, piperidinyl, piperazinyl, imidazolyl, phenyl, pyridinyl, tetrazolyl one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₄ alkyl)amino optionally substituted by one or more halogen atoms; or
aroyl;
- R₆: is a: C₁₋₄ alkyl optionally partially or fully halogenated and optionally substituted with R₂₆;
each R₇, R₈, R₉, R₁₀, R₁₂, R₁₃, R₁₄, R₁₅, R₁₇, R₁₉, R₂₅ and R₂₆ is independently: nitrile, phenyl, morpholino, piperidinyl, piperazinyl, imidazolyl, pyridinyl, tetrazolyl, amino or mono- or di-(C₁₋₄alkyl)amino optionally partially or fully halogenated;
- each R₁₁ and R₁₆: is independently:
hydrogen or C₁₋₄ alkyl optionally partially or fully halogenated;
- R₁₈: is independently:
hydrogen or a C₁₋₄ alkyl optionally independently substituted with oxo or R₂₅;
- R₂₀: is independently:
C₁₋₁₀ alkyl optionally partially or fully halogenated, phenyl, or pyridinyl;
- R₂₁: is independently:
hydrogen or C₁₋₃ alkyl optionally partially or fully halogenated;
- each R₂₂, R₂₃ and R₂₄: is independently:
hydrogen, C₁₋₆ alkyl optionally partially or fully halogenated, said C₁₋₆ alkyl is optionally interrupted by one or more O, N or S, said C₁₋₆ alkyl also being independently optionally substituted by mono- or di-(C₁₋₃alkyl)aminocarbonyl, phenyl, pyridinyl, amino or mono- or di-(C₁₋₄alkyl)amino each of which is optionally partially or fully halogenated and optionally substituted with mono- or di-(C₁₋₃alkyl)amino;
- or R₂₃ and R₂₄: taken together optionally form a heterocyclic or heteroaryl ring;
m = 0, 1 or 2;
W is O or S and
pharmaceutically acceptable derivatives thereof.

In another preferred embodiment the invention relates to pharmaceutical compositions comprising **1** and **2d**, **characterized in that** the p38 kinase inhibitor **2d** is selected from the compounds of formula **(VI)** wherein
- G: is: phenyl, naphthyl, benzocyclobutanyl, dihydronaphthyl, tetrahydronaphthyl, benzocycloheptanyl, benzocycloheptenyl, indanyl, indenyl;
pyridinyl, pyridonyl, quinolinyl, dihydroquinolinyl, tetrahydroquinoyl, isoquino- linyl, tetrahydroisoquinoyl, pyridazinyl, pyrimidinyl, pyrazinyl, benzimidazolyl, benzthiazolyl, benzoxazolyl, benzofuranyl, benzothiophenyl, benzpyrazolyl, dihydrobenzofuranyl, dihydrobenzothiophenyl, benzooxazolonyl, benzo[1,4]ox- azin-3-onyl, benzodioxolyl, benzo[1,3]dioxol-2-onyl, benzofuran-3-onyl, tetra- hydrobenzopyranyl, indolyl, indolinyl, indolonyl, indolinonyl, phthalimidyl, oxe- tanyl, pyrrolidinyl, tetrahydrofuranyl, tetrahydrothiophenyl, piperidinyl, pipera- zinyl, morpholinyl, tetrahydropyranyl, dioxanyl, tetramethylene sulfonyl, tetra- methylene sulfoxidyl, oxazolinyl, thiazolinyl, imidazolinyl, tertrahydropyridinyl, homopiperidinyl, pyrrolinyl, tetrahydropyrimidinyl, decahydroquinolinyl, deca- hydroisoquinolinyl, thiomorpholinyl, thiazolidinyl, dihydrooxazinyl, dihydropy- ranyl, oxocanyl, heptacanyl, thioxanyl or dithianyl;
wherein G is substituted by one or more R₁, R₂ or R₃.

In a further preferred embodiment the invention relates to pharmaceutical compositions comprising **1** and **2d**, **characterized in that** the p38 kinase inhibitor **2d** is selected from the compounds of formula **(VI)** wherein
- G is: phenyl, pyridinyl, pyridonyl, naphthyl, quinolinyl, isoquinolinyl, pyrazinyl, benzimidazolyl, benzoxazolyl, benzofuranyl, benzothiophenyl, benzpyrazolyl, dihydrobenzofuranyl, dihydrobenzothiophenyl, indanyl, indenyl, indolyl, indolinyl, indolonyl or indolinonyl, wherein G is substituted by one or more R₁, R₂ or R₃;
- Ar is:: naphthyl, quinolinyl, isoquinolinyl, tetrahydronaphthyl, tetrahydroquinolinyl, tetrahydroisoquinolinyl, indanyl, indenyl or indolyl each being optionally substituted by one or more R₄ or R₅ groups;
- X is:: phenyl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, piperazinyl, pyridazinyl or pyrazinyl
- Y is:: a bond or
a C₁₋₄ saturated or unsaturated carbon chain wherein one of the carbon atoms is optionally replaced by O, N, or S(O)ₘ and wherein Y is optionally independently substituted with one to two oxo groups, phenyl or one or more C₁₋₄ alkyl optionally substituted by one or more halogen atoms;
- Z is:: phenyl, pyridinyl, pyrimidinyl, pyridazinyl, pyrazinyl, imidazolyl, furanyl, thienyl, dihydrothiazolyl, dihydrothiazolyl sulfoxidyl, pyranyl, pyrrolidinyl which are optionally substituted with one to three nitrile, C₁₋₃ alkyl, C₁₋₃ alkoxy, amino, mono- or di-(C₁₋₃ alkyl)amino, CONH₂ or OH;
tetrahydropyranyl, tetrahydrofuranyl, 1,3-dioxolanonyl, 1,3-dioxanonyl, 1,4- dioxanyl, morpholinyl, thiomorpholinyl, thiomorpholino sulfoxidyl, piperidinyl, piperidinonyl, piperazinyl, tetrahydropyrimidonyl, pentamethylene sulfidyl, pentamethylene sulfoxidyl, pentamethylene sulfonyl, tetramethylene sulfidyl, tetramethylene sulfoxidyl or tetramethylene sulfonyl which are optionally substituted with one to three nitrile, C₁₋₃ alkyl, C₁₋₃ alkoxy, amino, mono- or di- (C₁₋₃ alkyl)amino, CONH₂, or OH;
nitrile, C₁₋₆ alkyl-S(O)ₘ, halogen, hydroxy, C₁₋₄ alkoxy, amino, mono- or di-(C₁₋₆ alkyl)amino, mono- or di-(C₁₋₃ alkyl)aminocarbonyl or NH₂C(O);
- each R₁: is independently:
C₃₋₆ alkyl optionally partially or fully halogenated, and optionally substituted with one to three C₃₋₆cycloalkyl, phenyl, thienyl, furyl, isoxazolyl or isothiazolyl; each of the aforementioned being optionally substituted with one to three groups selected from halogen, C₁₋₃ alkyl which is optionally partially or fully halogenated, hydroxy, nitrile or C₁₋₃alkoxy which is optionally partially or fully halogenated;
cyclopropyl, cyclobutyl, cyclopentanyl, cyclohexanyl, bicyclopentanyl or bicyclohexanyl, each being optionally partially or fully halogenated and optionally substituted with one to three C₁₋₃ alkyl groups optionally partially or fully halogenated, CN, hydroxyC₁₋₃alkyl or phenyl; or an analog of such cycloalkyl group wherein one to three ring methylene groups are independently replaced by O, S, CHOH, >C=O, >C=S or NH; or
silyl containing three C₁₋₄ alkyl groups optionally partially or fully halogenated;
- R₂ is: independently:
halogen, C₁₋₃ alkoxy, C₁₋₃ alkyl-S(O)ₘ optionally partially or fully halogenated, phenylsulfonyl or nitrile;
- R₃ is: independently:
phenyl, morpholino, pyridinyl, pyrimidinyl, pyrazinyl, pyrrolyl, pyrrolylidinyl, imidazolyl, pyrazolyl, each being optionally substituted with one to three phenyl, naphthyl, heterocycle or heteroaryl as hereinabove described in this paragraph, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, oxo, hydroxy, nitrile, C₁₋₃ alkyloxy optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), a mono- or di-(C₁₋₃alkyl)aminocarbonyl, C₁₋₅ alkyl-C(O)-C₁₋₄ alkyl, mono- or di- (C₁₋₃alkyl)amino, mono- or di-(C₁₋₃)alkylamino-C₁₋₅ alkyl, mono- or di- (C₁₋₃alkyl)amino-S(O)₂, R₇-C₁₋₅ alkyl, R₈-C₁₋₅ alkoxy, R₉-C(O)-C₁₋₅ alkyl, R₁₀-C₁- ₅ alkyl(R₁₁)N, carboxy-mono- or di-(C₁₋₅)-alkyl-amino;
C₁₋₃ alkyl or C₁₋₄ alkoxy each being optionally partially or fully halogenated or optionally substituted with R₁₇;
OR₁₈ or C₁₋₆ alkyl optionally substituted with OR₁₈;
amino or mono- or di- (C₁-₅ alkyl)amino optionally substituted with R₁₉;
R₂₀C(O)N(R₂₁)-, R₂₂O- ; R₂₃R₂₄NC(O)-; R₂₆CH₂C(O)N(R₂₁)- or R₂₆C(O)CH₂N(R₂₁)-;
C₂-₄alkenyl substituted by R₂₃R₂₄NC(O)-; or
C₂₋₄ alkynyl branched or unbranched carbon chain optionally partially or fully halogenated and optionally independently substituted with one to two oxo groups, pyrroldinyl, pyrrolyl, morpholinyl, piperidinyl, piperazinyl, imidazolyl, phenyl, pyridinyl, tetrazolyl or one or more C₁₋₄ alkyl optionally substituted by one or more halogen atoms; and
- R₂₃ and R₂₄: taken together optionally form imidazolyl, piperidinyl, morpholinyl, piperazinyl or a pyridinyl ring.

In yet another preferred embodiment the invention relates to pharmaceutical compositions comprising **1** and **2d**, **characterized in that** the p38 kinase inhibitor **2d** is selected from the compounds of formula **(VI)** wherein:
- G is: phenyl, pyridinyl, pyridonyl, naphthyl, quinolinyl, isoquinolinyl, pyrazinyl, benzothiophenyl, dihydrobenzofuranyl, dihydrobenzothiophenyl, indanyl, indolyl, indolinyl, indolonyl or indolinonyl, wherein G is substituted by one or more R₁, R₂ or R₃;
- Ar is: naphthyl;
- X is: phenyl, imidazolyl, pyridinyl, pyrimidinyl, piperdinyl, piperazinyl, pyridazinyl or pyrazinyl each being optionally independently substituted with one to three C₁- ₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y is:: a bond or
a C₁₋₄ saturated carbon chain wherein one of the carbon atoms is optionally replaced by O, N or S and wherein Y is optionally independently substituted with an oxo group;
- Z is:: phenyl, pyridinyl, pyrimidinyl, pyridazinyl, pyrazinyl, imidazolyl, dihydrothiazolyl, dihydrothiazolyl sulfoxide, pyranyl or pyrrolidinyl which are optionally substituted with one to two C₁₋₂ alkyl or C₁₋₂ alkoxy;
tetrahydropyranyl, morpholinyl, thiomorpholinyl, thiomorpholino sulfoxidyl, piperidinyl, piperidinonyl, piperazinyl or tetrahydropyrimidonyl which are optionally substituted with one to two C₁₋₂ alkyl or C₁₋₂ alkoxy; or
C₁₋₃ alkoxy;
- each R₁: is independently: C₃₋₅ alkyl optionally partially or fully halogenated, and optionally substituted with phenyl substituted with zero to three halogen, C₁₋₃ alkyl which is optionally partially or fully halogenated, hydroxy, nitrile or C₁₋₃alkoxy which is optionally partially or fully halogenated;
cyclopropyl, cyclobutyl, cyclopentanyl, cyclohexanyl, bicyclopentanyl or bicyclohexanyl, each being optionally partially or fully halogenated and optionally substituted with one to three C₁₋₃ alkyl groups optionally partially or fully halogenated, CN, hydroxyC₁₋₃alkyl or phenyl; and an analog of cyclopropyl, cyclobutyl, cyclopentanyl, cyclohexanyl, bicyclopentanyl or bicyclohexanyl wherein one ring methylene group is replaced by O; and
silyl containing three C₁₋₂ independently alkyl groups optionally partially or fully halogenated;
- each R₂: is independently:
bromo, chloro, fluoro, methoxy, methylsulfonyl or nitrile;
- each R₃: is independently:
phenyl, morpholino, pyridinyl, pyrimidinyl, pyrrolylidinyl, 2,5-pyrrolidin-dionyl, imidazolyl, pyrazolyl, each of the aforementioned is optionally substituted with one to three C₁₋₃ alkyl which is optionally partially or fully halogenated, halogen, oxo, hydroxy, nitrile and C₁₋₃ alkyloxy optionally partially or fully halogenated;
C₁₋₃ alkyl or C₁₋₃ alkoxy each being optionally partially or fully halogenated or optionally substituted with R₁₇;
OR₁₈ or C₁₋₃ alkyl optionally substituted with OR₁₈;
amino or mono- or di-(C₁₋₃ alkyl)amino optionally substituted with R₁₉;
R₂₀C(O)N(R₂₁)-, R₂₂O- ; R₂₃R₂₄NC(O)-; R₂₆CH₂C(O)N(R₂₁)- or
R₂₆C(O)CH₂N(R₂₁)-;
C₂₋₄ alkenyl substituted by R₂₃R₂₄NC(O)-; or
C₂₋₄ alkynyl substituted with pyrroldinyl or pyrrolyl;
and
- R₂₃ and R₂₄: taken together optionally form morpholino.

In yet another preferred embodiment the invention relates to pharmaceutical compositions comprising **1** and **2d**, **characterized in that** the p38 kinase inhibitor **2d** is selected from the compounds of formula **(VI)** wherein
- G is: phenyl, pyridinyl, pyridonyl, naphthyl, quinolinyl, isoquinolinyl, dihydrobenzofuranyl, indanyl, indolinyl, indolonyl, or indolinonyl, wherein G is substituted by one or more R₁, R₂ or R₃;
- Ar is: 1-naphthyl;
- X is:: phenyl, imidazolyl, pyridinyl, pyrimidinyl, piperdinyl, piperazinyl, pyridazinyl or pyrazinyl;
- Y is:: a bond or
-CH₂-, -CH₂CH₂-, -C(O)-, -O-, -S-, -NH-CH₂CH₂CH₂-, -N(CH₃)-, or -NH-;
- each R₁: is independently:
C₃₋₅ alkyl optionally partially or fully halogenated, and optionally substituted with phenyl;
cyclopropyl, cyclopentanyl, cyclohexanyl and bicyclopentanyl optionally substituted with one to three methyl groups optionally partially or fully halogenated, CN, hydroxymethyl or phenyl; or 2-tetrahydrofuranyl substituted by methyl; or trimethyl silyl;
- each R₃: is independently:
phenyl, morpholinyl, pyridinyl, pyrimidinyl, pyrrolylidinyl, 2,5-pyrrolidin-dionyl, imidazolyl or pyrazolyl, wherein any of the aforementioned is optionally substituted with C₁₋₂ alkyl which is optionally partially or fully halogenated;
C₁₋₃ alkyl or C₁₋₃ alkoxy each being optionally partially or fully halogenated or optionally substituted with diethylamino;
OR₁₈ or C₁₋₃ alkyl optionally substituted with OR₁₈;
amino or mono- or di-(C₁₋₃ alkyl)amino optionally substituted with R₁₉;
CH₃C(O)NH-, R₂₂O- ; R₂₃R₂₄NC(O)-; R₂₆CH₂C(O)N(R₂₁)- or R₂₆C(O)CH₂N(R₂₁)-;
C₂₋₄alkenyl substituted by R₂₃R₂₄NC(O)-; or
C₂₋₄ alkynyl substituted with pyrroldinyl or pyrrolyl;
- R₂₃ and R₂₄: are H or R₂₃ and R₂₄ taken together optionally form morpholino; and
- R₂₆ is: morpholino.

In a further preferred embodiment the invention relates to pharmaceutical compositions comprising **1** and **2d**, **characterized in that** the p38 kinase inhibitor **2d** is selected from the compounds of formula **(VI)** wherein
- G is: phenyl, pyridinyl or naphthyl wherein G is substituted by one or more R₁, R₂ or R₃;
- X is:: imidazolyl or pyridinyl;
- Y is:: -CH₂-, -NH-CH₂CH₂CH₂- or -NH-;
- Z is: morpholino;
- each R₁: is independently:
tert-butyl, sec-butyl, tert-amyl or phenyl;
- R₂: is chloro;
- R₃ is: independently:
methyl, methoxy, methoxymethyl, hydroxypropyl, acetamide, morpholino or morpholinocarbonyl.

In yet a further preferred embodiment the invention relates to pharmaceutical compositions comprising **1** and **2d**, **characterized in that** the p38 kinase inhibitor **2d** is selected from the compounds of formula **(VI)** wherein X is pyridinyl.

In yet a still further preferred embodiment the invention relates to pharmaceutical compositions comprising **1** and **2d**, **characterized in that** the p38 kinase inhibitor **2d** is selected from the compounds of formula **(VI)** wherein the pyridinyl is attached to Ar via the 3-pyridinyl position.

Preferably the invention relates to pharmaceutical compositions comprising **1** and **2d**, **characterized in that** the p38 kinase inhibitor **2d** is selected from the following compounds of formula **(VI):**
1-(3-cyano-phenyl)-3-[4-(6-morpholin-4-ylmethyl-pyridin-3-yl)-naphthalen-1-yl]-urea 1-(3-fluoro-phenyl)-3-[4-(6-morpholin-4-ylmethyl-pyridin-3-yl)-naphthalen-1-yl]-urea
1-(4-chloro-2-trifluoromethyl-phenyl)-3-[4-(6-morpholin-4-ylmethyl-pyridin-3-yl)-naphthalen-1-yl]-urea
1-(2-chloro-5-trifluoromethyl-phenyl)-3-[4-(6-morpholin-4-ylmethyl-pyridin-3-yl)-naphthalen-1-yl]-urea
1-(3,4-dimethyl-phenyl)-3-[4-(6-morpholin-4-ylmethyl-pyridin-3-yl)-naphthalen-1-yl]-urea
1-(3-iodo-phenyl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-m-tolyl-urea
1-(4-methylsulfanyl-phenyl)-3-[4-(6-morpholin-4-ylmethyl-pyridin-3-yl)-naphthalen-1-yl]-urea
1-(3-chloro-4-methyl-phenyl)-3-[4-(6-morpholin-4-ylmethyl-pyridin-3-yl)-naphthalen-1-yl]-urea
1-(4-chloro-3-nitro-phenyl)-3-[4-(6-morpholin-4-ylmethyl-pyridin-3-yl)-naphthalen-1-yl]-urea
1-(2,5-dichloro-phenyl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-naphthalen-2-yl-urea
1-[4-(6-morpholin-4-yl methyl-pyridin-3-yl)-naphthalen-1-yl]-3-phenyl-urea
1-(3-chloro-phenyl)-3-[4-(6-morpholin-4-ylmethyl-pyridin-3-yl)-naphthalen-1-yl]-urea
1-(4-chloro-3-trifluoromethyl-phenyl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-(2,4,6-trichloro-phenyl)-urea
1-(2-methyl-3-nitro-phenyl)-3-[4-(6-morpholin-4-ylmethyl-pyridin-3-yl)-naphthalen-1-yl]-urea
1-(4-methyl-2-nitro-phenyl)-3-[4-(6-morpholin-4-ylmethyl-pyridin-3-yl)-naphthalen-1-yl]-urea
1-(2,3-dichloro-phenyl)-3-[4-(6-morpholin-4-ylmethyl-pyridin-3-yl)-naphthalen-1-yl]-urea
1-(2-methoxy-5-methyl-phenyl)-3-[4-(6-morpholin-4-ylmethyl-pyridin-3-yl)-naphthalen-1-yl]-urea
1-(2-chloro-6-methyl-phenyl)-3-[4-(6-morpholin-4-ylmethyl-pyridin-3-yl)-naphthalen-1-yl]-urea
1-(2,4-dichloro-phenyl)-3-[4-(6-morpholin-4-ylmethyl-pyridin-3-yl)-naphthalen-1-yl]-urea
1-(4-methyl-3-nitro-phenyl)-3-[4-(6-morpholin-4-ylmethyl-pyridin-3-yl)-naphthalen-1-yl]-urea
1-(2,4-dimethyl-phenyl)-3-[4-(6-morpholin-4-ylmethyl-pyridin-3-yl)-naphthalen-1-yl]-urea
1-(2,3-dimethyl-phenyl)-3-[4-(6-morpholin-4-ylmethyl-pyridin-3-yl)-naphthalen-1-yl]-urea
1-(4-cyano-phenyl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-(3,4,5-trimethoxyphenyl)-urea
1-biphenyl-4-yl-3-[4-(6-morpholin-4-ylmethyl-pyridin-3-yl)-naphthalen-1-yl]-urea
1-(2,5-difluoro-phenyl)-3-[4-(6-morpholin-4-ylmethyl-pyridin-3-yl)-naphthalen-1-yl]-urea
1-(3-chloro-2-methoxy-phenyl)-3-[4-(6-morpholin-4-ylmethyl-pyridin-3-yl)-naphthalen-1-yl]-urea
1-(2-fluoro-3-trifluoromethyl-phenyl)-3-[4-(6-morpholin-4-ylmethyl-pyridin-3-yl)-naphthalen-1-yl]-urea
1-(4-benzyloxy-phenyl)-3-[4-(6-morpholin-4-ylmethyl-pyridin-3-yl)-naphthalen-1-yl]-urea
1-(2-methylsulfanyl-phenyl)-3-[4-(6-morpholin-4-ylmethyl-pyridin-3-yl)-naphthalen-1-yl]-urea
1-(2-fluoro-6-trifluoromethyl-phenyl)-3-[4-(6-morpholin-4-ylmethyl-pyridin-3-yl)-naphthalen-1-yl]-urea
1-(4-fluoro-3-trifluoromethyl-phenyl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-(2,4,5-trimethyl-phenyl)-urea
1-[4-(6-morpholin-4-ylmethyl-pyridin-3-yl)-naphthalen-1-yl]-3-(4-trifluoromethylphenyl)-urea
1-(3-methylsulfanyl-phenyl)-3-[4-(6-morpholin-4-ylmethyl-pyridin-3-yl)-naphthalen-1-yl]-urea
1-(2-methoxy-phenyl)-3-[4-(6-morpholin-4-ylmethyl-pyridin-3-yl)-naphthalen-1-yl]-urea
1-(2-fluoro-5-trifluoromethyl-phenyl)-3-[4-(6-morpholin-4-ylmethyl-pyridin-3-yl)-naphthalen-1-yl]-urea
1-(4-methoxy-2-methyl-phenyl)-3-[4-(6-morpholin-4-ylmethyl-pyridin-3-yl)-naphthalen-1-yl]-urea
1-(2-fluoro-5-nitro-phenyl)-3-[4-(6-morpholin-4-ylmethyl-pyridin-3-yl)-naphthalen-1-yl]-urea
1-(4-ethoxy-phenyl)-3-[4-(6-morpholin-4-ylmethyl-pyridin-3-yl)-naphthalen-1-yl]-urea
1-(2,5-dimethoxy-phenyl)-3-[4-(6-morpholin-4-ylmethyl-pyridin-3-yl)-naphthalen-1-yl]-urea
1-(4,5-dimethyl-2-nitro-phenyl)-3-[4-(6-morpholin-4-ylmethyl-pyridin-3-yl)-naphthalen-1-yl]-urea
1-(5-chloro-2-methyl-phenyl)-3-[4-(6-morpholin-4-ylmethyl-pyridin-3-yl)-naphthalen-1-yl]-urea
1-(2-isopropyl-6-methyl-phenyl)-3-[4-(6-morpholin-4-ylmethyl-pyridin-3-yl)-naphthalen-1-yl]-urea
1-(2-difluoromethoxy-phenyl)-3-[4-(6-morpholin-4-ylmethyl-pyridin-3-yl)-naphthalen-1-yl]-urea
1-(4-isopropyl-phenyl)-3-[4-(6-morpholin-4-ylmethyl-pyridin-3-yl)-naphthalen-1-yl]-urea
1-(4-methoxy-phenyl)-3-[4-(6-morpholin-4-ylmethyl-pyridin-3-yl)-naphthalen-1-yl]-urea
1-(3-ethyl-phenyl)-3-[4-(6-morpholin-4-ylmethyl-pyridin-3-yl)-naphthalen-1-yl]-urea
1-(2-ethoxy-phenyl)-3-[4-(6-morpholin-4-ylmethyl-pyridin-3-yl)-naphthalen-1-yl]-urea
1-(4-butoxy-phenyl)-3-[4-(6-morpholin-4-ylmethyl-pyridin-3-yl)-naphthalen-1-yl]-urea
4-{3-[4-(6-morpholin-4-ylmethyl-pyridin-3-yl)-naphthalen-1-yl]-ureido}-benzoic acid ethyl ester
1-(4-butyl-2-methyl-phenyl)-3-[4-(6-morpholin-4-ylmethyl-pyridin-3-yl)-naphthalen-1-yl]-urea
1-(2,6-dibromo-4-isopropyl-phenyl)-3-[4-(6-morpholin-4-ylmethyl-pyridin-3-yl)-naphthalen-1-yl]-urea
1-(3-methoxy-phenyl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-(4-trifluoromethylsulfanyl-phenyl)-urea
5-{3-[4-(6-morpholin-4-ylmethyl-pyridin-3-yl)-naphthalen-1-yl]-ureido}-isophthalic acid dimethyl ester
1-(3-cyclopentyloxy-4-methoxy-phenyl)-3-[4-(6-morpholin-4-ylmethyl-pyridin-3-yl)-naphthalen-1-yl]-urea
3-{3-[4-(6-morpholin-4-ylmethyl-pyridin-3-yl)-naphthalen-1-yl]-ureido}-benzoic acid ethyl ester
1-(5-tert-butyl-2-hydroxy-phenyl)-3-[4-(6-morpholin-4-ylmethyl-pyridin-3-yl)-naphthalen-1-yl]-urea
1-(2-hydroxymethyl-4-phenyl-cyclohexyl)-3-[4-(6-morpholin-4-ylmethyl-pyridin-3-yl)-naphthalen-1-yl]-urea
1-(2-methylsulfanyl-5-trifluoromethyl-phenyl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-(4-pentyloxy-biphenyl-3-yl)-urea
4-methoxy-3-{3-[4-(6-morpholin-4-ylmethyl-pyridin-3-yl)-naphthalen-1-yl]-ureido}-benzoic acid methyl ester
1-(2,5-diethoxy-phenyl)-3-[4-(6-morpholin-4-ylmethyl-pyridin-3-yl)-naphthalen-1-yl]-urea
1-benzothiazol-6-yl-3-[4-(6-morpholin-4-ylmethyl-pyridin-3-yl)-naphthalen-1-yl]-urea
N-(2,5-diethoxy-4-{3-[4-(6-morpholin-4-ylmethyl-pyridin-3-yl)-naphthalen-1-yl]-ureido}-phenyl)-benzamide
1-[4-(6-morpholin-4-ylmethyl-pyridin-3-yl)-naphthalen-1-yl]-3-(3-phenoxy-phenyl)-urea
1-(5-ethanesulfonyl-2-methoxy-phenyl)-3-[4-(6-morpholin-4-ylmethyl-pyridin-3-yl)-naphthalen-1-yl]-urea
4-methoxy-3-{3-[4-(6-morpholin-4-ylmethyl-pyridin-3-yl)-naphthalen-1-yl]-ureido}-N-phenyl-benzamide
1-(2-methyl-1,3-dioxo-2,3-dihydro-1H-isoindol-5-yl)-3-[4-(6-morpholin-4-ylmethyl-pyridin-3-yl)-naphthalen-1-yl]-urea
1-(2,3-dimethyl-1 H-indol-5-yl)-3-[4-(6-morpholin-4-ylmethyl-pyridin-3-yl)-naphthalen-1-yl]-urea
N-butyl-4-methoxy-3-{3-[4-(6-morpholin-4-ylmethyl-pyridin-3-yl)-naphthalen-1-yl]-ureido}-benzenesulfonamide
1-[3-(2-methyl-[1,3]dioxolan-2-yl)-phenyl]-3-[4-(6-morpholin-4-ylmethyl-pyridin-3-yl)-naphthalen-1-yl]-urea
1-(3-methoxy-5-trifluoromethyl-phenyl)-3-[4-(6-morpholin-4-ylmethyl-pyridin-3-yl)-naphthalen-1-yl]-urea
1-(2,4-dimethoxy-phenyl)-3-[4-(6-morpholin-4-ylmethyl-pyridin-3-yl)-naphthalen-1-yl]-urea
1-(2-methyl-4-nitro-phenyl)-3-[4-(6-morpholin-4-ylmethyl-pyridin-3-yl)-naphthalen-1-yl]-urea
1-(2-methoxy-4-nitro-phenyl)-3-[4-(6-morpholin-4-ylmethyl-pyridin-3-yl)-naphthalen-1-yl]-urea
1-(4-chloro-2-nitro-phenyl)-3-[4-(6-morpholin-4-ylmethyl-pyridin-3-yl)-naphthalen-1-yl]-urea
1-(5-chloro-2-methoxy-phenyl)-3-[4-(6-morpholin-4-ylmethyl-pyridin-3-yl)-naphthalen-1-yl]-urea
1-(3,5-dimethoxy-phenyl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-(4-trifluoromethoxyphenyl)-urea
1-[4-(6-morpholin-4-ylmethyl-pyridin-3-yl)-naphthalen-1-yl]-3-(3-trifluoromethylsulfanyl-phenyl)-urea
1-[4-(6-morpholin-4-ylmethyl-pyridin-3-yl)-naphthalen-1-yl]-3-(2-phenoxy-phenyl)-urea
1-(2-methoxy-5-nitro-phenyl)-3-[4-(6-morpholin-4-ylmethyl-pyridin-3-yl)-naphthalen-1-yl]-urea
1-(5-chloro-2,4-dimethoxy-phenyl)-3-[4-(6-morpholin-4-ylmethyl-pyridin-3-yl)-naphthalen-1-yl]-urea
1-(3,5-bis-trifluoromethyl-phenyl)-3-[4-(6-morpholin-4-ylmethyl-pyridin-3-yl)-naphthalen-1-yl]-urea
1-(2-tert-butyl-5-methyl-pyridin-4-yl)-3-[4-(6-morpholin-4-ylmethyl-pyridin-3-yl)-naphthalen-1-yl]-urea
1-(3-methyl-naphthalen-2-yl)-3-[4-(6-morpholin-4-ylmethyl-pyridin-3-yl)-naphthalen-1-yl]-urea
1-(3-tert-butyl-phenyl)-3-[4-(6-morpholin-4-ylmethyl-pyridin-3-yl)-naphthalen-1-yl]-urea
1-(4-methyl-biphenyl-3-yl)-3-[4-(6-morpholin-4-ylmethyl-pyridin-3-yl)-naphthalen-1-yl]-urea
1-(4-tert-butyl-biphenyl-2-yl)-3-[4-(6-morpholin-4-ylmethyl-pyridin-3-yl)-naphthalen-1-yl]-urea
1-(5-chloro-2,4-dimethoxy-phenyl)-3-[4-(6-morpholin-4-ylmethyl-pyridin-3-yl)-naphthalen-1-yl]-urea
1-(5-isopropyl-2-methyl-phenyl)-3-[4-(6-morpholin-4-ylmethyl-pyridin-3-yl)-naphthalen-1-yl]-urea
1-(5-sec-butyl-2-methoxy-phenyl)-3-[4-(6-morpholin-4-ylmethyl-pyridin-3-yl)-naphthalen-1-yl]-urea
1-(5-tert-butyl-2-methoxy-3-propyl-phenyl)-3-[4-(6-morpholin-4-ylmethyl-pyridin-3-yl)-naphthalen-1-yl]-urea
1-(5-tert-butyl-2-methoxymethyl-phenyl)-3-[4-(6-morpholin-4-ylmethyl-pyridin-3-yl)-naphthalen-1-yl]-urea
1-(5-tert-butyl-2-methoxy-phenyl)-3-[4-(6-morpholin-4-ylmethyl-pyridin-3-yl)-naphthalen-1-yl]-urea
1-(5-tert-butyl-2-methyl-phenyl)-3-(4-{6-[(3-methoxy-propyl)-methyl-amino]-pyridin-3-yl}-naphthalen-1-yl)-urea
1-(5-tert-butyl-2-methyl-phenyl)-3-[4-(4-morpholin-4-ylmethyl-imidazol-1-yl)-naphthalen-1-yl]-urea
1-(5-tert-butyl-2-methyl-phenyl)-3-[4-(6-morpholin-4-ylmethyl-pyridin-3-yl)-naphthalen-1-yl]-urea
1-(5-tert-butyl-2-methyl-phenyl)-3-{4-[6-(3-methoxy-propylamino)-pyridin-3-yl]-naphthalen-1-yl}-urea
1-(5-tert-butyl-2-methyl-pyridin-3-yl)-3-[4-(6-morpholin-4-ylmethyl-pyridin-3-yl)-naphthalen-1-yl]-urea
1-(5-tert-butyl-2-morpholin-4-yl-phenyl)-3-[4-(6-morpholin-4-ylmethyl-pyridin-3-yl)-naphthalen-1-yl]-urea
1-(6-tert-butyl-2-chloro-3-methyl-pyridin-4-yl)-3-[4-(6-morpholin-4-ylmethyl-pyridin-3-yl)-naphthalen-1-yl]-urea
1-[4-(6-morpholin-4-ylmethyl-pyridin-3-yl)-naphthalen-1-yl]-3-(3-trifluoromethylphenyl)-urea
1-[4-(6-morpholin-4-ylmethyl-pyridin-3-yl)-naphthalen-1-yl]-3-(4-trifluoromethoxyphenyl)-urea
1-[5-(1,1-dimethyl-propyl)-2-methoxy-phenyl]-3-[4-(6-morpholin-4-ylmethyl-pyridin-3-yl)-naphthalen-1-yl]-urea
1-[5-tert-butyl-2-(1H-pyrazol-4-yl)-phenyl]-3-[4-(6-morpholin-4-ylmethyl-pyridin-3-yl)-naphthalen-1-yl]-urea
1-[5-tert-butyl-2-(2-methyl-pyrimidin-5-yl)-phenyl]-3-[4-(6-morpholin-4-ylmethyl-pyridin-3-yl)-naphthalen-1-yl]-urea
1-[5-tert-butyl-2-(3-hydroxy-propyl)-phenyl]-3-[4-(6-morpholin-4-ylmethyl-pyridin-3-yl)-naphthalen-1-yl]-urea
1-[5-tert-butyl-2-(3-morpholin-4-yl-3-oxo-propyl)-phenyl]-3-[4-(6-morpholin-4-ylmethyl-pyridin-3-yl)-naphthalen-1-yl]-urea
1-[5-tert-butyl-2-(morpholine-4-carbonyl)-phenyl]-3-[4-(6-morpholin-4-ylmethyl-pyridin-3-yl)-naphthalen-1-yl]-urea
N-(5-tert-butyl-2-methoxy-3-{3-[4-(6-morpholin-4-ylmethyl-pyridin-3-yl)-naphthalen-1-yl]-ureido}-phenyl)-acetamide
1-(2-tert-butyl-5-methyl-pyridin-4-yl)-3-[4-(6-morpholin-4-ylmethyl-pyridin-3-yl)-naphthalen-1-yl]-urea;
1-(3-methyl-naphthalen-2-yl)-3-[4-(6-morpholin-4-ylmethyl-pyridin-3-yl)-naphthalen-1-yl]-urea;
1-(3-tert-butyl-phenyl)-3-[4-(4-morpholin-4-ylmethyl-phenyl)-naphthalen-1-yl]-urea;
1-(3-tert-butyl-phenyl)-3-[4-(6-morpholin-4-ylmethyl-pyridin-3-yl)-naphthalen-1-yl]-urea;
1-(4-methyl-biphenyl-3-yl)-3-[4-(6-morpholin-4-ylmethyl-pyridin-3-yl)-naphthalen-1-yl]-urea;
1-(4-tert-butyl-biphenyl-2-yl)-3-[4-(6-morpholin-4-ylmethyl-pyridin-3-yl)-naphthalen-1-yl]-urea;
1-(5-chloro-2,4-dimethoxy-phenyl)-3-[4-(6-morpholin-4-ylmethyl-pyridin-3-yl)-naphthalen-1-yl]-urea;
1-(5-isopropyl-2-methyl-phenyl)-3-[4-(6-morpholin-4-ylmethyl-pyridin-3-yl)-naphthalen-1-yl]-urea;
1-(5-sec-butyl-2-methoxy-phenyl)-3-[4-(6-morpholin-4-ylmethyl-pyridin-3-yl)-naphthalen-1-yl]-urea;
1-(5-tert-butyl-2-methoxy-3-propyl-phenyl)-3-[4-(6-morpholin-4-ylmethyl-pyridin-3-yl)-naphthalen-1-yl]-urea;
1-(5-tert-butyl-2-methoxymethyl-phenyl)-3-[4-(6-morpholin-4-ylmethyl-pyridin-3-yl)-naphthalen-1-yl]-urea;
1-(5-tert-butyl-2-methoxy-phenyl)-3-[4-(2-morpholin-4-ylmethyl-pyrimidin-5-yl)-naphthalen-1-yl]-urea;
1-(5-tert-butyl-2-methoxy-phenyl)-3-[4-(4-thiomorpholin-4-ylmethyl-phenyl)-naphthalen-1-yl]-urea;
1-(5-tert-butyl-2-methoxy-phenyl)-3-[4-(6-morpholin-4-ylmethyl-pyridin-3-yl)-naphthalen-1-yl]-urea;
1-(5-tert-butyl-2-methoxy-phenyl)-3-[4-(6-morpholin-4-ylmethyl-phenyl)-naphthalen-1-yl]-urea;
1-(5-tert-butyl-2-methoxy-phenyl)-3-{4-[4-(tetrahydro-pyran-4-ylamino)-phenyl]-naphthalen-1-yl}-urea;
1-(5-tert-butyl-2-methoxy-phenyl)-3-{4-[6-(4-methyl-piperazin-1-ylmethyl)-pyridin-3-yl]-naphthalen-1-yl}-urea;
1-(5-tert-butyl-2-methyl-phenyl)-3-(4-{6-[(3-methoxy-propyl)-methyl-amino]-pyridin-3-yl}-naphthalen-1-yl)-urea;
1-(5-tert-butyl-2-methyl-phenyl)-3-[4-(4-morpholin-4-ylmethyl-imidazol-1-yl)-naphthalen-1-yl]-urea;
1-(5-tert-butyl-2-methyl-phenyl)-3-[4-(4-morpholin-4-ylmethyl-phenyl)-naphthalen-1-yl]-urea;
1-(5-tert-butyl-2-methyl-phenyl)-3-[4-(6-morpholin-4-ylmethyl-pyridin-3-yl)-naphthalen-1-yl]-urea;
1-(5-tert-butyl-2-methyl-phenyl)-3-{4-[6-(3-methoxy-propylamino)-pyridin-3-yl]-naphthalen-1-yl}-urea;
1-(5-tert-butyl-2-methyl-pyridin-3-yl)-3-[4-(6-morpholin-4-ylmethyl-pyridin-3-yl)-naphthalen-1-yl]-urea;
1-(5-tert-butyl-2-morpholin-4-yl-phenyl)-3-[4-(6-morpholin-4-ylmethyl-pyridin-3-yl)-naphthalen-1-yl]-urea;
1-(6-tert-butyl-2-chloro-3-methyl-pyridin-4-yl)-3-[4-(6-morpholin-4-ylmethyl-pyridin-3-yl)-naphthalen-1-yl]-urea;
1-(6-tert-butyl-2-chloro-3-methyl-pyridin-4-yl)-3-[4-(6-thiomorpholin-4-ylmethyl-pyridin-3-yl)-naphthalen-1-yl]-urea;
1-[2-methoxy-5-(1-methyl-cyclopropyl)-phenyl]-3-[4-(2-morpholin-4-ylmethyl-pyrimidin-5-yl)-naphthalen-1-yl]-urea;
1-[4-(6-morpholin-4-ylmethyl-pyridin-3-yl)-naphthalen-1-yl]-3-(3-trifluoromethylphenyl)-urea;
1-[4-(6-morpholin-4-ylmethyl-pyridin-3-yl)-naphthalen-1-yl]-3-(4-trifluoromethoxyphenyl)-urea;
1-[5-(1,1 -dimethyl-propyl)-2-methoxy-phenyl]-3-[4-(4-thiomorpholin-4-ylmethyl-phenyl)-naphthalen-1-yl]-urea;
1-[5-(1,1 -dimethyl-propyl)-2-methoxy-phenyl]-3-[4-(6-morpholin-4-ylmethyl-pyridin-3-yl)-naphthalen-1-yl]-urea;
1-[5-(1-cyano-cyclopropyl)-2-methoxy-phenyl]-3-[4-(2-morpholin-4-ylmethyl-pyrimidin-5-yl)-naphthalen-1-yl]-urea;
1-[5-tert-butyl-2-(1 H-pyrazol-4-yl)-phenyl]-3-[4-(6-morpholin-4-ylmethyl-pyridin-3-yl)-naphthalen-1-yl]-urea;
1-[5-tert-butyl-2-(2-methyl-pyrimidin-5-yl)-phenyl]-3-[4-(5-pyridin-4-ylmethyl-pyridin-2-yl)-naphthalen-1-yl]-urea;
1-[5-tert-butyl-2-(2-methyl-pyrimidin-5-yl)-phenyl]-3-[4-(6-morpholin-4-ylmethyl-pyridin-3-yl)-naphthalen-1-yl]-urea;
1-[5-tert-butyl-2-(3-hydroxy-propyl)-phenyl]-3-[4-(6-morpholin-4-ylmethyl-pyridin-3-yl)-naphthalen-1-yl]-urea;
1-[5-tert-butyl-2-(3-morpholin-4-yl-3-oxo-propyl)-phenyl]-3-[4-(6-morpholin-4-ylmethyl-pyridin-3-yl)-naphthalen-1-yl]-urea;
1-[5-tert-butyl-2-(morpholine-4-carbonyl)-phenyl]-3-[4-(6-morpholin-4-ylmethyl-pyridin-3-yl)-naphthalen-1-yl]-urea;
2-[4-tert-butyl-2-(3-{4-[6-(2,6-dimethyl-morpholin-4-ylmethyl)-pyridin-3-yl]-naphthalen-1-yl}-ureido)-phenoxy]-acetamide;
3-{4-[3-(5-tert-butyl-2-methoxy-phenyl)-ureido]-naphthalen-1-yl}-benzamide;
4-tert-butyl-2-(3-[4-(2-chloro-4-morpholin-4-ylmethyl-phenyl)-naphthalen-1-yl]-ureido)-benzamide;
and the pharmaceutically acceptable derivatives thereof.

More preferably the invention relates to pharmaceutical compositions comprising **1** and **2d**, **characterized in that** the p38 kinase inhibitor **2d** is selected from the following compounds of formula **(VI):**
1-(2-tert-butyl-5-methyl-pyridin-4-yl)-3-[4-(6-morpholin-4-ylmethyl-pyridin-3-yl)-naphthalen-1-yl]-urea;
1-(3-tert-butyl-phenyl)-3-[4-(6-morpholin-4-ylmethyl-pyridin-3-yl)-naphthalen-1-yl]-urea;
1-(4-methyl-biphenyl-3-yl)-3-[4-(6-morpholin-4-ylmethyl-pyridin-3-yl)-naphthalen-1-yl]-urea;
1-(4-tert-butyl-biphenyl-2-yl)-3-[4-(6-morpholin-4-ylmethyl-pyridin-3-yl)-naphthalen-1-yl]-urea;
1-(5-isopropyl-2-methyl-phenyl)-3-[4-(6-morpholin-4-ylmethyl-pyridin-3-yl)-naphthalen-1-yl]-urea;
1-(5-sec-butyl-2-methoxy-phenyl)-3-[4-(6-morpholin-4-ylmethyl-pyridin-3-yl)-naphthalen-1-yl]-urea;
1-(5-tert-butyl-2-methoxymethyl-phenyl)-3-[4-(6-morpholin-4-ylmethyl-pyridin-3-yl)-naphthalen-1-yl]-urea;
1-(5-tert-butyl-2-methoxy-phenyl)-3-[4-(6-morpholin-4-ylmethyl-pyridin-3-yl)-naphthalen-1-yl]-urea;
1-(5-tert-butyl-2-methyl-phenyl)-3-(4-{6-[(3-methoxy-propyl)-methyl-amino]-pyridin-3-yl}-naphthalen-1-yl)-urea;
1-(5-tert-butyl-2-methyl-phenyl)-3-[4-(6-morpholin-4-ylmethyl-pyridin-3-yl)-naphthalen-1-yl]-urea;
1-(5-tert-butyl-2-methyl-pyridin-3-yl)-3-[4-(6-morpholin-4-ylmethyl-pyridin-3-yl)-naphthalen-1-yl]-urea;
1-[5-(1,1 -dimethyl-propyl)-2-methoxy-phenyl]-3-[4-(6-morpholin-4-ylmethyl-pyridin-3-yl)-naphthalen-1-yl]-urea;
1-[5-tert-butyl-2-(1 H-pyrazol-4-yl)-phenyl]-3-[4-(6-morpholin-4-ylmethyl-pyridin-3-yl)-naphthalen-1-yl]-urea;
1-[5-tert-butyl-2-(2-methyl-pyrimidin-5-yl)-phenyl]-3-[4-(6-morpholin-4-ylmethyl-pyridin-3-yl)-naphthalen-1-yl]-urea;
1-[5-tert-butyl-2-(3-hydroxy-propyl)-phenyl]-3-[4-(6-morpholin-4-ylmethyl-pyridin-3-yl)-naphthalen-1-yl]-urea;
1-[5-tert-butyl-2-(morpholine-4-carbonyl)-phenyl]-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-acetamide
and the pharmaceutically acceptable derivatives thereof.

In another preferred embodiment the invention relates to pharmaceutical compositions comprising **1** and **2d**, **characterized in that** the p38 kinase inhibitor **2d** is selected from the following compounds of formula **(VII)** as disclosed in WO 00/55139 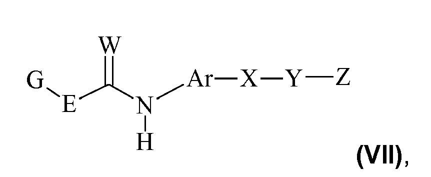 wherein:
- E is: carbon or a heteroatom group chosen from -O-, -NH- and -S-;
- Gis:: an aromatic C₆₋₁₀ carbocycle or a nonaromatic C₃₋₁₀carbocycle saturated or unsaturated;
a 6-14 membered monocyclic, bicyclic or tricyclic heteroaryl containing 1 or more heteroatoms chosen from O, N and S;
a 6-8 membered monocyclic heterocycle containing one or more heteroatoms chosen from O, N and S;
or
an 8-11 membered bicyclic heterocycle, containing one or more heteroatoms chosen from O, N and S;
wherein G is optionally substituted by one or more R₁, R₂ or R₃;
- Ar is:: phenyl, naphthyl, quinolinyl, isoquinolinyl, tetrahydronaphthyl, tetrahydroqui- nolinyl, tetrahydroisoquinolinyl, benzimidazolyl, benzofuranyl, dihydrobenzo- furanyl, indolinyl, benzothienyl, dihydrobenzothienyl, indanyl, indenyl or indolyl each being optionally substituted by one or more R₄ or R₅;
- X is:: a C₅₋₈ cycloalkyl or cycloalkenyl optionally substituted with one to two oxo groups or one to three C₁₋₄ alkyl, C₁₋₄ alkoxy or C₁₋₄ alkylamino chains each being branched or unbranched;
aryl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl; each being optionally independently substituted with one to three C₁₋₄ alkyl, C₁₋₄alkoxy, hydroxy, nitrile, amino, mono- or di-(C₁₋₃ alkyl)amino, mono- or di- (C₁₋₃ alkylamino)carbonyl, NH₂C(O), C₁₋₆ alkyl-S(O)ₘ or halogen;
- Y is:: a bond or a C₁₋₄ saturated or unsaturated branched or unbranched carbon chain optionally partially or fully halogenated, wherein one or more C atoms are optionally replaced by O, N, or S(O)ₘ and wherein Y is optionally indepen- dently substituted with one to two oxo groups, nitrile, phenyl or one or more C₁₋₄ alkyl optionally substituted by one or more halogen atoms;
- Z is:: aryl, heteroaryl selected from pyridinyl, piperazinyl, pyrimidinyl, pyridazinyl, pyrazinyl, imidazolyl, pyrazolyl, triazolyl, tetrazolyl, furanyl, thienyl and pyranyl, heterocycle selected from tetrahydropyrimidonyl, cyclohexanonyl, cyclohexan- olyl, 2-oxa- or 2-thia-5-aza-bicyclo[2.2.1]heptanyl, pentamethylene sulfidyl, pentamethylene sulfoxidyl, pentamethylene sulfonyl, tetramethylene sulfidyl, tetramethylene sulfoxidyl or tetramethylene sulfonyl, tetrahydropyranyl, tetrahydrofuranyl, 1,3-dioxolanonyl, 1,3-dioxanonyl, 1,4-dioxanyl, morpholino, thiomorpholino, thiomorpholino sulfoxidyl, thiomorpholino sulfonyl, piperidinyl, piperidinonyl, pyrrolidinyl and dioxolanyl,
each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group is optionally substituted with one to two halogen, C₁₋₆ alkyl or C₁₋₆ alkoxy;
or Z is optionally substituted with one to three aryl, heterocycle or heteroaryl as hereinabove described in this paragraph each in turn is optionally substituted by halogen, C₁₋₆ alkyl or C₁₋₆ alkoxy;
or Z is hydroxy, halogen, nitrile, amino wherein the N atom is optionally independently mono- or di-substituted by C₁₋₃acyl, C₁₋₆alkyl or C₁₋₃alkoxy- C₁₋₃alkyl, C₁₋₆alkyl branched or unbranched, C₁₋₆alkoxy, C₁₋₃acylamino, nitrileC₁₋₄alkyl, C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
- each R₁: is independently:
C₁₋₁₀ alkyl branched or unbranched optionally partially or fully halogenated, wherein one or more C atoms are optionally independently replaced by O, N or S(O)ₘ, and wherein said C₁₋₁₀ alkyl is optionally substituted with one to three C₃₋₁₀ cycloalkyl, hydroxy, oxo, phenyl, naphthyl, pyridinyl, pyrimidinyl, pyrazinyl, pyridazinyl, pyrrolyl, pyrrolidinyl, imidazolyl, pyrazolyl, thienyl, furyl, dioxolanyl, isoxazolyl or isothiazolyl; each of the aforementioned being opti- onally substituted with one to five groups selected from halogen, C₁₋₆ alkyl which is optionally partially or fully halogenated, C₃₋₈ cycloalkanyl, C₅₋₈ cyclo- alkenyl, hydroxy, nitrile, C₁₋₃ alkoxy which is optionally partially or fully halo- genated or NH₂C(O), mono- or di(C₁₋₃alkyl)amino, and mono- or di(C₁₋₃alkyl)- aminocarbonyl;
- or R₁: is cyclopropyloxy, cyclobutyloxy, cyclopentyloxy, cyclohexyloxy, or cycloheptyloxy each being optionally partially or fully halogenated and optionally substituted with one to three C₁₋₃ alkyl groups optionally partially or fully halogenated, nitrile, hydroxyC₁₋₃alkyl or aryl; or an analog of such cycloalkyl group wherein one to three ring methylene groups are independently replaced by O, S(O)ₘ, CHOH, >C=O, >C=S or NH;
phenyloxy or benzyloxy each being optionally partially or fully halogenated and optionally substituted with one to three C₁₋₃ alkyl groups optionally partially or fully halogenated, nitrile, hydroxyC₁₋₃alkyl or aryl; or an analog of such cycloaryl group wherein one to two ring methyne groups are independently replaced by N;
cyclopropyl, cyclobutyl, cyclopentyl, cyclohexyl, cycloheptyl, bicyclopentanyl, bicyclohexanyl or bicycloheptanyl, each being optionally partially or fully halogenated and optionally substituted with one to three C₁₋₃ alkyl optionally partially or fully halogenated, nitrile, hydroxyC₁₋₃alkyl or aryl; or an analog of such cycloalkyl group wherein one to three ring methylene groups are independently replaced by O, S(O)ₘ, CHOH, >C=O, >C=S or NH;
C₃₋₁₀ branched or unbranced alkenyl each being optionally partially or fully halogenated, and optionally substituted with one to three C₁₋₅ branched or unbranched alkyl, phenyl, naphthyl, pyridinyl, pyrimidinyl, pyrazinyl, pyrida- zinyl, pyrrolyl, imidazolyl, pyrazolyl, thienyl, furyl, isoxazolyl or isothiazolyl, each of the aforementioned being substituted with one to five halogen, C₁₋₆ alkyl which is optionally partially or fully halogenated, cyclopropanyl, cyclobu- tanyl, cyclopentanyl, cyclohexanyl, cycloheptanyl, bicyclopentanyl, bicyclo- hexanyl and bicycloheptanyl, hydroxy, nitrile, C₁₋₃ alkyloxy which is optionally partially or fully halogenated, NH₂C(O), mono- or di(C₁₋₃alkyl)aminocarbonyl; the C₃₋₁₀ branched or unbranced alkenyl being optionally interrupted by one or more heteroatoms chosen from O, N and S(O)ₘ;
cyclopentenyl, cyclohexenyl, cyclohexadienyl, cycloheptenyl, cycloheptadienyl, bicyclohexenyl or bicycloheptenyl, wherein such cycloalkenyl group is option- ally substituted with one to three C₁₋₃ alkyl groups;
oxo, nitrile, halogen;
silyl containing three C₁₋₄ alkyl groups optionally partially or fully halogenated; or
C₃₋₆ alkynyl branched or unbranched carbon chain optionally partially or fully halogenated, wherein one or more methylene groups are optionally replaced by O, NH or S(O)ₘ and wherein said alkynyl group is optionally independently substituted with one to two oxo groups, hydroxy, pyrroldinyl, pyrrolyl, tetrahy- dropyranyl, one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
- each R₂, R₄, and R₅: is a C₁₋₆ branched or unbranched alkyl optionally partially or fully halogenated, C₁₋₆acyl, aroyl, C₁₋₄ branched or unbranched alkoxy, each being optionally partially or fully halogenated, halogen, methoxycarbonyl, C₁₋₃ alkyl-S(O)ₘ optionally partially or fully halogenated, or phenyl-S(O)ₘ;
OR₆, C₁₋₆ alkoxy, hydroxy, nitrile, nitro, halogen;
or amino-S(O)ₘ- wherein the N atom is optionally independently mono- or di- substituted by C₁₋₆alkyl or arylC₀₋₃alkyl, or amino wherein the N atom is option- ally independently mono- or di-substituted by C₁₋₃alkyl, arylC₀₋₃alkyl, C₁₋₆acyl, C₁₋₆alkyl-S(O)ₘ- or arylC₀₋₃alkyl-S(O)ₘ-, each of the aforementioned alkyl and aryl in this subparagraph are optionally partially or fully halogenated and optionally substituted with one to two C₁₋₆ alkyl or C₁₋₆ alkoxy;
- each R₃: is independently:
phenyl, naphthyl, morpholino, pyridinyl, pyrimidinyl, pyrazinyl, pyridazinyl, pyrrolyl, pyrrolidinyl, imidazolyl, pyrazolyl, thiazolyl, oxazoyl, [1,3,4]oxadiazol, triazolyl, tetrazolyl, thienyl, furyl, tetrahydrofuryl, isoxazolyl, isothiazolyl, quino- linyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl or indazolyl, each of the aforementioned is optionally substituted with one to three phenyl, naphthyl, heterocycle or heteroaryl as hereinabove described in this para- graph, C₁₋₆ branched or unbranched alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, hydroxy, oxo, nitrile, C₁₋₃ alkoxy optionally partially or fully halogenated, phenyloxy, naphthyloxy, heteroaryloxy or heterocyclicoxy wherein the heterocyclic or heteroaryl moiety is as herein- above described in this paragraph, nitro, amino, mono- or di-(C₁₋₃alky)lamino, phenylamino, naphthylamino, heteroaryl or heterocyclic amino wherein the heteroaryl heterocyclic moiety is as hereinabove described in this paragraph, NH₂C(O), a mono- or di-(C₁₋₃alkyl) aminocarbonyl, C₁₋₅ alkyl-C(O)-C₁₋₄ alkyl, amino-C₁₋₅ alkyl, mono- or di-(C₁₋₅alkyl)amino, mono- or di-(C₁₋₃alkyl)amino- C₁₋₅ alkyl, amino-S(O)₂, di-(C₁₋₃alkyl)amino-S(O)₂, R₇-C₁₋₅ alkyl, R₈-C₁₋₅ alkoxy, R₉-C(O)-C₁₋₅ alkyl, R₁₀-C₁₋₅ alkyl(R₁₁)N, carboxy-mono- or di-(C₁₋₅alkyl)-amino;
a fused aryl selected from benzocyclobutanyl, indanyl, indenyl, dihydronaph- thyl, tetrahydronaphthyl, benzocycloheptanyl and benzocycloheptenyl, or a fused heteroaryl selected from cyclopentenopyridinyl, cyclohexanopyridinyl, cyclopentanopyrimidinyl, cyclohexanopyrimidinyl, cyclopentanopyrazinyl, cy- clohexanopyrazinyl, cyclopentanopyridazinyl, cyclohexanopyridazinyl, cyclo- pentanoquinolinyl, cyclohexanoquinolinyl, cyclopentanoisoquinolinyl, cyclohe- xanoisoquinolinyl, cyclopentanoindolyl, cyclohexanoindolyl, cyclopentanobenz- imidazolyl, cyclohexanobenzimidazolyl, cyclopentanobenzoxazolyl, cyclohex- anobenzoxazolyl, cyclopentanoimidazolyl, cyclohexanoimidazolyl, cyclopen- tanothienyl and cyclohexanothienyl; wherein the fused aryl or fused heteroaryl ring is independently substituted with zero to three phenyl, naphthyl, pyridinyl, pyrimidinyl, pyrazinyl, pyridazinyl, pyrrolyl, imidazolyl, pyrazolyl, thienyl, furyl, isoxazolyl, isothiazolyl, C₁₋₆ alkyl which is optionally partially or fully halo- genated, halogen, nitrile, C₁₋₃ alkyloxy which is optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naph- thylamino, heteroaryl or heterocyclic amino wherein the heteroaryl or hetero- cyclic moiety is as hereinabove described in this paragraph, NH₂C(O), mono- or di-(C₁₋₃alkyl)aminocarbonyl, C₁₋₄ alkyl-OC(O), C₁₋₅ alkyl-C(O)-C₁₋₄ alkyl, amino-C₁₋₅ alkyl, mono- or di-(C₁₋₃)alkylamino-C₁₋₅ alkyl, R₁₂-C₁₋₅ alkyl, R₁₃-C₁₋₅ alkoxy, R₁₄-C(O)-C₁₋₅ alkyl or R₁₅-C₁₋₅ alkyl(R₁₆)N;
cyclopropanyl, cyclobutanyl, cyclopentanyl, cyclohexanyl, cycloheptanyl, bicyclopentanyl, bicyclohexanyl or bicycloheptanyl, each being optionally be partially or fully halogenated and optionally substituted with one to three C₁-₃ alkyl groups, or an analog of such cycloalkyl group wherein one to three ring methylene groups are independently replaced by O, S, CHOH, >C=O, >C=S or NH;
cyclopentenyl, cyclohexenyl, cyclohexadienyl, cycloheptenyl, cycloheptadienyl, bicyclohexenyl or bicycloheptenyl, each optionally substituted with one to three C₁₋₃ alkyl groups;
C₁-₄ alkyl-phenyl-C(O)-C₁₋₄ alkyl-, C₁₋₄ alkyl-C(O)-C₁₋₄ alkyl- or C₁₋₄ alkyl- phenyl-S(O)ₘ-C₁₋₄ alkyl-;
C₁₋₆ alkyl or C₁₋₆ branched or unbranched alkoxy each of which is optionally partially or fully halogenated or optionally substituted with R₁₇;
OR₁₈ or C₁₋₆ alkyl optionally substituted with OR₁₈;
amino or mono- or di-(C₁₋₅alkyl)amino optionally substituted with R₁₉;
R₂₀C(O)N(R₂₁)-, R₂₂O- Or R₂₃R₂₄NC(O)-; R₂₆(CH₂)ₘC(O)N(R₂₁)-, R₂₃R₂₄NC(O)- C₁₋₃alkoxy or R₂₆C(O)(CH2)ₘN(R₂₁)-;
C₂₋₆alkenyl substituted by R₂₃R₂₄NC(O)-;
C₂₋₆ alkynyl branched or unbranched carbon chain, optionally partially or fully halogenated, wherein one or more methylene groups are optionally replaced by O, NH, S(O)ₘ and wherein said alkynyl group is optionally independently substituted with one to two oxo groups, pyrroldinyl, pyrrolyl, morpholino, piper- idinyl, piperazinyl, imidazolyl, phenyl, pyridinyl, tetrazolyl one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₄ alkyl)amino optionally substituted by one or more halogen atoms;
C₁₋₆acyl or aroyl;
- R₆: is a: C₁₋₄ alkyl optionally partially or fully halogenated and optionally substituted with R₂₆;
- each R₇, R₈, R₉, R₁₀, R₁₂, R₁₃,:
- R₁₄, R₁₅, R₁₇ R₁₉, R₂₅ and R₂₆: is independently:
nitrile, phenyl, morpholino, piperidinyl, piperazinyl, imidazolyl, pyridinyl, tetrazolyl, amino or mono- or di-(C₁₋₄alkyl)amino optionally partially or fully halogenated;
- each R₁₁ and R₁₆: is independently:
hydrogen or C₁₋₄ alkyl optionally partially or fully halogenated;
- R₁₈ is: independently:
hydrogen or a C₁₋₄ alkyl optionally independently substituted with oxo or R₂₅;
- R₂₀ is: independently:
C₁₋₁₀ alkyl optionally partially or fully halogenated, phenyl, or pyridinyl;
- R₂₁ is: independently:
hydrogen or C₁₋₃ alkyl optionally partially or fully halogenated;
- each R₂₂, R₂₃ and R₂₄: is independently:
hydrogen, C₁₋₆ alkyl optionally partially or fully halogenated, said C₁₋₆ alkyl is optionally interrupted by one or more O, N or S, said C₁₋₆ alkyl also being independently optionally substituted by mono- or di-(C₁₋₃alkyl)aminocarbonyl, phenyl, pyridinyl, amino or mono- or di-(C₁₋₄alkyl)amino each of which is optionally partially or fully halogenated and optionally substituted with mono- or di-(C₁₋₃alkyl)amino;
- or R₂₃ and R₂₄: taken together optionally form a heterocyclic or heteroaryl ring;

m = 0, 1 or 2;
W is O or S and
pharmaceutically acceptable derivatives thereof.

In a preferred embodiment the invention relates to pharmaceutical compositions comprising **1** and **2d**, **characterized in that** the p38 kinase inhibitor **2d** is selected from the following compounds of formula **(VII)** wherein:
- E is: -CH₂-, -NH- or -O-;
- W is: O; and
- G is:: phenyl, naphthyl, benzocyclobutanyl, dihydronaphthyl, tetrahydronaphthyl, benzocycloheptanyl, benzocycloheptenyl, indanyl, indenyl;
pyridinyl, pyridonyl, quinolinyl, dihydroquinolinyl, tetrahydroquinoyl, isoqui- nolinyl, tetrahydroisoquinoyl, pyridazinyl, pyrimidinyl, pyrazinyl, benzimidazolyl, benzthiazolyl, benzooxazolyl, benzofuranyl, benzothiophenyl, benzpyrazolyl, dihydrobenzofuranyl, dibenzofuranyl, dihydrobenzothiophenyl, benzooxazol- onyl, benzo[1,4]oxazin-3-onyl, benzodioxolyl, benzo[1,3]dioxol-2-onyl, benzo- furan-3-onyl, tetrahydrobenzopyranyl, indolyl, 2,3-dihydro-1 H-indolyl, indolinyl, indolonyl, indolinonyl, phthalimidyl, chromoyl;
oxetanyl, pyrrolidinyl, tetrahydrofuranyl, tetrahydrothiophenyl, piperidinyl, pip- erazinyl, morpholino, tetrahydropyranyl, dioxanyl, tetramethylene sulfonyl, tetramethylene sulfoxidyl, oxazolinyl, 3,4-dihydro-2H-benzo[1,4]oxazinyl, thiazolinyl, imidazolinyl, tertrahydropyridinyl, homopiperidinyl, pyrrolinyl, tetrahydropyrimidinyl, decahydroquinolinyl, decahydroisoquinolinyl, thiomorph- olino, thiazolidinyl, dihydrooxazinyl, dihydropyranyl, oxocanyl, heptacanyl, thioxanyl or dithianyl;
wherein G is optionally substituted by one or more R₁, R₂ or R₃.

In yet another preferred embodiment the invention relates to pharmaceutical compositions comprising **1** and **2d, characterized in that** the p38 kinase inhibitor **2d** is selected from the following compounds of formula **(VII)** wherein:
- E is: -NH-;
- G is: phenyl, pyridinyl, pyridonyl, naphthyl, quinolinyl, isoquinolinyl, pyrazinyl, benzimidazolyl, benzooxazolyl, benzooxazolonyl, benzofuranyl, benzothio- phenyl, benzpyrazolyl, dihydrobenzofuranyl, dihydrobenzothiophenyl, 3,4- dihydro-2H-benzo[1,4]oxazinyl, indanyl, indenyl, indolyl, indolinyl, indolonyl, 2,3-dihydro-1H-indolyl or indolinonyl, wherein G is optionally substituted by one or more R₁, R₂ or R₃;
- Ar is:: naphthyl, quinolinyl, isoquinolinyl, tetrahydronaphthyl, tetrahydroquinolinyl, tetrahydroisoquinolinyl, indanyl, indenyl or indolyl each being optionally substituted by one or more R₄ or R₅ groups;
- X is:: phenyl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, piperazinyl, pyridazinyl or pyrazinyl; each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y is:: a bond or
a C₁₋₄ saturated or unsaturated carbon chain wherein one or more of the C atoms is optionally replaced by O, N, or S(O)ₘ and wherein Y is optionally independently substituted with one to two oxo groups, nitrile, phenyl or one or more 6₁₋₄ alkyl optionally substituted by one or more halogen atoms;
- Z is:: phenyl, heteroaryl selected from pyridinyl, piperazinyl, pyrimidinyl, pyridazinyl, pyrazinyl, imidazolyl, furanyl, thienyl and pyranyl, heterocycle selected from 2- oxa-5-aza-bicyclo[2.2.1 ]heptanyl, tetrahydropyrimidonyl, pentamethylene sulfidyl, pentamethylene sulfoxidyl, pentamethylene sulfonyl, tetramethylene sulfidyl, tetramethylene sulfoxidyl tetramethylene sulfonyl, tetrahydropyranyl, tetrahydrofuranyl, 1,3-dioxolanonyl, 1,3-dioxanonyl, 1,4-dioxanyl, morpholino, thiomorpholino, thiomorpholino sulfoxidyl, piperidinyl, piperidinonyl, dihydro- thiazolyl, dihydrothiazolyl sulfoxidyl, pyrrolidinyl and dioxolanyl which are optionally substituted with one to three nitrile, C₁₋₃ alkyl, C₁₋₃ alkoxy, amino, mono- or di-(C₁₋₃ alkyl)amino, CONH₂ or OH;
or Z is optionally substituted by phenyl, heterocycle or heteroaryl as hereinabove described in this paragraph each in turn is optionally substituted by halogen, C₁₋₃ alkyl or C₁₋₃ alkoxy;
or Z is nitrile, nitrileC₁₋₃ alkyl, C₁₋₆ alkyl-S(O)ₘ, halogen, hydroxy, C₁₋₃ alkyl, C₁₋₃ acylamino, C₁₋₄ alkoxy, amino, mono- or di-(C₁₋₃ alkyl)aminocarbonyl, or amino mono or di-substituted by aminoC₁₋₆ alkyl or C₁₋₃alkoxyC₁₋₃akkyl;
- each R₁: is independently:
C₁₋₆ alkyl branched or unbranched optionally partially or fully halogenated, wherein one or more C atoms are optionally independently replaced by O, N or S(O)ₘ, and wherein said C₁₋₆ alkyl is optionally substituted with one to three C₃₋₆cycloalkyl, oxo, phenyl, dioxolanyl, pyrrolidinyl, furyl, isoxazolyl or isothiazolyl; each of the aforementioned being optionally substituted with one to three groups selected from halogen, 6₁₋₃ alkyl which is optionally partially or fully halogenated, hydroxy, nitrile and C₁₋₃alkoxy which is optionally partially or fully halogenated;
cyclopropyl, cyclobutyl, cyclopentanyl, cyclohexanyl, bicyclopentanyl or bicyclohexanyl, each being optionally partially or fully halogenated and optionally substituted with one to three C₁₋₃ alkyl groups optionally partially or fully halogenated, nitrile, hydroxyC₁₋₃alkyl or phenyl; or an analog of such cycloalkyl group wherein one to three ring methylene groups are independently replaced by O, S, CHOH, >C=O, >C=S or NH;
oxo;
C₃₋₆ alkynyl branched or unbranched carbon chain optionally partially or fully halogenated, wherein one or more methylene groups are optionally replaced by O, NH or S(O)ₘ and wherein said alkynyl group is optionally independently substituted with one to two oxo groups, hydroxy, pyrroldinyl, pyrrolyl, tetrahydropyranyl, C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
or
silyl containing three C₁₋₄ alkyl groups optionally partially or fully halogenated;
- R₂: is independently:
a C₁₋₅ branched or unbranched alkyl optionally partially or fully halogenated, acetyl, aroyl, C₁₋₄ branched or unbranched alkoxy, each being optionally partially or fully halogenated, halogen, methoxycarbonyl, C₁₋₂ alkyl-S(O)ₘ optionally partially or fully halogenated, or phenyl-S(O)ₘ;
C₁₋₃ alkoxy, hydroxy, nitrile, nitro, halogen;
or amino-S(O)ₘ- wherein the N atom is optionally independently mono- or di- substituted by C₁₋₃alkyl or arylC₀₋₃alkyl, or amino wherein the N atom is optionally independently mono- or di-substituted by C₁₋₃alkyl, arylC₀₋₃alkyl, C₁₋₃acyl, C₁₋₄alkyl-S(O)ₘ- or arylC₀₋₃alkyl-S(O)ₘ-, each of the aforementioned alkyl and aryl in this subparagraph are optionally partially or fully halogenated and optionally substituted with one to two C₁₋₃ alkyl or C₁₋₃ alkoxy;
- R₃ is: independently:
phenyl, morpholino, pyridinyl, pyrimidinyl, pyrazinyl, pyrrolyl, pyrrolidinyl, imidazolyl, [1,3,4]oxadiazol, pyrazolyl, each is optionally substituted with one to three phenyl, naphthyl, heterocycle or heteroaryl as hereinabove described in this paragraph, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, oxo, hydroxy, nitrile, C₁₋₃ alkoxy optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), a mono- or di-(C₁₋₃alkyl)aminocarbonyl, C₁₋₅ alkyl-C(O)-C₁₋₄ alkyl, mono- or di- (C₁₋₃alkyl)amino, mono- or di-(C₁₋₃)alkylamino-C₁₋₅ alkyl, mono- or di- (C₁₋₃alkyl)amino-S(O)₂, R₇-C₁₋₅ alkyl, R₈-C₁₋₅ alkoxy, R₉-C(O)-C₁₋₅ alkyl, R₁₀- C₁₋₅ alkyl(R₁₁)N, carboxy-mono- or di-(C₁₋₅)-alkyl-amino;
C₁₋₃ alkyl or C₁₋₄ alkoxy each being optionally partially or fully halogenated or optionally substituted with R₁₇;
OR₁₈ or C₁₋₆ alkyl optionally substituted with OR₁₈;
amino or mono- or di- (C₁₋₅ alkyl)amino optionally substituted with R₁₉;
R₂₀C(O)N(R₂₁)-, R₂₂O- R₂₃R₂₄NC(O)-; R₂₆CH₂C(O)N(R₂₁)-, R₂₃R₂₄NC(O)-C₁₋₂alkoxy or R₂₆C(O)CH₂N(R₂₁)-;
C₂₋₄alkenyl substituted by R₂₃R₂₄NC(O)-; or
C₂₋₄ alkynyl branched or unbranched carbon chain optionally partially or fully halogenated wherein one of the methylene groups is optionally replaced by O, and optionally independently substituted with one to two oxo groups, pyrroldinyl, pyrrolyl, morpholino, piperidinyl, piperazinyl, imidazolyl, phenyl, pyridinyl, tetrazolyl or one or more C₁₋₄ alkyl optionally substituted by one or more halogen atoms;
C₁₋₃acyl; and
- R₂₃ and R₂₄: taken together optionally form imidazolyl, piperidinyl, morpholino, piperazinyl or a pyridinyl ring.

In yet another preferred embodiment the invention relates to pharmaceutical compositions comprising **1** and **2d, characterized in that** the p38 kinase inhibitor **2d** is selected from the following compounds of formula **(VII)** wherein:
- G: is phenyl, pyridinyl, pyridonyl, naphthyl, quinolinyl, isoquinolinyl, pyrazinyl, 3,4- dihydro-2H-benzo[1,4]oxazinyl, benzothiophenyl, dihydrobenzofuranyl, dihydrobenzothiophenyl, benzooxazolyl, indanyl, indolyl, indolinyl, indolonyl or indolinonyl, wherein G is optionally substituted by one or more R₁, R₂ or R₃;
- Ar: is naphthyl;
- X: is phenyl, imidazolyl, pyridinyl, pyrimidinyl, piperdinyl, piperazinyl, pyridazinyl or pyrazinyl each being optionally independently substituted with one to three C₁₋₄ alkyl, C₁₋₄alkoxy, hydroxy, nitrile, amino, mono- or di-(C₁₋₃ alkyl)amino, mono- or di-(C₁-₃ alkylamino)carbonyl, NH₂C(O), C₁₋₆ alkyl-S(O)ₘ or halogen;
- Y: is: a bond or
a C₁₋₄ saturated carbon chain wherein one or more of the C atoms is optionally replaced by O, N or S and wherein Y is optionally independently substituted with nitrile or oxo;
- Z is:: phenyl, pyridinyl, pyrimidinyl, pyridazinyl, pyrazinyl, imidazolyl, dihydrothiazolyl, dihydrothiazolyl sulfoxide, pyranyl, pyrrolidinyl, phenylpiperazinyl, tetrahydropyranyl, tetrahydrofuranyl, dioxolanyl, 2-oxa-5-aza- bicyclo[2.2.1]heptanyl, morpholino, thiomorpholino, thiomorpholino sulfoxidyl, piperidinyl, piperidinonyl, piperazinyl or tetrahydropyrimidonyl each of which are optionally substituted with one to two C₁₋₂ alkyl or C₁₋₂ alkoxy;
or Z is hydroxy, C₁₋₃ alkyl, C₁₋₃ alkoxy, C₁₋₃ acylamino, C₁₋₃ alkylsulfonyl, nitrile C₁₋₃ alkyl or amino mono or di-substituted by C₁₋₃ alkoxyC₁₋₃alkyl;
- each R₁: is independently: C₁₋₅ alkyl branched or unbranched optionally partially or fully halogenated, wherein one or more C atoms are optionally independently replaced by O, N or S(O)ₘ, and wherein said C₁₋₅ alkyl is optionally substituted with oxo, dioxolanyl, pyrrolidinyl, furyl or phenyl each optionally substituted with one to three halogen, C₁₋₃ alkyl which is optionally partially or fully halogenated, hydroxy, nitrile and C₁- ₃alkoxy which is optionally partially or fully halogenated;
cyclopropyl, cyclobutyl, cyclopentanyl, cyclohexanyl, bicyclopentanyl or bicyclohexanyl, each being optionally partially or fully halogenated and optionally substituted with one to three C₁₋₃ alkyl groups optionally partially or fully halogenated, nitrile, hydroxyC₁₋₃alkyl or phenyl; and an analog of cyclopropyl, cyclobutyl, cyclopentanyl, cyclohexanyl, bicyclopentanyl or bicyclohexanyl wherein one ring methylene group is replaced by O;
oxo;
C₂₋₄ alkynyl optionally partially or fully halogenated wherein one or more methylene groups are optionally replaced by O, and optionally independently substituted with one to two oxo groups, hydroxy, pyrroldinyl, pyrrolyl, tetrahydropyranyl, C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
or
silyl containing three C₁₋₂ alkyl groups optionally partially or fully halogenated;
- each R₂: is independently:
a C₁₋₄ alkyl optionally partially or fully halogenated, C₁₋₄ alkoxy optionally partially or fully halogenated, bromo, chloro, fluoro, methoxycarbonyl, methyl-S(O)ₘ, ethyl-S(O)ₘ each optionally partially or fully halogenated or phenyl-S(O)ₘ; or R₂ is mono- or di-C₁₋₃acylamino, amino-S(O)ₘ or S(O)ₘamino wherein the N atom is mono- or di-substituted by C₁₋₃alkyl or phenyl, nitrile, nitro or amino;
- each R₃: is independently:
phenyl, morpholino, pyridinyl, pyrimidinyl, pyrrolidinyl, 2,5-pyrrolidin-dionyl, imidazolyl, [1,3,4]oxadiazol, pyrazolyl, each of the aforementioned is optionally substituted with one to three C₁₋₃ alkyl which is optionally partially or fully halogenated, halogen, oxo, hydroxy, nitrile and C₁₋₃ alkoxy optionally partially or fully halogenated;
C₁₋₃ alkyl or C₁₋₃ alkoxy optionally partially or fully halogenated or optionally substituted with R₁₇;
OR₁₈ or C₁₋₃ alkyl optionally substituted with OR₁₈; amino or mono- or di-(C₁₋₃ alkyl)amino optionally substituted with R₁₉;
R₂₀C(O)N(R₂₁)-, R₂₂O- ; R₂₃R₂₄NC(O)-; R₂₆CH₂C(O)N(R₂₁)-, NH₂C(O)methoxy or R₂₆C(O)CH₂N(R₂₁)-;
C₂₋₄ alkenyl substituted by R₂₃R₂₄NC(O)-; or
C₂₋₄ alkynyl substituted with pyrroldinyl or pyrrolyl;
C₁₋₃acyl and
- R₂₃ and R₂₄: taken together optionally form morpholino.

In another preferred embodiment the invention relates to pharmaceutical compositions comprising **1** and **2d, characterized in that** the p38 kinase inhibitor **2d** is selected from the following compounds of formula **(VII)** wherein:
- G: is phenyl, pyridinyl, pyridonyl, 2-naphthyl, quinolinyl, isoquinolinyl, dihydrobenzofuranyl, indanyl, 5-indolyl, 3-oxo-3,4-dihydro-2H-benzo[1,4]oxazin- 8-yl, benzooxalolyl, 2,3-dihydrobenzooxazol-7-yl, 2-oxo-2,3-dihydro-1H-indol-5- yl, indolinyl, indolonyl, or indolinonyl , wherein G is optionally substituted by one or more R₁, R₂ or R₃;
- Ar: is 1-naphthyl;
- X is:: phenyl, imidazolyl, pyridinyl, pyrimidinyl, piperdinyl, piperazinyl, pyridazinyl or pyrazinyl;
- Y is:: a bond or
-CH₂-, -CH₂CH₂-, -C(O)-, -O-, -S-, -NH-CH₂CH₂CH₂- , -N(CH₃)-, CH₂(CN)CH₂-NH-CH₂ or -NH-;
- Z: is morpholino, dioxolanyl, tetrahydrofuranyl, pyridinyl, 2-oxa-5-aza- bicyclo[2.2.1]heptanyl, C₁₋₃alkoxyphenylpiperazinyl, hydroxy, C₁₋₃alkyl, N,N-diC₁₋₃alkoxyC₁₋₃alkylamino, C₁₋₃acylamino, C₁₋₃alkylsulfonyl or nitrileC₁₋₃alkyl;
- each R₁: is independently:
C₁₋₅ alkyl optionally partially or fully halogenated wherein one or more C atoms are optionally independently replaced by O or N, and wherein said C₁₋₅ alkyl is optionally substituted with oxo, dioxolanyl, pyrrolidinyl, furyl or phenyl optionally substituted by C₁₋₃alkoxy;
cyclopropyl, cyclopentanyl, cyclohexanyl and bicyclopentanyl optionally substituted with one to three methyl groups optionally partially or fully halogenated, nitrile, hydroxymethyl or phenyl; or 2-tetrahydrofuranyl substituted by methyl; or trimethyl silyl;
propynyl substituted hydroxy or tetrahydropyran-2-yloxy;
- R₂: is is mono- or di-C₁₋₃acylamino, amino-S(O)ₘ or S(O)ₘ amino wherein the N atom is mono- or di-substituted by C₁₋₃alkyl or phenyl, bromo, chloro, fluoro, nitrile, nitro, amino, _methylsulfonyl optionally partially or fully halogenated or phenylsulfonyl;
- each R₃: is independently:
phenyl, morpholino, pyridinyl, pyrimidinyl, pyrrolidinyl, 2,5-pyrrolidin-dionyl, imidazolyl, [1,3,4]oxadiazol or pyrazolyl, each is optionally substituted with C₁₋₂ alkyl which is optionally partially or fully halogenated;
C₁₋₃ alkyl or C₁₋₃ alkoxy each being optionally partially or fully halogenated or optionally substituted with diethylamino;
OR₁₈ or C₁₋₃ alkyl optionally substituted with OR₁₈;
amino or mono- or di-(C₁₋₃ alkyl)amino optionally substituted with R₁₉;
CH₃C(O)NH-, R₂₂O- ; R₂₃R₂₄NC(O)-; R₂₆CH₂C(O)N(R₂₁)-, NH₂C(O)methoxy or R₂₆C(O)CH₂N(R₂₁)-;
C₂₋₄alkenyl substituted by R₂₃R₂₄NC(O)-; or
C₂₋₄ alkynyl substituted with pyrroldinyl or pyrrolyl;
C₁₋₂acyl; and
- R₂₃ and R₂₄: are H or R₂₃ and R₂₄ taken together optionally form morpholino; and
- R₂₆: is morpholino.

In another preferred embodiment the invention relates to pharmaceutical compositions comprising **1** and **2d, characterized in that** the p38 kinase inhibitor **2d** is selected from the following compounds of formula **(VII)** wherein:
- G is: phenyl, pyridinyl, 5-indolyl, 3-oxo-3,4-dihydro-2H-benzo[1,4]oxazin-8-yl, benzooxalolyl, 2,3-dihydrobenzooxazol-7-yl, 2-oxo-2,3-dihydro-1 H-indol-5-yl or 2- naphthyl wherein G is optionally substituted by one or more R₁, R₂ or R₃;
- X is:: imidazolyl, pyridinyl, pyrimidinyl or pyrazinyl;
- Y is:: a bond, CH₂(CN)CH₂-NH-CH₂, -CH₂-, -NH-CH₂CH₂CH₂- or -NH-;
- Z: is morpholin-4yl, dioxolan-2yl, tetrahydrofuranyl, pyridinyl, 2-oxa-5-aza- bicyclo[2.2.1]hept-5yl, methoxyphenylpiperazinyl, hydroxy, methyl, N,N- dimethoxyethylamino, acetylamino, methylsulfonyl or cyanoethyl;
- each R₁: is independently:
tert-butyl, sec-butyl, tert-amyl, phenyl, tetrahydropyran-2-yloxypropynyl, hydroxypropynyl, trihalomethyl, 2,2-diethylpropionyl or cyclohexanyl;
- R₂: is chloro, nitro, amino, nitrile, methylsulfonylamino, diacetylamino, phenylsulfonylamino, N,N-di(methylsulfonyl)amino, methylsulfonyl or trihalomethylsulfonyl;
- R₃: is independently:
methyl, C₁₋₃ alkoxy, methoxymethyl, hydroxypropyl, dimethylamino, C₁₋₄alkylamino, NH₂C(O)methoxy, acetyl, pyrrolidinyl, imidazolyl, pyrazolyl, morpholino or morpholinocarbonyl.

In yet another preferred embodiment the invention relates to pharmaceutical compositions comprising **1** and **2d, characterized in that** the p38 kinase inhibitor **2d** is selected from the following compounds of formula **(VII)** wherein X is pyridinyl.

In still another preferred embodiment the invention relates to pharmaceutical compositions comprising **1** and **2d, characterized in that** the p38 kinase inhibitor **2d** is selected from the following compounds of formula **(VII)** wherein the pyridinyl is attached to Ar via the 3-pyridinyl position.

Preferably the invention relates to pharmaceutical compositions comprising 1 and **2d, characterized in that** the p38 kinase inhibitor **2d** is selected from the following compounds of formula **(VII):**
1-(4-tert-butyl-phenyl)-3-[4-(6-morpholin-4-ylmethyl-pyridin-3-yl)-naphthalen-1-yl]-urea;
1-(5-tert-butyl-2-methyl-phenyl)-3-[4-(4-morpholin-4-ylmethyl-piperidin-1-yl)-naphthalen-1-yl]-urea;
1-(6-chloro-4-trifluoromethyl-pyridin-2-yl)-3-[4-(6-morpholin-4-ylmethyl-pyridin-3-yl)-naphthalen-1-yl]-urea;
1-(4-difluoromethoxy-phenyl)-3-[4-(6-morpholin-4-ylmethyl-pyridin-3-yl)-naphthalen-1-yl]-urea;
1-(3-methyl-naphthalen-2-yl)-3-[4-(6-morpholin-4-ylmethyl-pyridin-3-yl)-naphthalen-1-yl]-urea;
1-[2-methoxy-5-(1-methyl-1-phenyl-ethyl)-phenyl]-3-[4-(6-morpholin-4-ylmethyl-pyridin-3-yl)-naphthalen-1-yl]-urea;
(5-tert-butyl-2-methyl-phenyl)-carbamic acid 3-(5-{4-[3-(5-tert-butyl-2-methyl-phenyl)-ureido]-naphthalen-1-yl}-pyridin-2-ylamino)-propyl ester;
1-(6-tert-butyl-benzo[1,3]dioxol-5-yl)-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-acetamide;
1,3-bis-[4-(6-morpholin-4-ylmethyl-pyridin-3-yl)-naphthalen-1-yl]-urea;
1-[5-tert-butyl-3-(2,2-dimethyl-[1,3]dioxolan-4-ylmethyl)-2-hydroxy-phenyl]-3-[4-(6-morpholin-4-ylmethyl-pyridin-3-yl)-naphthalen-1-yl]-urea;
1-[5-tert-butyl-2-(2-pyrrolidin-1-yl-ethoxy)-phenyl]-3-[4-(6-morpholin-4-ylmethyl-pyridin-3-yl)-naphthalen-1-yl]-urea;
1-[5-tert-butyl-3-(2,3-dihydroxy-propyl)-2-hydroxy-phenyl]-3-[4-(6-morpholin-4-ylmethyl-pyridin-3-yl)-naphthalen-1-yl]-urea;
1-(2,3-dimethyl-1 H-indol-5-yl)-3-[4-(6-morpholin-4-ylmethyl-pyridin-3-yl)-naphthalen-1-yl]-urea;
1-[4-(6-morpholin-4-ylmethyl-pyridin-3-yl)-naphthalen-1-yl]-3-(2-p-tolyloxy-5-trifluoromethyl-phenyl)-urea;
1-[2-(2-methoxy-phenoxy)-5-trifluoromethyl-phenyl]-3-[4-(6-morpholin-4-ylmethyl-pyridin-3-yl)-naphthalen-1-yl]-urea;
1-[4-(6-morpholin-4-ylmethyl-pyhdin-3-yl)-naphthalen-1-yl]-3-naphthalen-1-yl-urea;
1-{5-tert-butyl-2-methyl-3-[3-(tetrahydro-pyran-2-yloxy)-prop-1-ynyl]-phenyl}-3-[4-(6-morpholin-4-ylmethyl-pyridin-3-yl)-naphthalen-1-yl]-urea;
1-{5-tert-butyl-2-[3-(tetrahydro-pyran-2-yloxy)-prop-1-ynyl]-phenyl}-3-[4-(6-morpholin-4-ylmethyl-pyridin-3-yl)-naphthalen-1-yl]-urea;
1-(5-hydroxymethyl-2-methyl-phenyl)-3-[4-(6-morpholin-4-ylmethyl-pyridin-3-yl)-naphthalen-1-yl]-urea;
1-(2-methoxy-dibenzofuran-3-yl)-3-[4-(6-morpholin-4-ylmethyl-pyridin-3-yl)-naphthalen-1-yl]-urea;
1-(2,5-di-tert-butyl-phenyl)-3-[4-(6-morpholin-4-ylmethyl-pyridin-3-yl)-naphthalen-1-yl]-urea;
1-[3-(4-bromo-1-methyl-1H-pyrazol-3-yl)-phenyl]-3-[4-(6-morpholin-4-ylmethyl-pyridin-3-yl)-naphthalen-1-yl]-urea;
1-(3-hydroxy-5,6,7,8-tetrahydro-naphthalen-2-yl)-3-[4-(6-morpholin-4-ylmethyl-pyridin-3-yl)-naphthalen-1-yl]-urea;
1-(1-acetyl-2,3-dihydro-1H-indol-5-yl)-3-[4-(6-morpholin-4-ylmethyl-pyridin-3-yl)-naphthalen-1-yl]-urea;
1-[4-(6-morpholin-4-ylmethyl-pyridin-3-yl)-naphthalen-1-yl]-3-(3-oxazol-5-yl-phenyl)-urea;
1-[4-(6-morpholin-4-ylmethyl-pyridin-3-yl)-naphthalen-1-yl]-3-(3-[1,3,4]oxadiazol-2-yl-phenyl)-urea;
1-(2-methoxy-5-trifluoromethyl-phenyl)-3-[4-(6-morpholin-4-ylmethyl-pyridin-3-yl)-naphthalen-1-yl]-urea;
furan-2-carboxylic acid (4-tert-butyl-2-{3-[4-(6-morpholin-4-ylmethyl-pyridin-3-yl)-naphthalen-1-yl]-ureido}-phenyl)-amide;
1-(2-methoxy-4-phenylamino-phenyl)-3-[4-(6-morpholin-4-ylmethyl-pyridin-3-yl)-naphthalen-1-yl]-urea;
1-(5-methoxy-2-methyl-phenyl)-3-[4-(6-morpholin-4-ylmethyl-pyridin-3-yl)-naphthalen-1-yl]-urea;
1-(3-hydroxy-naphthalen-2-yl)-3-[4-(6-morpholin-4-ylmethyl-pyridin-3-yl)-naphthalen-1-yl]-urea;
N,N-diethyl-4-methoxy-3-{3-[4-(6-morpholin-4-ylmethyl-pyridin-3-yl)-naphthalen-1-yl]-ureido}-benzenesulfonamide;
1-(2,2-difluoro-benzo[1,3]dioxol-5-yl)-3-[4-(6-morphol in-4-ylmethyl-pyridin-3-yl)-naphthalen-1-yl]-urea;
1-[5-(1,1 -dimethyl-propyl)-2-phenoxy-phenyl]-3-[4-(6-morpholin-4-ylmethyl-pyridin-3-yl)-naphthalen-1-yl]-urea;
1-[5-(2,2-dimethyl-propionyl)-2-methyl-phenyl]-3-[4-(6-morpholin-4-ylmethyl-pyridin-3-yl)-naphthalen-1-yl]-urea;
2-chloro-5-{3-[4-(6-morpholin-4-ylmethyl-pyridin-3-yl)-naphthalen-1-yl]-ureido}-benzoic acid isopropyl ester;
1-(4-amino-3,5-dibromo-phenyl)-3-[4-(6-morpholin-4-ylmethyl-pyridin-3-yl)-naphthalen-1-yl]-urea;
1-[5-tert-butyl-3-(3-hydroxy-prop-1-ynyl)-2-methyl-phenyl]-3-[4-(6-morpholin-4-ylmethyl-pyridin-3-yl)-naphthalen-1-yl]-urea;
1-[5-tert-butyl-2-(3-hydroxy-prop-1-ynyl)-phenyl]-3-[4-(6-morpholin-4-ylmethyl-pyridin-3-yl)-naphthalen-1-yl]-urea;
1-[5-tert-butyl-3-(2,2-dimethyl-[1,3]dioxolan-4-ylmethyl)-2-methoxy-phenyl]-3-[4-(6-morpholin-4-ylmethyl-pyridin-3-yl)-naphthalen-1-yl]-urea;
1-[5-tert-butyl-3-(2,3-dihydroxy-propyl)-2-methoxy-phenyl]-3-[4-(6-morpholin-4-ylmethyl-pyridin-3-yl)-naphthalen-1-yl]-urea;
1-(5-tert-butoxy-2-methoxy-phenyl)-3-[4-(6-morpholin-4-ylmethyl-pyridin-3-yl)-naphthalen-1-yl]-urea;
1-[5-(1-cyano-cyclopropyl)-2-methoxy-phenyl]-3-[4-(6-morpholin-4-ylmethyl-pyridin-3-yl)-naphthalen-1-yl]-urea;
1-[5-tert-butyl-3-(2-diethylamino-ethyl)-2-methoxy-phenyl]-3-[4-(6-morpholin-4-ylmethyl-pyridin-3-yl)-naphthalen-1-yl]-urea;
1-(5-tert-butyl-2-methoxy-phenyl)-3-[4-(6-[1,3]dioxolan-2-yl-pyridin-3-yl)-naphthalen-1-yl]-urea;
1-(5-tert-butyl-2-pyrrolidin-1-yl-phenyl)-3-[4-(6-morpholin-4-ylmethyl-pyridin-3-yl)-naphthalen-1-yl]-urea;
1-(5-tert-butyl-2-dimethylamino-phenyl)-3-[4-(6-morpholin-4-ylmethyl-pyridin-3-yl)-naphthalen-1-yl]-urea;
1-(5-tert-butyl-2-propoxy-phenyl)-3-[4-(6-morpholin-4-ylmethyl-pyridin-3-yl)-naphthalen-1-yl]-urea;
1-(5-tert-butyl-2-methoxy-phenyl)-3-[4-(6-hydroxymethyl-pyridin-3-yl)-naphthalen-1-yl]-urea;
1-(5-tert-butyl-2-methoxy-phenyl)-3-{4-[6-(2,6-dimethyl-morpholin-4-ylmethyl)-pyridin-3-yl]-naphthalen-1-yl}-urea;
2-(5-tert-butyl-2-methoxy-phenyl)-N-[4-(6-morpholin-4-ylmethyl-pyridin-3-yl)-naphthalen-1-yl]-acetamide;
1-(2-methoxy-5-phenoxy-phenyl)-3-[4-(6-morpholin-4-ylmethyl-pyridin-3-yl)-naphthalen-1-yl]-urea;
1-(3,3-dimethyl-2-oxo-2,3-dihydro-1H-indol-7-yl)-3-[4-(6-morpholin-4-ylmethyl-pyridin-3-yl)-naphthalen-1-yl]-urea;
1-(5-tert-butyl-2-cyclopentyloxy-phenyl)-3-[4-(6-morpholin-4-ylmethyl-pyridin-3-yl)-naphthalen-1-yl]-urea;
1-(5-tert-butyl-2-methoxy-phenyl)-3-{4-[6-(3-pyridin-3-yl-pyrrolidin-1-ylmethyl)-pyridin-3-yl]-naphthalen-1-yl}-urea;
1-(5-cyclohexyl-2-methoxy-phenyl)-3-[4-(6-morpholin-4-ylmethyl-pyridin-3-yl)-naphthalen-1-yl]-urea;
1-(2,4-dimethoxy-5-trifluoromethyl-phenyl)-3-[4-(6-morpholin-4-ylmethyl-pyridin-3-yl)-naphthalen-1-yl]-urea;
1-(6-tert-butyl-3-oxo-3,4-dihydro-2H-benzo[1,4]oxazin-7-yl)-3-[4-(6-morpholin-4-ylmethyl-pyridin-3-yl)-naphthalen-1-yl]-urea;
1-(5-tert-butyl-2-methoxy-3-nitro-phenyl)-3-[4-(6-morpholin-4-ylmethyl-pyridin-3-yl)-naphthalen-1-yl]-urea;
1-(3-amino-5-tert-butyl-2-methoxy-phenyl)-3-[4-(6-methyl-pyridin-3-yl)-naphthalen-1-yl]-urea;
N-acetyl-N-(5-tert-butyl-2-methoxy-3-{3-[4-(6-morpholin-4-ylmethyl-pyridin-3-yl)-naphthalen-1-yl]-ureido}-phenyl)-acetamide;
1-(6-tert-butyl-4-methyl-3-oxo-3,4-dihydro-2H-benzo[1,4]oxazin-8-yl)-3-[4-(6-morpholin-4-ylmethyl-pyridin-3-yl)-naphthalen-1-yl]-urea;
1-[6-tert-butyl-4-(2-morpholin-4-yl-ethyl)-3-oxo-3,4-dihydro-2H-benzo[1,4]oxazin-8-yl]-3-[4-(6-morpholin-4-ylmethyl-pyridin-3-yl)-naphthalen-1-yl]-urea;
1-(5-tert-butyl-2-ethoxy-phenyl)-3-[4-(6-morpholin-4-ylmethyl-pyridin-3-yl)-naphthalen-1-yl]-urea;
1-(5-tert-butyl-2-isopropoxy-phenyl)-3-[4-(6-morpholin-4-ylmethyl-pyridin-3-yl)-naphthalen-1-yl]-urea;
1-(5-tert-butyl-2-imidazol-1-yl-phenyl)-3-[4-(6-morpholin-4-ylmethyl-pyridin-3-yl)-naphthalen-1-yl]-urea;
N-(5-tert-butyl-2-methoxy-4-{3-[4-(6-morpholin-4-ylmethyl-pyridin-3-yl)-naphthalen-1-yl]-ureido}-phenyl)-methanesulfonamide;
1-(5-tert-butyl-3-ethylamino-2-methoxy-phenyl)-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-bis(methanesulfon)amide;
1-[5-tert-butyl-2-(1-methyl-1H-pyrazol-4-yl)-phenyl]-3-[4-(6-morpholin-4-ylmethyl-pyridin-3-yl)-naphthalen-1-yl]-urea;
1-(2-methanesulfinyl-5-trifluoromethyl-phenyl)-3-[4-(6-morpholin-4-ylmethyl-pyridin-3-yl)-naphthalen-1-yl]-urea;
1-(2-ethanesulfonyl-5-trifluoromethyl-phenyl)-3-[4-(6-morpholin-4-ylmethyl-pyridin-3-yl)-naphthalen-1-yl]-urea;
1-[4-(6-{[bis-(2-methoxy-ethyl)-amino]-methyl}-pyridin-3-yl)-naphthalen-1-yl]-3-(5-tert-butyl-2-methoxy-phenyl)-urea;
1-(5-tert-butyl-2-methoxy-phenyl)-3-{4-[6-(3-dimethylamino-pyrrolidin-1-ylmethyl)-pyridin-3-yl]-naphthalen-1-yl}-urea;
N-[1-(5-{4-[3-(5-tert-butyl-2-methoxy-phenyl)-ureido]-naphthalen-1-yl}-pyridin-2-ylmethyl)-pyrrolidin-3-yl]-acetamide;
1-(1-acetyl-3,3-dimethyl-2,3-dihydro-1H-indol-5-yl)-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-propionamide;
1-(5-tert-butyl-2-methyl-benzooxazol-7-yl)-3-[4-(6-morpholin-4-ylmethyl-pyridin-3-yl)-naphthalen-1-yl]-urea;
1-[4-(6-morpholin-4-ylmethyl-pyridin-3-yl)-naphthalen-1-yl]-3-(3-trifluoromethanesulfonyl-phenyl)-urea;
N-(5-tert-butyl-2-methoxy-3-{3-[4-(6-morpholin-4-ylmethyl-pyridin-3-yl)-naphthalen-1-yl]-ureido}-phenyl)-isobutyramide;
2-(4-tert-butyl-2-{3-[4-(6-morpholin-4-ylmethyl-pyridin-3-yl)-naphthalen-1-yl]-ureido}-phenoxy)-acetamide;
1-(5-tert-butyl-2-oxo-2,3-dihydro-benzooxazol-7-yl)-3-[4-(6-morpholin-4-ylmethyl-pyridin-3-yl)-naphthalen-1-yl]-urea;
1-(6-tert-butyl-3-cyano-2-methoxymethoxy-pyridin-4-yl)-3-[4-(6-morpholin-4-ylmethyl-pyridin-3-yl)-naphthalen-1-yl]-urea;
1-(6-tert-butyl-3-cyano-2-hydroxy-pyridin-4-yl)-3-[4-(6-morpholin-4-ylmethyl-pyridin-3-yl)-naphthalen-1-yl]-urea;
1-(5-tert-butyl-3-cyano-2-methoxy-phenyl)-3-[4-(6-morpholin-4-ylmethyl-pyridin-3-yl)-naphthalen-1-yl]-urea;
1-[4-(6-morpholin-4-ylmethyl-pyhdin-3-yl)-naphthalen-1-yl]-3-(1,3,3-thmethyl-2,3-dihydro-1H-indol-5-yl)-urea;
1-(5-tert-butyl-benzooxazol-7-yl)-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-benzenesulfonamide;
ethanesulfonicacid(5-tert-butyl-2-methoxy-3-{3-[4-(6-morpholin-4-ylmethyl-pyridin-3-yl)-naphthalen-1-yl]-ureido}-phenyl)-amide;
1-(5-tert-butyl-2-methoxy-phenyl)-3-[4-(4-morpholin-4-ylmethyl-piperidin-1-yl)-naphthalen-1-yl]-urea;
1-[5-tert-butyl-2-(1-methyl-1H-pyrazol-4-yl)-phenyl]-3-[4-(4-morpholin-4-ylmethyl-piperidin-1-yl)-naphthalen-1-yl]-urea;
1-(5-tert-butyl-2-methoxy-phenyl)-3-[4-(2-morpholin-4-ylmethyl-pyrimidin-5-yl)-naphthalen-1-yl]-urea;
1-(5-tert-butyl-2-methylsulfanyl-phenyl)-3-[4-(6-morpholin-4-ylmethyl-pyridin-3-yl)-naphthalen-1-yl]-urea;
1-(5-tert-butyl-2-methoxy-pyridin-3-yl)-3-[4-(6-morpholin-4-ylmethyl-pyridin-3-yl)-naphthalen-1-yl]-urea;
2,2,2-trifluoro-ethanesulfonicacid(5-tert-butyl-2-methoxy-3-{3-[4-(6-morpholin-4-ylmethyl-pyridin-3-yl)-naphthalen-1-yl]-ureido}-phenyl)-amide;
N-(5-(4-[3-(5-tert-butyl-2-methyl-phenyl)-ureido]-naphthalen-1-yl)-pyrazin-2-yl)-methanesulfonamide;
1-[4-(6-{[bis-(2-cyano-ethyl)-amino]-methyl}-pyridin-3-yl)-naphthalen-1-yl]-3-(5-tert-butyl-2-methoxy-phenyl)-urea;
1-(5-tert-butyl-2-methoxy-phenyl)-3-{4-[6-(4-methyl-piperazin-1-ylmethyl)-pyridin-3-yl]-naphthalen-1-yl}-urea;
1-(5-tert-butyl-2-methoxy-phenyl)-3-[4-(6-thiomorpholin-4-ylmethyl-pyridin-3-yl)-naphthalen-1-yl]-urea;
1-(5-tert-butyl-2-methoxy-phenyl)-3-{4-[6-(2,6-dimethyl-piperidin-1-ylmethyl)-pyridin-3-yl]-naphthalen-1-yl}-urea;
1-(5-tert-butyl-2-methoxy-phenyl)-3-{4-[6-(1-oxo-tetrahydro-thiopyran-4-ylamino)-pyridin-3-yl]-naphthalen-1-yl}-urea;
1-(5-tert-Butyl-2-methoxy-phenyl)-3-{4-[6-(tetrahydro-pyran-4-ylamino)-pyhdin-3-yl]-naphthalen-1-yl}-urea;
1-(5-tert-butyl-2-methoxy-phenyl)-3-[4-(6-{[(2-cyano-ethyl)-(tetrahydro-furan-2-ylmethyl)-amino]-methyl}-pyridin-3-yl)-naphthalen-1-yl]-urea;
1-(5-tert-butyl-2-methoxy-phenyl)-3-{4-[6-(2-methoxymethyl-morpholin-4-ylmethyl)-pyridin-3-yl]-naphthalen-1-yl}-urea;
1-(5-tert-butyl-2-methoxy-phenyl)-3-(4-{6-[(2-morpholin-4-yl-ethylamino)-methyl]-pyhdin-3-yl}-naphthalen-1-yl)-urea;
1-(5-tert-butyl-2-methoxy-phenyl)-3-{4-[6-(2-methyl-3-oxo-piperazin-1-ylmethyl)-pyridin-3-yl]-naphthalen-1-yl}-urea;
1-(5-(4-[3-(5-tert-butyl-2-methoxy-phenyl)-ureido]-naphthalen--yl)-pyridin-2-ylmethyl)-piperidine-3-carboxylicacidamide;
1-(5-(4-[3-(5-tert-butyl-2-methoxy-phenyl)-ureido]-naphthalen-1-yl)-pyridin-2-ylmethyl)-piperidine-4-carboxylicacidamide;
1-(5-tert-butyl-2-methoxy-phenyl)-3-{4-[6-(1-oxo-1l4-thiomorpholin-4-ylmethyl)-pyridin-3-yl]-naphthalen-1-yl}-urea;
1-(3,3-dimethyl-2-oxo-2,3-dihydro-1H-indol-5-yl)-3-[4-(6-morpholin-4-ylmethyl-pyridin-3-yl)-naphthalen-1-yl]-urea;
1-(5-tert-butyl-2-methoxy-phenyl)-3-{4-[6-(3-oxo-piperazin-1-ylmethyl)-pyridin-3-yl]-naphthalen-1-yl}-urea;
1-{4-[6-(4-acetyl-piperazin-1-ylmethyl)-pyridin-3-yl]-naphthalen-1-yl}-3-(5-tert-butyl-2-methoxy-phenyl)-urea;
4-(5-{4-[3-(5-tert-butyl-2-methoxy-phenyl)-ureido]-naphthalen-1-yl}-pyridin-2-ylmethyl)-piperazine-1-carboxylicacidethylester;
1-(5-tert-butyl-2-methoxy-phenyl)-3-(4-{6-[(2-pyridin-3-yl-ethylamino)-methyl]-pyridin-3-yl}-naphthalen-1-yl)-urea;
1-(5-tert-butyl-2-methoxy-phenyl)-3-(4-{6-[(tetrahydro-furan-3-ylamino)-methyl]-pyridin-3-yl}-naphthalen-1-yl)-urea;
1-(5-tert-butyl-2-methoxy-phenyl)-3-[4-(6-{[(2-cyano-ethyl)-pyridin-3-ylmethyl-amino]-methyl}-pyridin-3-yl)-naphthalen-1-yl]-urea;
1-(5-tert-butyl-2-methoxy-phenyl)-3-(4-{6-[(2-methylsulfanyl-ethylamino)-methyl]-pyridin-3-yl}-naphthalen-1-yl)-urea;
1-(5-tert-butyl-2-methoxy-phenyl)-3-{4-[6-(2-oxa-5-aza-bicyclo[2.2.1]hept-5-ylmethyl)-pyridin-3-yl]-naphthalen-1-yl}-urea;
1-(5-tert-butyl-2-methoxy-phenyl)-3-{4-[6-(2,6-dimethyl-morpholin-4-ylmethyl)-pyridin-3-yl]-naphthalen-1-yl}-urea;
1-(5-tert-butyl-2-methoxy-phenyl)-3-(4-{6-[(2-piperazin-1-yl-ethylamino)-methyl]-pyridin-3-yl}-naphthalen-1-yl)-urea;
1-(5-tert-butyl-2-methoxy-phenyl)-3-{4-[6-(4-pyrimidin-2-yl-piperazin-1-ylmethyl)-pyridin-3-yl]-naphthalen-1-yl}-urea;
1-(5-tert-butyl-2-methoxy-phenyl)-3-{4-[6-(4-pyridin-2-yl-piperazin-1-ylmethyl)-pyridin-3-yl]-naphthalen-1-yl}-urea;
1-(5-tert-butyl-2-methoxy-phenyl)-3-(4-{6-[4-(3-methoxy-phenyl)-piperazin-1-ymethyl]-pyhdin-3-yl}-naphthalen-1-yl)-urea;
1-(5-tert-butyl-2-methoxy-phenyl)-3-{4-[6-(morpholine-4-carbonyl)-pyridin-3-yl]-naphthalen-1-yl}-urea;
1-(5-tert-butyl-2-methoxy-phenyl)-3-{4-[6-(2-thia-5-aza-bicyclo[2.2.1]hept-5-ylmethyl)-pyridin-3-yl]-naphthalen-1-yl}-urea;
1-(5-tert-butyl-2-methoxy-phenyl)-3-[4-(5-morpholin-4-ylmethyl-pyrazin-2-yl)-naphthalen-1-yl]-urea;
1-(6-tert-butyl-3-oxo-3,4-dihydro-2H-benzo[1,4]oxazin-8-yl)-3-[4-(6-morpholin-4-ylmethyl-pyridin-3-yl)-naphthalen-1-yl]-urea;
1-(3-amino-5-tert-butyl-2-methoxy-phenyl)-3-[4-(6-morpholin-4-ylmethyl-pyridin-3-yl)-naphthalen-1-yl]-urea;
N-(5-{4-[3-(5-tert-butyl-2-methoxy-phenyl)-ureido]-naphthalen-1-yl}-pyridin-2-yl)-acetamide;
N-(5-tert-butyl-2-methoxy-3-{3-[4-(6-morpholin-4-ylmethyl-pyridin-3-yl)-naphthalen-1-yl]-ureido}-phenyl)-N-methyl-acetamide;
N-(5-tert-butyl-2-methoxy-3-{3-[4-(6-morpholin-4-ylmethyl-pyridin-3-yl)-naphthalen-1-yl]-ureido}-phenyl)-2,2,2-trifluoro-acetamide;
1-(5-tert-butyl-2-methoxy-phenyl)-3-{4-[6-(pyridin-3-yloxy)-pyridin-3-yl]-naphthalen-1-yl}-urea;
1-(5-tert-butyl-2-methoxy-phenyl)-3-{4-[6-(pyridin-3-ylamino)-pyhdin-3-yl]-naphthalen-1-yl}-urea;
[4-(6-morpholin-4-ylmethyl-pyridin-3-yl)-naphthalen-1-yl]-carbamicacid3-tert-butylphenylester;
N-(5-tert-butyl-2-methoxy-3-{3-[4-(6-morpholin-4-ylmethyl-pyridin-3-yl)-naphthalen-1-yl]-ureido}-phenyl)-methanesulfonamideand
and the pharmaceutically acceptable derivatives thereof.

In another preferred embodiment the invention relates to pharmaceutical compositions comprising **1** and **2d, characterized in that** the p38 kinase inhibitor **2d** is selected from the following compounds of formula **(VII):**
1-(3-methyl-naphthalen-2-yl)-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-acetamide;
1-[5-tert-butyl-3-(2,3-dihydroxy-propyl)-2-hydroxy-phenyl]-3-[4-(6-morpholin-4-ylmethyl-pyridin-3-yl)-naphthalen-1-yl]-urea;
1-(2,3-dimethyl-1 H-indol-5-yl)-3-[4-(6-morpholin-4-ylmethyl-pyridin-3-yl)-naphthalen-1-yl]-urea;
1-{5-tert-butyl-2-methyl-3-[3-(tetrahydro-pyran-2-yloxy)-prop-1-ynyl]-phenyl}-3-[4-(6-morpholin-4-ylmethyl-pyridin-3-yl)-naphthalen-1-yl]-urea;
1-(2-methoxy-5-trifluoromethyl-phenyl)-3-[4-(6-morpholin-4-ylmethyl-pyridin-3-yl)-naphthalen-1-yl]-urea;
1-[5-(2,2-dimethyl-propionyl)-2-methyl-phenyl]-3-[4-(6-morpholin-4-ylmethyl-pyridin-3-yl)-naphthalen-1-yl]-urea;
1-[5-tert-butyl-3-(3-hydroxy-prop-1-ynyl)-2-methyl-phenyl]-3-[4-(6-morphol in-4-ylmethyl-pyridin-3-yl)-naphthalen-1-yl]-urea;
1-[5-tert-butyl-2-(3-hydroxy-prop-1-ynyl)-phenyl]-3-[4-(6-morpholin-4-ylmethyl-pyridin-3-yl)-naphthalen-1-yl]-urea;
1-[5-tert-butyl-3-(2,2-dimethyl-[1,3]dioxolan-4-ylmethyl)-2-methoxy-phenyl]-3-[4-(6-morpholin-4-ylmethyl-pyridin-3-yl)-naphthalen-1-yl]-urea;
1-[5-tert-butyl-3-(2,3-dihydroxy-propyl)-2-methoxy-phenyl]-3-[4-(6-morpholin-4-ylmethyl-pyridin-3-yl)-naphthalen-1-yl]-urea;
1-(5-tert-butoxy-2-methoxy-phenyl)-3-[4-(6-morpholin-4-ylmethyl-pyridin-3-yl)-naphthalen-1-yl]-urea;
1-[5-(1-cyano-cyclopropyl)-2-methoxy-phenyl]-3-[4-(6-morpholin-4-ylmethyl-pyridin-3-yl)-naphthalen-1-yl]-urea;
1-[5-tert-butyl-3-(2-diethylamino-ethyl)-2-methoxy-phenyl]-3-[4-(6-morpholin-4-ylmethyl-pyridin-3-yl)-naphthalen-1-yl]-urea;
1-(5-tert-butyl-2-methoxy-phenyl)-3-[4-(6-[1,3]dioxolan-2-yl-pyridin-3-yl)-naphthalen-1-yl]-urea;
1-(5-tert-butyl-2-pyrrolidin-1-yl-phenyl)-3-[4-(6-morpholin-4-ylmethyl-pyridin-3-yl)-naphthalen-1-yl]-urea;
1-(5-tert-butyl-2-dimethylamino-phenyl)-3-[4-(6-morpholin-4-ylmethyl-pyridin-3-yl)-naphthalen-1-yl]-urea;
1-(5-tert-butyl-2-propoxy-phenyl)-3-[4-(6-morpholin-4-ylmethyl-pyridin-3-yl)-naphthalen-1-yl]-urea;
1-(5-tert-butyl-2-methoxy-phenyl)-3-[4-(6-hydroxymethyl-pyridin-3-yl)-naphthalen-1-yl]-urea;
1-(5-tert-butyl-2-methoxy-phenyl)-3-{4-[6-(2,6-dimethyl-morpholin-4-ylmethyl)-pyridin-3-yl]-naphthalen-1-yl}-urea;
1-(5-cyclohexyl-2-methoxy-phenyl)-3-[4-(6-morphol in-4-ylmethyl-pyrid in-3-yl)-naphthalen-1-yl]-urea;
1-(2,4-dimethoxy-5-trifluoromethyl-phenyl)-3-[4-(6-morpholin-4-ylmethyl-pyridin-3-yl)-naphthalen-1-yl]-urea;
1-(5-tert-butyl-2-methoxy-3-nitro-phenyl)-3-[4-(6-morpholin-4-ylmethyl-pyridin-3-yl)-naphthalen-1-yl]-urea;
1-(3-amino-5-tert-butyl-2-methoxy-phenyl)-3-[4-(6-methyl-pyridin-3-yl)-naphthalen-1-yl]-urea;
N-acetyl-N-(5-tert-butyl-2-methoxy-3-{3-[4-(6-morphol in-4-ylmethyl-pyrid in-3-yl)-naphthalen-1-yl]-ureido}-phenyl)-acetamide;
1-(6-tert-butyl-4-methyl-3-oxo-3,4-dihydro-2H-benzo[1,4]oxazin-8-yl)-3-[4-(6-morpholin-4-ylmethyl-pyridin-3-yl)-naphthalen-1-yl]-urea;
1-(5-tert-butyl-2-ethoxy-phenyl)-3-[4-(6-morpholin-4-ylmethyl-pyridin-3-yl)-naphthalen-1-yl]-urea;
1-(5-tert-butyl-2-isopropoxy-phenyl)-3-[4-(6-morpholin-4-ylmethyl-pyridin-3-yl)-naphthalen-1-yl]-urea;
1-(5-tert-butyl-2-imidazol-1-yl-phenyl)-3-[4-(6-morphol in-4-ylmethyl-pyrid in-3-yl)-naphthalen-1-yl]-urea;
1-(5-tert-butyl-3-ethylamino-2-methoxy-phenyl)-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-bis(methanesulfon)amide;
1-[5-tert-butyl-2-(1-methyl-1H-pyrazol-4-yl)-phenyl]-3-[4-(6-morpholin-4-ylmethyl-pyridin-3-yl)-naphthalen-1-yl]-urea;
1-(2-methanesulfinyl-5-trifluoromethyl-phenyl)-3-[4-(6-morpholin-4-ylmethyl-pyridin-3-yl)-naphthalen-1-yl]-urea;
1-[4-(6-{[bis-(2-methoxy-ethyl)-amino]-methyl}-pyridin-3-yl)-naphthalen-1-yl]-3-(5-tert-butyl-2-methoxy-phenyl)-urea;
N-[1-(5-{4-[3-(5-tert-butyl-2-methoxy-phenyl)-ureido]-naphthalen-1-yl}-pyridin-2-ylmethyl)-pyrrolidin-3-yl]-acetamide;
1-(1-acetyl-3,3-dimethyl-2,3-dihydro-1H-indol-5-yl)-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-propionamide;
1-(5-tert-butyl-2-methyl-benzooxazol-7-yl)-3-[4-(6-morpholin-4-ylmethyl-pyridin-3-yl)-naphthalen-1-yl]-urea;
1-[4-(6-morpholin-4-ylmethyl-pyridin-3-yl)-naphthalen-1-yl]-3-(3-trifluoromethanesulfonyl-phenyl)-urea;
N-(5-tert-butyl-2-methoxy-3-{3-[4-(6-morpholin-4-ylmethyl-pyridin-3-yl)-naphthalen-1-yl]-ureido}-phenyl)-isobutyramide;
2-(4-tert-butyl-2-{3-[4-(6-morpholin-4-ylmethyl-pyridin-3-yl)-naphthalen-1-yl]-ureido}-phenoxy)-acetamide;
1-(5-tert-butyl-2-oxo-2,3-dihydro-benzooxazol-7-yl)-3-[4-(6-morpholin-4-ylmethyl-pyridin-3-yl)-naphthalen-1-yl]-urea;
1-(5-tert-butyl-3-cyano-2-methoxy-phenyl)-3-[4-(6-morpholin-4-ylmethyl-pyridin-3-yl)-naphthalen-1-yl]-urea;
1-(5-tert-butyl-benzooxazol-7-yl)-3-[4-(6-morpholin-4-ylmethyl-pyridin-3-yl)-naphthalen-1-yl]-urea;
N-(5-tert-butyl-2-methoxy-3-{3-[4-(6-morpholin-4-ylmethyl-pyridin-3-yl)-naphthalen-1-yl]-ureido}-phenyl)-benzenesulfonamide;
ethanesulfonicacid(5-tert-butyl-2-methoxy-3-{3-[4-(6-morpholin-4-ylmethyl-pyridin-3-yl)-naphthalen-1-yl]-ureido}-phenyl)-amide;
1-(5-tert-butyl-2-methoxy-phenyl)-3-[4-(2-morpholin-4-ylmethyl-pyrimidin-5-yl)-naphthalen-1-yl]-urea;
1-(5-tert-butyl-2-methylsulfanyl-phenyl)-3-[4-(6-morpholin-4-ylmethyl-pyridin-3-yl)-naphthalen-1-yl]-urea;
1-(5-tert-butyl-2-methoxy-pyridin-3-yl)-3-[4-(6-morpholin-4-ylmethyl-pyridin-3-yl)-naphthalen-1-yl]-urea;
2,2,2-trifluoro-ethanesulfonicacid(5-tert-butyl-2-methoxy-3-{3-[4-(6-morpholin-4-ylmethyl-pyridin-3-yl)-naphthalen-1-yl]-ureido}-phenyl)-amide;
N-(5-{4-[3-(5-tert-butyl-2-methyl-phenyl)-ureido]-naphthalen-1-yl}-pyrazin-2-yl)-methanesulfonamide;
1-[4-(6-{[bis-(2-cyano-ethyl)-amino]-methyl}-pyridin-3-yl)-naphthalen-1-yl]-3-(5-tert-butyl-2-methoxy-phenyl)-urea;
1-(5-tert-butyl-2-methoxy-phenyl)-3-{4-[6-(4-methyl-piperazin-1-ylmethyl)-pyridin-3-yl]-naphthalen-1-yl}-urea;
1-(5-tert-butyl-2-methoxy-phenyl)-3-[4-(6-thiomorpholin-4-ylmethyl-pyridin-3-yl)-naphthalen-1-yl]-urea;
1-(5-tert-butyl-2-methoxy-phenyl)-3-{4-[6-(2,6-dimethyl-piperidin-1-ylmethyl)-pyridin-3-yl]-naphthalen-1-yl}-urea;
1-(5-tert-butyl-2-methoxy-phenyl)-3-{4-[6-(1-oxo-tetrahydro-thiopyran-4-ylamino)-pyridin-3-yl]-naphthalen-1-yl}-urea;
1-(5-tert-butyl-2-methoxy-phenyl)-3-{4-[6-(tetrahydro-pyran-4-ylamino)-pyhdin-3-yl]-naphthalen-1-yl}-urea;
1-(5-tert-butyl-2-methoxy-phenyl)-3-[4-(6-{[(2-cyano-ethyl)-(tetrahydro-furan-2-ylmethyl)-amino]-methyl}-pyridin-3-yl)-naphthalen-1-yl]-urea;
1-(5-tert-butyl-2-methoxy-phenyl)-3-{4-[6-(2-methoxymethyl-morpholin-4-ylmethyl)-pyridin-3-yl]-naphthalen-1-yl}-urea;
1-(5-tert-butyl-2-methoxy-phenyl)-3-{4-[6-(2-methyl-3-oxo-piperazin-1-ylmethyl)-pyridin-3-yl]-naphthalen-1-yl}-urea;
1-(5-{4-[3-(5-tert-butyl-2-methoxy-phenyl)-ureido]-naphthalen-1-yl}-pyridin-2-ylmethyl)-piperidine-3-carboxylic acid amide;
1-(5-tert-butyl-2-methoxy-phenyl)-3-{4-[6-(1-oxo-1I4-thiomorpholin-4-ylmethyl)-pyridin-3-yl]-naphthalen-1-yl}-urea;
1-(3,3-dimethyl-2-oxo-2,3-dihydro-1 H-indol-5-yl)-3-[4-(6-morpholin-4-ylmethyl-pyridin-3-yl)-naphthalen-1-yl]-urea;
1-(5-tert-butyl-2-methoxy-phenyl)-3-{4-[6-(3-oxo-piperazin-1-ylmethyl)-pyridin-3-yl]-naphthalen-1-yl}-urea;
1-(5-tert-butyl-2-methoxy-phenyl)-3-(4-{6-[(tetrahydro-furan-3-ylamino)-methyl]-pyridin-3-yl}-naphthalen-1-yl)-urea;
1-(5-tert-butyl-2-methoxy-phenyl)-3-[4-(6-{[(2-cyano-ethyl)-pyridin-3-ylmethyl-amino]-methyl}-pyridin-3-yl)-naphthalen-1-yl]-urea;
1-(5-tert-butyl-2-methoxy-phenyl)-3-{4-[6-(2-oxa-5-aza-bicyclo[2.2.1]hept-5-ylmethyl)-pyridin-3-yl]-naphthalen-1-yl}-urea;
1-(5-tert-butyl-2-methoxy-phenyl)-3-{4-[6-(2,6-dimethyl-morpholin-4-ylmethyl)-pyridin-3-yl]-naphthalen-1-yl}-urea;
1-(5-tert-butyl-2-methoxy-phenyl)-3-(4-{6-[4-(3-methoxy-phenyl)-piperazin-1-ylmethyl]-pyridin-3-yl}-naphthalen-1-yl)-urea;
1-(5-tert-butyl-2-methoxy-phenyl)-3-{4-[6-(morpholine-4-carbonyl)-pyridin-3-yl]-naphthalen-1-yl}-urea;
1-(5-tert-butyl-2-methoxy-phenyl)-3-[4-(5-morpholin-4-ylmethyl-pyrazin-2-yl)-naphthalen-1-yl]-urea;
1-(6-tert-butyl-3-oxo-3,4-dihydro-2H-benzo[1,4]oxazin-8-yl)-3-[4-(6-morpholin-4-ylmethyl-pyridin-3-yl)-naphthalen-1-yl]-urea;
1-(3-amino-5-tert-butyl-2-methoxy-phenyl)-3-[4-(6-morpholin-4-ylmethyl-pyridin-3-yl)-naphthalen-1-yl]-urea;
N-(5-{4-[3-(5-tert-butyl-2-methoxy-phenyl)-ureido]-naphthalen-1-yl}-pyridin-2-yl)-acetamide;
N-(5-tert-butyl-2-methoxy-3-{3-[4-(6-morpholin-4-ylmethyl-pyridin-3-yl)-naphthalen-1-yl]-ureido}-phenyl)-N-methyl-acetamide;
N-(5-tert-butyl-2-methoxy-3-{3-[4-(6-morpholin-4-ylmethyl-pyridin-3-yl)-naphthalen-1-yl]-ureido}-phenyl)-2,2,2-trifluoro-acetamide;
1-(5-tert-butyl-2-methoxy-phenyl)-3-{4-[6-(pyridin-3-yloxy)-pyridin-3-yl]-naphthalen-1-yl}-urea;
[4-(6-morpholin-4-ylmethyl-pyridin-3-yl)-naphthalen-1-yl]-carbamicacid3-tert-butylphenylester;
N-(5-tert-butyl-2-methoxy-3-{3-[4-(6-morpholin-4-ylmethyl-pyridin-3-yl)-naphthalen-1-yl]-ureido}-phenyl)-methanesulfonamide and
and the pharmaceutically acceptable derivatives thereof.

Particularily preferred the invention relates to pharmaceutical compositions comprising **1** and **2d**, **characterized in that** the p38 kinase inhibitor **2d** is selected from the following compounds: 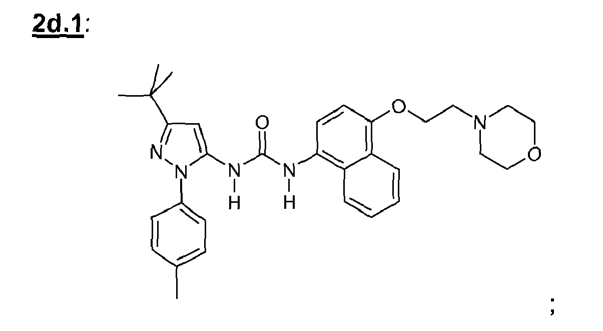 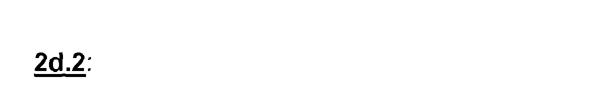 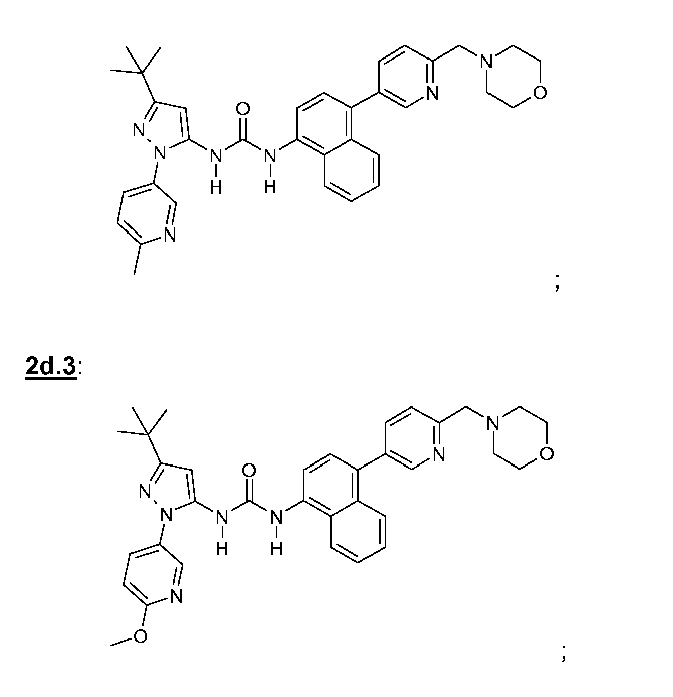 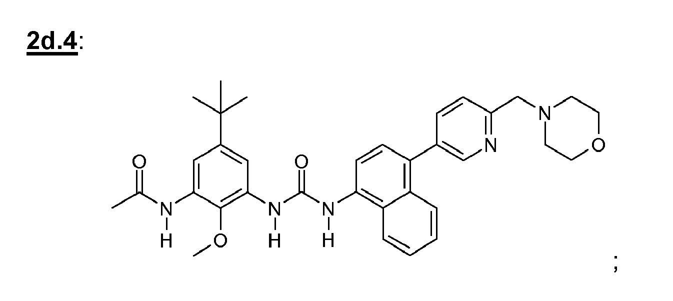 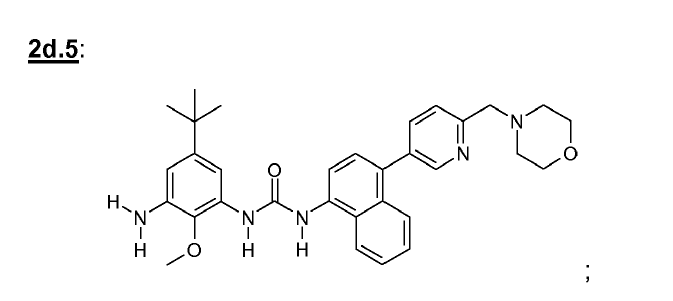 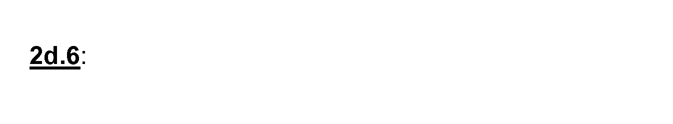 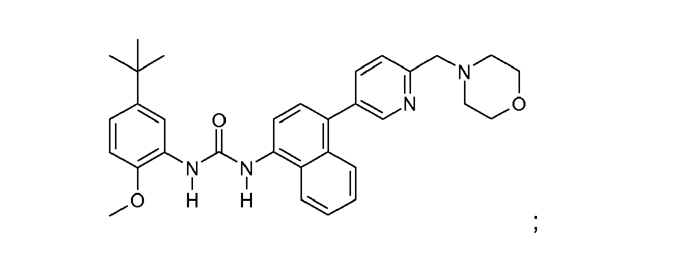 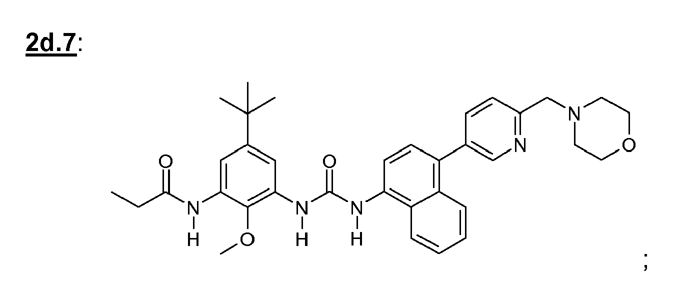 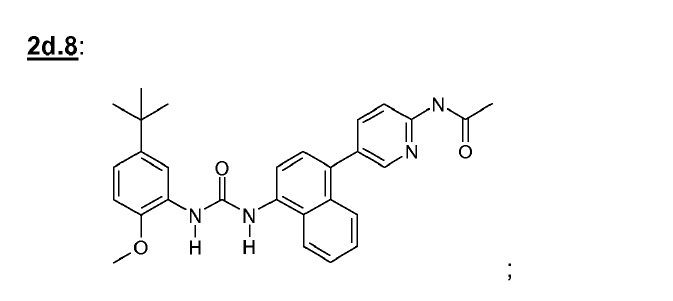 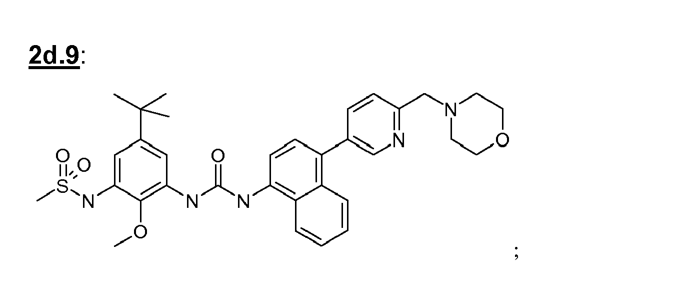 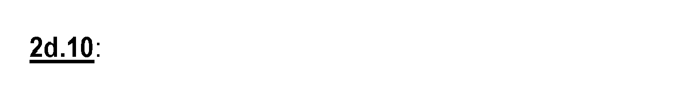 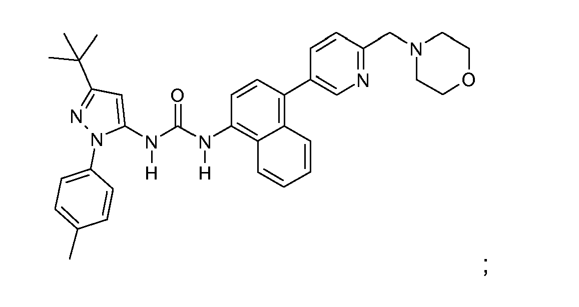 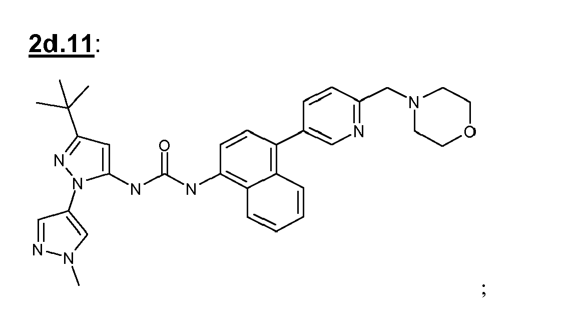 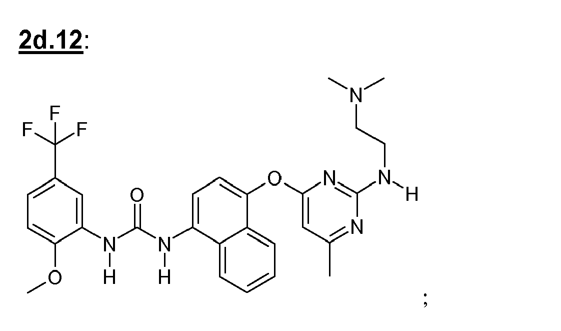 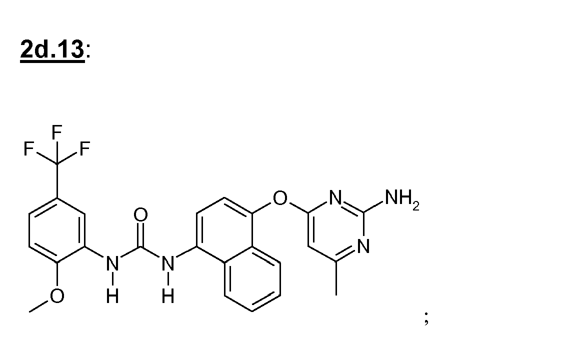 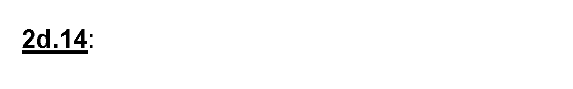 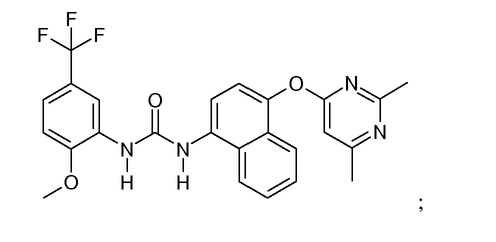 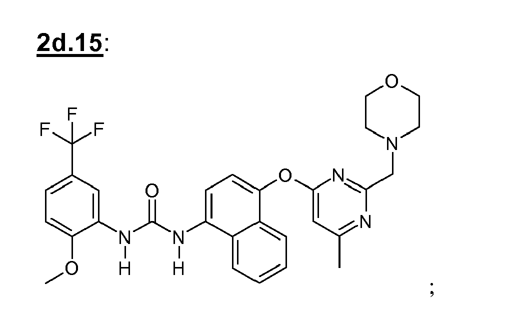 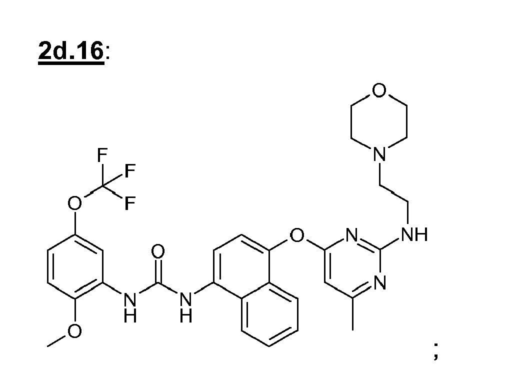 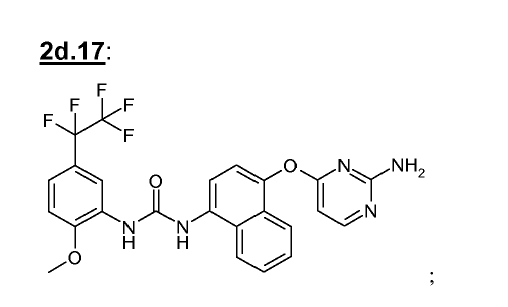 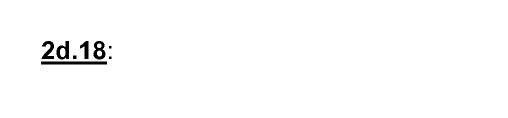 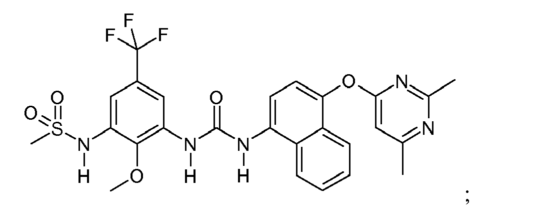 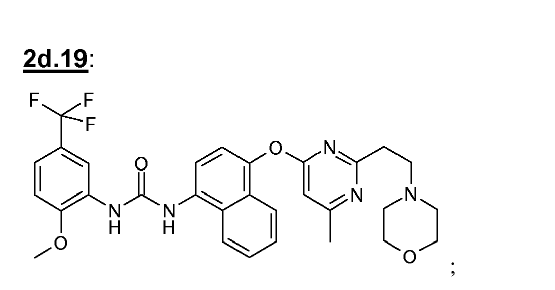 and the pharmaceutically acceptable derivatives thereof.

Any reference to the abovementioned p38 kinase inhibitors **2d** within the scope of the present invention includes a reference to any pharmaceutically acceptable acid addition salts thereof which may exist. By the physiologically or pharmaceutically acceptable acid addition salts which may be formed from **2d** are meant, according to the invention, pharmaceutically acceptable salts selected from among the salts of hydrochloric, hydrobromic, sulfuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids.
Any reference to the abovementioned p38 kinase inhibitors **2d** within the scope of the present invention includes a reference to any alkali metal and alkaline earth metal salts thereof which may exist. If the compounds **2d** are present in the form of their basic salts, the sodium or potassium salts are particularly preferred.

The pharmaceutical combinations of **1** and **2d** according to the invention are preferably administered by parenteral or oral route or by inhalation, the latter being particularly preferred. For oral or parenteral administration the pharmaceutical compositions according to the invention may be administered e.g. in the form of solutions and tablets. For inhalation, as preferred according to the invention, suitable inhalable powders may be used which are packed into suitable capsules (inhalettes) and administered using suitable powder inhalers. Alternatively, the drug may be inhaled by the application of suitable inhalation aerosols. These include inhalation aerosols which contain HFA134a, HFA227 or a mixture thereof as propellant gas. The drug may also be inhaled using suitable solutions of the pharmaceutical combination consisting of **1** and **2d.**

Especially preferred pharmaceutical compositions according to the invention comprise one EGFR kinase inhibitors **1** selected from the group consisting of
**1.1, 1.4, 1.6, 1.8, 1.9, 1.14, 1.17, 1.19, 1.21, 1.23, 1.24, 1.27, 1.28, 1.30, 1.34, 1.35, 1.37, 1.38, 1.40, 1.42, 1.43, 1.44, 1.48, 1.52, 1.55, 1.57, 1.59, 1.60, 1.63, 1.64, 1.66, 1.67, 1.69, 1.70, 1.71, 1.72, 1.78, 1.82, 1.83, 1.84, 1.88, 1.90, 1.91, 1.94** and **1.95**
and one p38 kinase inhibitors selected from the group consisting of
**2d.1, 2d.2, 2d.3, 2d.4, 2d.5, 2d.6, 2d.7, 2d.8, 2d.9, 2d.10, 2d.11, 2d.12, 2d.13, 2d.14, 2d.15, 2d.16, 2d.17, 2d.18**, and **2d.19,**
either as free bases or pharmacologically acceptable acid addition salts, e.g.
**1.1** and **2d.1, 1.4** and **2d.1, 1.6** and **2d.1, 1.8** and **2d.1, 1.9** and **2d.1, 1.14** and **2d.1, 1.17** and **2d.1, 1.19** and **2d.1, 1.21** and **2d.1, 1.23** and **2d.1**, **1.24** and **2d.1**, **1.27** and **2d.1, 1.28** and **2d.1, 1.30** and **2d.1, 1.34** and **2d.1**, **1.35** and **2d.1, 1.37** and **2d.1, 1.38** and **2d.1, 1.40** and **2d.1, 1.42** and **2d.1, 1.43** and **2d.1, 1.44** and **2d.1, 1.48** and **2d.1, 1.52** and **2d.1, 1.55** and **2d.1, 1.57** and **2d.1, 1.59** and **2d.1**, **1.60** and **2d.1, 1.63** and **2d.1, 1.64** and **2d.1, 1.66** and **2d.1, 1.67** and **2d.1, 1.69** and **2d.1, 1.70** and **2d.1, 1.71** and **2d.1, 1.72** and **2d.1, 1.78** and **2d.1, 1.82** and **2d.1, 1.83** and **2d.1, 1.84** and **2d.1, 1.88** and **2d.1, 1.90** and **2d.1, 1.91** and **2d.1**, **1.94** and **2d.1, 1.95** and **2d.1;**
**1.1** and **2d.2, 1.4** and **2d.2, 1.6** and **2d.2, 1.8** and **2d.2, 1.9** and **2d.2, 1.14** and **2d.2, 1.17** and **2d.2, 1.19** and **2d.2, 1.21** and **2d.2, 1.23** and **2d.2, 1.24** and **2d.2, 1.27** and **2d.2, 1.28** and **2d.2, 1.30** and **2d.2, 1.34** and **2d.2, 1.35** and **2d.2, 1.37** and **2d.2, 1.38** and **2d.2, 1.40** and **2d.2, 1.42** and **2d.2, 1.43** and **2d.2, 1.44** and **2d.2, 1.48** and **2d.2, 1.52** and **2d.2, 1.55** and **2d.2, 1.57** and **2d.2, 1.59** and **2d.2, 1.60** and **2d.2, 1.63** and **2d.2, 1.64** and **2d.2, 1.66** and **2d.2, 1.67** and **2d.2, 1.69** and **2d.2, 1.70** and **2d.2, 1.71** and **2d.2, 1.72** and **2d.2, 1.78** and **2d.2, 1.82** and **2d.2, 1.83** and **2d.2, 1.84** and **2d.2, 1.88** and **2d.2,** 1.90 and **2d.2, 1.91** and **2d.2, 1.94** and **2d.2, 1.95** and **2d.2;**
etc.
**1.1** and **2d.19, 1.4** and **2d.19, 1.6** and **2d.19, 1.8** and **2d.19, 1.9** and **2d.19, 1.14** and **2d.19, 1.17** and **2d.19, 1.19** and **2d.19, 1.21** and **2d.19, 1.23** and **2d.19, 1.24** and **2d.19, 1.27** and **2d.19, 1.28** and **2d.19, 1.30** and **2d.19, 1.34** and **2d.19, 1.35** and **2d.19, 1.37** and **2d.19, 1.38** and **2d.19,** 1.40 and **2d.19, 1.42** and **2d.19, 1.43** and **2d.19, 1.44** and **2d.19, 1.48** and **2d.19, 1.52** and **2d.19, 1.55** and **2d.19, 1.57** and **2d.19, 1.59** and **2d.19, 1.60** and **2d.19, 1.63** and **2d.19, 1.64** and **2d.19, 1.66** and **2d.19, 1.67** and **2d.19, 1.69** and **2d.19, 1.70** and **2d.19, 1.71** and **2d.19, 1.72** and **2d.19, 1.78** and **2d.19, 1.82** and **2d.19, 1.83** and **2d.19, 1.84** and **2d.19, 1.88** and **2d.19, 1.90** and **2d.19, 1.91** and **2d.19, 1.94** and **2d.19, 1.95** and **2d.19.**

The proportions in which the active substances **1** and **2d** may be used in the active substance combinations according to the invention are variable. Active substances **1** and **2d** may possibly be present in the form of their solvates or hydrates. Depending on the choice of the compounds **1** and **2d,** the weight ratios which may be used within the scope of the present invention vary on the basis of the different molecular weights of the various compounds and their different potencies.

As a rule, the pharmaceutical combinations according to the invention may contain compounds **1** and **2d** in ratios by weight ranging from 100:1 to 1:100, preferably from 50:1 to 1:50, more preferred from 25:1 to 1:25, most preferred 20:1 to 1:20.

For example, without restricting the scope of the invention thereto, preferred combinations may contain **1** and **2d** in the following weight ratios:
100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100.

The pharmaceutical compositions according to the invention containing the combinations of **1** and **2d** are normally administered so that **1** and **2d** are present together in doses of about 100 to 50000 µg, preferably 1000 to 25000 µg, more preferably 1500 to 10000µg, better still from about 2000 to about 7000 µg, more preferably 2500 to 6000µg per single dose. For example about 3000 to about 5500 µg of the combination of **1** and **2d** according to the invention may be administered once or twice daily to the patient in need thereof. For example, combinations of **1** and **2d** according to the invention contain a quantity of **1** and **2d** such that the total dosage per single dose is about 100µg, 150µg, 200µg, 250µg, 300µg etc. (add stepwise 50µg) up to 50000µg, or similar.

The suggested dosages per single dose specified above are not to be regarded as being limited to the numerical values actually stated, but are intended as dosages which are disclosed by way of example. Of course, dosages which may fluctuate about the abovementioned numerical values within a range of about +/- 2.5 µg are also included in the values given above by way of example. In these dosage ranges, the active substances **1** and **2d** may be present in the weight ratios given above.

For example, without restricting the scope of the invention thereto, the combinations of **1** and **2d** according to the invention may contain a quantity of **1** and p38 kinase inhibitor **2d** such that, in each individual dose,
100µg of **1** and 1000µg of **2d**, 100µg of **1** and 1500µg of **2d**, 100µg of **1** and 2000µg of **2d**, 100µg of **1** and 2500µg of **2d**, 100µg of **1** and 3000µg of **2d**, 100µg of **1** and 3500µg of **2d**, 100µg of **1** and 4000µg of **2d**, 100µg of **1** and 4500µg of **2d**, 100µg of **1** and 5000µg of **2d**, 100µg of **1** and 6000µg of **2d**, 100µg of **1** and 7000µg of **2d**,
100µg of **1** and 8000µg of **2d,** 100µg of **1** and 9000µg of **2d,** 100µg of **1** and 1000µg of **2d**,
200µg of **1** and 1000µg of **2d**, 200µg of **1** and 1500µg of **2d**, 200µg of **1** and 2000µg of **2d,** 200µg of **1** and 2500µg of **2d,** 200µg of **1** and 3000µg of **2d,** 200µg of **1** and 3500µg of **2d,** 200µg of **1** and 4000µg of **2d,** 200µg of **1** and 4500µg of **2d,** 200µg of **1** and 5000µg of **2d,** 200µg of **1** and 6000µg of **2d,** 200µg of **1** and 7000µg of **2d,** 200µg of **1** and 8000µg of **2d,** 200µg of **1** and 9000µg of **2d,** 200µgof **1** and 10000µg of **2d**,
500µg of **1** and 10OOµg of **2d,** 500µg of **1** and 1500µg of **2d**, 500µg of **1** and 2000µg of **2d,** 500µg of **1** and 2500µg of **2d,** 500µg of **1** and 3000µg of **2d,** 500µg of **1** and 3500µg of **2d,** 500µg of **1** and 4000µg of **2d,** 500µg of **1** and 4500µg of **2d,** 500µg of **1** and 5000µg of **2d,** 500µg of **1** and 6000µg of **2d,** 500µg of **1** and 7000µg of **2d,** 500µg of **1** and 8000µg of **2d,** 500µg of **1** and 9000µg of **2d,** 500µg of **1** and 10000µg of **2d**,
1000µg of **1** and 1000µg of **2d,** 1000µg of **1** and 1500µg of **2d,** 1000µg of **1** and 2000µg of **2d,** 1000µg of **1** and 2500µg of **2d,** 1000µg of **1** and 3000µg of **2d,** 1000µg of **1** and 3500µg of **2d,** 1000µg of **1** and 4000µg of **2d,** 1000µg of **1** and 4500µg of **2d,** 1000µg of **1** and 5000µg of **2d,** 1000µg of **1** and 6000µg of **2d,** 1000µg of **1** and 7000µg of **2d,** 1000µg of **1** and 8000µof **2d,** 1000µg of **1** and 9000µg of **2d**, 1000µg of **1** and 1000µg of **2d,**
5000µg of **1** and 1000µg of **2d,** 5000µg of **1** and 1500µg of **2d,** 5000µgof **1** and 2000µg of **2d,** 5000µg of **1** and 2500µg of **2d,** 5000µg of **1** and 3000µg of **2d,** 5000µg of **1** and 3500µg of **2d,** 5000µg of **1** and 4000µg of **2d,** 5000µg of **1** and 4500µg of **2d,** 5000µg of **1** and 5000µg of **2d,** 5000µg of **1** and 6000µg of **2d,** 5000µg of **1** and 7000µg of **2d,** 5000µg of **1** and 8000µg of **2d,** 5000µg of **1** and 9000µg of **2d**, 5000µg of **1** and 10000µg of **2d,**
10000µg of **1** and 1000µg of **2d,** 10000µg of **1** and 1500µg of **2d,** 10000µg of **1** and 2000µg of **2d,** 10000µg of **1** and 2500µg of **2d,** 10000µg of **1** and 3000µg of **2d,** 10000µg of **1** and 3500µg of **2d,** 10000µg of **1** and 4000µg of **2d,** 10000µg of **1** and 4500µg of **2d,** 10000µg of **1** and 5000µg of **2d,** 10000µg of **1** and 6000µg of **2d,** 10000µg of **1** and 7000µg of **2d,** 10000µg of **1** and 8000µg of **2d,** 10000µg of **1** and 9000µg of **2d,** 10000µg of **1** and 10000µg of **2d,**
25000µg of **1** and 1000µg of **2d,** 25000µg of **1** and 1500µg of **2d,** 25000µg of **1** and 2000µg of **2d,** 25000µg of **1** and 2500µg of **2d,** 25000µg of **1** and 3000µg of **2d,** 25000µg of **1** and 3500µg of **2d,** 25000µg of **1** and 4000µg of **2d,** 25000µg of **1** and 4500µg of **2d,** 25000µg of **1** and 5000µg of **2d,** 25000µg of **1** and 6000µg of **2d,** 25000µof **1** and 7000µg of **2d,** 25000µg of **1** and 8000µg of **2d,** 25000µg of **1** and 9000µg of **2d**, 25000µg of **1** and 10000µg of **2d,**
50000µg of **1** and 1000µg of **2d,** 50000µg of **1** and 1500µg of **2d,** 50000µg of **1** and 2000µg of **2d,** 50000µg of **1** and 2500µg of **2d,** 50000µg of **1** and 3000µg of **2d,** 50000µg of **1** and 3500µg of **2d,** 50000µg of **1** and 4000µg of **2d,** 50000µg of **1** and 4500µg of **2d,** 50000µg of **1** and 5000µg of **2d,** 50000µg of **1** and 6000µg of **2d,** 50000µg of **1** and 7000µg of **2d,** 50000µg of **1** and 8000µg of **2d,** 50000µg of **1** and 9000µg of **2d**, 50000µg of **1** and 10000µg of **2d**, are administered.

### Pharmaceutical compositions comprising an EGFR kinase inhibitor 1 and an NK₁ antagonist 2e:

One embodiment of the invention is a pharmaceutical composition comprising an EGFR kinase inhibitor **1** and an NK₁ antagonist **2e.** Binary compositions containing only one active **1** and one active **2e**, optionally together with one or more pharmaceutically acceptable excipients or carriers, are preferred. In the pharmaceutical combinations according to the invention preferred NK₁ antagonists **2e** are selected from the group consisting of N-[2-(3,5-bis-trifluoromethyl-phenyl)-ethyl]-2-{4-cydopropylmethyl-piperazin-1-yl}-N-methyl-2-phenyl-acetamide (BIIF 1149), CP-122721, FK-888, NKP 608C, NKP 608A, CGP 60829, SR 48968(Saredutant), SR 140333 (Nolpitantium besilate/chloride), LY 303 870 (Lanepitant), MEN-11420 (Nepadutant), SB 223412, MDL-105172A, MDL-103896, MEN-11149, MEN-11467, DNK 333A, SR-144190, YM-49244, YM-44778, ZM-274773, MEN-10930, S-19752, Neuronorm, YM-35375, DA-5018, Aprepitant (MK-869), L-754030, CJ-11974, L-758298, DNK-33A, 6b-I, CJ-11974, TAK-637, GR 205171 and the arylglycine amide derivates of general formula (**VIII**) 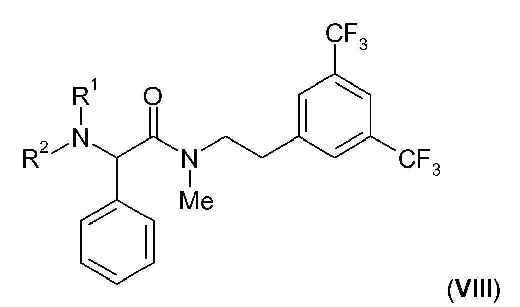 wherein
R¹ and R² together with the N-atom they are bound to form a ring of formula 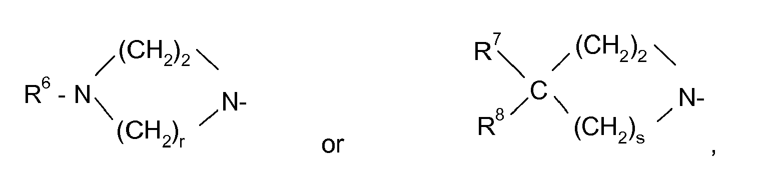 wherein r and s independently denote the number 2 or 3;
R⁶ denotes H, -C₁-C₅-alkyl, C₃-C₅-alkenyl, propinyl, hydroxy(C₂-C₄)alkyl, methoxy(C₂-C₄)alkyl, di(C₁-C₃)alkylamino(C₂-C₄)alkyl, amino(C₂-C₄)alkyl, amino, di(C₁-C₃)alkylamino, monofluoro- up to perfluoro(C₁-C₂)alkyl, N-methylpiperidinyl, pyridyl, pyrimidinyl, pyrazinyl or pyridazinyl,
R⁷ denotes any of the groups defined under (a) to (d):
(a) hydroxy
(b) 4-piperidinopiperidyl,
(c) 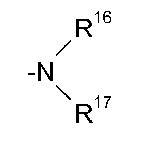 wherein R¹⁶ and R¹⁷ independently denote
   H, (C₁-C₄)alkyl, (C₃-C₆)cycloalkyl, hydroxy(C₂-C₄)alkyl, dihydroxy(C₂-C₄)alkyl, (C₁-C₃)alkoxy(C₂-C₄)alkyl, phenyl(C₁-C₄)alkyl or di(C₁-C₃)alkylamino(C₂-C₄)alkyl, and
R⁸ denotes H,
optionally in the form of enantiomers, mixtures of enantiomers or the racemates.

The compounds of formula (**VIII**) mentioned hereinbefore are described in WO 96/32386, WO 97/32865 and WO 02/32865. The disclosure of these international patent applications is incorporated herein by reference in its entirety.

According to the instant invention preferred NK₁ antagonists **2e** are selected from the group consisting of BIIF 1149, CP-122721, CGP 60829, MK-869, CJ-11974, GR 205171 and the
arylglycine amide derivates of general formula (**VIII**), wherein
R¹ and R² together with the N-atom they are bound to form a ring of formula 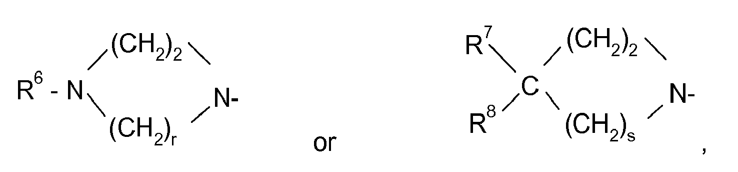 wherein r and s independently denote the number 2 or 3;
R⁶ denotes H, -C₁-C₅-alkyl, C₃-C₅-alkenyl, propinyl, hydroxy(C₂-C₄)alkyl, methoxy-(C₂-C₄)alkyl, di(C₁-C₃)alkylamino(C₂-C₄)alkyl, amino(C₂-C₄)alkyl, amino, di-(C₁-C₃)alkylamino, monofluoro- up to perfluoro(C₁-C₂)alkyl, N-methylpiperidinyl, pyridyl, pyrimidinyl, pyrazinyl or pyridazinyl,
R⁷ denotes the group 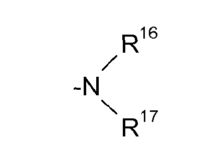 wherein R¹⁶ and R¹⁷ independently denote
H, (C₁-C₄)alkyl, (C₃-C₆)cycloalkyl, hydroxy(C₂-C₄)alkyl, dihydroxy(C₂-C₄)alkyl, (C₁-C₃)alkoxy(C₂-C₄)alkyl, phenyl(C₁-C₄)alkyl or di(C₁-C₃)alkylamino(C₂-C₄)alkyl, and
R⁸ denotes H,
optionally in the form of enantiomers, mixtures of enantiomers or the racemates.

More preferred NK₁ antagonists **2e** according to the instant invention are selected from the group consisting of BIIF 1149 and the arylglycine amide derivates of general formula (**VIII**), wherein
R¹ and R² together with the N-atom they are bound to form a ring of formula 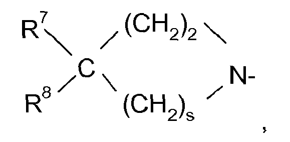 wherein s denotes the number 2;
R⁷ denotes the group 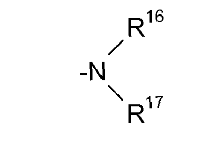 wherein R¹⁶ and R¹⁷ independently denote
H, (C₁-C₄)alkyl, (C₃-C₆)cycloalkyl, hydroxy(C₂-C₄)alkyl, dihydroxy(C₂-C₄)alkyl, and
R⁸ denotes H,
optionally in the form of enantiomers, mixtures of enantiomers or the racemates.

Even more preferred representatives of component **2e** are selected from the group consisting of N-[2-(3,5-bis-trifluoromethyl-phenyl)-ethyl]-2-{4-[(3-hydroxy-propyl)-methyl-amino]-piperidin-1-yl}-N-methyl-2-phenyl-acetamide **2e.1**, N-[2-(3,5-bis-trifluoromethyl-phenyl)-ethyl]-2-[4-(2-hydroxy-1-hydroxymethyl-ethylamino)-piperidin-1-yl]-N-methyl-2-phenylacetamide **2e.2**, N-[2-(3,5-bis-trifluoromethyl-phenyl)-ethyl]-2-[4-(cyclopropylmethyl-methyl-amino)-piperidin-1-yl]-N-methyl-2-phenyl-acetamide **2e.3**, N-[2-(3,5-bis-trifluoromethyl-phenyl)-ethyl]-2-{4-[(2-hydroxy-ethyl)-(3-hydroxypropyl)-amino]-piperidin-1-yl}-N-methyl-2-phenyl-acetamide **2e.4** and N-[2-(3,5-bis-trifluoromethyl-phenyl)-ethyl]-2-{4-[cydopropylmethyl-(3-hydroxy-propyl)-amino]-piperidin-1-yl}-N-methyl-2-phenyl-acetamide **2e.5**, optionally in the form of enantiomers, mixtures of enantiomers or the racemates.

Of exceptional importance according to the invention is N-[2-(3,5-bis-trifluoromethylphenyl)-ethyl]-2-[4-(2-hydroxy-1-hydroxymethyl-ethylamino)-piperidin-1-yl]-N-methyl-2-phenylacetamide as component **2e**, optionally in the form of its enantiomers, preferably in form of the (S)-enantiomer, and optionally in the form of mixtures of enantiomers or the racemates.

As far as not defined specifically, in the definitions used hereinbefore an alkyl group (also as part of other groups) is meant to include branched as well as unbranched alkyl groups having one to five carbon atoms, e.g. methyl, ethyl, propyl, 1-methylethyl (isopropyl), n-butyl, 1-methylpropyl, 2-methylpropyl, 1,1-dimethylethyl (tert.butyl), etc. The groups defined as propyl, butyl and pentyl encompass the respective isomers thereof. Hydroxy- or dihydroxyalkyl groups are meant to be alkyl groups substituted by one or two hydroxy groups.

Alkenyl groups (also as part of other groups) are meant to include branched as well as unbranched alkenyl groups having one to five carbon atoms possessing at least one double bond, e.g.,propenyl, iso-propenyl, butenyl etc.

Cycloalkyl is meant to comprise a saturated cyclic hydrocarbon moiety having three to six carbon atoms, e.g. cyclopropyl, cyclobutyl, cyclopentyl, cyclohexyl, cyclopropylmethyl, cylobutylmethyl, cyclopentylmethyl, cylopropylethyl, cyclobutylethyl etc.

Alkyloxy is synonymous for alkoxy and means a branched or unbranched alkyl group bound via an oxygen atom. The methoxy group is preferred.

Any reference to NK₁ receptor antagonists **2e** within the scope of the present invention includes a reference to the salts, preferably pharmacologically acceptable acid addition salts, or derivatives which may be formed from the NK₁ antagonists. Examples of pharmacologically acceptable acid addition salts of the NK₁ antagonists **2e** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid and maleic acid. Preferred salts are selected from the group consisting of acetate, hydrochloride, hydrobromide, sulphate, phosphate, maleate and methanesulphonate.

The pharmaceutical combinations of **1** and **2e** according to the invention are preferably administered by inhalation. For inhalation suitable inhalable powders may be used which are packed into suitable capsules (inhalettes) and administered using suitable powder inhalers. Alternatively, the drug may be inhaled by the application of suitable inhalation aerosols. These include inhalation aerosols which contain HFA134a, HFA227 or a mixture thereof as propellant gas. The drug may also be inhaled using suitable solutions of the pharmaceutical combination consisting of **1** and **2e**.

Especially preferred pharmaceutical compositions according to the invention comprise the following specific combinations of EGFR kinase inhibitors **1** and NK₁ antagonist **2e**, either as free bases or pharmacologically acceptable acid addition salts:
**1.1** and **2e.1, 1.4** and **2e.1, 1.6** and **2e.1, 1.8** and **2e.1, 1.9** and **2e.1, 1.14** and **2e.1, 1.17** and **2e.1, 1.19** and **2e.1, 1.21** and **2e.1, 1.23** and **2e.1, 1.24** and **2e.1, 1.27** and **2e.1, 1.28** and **2e.1, 1.30** and **2e.1, 1.34** and **2e.1, 1.35** and **2e.1, 1.37** and **2e.1, 1.38** and **2e.1, 1.40** and **2e.1, 1.42** and **2e.1, 1.43** and **2e.1, 1.44** and **2e.1, 1.48** and **2e.1, 1.52** and **2e.1, 1.55** and **2e.1, 1.57** and **2e.1, 1.59** and **2e.1, 1.60** and **2e.1, 1.63** and **2e.1, 1.64** and **2e.1, 1.66** and **2e.1, 1.67** and **2e.1, 1.69** and **2e.1, 1.70** and **2e.1, 1.71** and **2e.1, 1.72** and **2e.1, 1.78** and **2e.1, 1.82** and **2e.1, 1.83** and **2e.1, 1.84** and **2e.1, 1.88** and **2e.1, 1.90** and **2e.1, 1.91** and **2e.1, 1.94** and **2e.1, 1.95** and **2e.1;**
**1.1** and **2e.2, 1.4** and **2e.2, 1.6** and **2e.2, 1.8** and **2e.2, 1.9** and **2e.2, 1.14** and **2e.2, 1.17** and **2e.2, 1.19** and **2e.2, 1.21** and **2e.2, 1.23** and **2e.2, 1.24** and **2e.2, 1.27** and **2e.2, 1.28** and **2e.2, 1.30** and **2e.2, 1.34** and **2e.2, 1.35** and **2e.2, 1.37** and **2e.2, 1.38** and **2e.2, 1.40** and **2e.2, 1.42** and **2e.2, 1.43** and **2e.2, 1.44** and **2e.2, 1.48** and **2e.2, 1.52** and **2e.2, 1.55** and **2e.2, 1.57** and **2e.2, 1.59** and **2e.2, 1.60** and **2e.2, 1.63** and **2e.2, 1.64** and **2e.2, 1.66** and **2e.2, 1.67** and **2e.2, 1.69** and **2e.2, 1.70** and **2e.2, 1.71** and **2e.2, 1.72** and **2e.2, 1.78** and **2e.2, 1.82** and **2e.2, 1.83** and **2e.2, 1.84** and **2e.2, 1.88** and **2e.2, 1.90** and **2e.2, 1.91** and **2e.2, 1.94** and **2e.2, 1.95** and **2e.2;**
**1.1** and **2e.3, 1.4** and **2e.3, 1.6** and **2e.3, 1.8** and **2e.3, 1.9** and **2e.3, 1.14** and **2e.3, 1.17** and **2e.3, 1.19** and **2e.3, 1.21** and **2e.3, 1.23** and **2e.3, 1.24** and **2e.3, 1.27** and **2e.3, 1.28** and **2e.3, 1.30** and **2e.3, 1.34** and **2e.3, 1.35** and **2e.3, 1.37** and **2e.3, 1.38** and **2e.3, 1.40** and **2e.3, 1.42** and **2e.3, 1.43** and **2e.3, 1.44** and **2e.3, 1.48** and **2e.3, 1.52** and **2e.3, 1.55** and **2e.3, 1.57** and **2e.3, 1.59** and **2e.3, 1.60** and **2e.3, 1.63** and **2e.3, 1.64** and **2e.3, 1.66** and **2e.3, 1.67** and **2e.3, 1.69** and **2e.3, 1.70** and **2e.3, 1.71** and **2e.3, 1.72** and **2e.3, 1.78** and **2e.3, 1.82** and **2e.3, 1.83** and **2e.3, 1.84** and **2e.3, 1.88** and **2e.3, 1.90** and **2e.3, 1.91** and **2e.3, 1.94** and **2e.3, 1.95** and **2e.3;**
**1.1** and **2e.4, 1.4** and **2e.4, 1.6** and **2e.4, 1.8** and **2e.4, 1.9** and **2e.4, 1.14** and **2e.4, 1.17** and **2e.4, 1.19** and **2e.4, 1.21** and **2e.4, 1.23** and **2e.4, 1.24** and **2e.4, 1.27** and **2e.4, 1.28** and **2e.4, 1.30** and **2e.4, 1.34** and **2e.4, 1.35** and **2e.4, 1.37** and **2e.4, 1.38** and **2e.4, 1.40** and **2e.4, 1.42** and **2e.4, 1.43** and **2e.4, 1.44** and **2e.4, 1.48** and **2e.4, 1.52** and **2e.4, 1.55** and **2e.4, 1.57** and **2e.4, 1.59** and **2e.4, 1.60** and **2e.4, 1.63** and **2e.4, 1.64** and **2e.4, 1.66** and **2e.4, 1.67** and **2e.4, 1.69** and **2e.4, 1.70** and **2e.4, 1.71** and **2e.4, 1.72** and **2e.4, 1.78** and **2e.4, 1.82** and **2e.4, 1.83** and **2e.4, 1.84** and **2e.4, 1.88** and **2e.4, 1.90** and **2e.4, 1.91** and **2e.4, 1.94** and **2e.4, 1.95** and **2e.4;**
**1.1** and **2e.5, 1.4** and **2e.5, 1.6** and **2e.5, 1.8** and **2e.5, 1.9** and **2e.5, 1.14** and **2e.5, 1.17** and **2e.5, 1.19** and **2e.5, 1.21** and **2e.5, 1.23** and **2e.5, 1.24** and **2e.5, 1.27** and **2e.5, 1.28** and **2e.5, 1.30** and **2e.5, 1.34** and **2e.5, 1.35** and **2e.5, 1.37** and **2e.5, 1.38** and **2e.5, 1.40** and **2e.5, 1.42** and **2e.5, 1.43** and **2e.5, 1.44** and **2e.5, 1.48** and **2e.5, 1.52** and **2e.5, 1.55** and **2e.5, 1.57** and **2e.5, 1.59** and **2e.5, 1.60** and **2e.5, 1.63** and **2e.5, 1.64** and **2e.5, 1.66** and **2e.5, 1.67** and **2e.5, 1.69** and **2e.5, 1.70** and **2e.5, 1.71** and **2e.5, 1.72** and **2e.5, 1.78** and **2e.5, 1.82** and **2e.5, 1.83** and **2e.5, 1.84** and **2e.5, 1.88** and **2e.5, 1.90** and **2e.5, 1.91** and **2e.5, 1.94** and **2e.5, 1.95** and **2e.5.**

The proportions in which the active substances **1** and **2e** may be used in the active substance combinations according to the invention are variable. Active substances **1** and **2e** may possibly be present in the form of their solvates or hydrates. Depending on the choice of the compounds **1** and **2e,** the weight ratios which may be used within the scope of the present invention vary on the basis of the different molecular weights of the various compounds and their different potencies.

As a rule, the pharmaceutical combinations according to the invention may contain compounds **1** and **2e** in ratios by weight ranging from 100:1 to 1:100, preferably from 50:1 to 1:50, more preferred from 25:1 to 1:25, most preferred 20:1 to 1:20.

With respect to the preferred subgroup of pharmaceutical combinations comprising an EGFR kinase inhibitor **1** and an NK₁ antagonist **2e** selected from the group consisting of BIIF 1149, CGP 60829, MK-869, CJ-11974, GR 205171, N-[2-(3,5-bis-trifluoromethyl-phenyl)-ethyl]-2-{4-[(3-hydroxy-propyl)-methyl-amino]-piperidin-1-yl}-N-methyl-2-phenyl-acetamide, N-[2-(3,5-bis-trifluoromethyl-phenyl)-ethyl]-2-[4-(2-hydroxy-1-hydroxymethyl-ethylamino)-piperidin-1-yl]-N-methyl-2-phenylacetamide, N-[2-(3,5-bis-trifluoromethyl-phenyl)-ethyl]-2-[4-(cyclopropylmethyl-methyl-amino)-piperidin-1-yl]-N-methyl-2-phenyl-acetamide, N-[2-(3,5-bis-trifluoromethyl-phenyl)-ethyl]-2-{4-[(2-hydroxy-ethyl)-(3-hydroxy-propyl)-amino]-piperidin-1-yl}-N-methyl-2-phenyl-acetamide, N-[2-(3,5-bis-trifluoromethyl-phenyl)-ethyl]-2-{4-[cyclopropylmethyl-(3-hydroxy-propyl)-amino]-piperidin-1-yl}-N-methyl-2-phenyl-acetamide and the arylglycine amide derivates of formula (**VIII**) the weight ratios of **1** : **2e** especially preferred are in the range of 50:1 to 4:1.

For example, without restricting the scope of the invention thereto, preferred combinations may contain **1** and **2e** in the following weight ratios:
100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100.

The pharmaceutical compositions according to the invention containing the combinations of **1** and **2e** are normally administered so that **1** and **2e** are present together in doses of about 100 to 50000 µg, preferably 1000 to 25000 µg, more preferably 1500 to 10000µg, better still from about 2000 to about 7000 µg, more preferably 2500 to 6000µg per single dose. For example about 3000 to about 5500 µg of the combination of **1** and **2e** according to the invention may be administered once or twice daily to the patient in need thereof. For example, combinations of **1** and **2e** according to the invention contain a quantity of **1** and **2e** such that the total dosage per single dose is about 100µg, 150µg, 200µg, 250µg, 300µg etc. (add stepwise 50µg) up to 50000µg, or similar.

The suggested dosages per single dose specified above are not to be regarded as being limited to the numerical values actually stated, but are intended as dosages which are disclosed by way of example. Of course, dosages which may fluctuate about the abovementioned numerical values within a range of about +/- 2.5 µg are also included in the values given above by way of example. In these dosage ranges, the active substances **1** and **2e** may be present in the weight ratios given above.

For example, without restricting the scope of the invention thereto, the combinations of **1** and **2e** according to the invention may contain a quantity of **1** and NK₁ antagonist **2e** such that, in each individual dose,
100µg of **1** and 1000µg of **2e,** 100µg of **1** and 1500µg of **2e,** 100µg of **1** and 2000µg of **2e,** 100µg of **1** and 2500µg of **2e,** 100µg of **1** and 3000µg of **2e,** 100µg of **1** and 3500µg of **2e,** 100µg of **1** and 4000µg of **2e,** 100µg of **1** and 4500µg of **2e,** 100µg of **1** and 5000µg of **2e,** 100µg of **1** and 6000µg of **2e,** 100µg of **1** and 7000µg of **2e,** 100µg of **1** and 8000µg of **2e,** 100µg of **1** and 9000µg of **2e,** 100µg of **1** and 10000µg of **2e,**
200µg of **1** and 1000µg of **2e,** 200µg of **1** and 1500µg of **2e,** 200µg of **1** and 2000µg of **2e,** 200µg of **1** and 2500µg of **2e,** 200µg of **1** and 3000µg of **2e,** 200µg of **1** and 3500µg of **2e,** 200µg of **1** and 4000µg of **2e,** 200µg of **1** and 4500µg of **2e,** 200µg of **1** and 5000µg of **2e,** 200µg of **1** and 6000µg of **2e,** 200µg of **1** and 7000µg of **2e,** 200µg of **1** and 8000µg of **2e**, 200µg of **1** and 9000µg of **2e**, 200µg of **1** and 10000µg of **2e**,
500µg of **1** and 1000µg of **2e,** 500µg of **1** and 1500µg of **2e,** 500µg of **1** and 2000µg of **2e,** 500µg of **1** and 2500µg of **2e,** 500µg of **1** and 3000µg of **2e,** 500µg of **1** and 3500µg of **2e,** 500µg of **1** and 4000µg of **2e,** 500µg of **1** and 4500µg of **2e,** 500µg of **1** and 5000µg of **2e,** 500µg of **1** and 6000µg of **2e,** 500µg of **1** and 7000µg of **2e,** 500µg of **1** and 8000µg of **2e,** 500µg of **1** and 9000µg of **2e,** 500µg of **1** and 10000µg of **2e**,
1000µg of **1** and 1000µg of **2e,** 1000µg of **1** and 1500µg of **2e,** 1000µg of **1** and 2000µg of **2e**, 1000µg of **1** and 2500µg of **2e**, 1000µg of **1** and 3000µg of **2e**, 1000µg of **1** and 3500µg of **2e,** 1000µg of **1** and 4000µg of **2e,** 1000µg of **1** and 4500µg of **2e,** 1000µg of **1** and 5000µg of **2e,** 1000µg of **1** and 6000µg of **2e,** 1000µg of **1** and 7000µg of **2e**, 1000µg of **1** and 8000µg of **2e**, 1000µg of **1** and 9000µg of **2e**, 1000µg of **1** and 10000µg of **2e**,
5000µg of **1** and 1000µg of **2e,** 5000µg of **1** and 1500µg of **2e,** 5000µg of **1** and 2000µg of **2e,** 5000µg of **1** and 2500µg of **2e,** 5000µg of **1** and 3000µg of **2e,** 5000µg of **1** and 3500µg of **2e,** 5000µg of **1** and 4000µg of **2e,** 5000µg of **1** and 4500µg of **2e,** 5000µg of **1** and 5000µg of **2e,** 5000µg of **1** and 6000µg of **2e,** 5000µg of **1** and 7000µg of **2e,** 5000µg of **1** and 8000µg of **2e,** 5000µg of **1** and 9000µg of **2e,** 5000µg of **1** and 10000µg of **2e,**
10000µg of **1** and 1000µg of **2e,** 10000µg of **1** and 1500µg of **2e,** 10000µg of **1** and 2000µg of **2e,** 10000µg of **1** and 2500µg of **2e,** 10000µg of **1** and 3000µg of **2e,** 10000µg of **1** and 3500µg of **2e,** 10000µg of **1** and 4000µg of **2e,** 10000µg of **1** and 4500µg of **2e,** 10000µg of **1** and 5000µg of **2e,** 10000µg of **1** and 6000µg of **2e,** 10000µg of **1** and 7000µg of **2e,** 10000µg of **1** and 8000µg of **2e,** 10000µg of **1** and 9000µg of **2e,** 10000µg of **1** and 10000µg of **2e,**
25000µg of **1** and 1000µg of **2e,** 25000µg of **1** and 1500µg of **2e,** 25000µg of **1** and 2000µg of **2e,** 25000µg of **1** and 2500µg of **2e,** 25000µg of **1** and 3000µg of **2e,** 25000µg of **1** and 3500µg of **2e,** 25000µg of **1** and 4000µg of **2e,** 25000µg of **1** and 4500µg of **2e,** 25000µg of **1** and 5000µg of **2e,** 25000µg of **1** and 6000µg of **2e,** 25000µg of **1** and 7000µg of **2e,** 25000µg of **1** and 8000µg of **2e,** 25000µg of **1** and 9000µg of **2e,** 25000µg of **1** and 10000µg of **2e,**
50000µg of **1** and 1000µg of **2e,** 50000µg of **1** and 1500µg of **2e,** 50000µg of **1** and 2000µg of **2e,** 50000µg of **1** and 2500µg of **2e,** 50000µg of **1** and 3000µg of **2e,** 50000µg of **1** and 3500µg of **2e,** 50000µg of **1** and 4000µg of **2e,** 50000µg of **1** and 4500µg of **2e,** 50000µg of **1** and 5000µg of **2e,** 50000µg of **1** and 6000µg of **2e,** 50000µg of **1** and 7000µg of **2e,** 50000µg of **1** and 8000µg of **2e,** 50000µg of **1** and 9000µg of **2e,** 50000µg of **1** and 10000µg of **2e,** are administered.

### Pharmaceutical compositions comprising an EGFR kinase inhibitor 1 and an endothelin-antagonist 2f:

One embodiment of the invention is a pharmaceutical composition comprising an EGFR kinase inhibitor **1** and an endothelin-antagonist **2f**. Binary compositions containing only one active **1** and one active **2f**, optionally together with one or more pharmaceutically acceptable excipients or carriers, are preferred. In the pharmaceutical combinations according to the invention preferred endothelin-antagonists **2f** are selected from the group consisting of Tezosentan **2f.1,** Bosentan **2f.2,** Enrasentan **2f.3,** Sixtasentan **2f.4,** T-0201 **2f.5,** BMS-193884 **2f.6,** K-8794 **2f.7,** PD-156123 **2f.8,** PD-156707 **2f.9,** PD-160874 **2f.10,** PD-180988 **2f.11,** S-0139 **2f.12** and ZD-1611 **2f.13.** According to the instant invention preferred endothelin-antagonists **2f** are selected from the group consisting **2f.1, 2f.2, 2f.3, 2f.4, 2f.5** and **2f.6,** the endothelin-antagonists **2f.1** and **2f.2** being particularly preferred.

Any reference to endothelin-antagonists **2f** within the scope of the present invention includes a reference to the salts, preferably pharmacologically acceptable acid addition salts, or derivatives which may be formed from the endothelin-antagonists. Examples of pharmacologically acceptable acid addition salts of the endothelin-antagonists **2f** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid and maleic acid. Preferred salts are selected from the group consisting of acetate, hydrochloride, hydrobromide, sulphate, phosphate, maleate and methanesulphonate.

Any reference to the abovementioned endothelin-antagonists **2f** within the scope of the present invention includes a reference to any alkali metal and alkaline earth metal salts thereof which may exist. If the compounds **2f** are present in the form of their basic salts, the sodium or potassium salts are particularly preferred.

The pharmaceutical combinations of **1** and **2f** according to the invention are preferably administered by parenteral or oral route or by inhalation, the latter being particularly preferred. For oral or parenteral administration the pharmaceutical compositions according to the invention may be administered e.g. in the form of solutions and tablets. For inhalation, as preferred according to the invention, suitable inhalable powders may be used which are packed into suitable capsules (inhalettes) and administered using suitable powder inhalers. Alternatively, the drug may be inhaled by the application of suitable inhalation aerosols. These include inhalation aerosols which contain HFA134a, HFA227 or a mixture thereof as propellant gas. The drug may also be inhaled using suitable solutions of the pharmaceutical combination consisting of **1** and **2f**.

Especially preferred pharmaceutical compositions according to the invention comprise the following specific combinations of EGFR kinase inhibitors **1** and endothelin-antagonists **2f**, either as free bases or pharmacologically acceptable acid addition salts:
**1.1** and **2f.1, 1.4** and **2f.1, 1.6** and **2f.1, 1.8** and **2f.1, 1.9** and **2f.1, 1.14** and **2f.1, 1.17** and **2f.1, 1.19** and **2f.1, 1.21** and **2f.1, 1.23** and **2f.1, 1.24** and **2f.1, 1.27** and **2f.1, 1.28** and **2f.1, 1.30** and **2f.1, 1.34** and **2f.1, 1.35** and **2f.1, 1.37** and **2f.1, 1.38** and **2f.1, 1.40** and **2f.1, 1.42** and **2f.1, 1.43** and **2f.1, 1.44** and **2f.1, 1.48** and **2f.1, 1.52** and **2f.1, 1.55** and **2f.1, 1.57** and **2f.1, 1.59** and **2f.1, 1.60** and **2f.1, 1.63** and **2f.1, 1.64** and **2f.1, 1.66** and **2f.1, 1.67** and **2f.1, 1.69** and **2f.1, 1.70** and **2f.1, 1.71** and **2f.1, 1.72** and **2f.1, 1.78** and **2f.1, 1.82** and **2f.1, 1.83** and **2f.1, 1.84** and **2f.1, 1.88** and **2f.1, 1.90** and **2f.1, 1.91** and **2f.1, 1.94** and **2f.1, 1.95** and **2f.1;**
**1.1** and **2f.2, 1.4** and **2f.2, 1.6** and **2f.2, 1.8** and **2f.2, 1.9** and **2f.2, 1.14** and **2f.2, 1.17** and **2f.2, 1.19** and **2f.2, 1.21** and **2f.2, 1.23** and **2f.2, 1.24** and **2f.2, 1.27** and **2f.2, 1.28** and **2f.2, 1.30** and **2f.2, 1.34** and **2f.2, 1.35** and **2f.2, 1.37** and **2f.2, 1.38** and **2f.2, 1.40** and **2f.2, 1.42** and **2f.2, 1.43** and **2f.2, 1.44** and **2f.2, 1.48** and **2f.2, 1.52** and **2f.2, 1.55** and **2f.2, 1.57** and **2f.2, 1.59** and **2f.2, 1.60** and **2f.2, 1.63** and **2f.2, 1.64** and **2f.2, 1.66** and **2f.2, 1.67** and **2f.2, 1.69** and **2f.2, 1.70** and **2f.2, 1.71** and **2f.2, 1.72** and **2f.2, 1.78** and **2f.2, 1.82** and **2f.2, 1.83** and **2f.2, 1.84** and **2f.2, 1.88** and **2f.2, 1.90** and **2f.2, 1.91** and **2f.2, 1.94** and **2f.2, 1.95** and **2f.2;**
**1.1** and **2f.3, 1.4** and **2f.3, 1.6** and **2f.3, 1.8** and **2f.3, 1.9** and **2f.3, 1.14** and **2f.3, 1.17** and **2f.3, 1.19** and **2f.3, 1.21** and **2f.3, 1.23** and **2f.3, 1.24** and **2f.3, 1.27** and **2f.3, 1.28** and **2f.3, 1.30** and **2f.3, 1.34** and **2f.3, 1.35** and **2f.3, 1.37** and **2f.3, 1.38** and **2f.3, 1.40** and **2f.3, 1.42** and **2f.3, 1.43** and **2f.3, 1.44** and **2f.3, 1.48** and **2f.3, 1.52** and **2f.3, 1.55** and **2f.3, 1.57** and **2f.3, 1.59** and **2f.3, 1.60** and **2f.3, 1.63** and **2f.3, 1.64** and **2f.3, 1.66** and **2f.3, 1.67** and **2f.3, 1.69** and **2f.3, 1.70** and **2f.3, 1.71** and **2f.3, 1.72** and **2f.3, 1.78** and **2f.3, 1.82** and **2f.3, 1.83** and **2f.3, 1.84** and **2f.3, 1.88** and **2f.3, 1.90** and **2f.3, 1.91** and **2f.3, 1.94** and **2f.3, 1.95** and **2f.3;**
**1.1** and **2f.4, 1.4** and **2f.4, 1.6** and **2f.4, 1.8** and **2f.4, 1.9** and **2f.4, 1.14** and **2f.4, 1.17** and **2f.4, 1.19** and **2f.4, 1.21** and **2f.4, 1.23** and **2f.4, 1.24** and **2f.4, 1.27** and **2f.4, 1.28** and **2f.4, 1.30** and **2f.4, 1.34** and **2f.4, 1.35** and **2f.4, 1.37** and **2f.4, 1.38** and **2f.4, 1.40** and **2f.4, 1.42** and **2f.4, 1.43** and **2f.4, 1.44** and **2f.4, 1.48** and **2f.4, 1.52** and **2f.4, 1.55** and **2f.4, 1.57** and **2f.4, 1.59** and **2f.4, 1.60** and **2f.4, 1.63** and **2f.4, 1.64** and **2f.4, 1.66** and **2f.4, 1.67** and **2f.4, 1.69** and **2f.4, 1.70** and **2f.4, 1.71** and **2f.4, 1.72** and **2f.4, 1.78** and **2f.4, 1.82** and **2f.4, 1.83** and **2f.4, 1.84** and **2f.4, 1.88** and **2f.4, 1.90** and **2f.4, 1.91** and **2f.4, 1.94** and **2f.4, 1.95** and **2f.4;**
**1.1** and **2f.5, 1.4** and **2f.5, 1.6** and **2f.5, 1.8** and **2f.5, 1.9** and **2f.5, 1.14** and **2f.5, 1.17** and **2f.5, 1.19** and **2f.5, 1.21** and **2f.5, 1.23** and **2f.5, 1.24** and **2f.5, 1.27** and **2f.5, 1.28** and **2f.5, 1.30** and **2f.5, 1.34** and **2f.5, 1.35** and **2f.5, 1.37** and **2f.5, 1.38** and **2f.5, 1.40** and **2f.5, 1.42** and **2f.5, 1.43** and **2f.5, 1.44** and **2f.5, 1.48** and **2f.5, 1.52** and **2f.5, 1.55** and **2f.5, 1.57** and **2f.5, 1.59** and **2f.5, 1.60** and **2f.5, 1.63** and **2f.5, 1.64** and **2f.5, 1.66** and **2f.5, 1.67** and **2f.5, 1.69** and **2f.5, 1.70** and **2f.5, 1.71** and **2f.5, 1.72** and **2f.5, 1.78** and **2f.5, 1.82** and **2f.5, 1.83** and **2f.5, 1.84** and **2f.5, 1.88** and **2f.5, 1.90** and **2f.5, 1.91** and **2f.5, 1.94** and **2f.5, 1.95** and **2f.5;**
**1.1** and **2f.6, 1.4** and **2f.6, 1.6** and **2f.6, 1.8** and **2f.6, 1.9** and **2f.6, 1.14** and **2f.6, 1.17** and **2f.6, 1.19** and **2f.6, 1.21** and **2f.6, 1.23** and **2f.6, 1.24** and **2f.6, 1.27** and **2f.6, 1.28** and **2f.6, 1.30** and **2f.6, 1.34** and **2f.6, 1.35** and **2f.6, 1.37** and **2f.6, 1.38** and **2f.6, 1.40** and **2f.6, 1.42** and **2f.6, 1.43** and **2f.6, 1.44** and **2f.6, 1.48** and **2f.6, 1.52** and **2f.6, 1.55** and **2f.6, 1.57** and **2f.6, 1.59** and **2f.6, 1.60** and **2f.6, 1.63** and **2f.6, 1.64** and **2f.6, 1.66** and **2f.6, 1.67** and **2f.6, 1.69** and **2f.6, 1.70** and **2f.6, 1.71** and **2f.6, 1.72** and **2f.6, 1.78** and **2f.6, 1.82** and **2f.6, 1.83** and **2f.6, 1.84** and **2f.6, 1.88** and **2f.6, 1.90** and **2f.6, 1.91** and **2f.6, 1.94** and **2f.6, 1.95** and **2f.6.**

The proportions in which the active substances **1** and **2f** may be used in the active substance combinations according to the invention are variable. Active substances **1** and **2f** may possibly be present in the form of their solvates or hydrates. Depending on the choice of the compounds **1** and **2f,** the weight ratios which may be used within the scope of the present invention vary on the basis of the different molecular weights of the various salt forms.

As a rule, the pharmaceutical combinations according to the invention may contain compounds **1** and **2f** in ratios by weight ranging from 100:1 to 1:100, preferably from 50:1 to 1:50, more preferred from 25:1 to 1:25, most preferred 20:1 to 1:20.

For example, without restricting the scope of the invention thereto, preferred combinations may contain **1** and an endothelin-antagonists **2f** in the following weight ratios:
100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100.

The pharmaceutical compositions according to the invention containing the combinations of **1** and **2f** are normally administered so that **1** and **2f** are present together in doses of about 100 to 50000 µg, preferably 1000 to 25000 µg, more preferably 1500 to 10000µg, better still from about 2000 to about 7000 µg, more preferably 2500 to 6000µg per single dose. For example about 3000 to about 5500 µg of the combination of **1** and **2f** according to the invention may be administered once or twice daily to the patient in need thereof. For example, combinations of **1** and **2f** according to the invention contain a quantity of **1** and an endothelin-antagonist **2f** (as for instance **2f.1, 2f.2, 2f.3, 2f.4** or **2f.5)** such that the total dosage per single dose is about 100µg, 150µg, 200µg, 250µg, 300µg etc. (add stepwise 50µg) up to 50000µg, or similar.

For example, without restricting the scope of the invention thereto, the combinations of **1** and **2f** according to the invention may contain a quantity of **1** and an endothelin-antagonist **2f** (as for instance **2f.1, 2f.2, 2f.3, 2f.4** or **2f.5)** in such an amount that the following quantities of the active substances are administered per single dose:
100µg of **1** and 1000µg of **2f,** 100µg of **1** and 1500µg of **2f,** 100µg of **1** and 2000µg of **2f,** 100µg of **1** and 2500µg of **2f,** 100µg of **1** and 3000µg of **2f,** 100µg of **1** and 3500µg of **2f,** 100µg of **1** and 4000µg of **2f,** 100µg of **1** and 4500µg of **2f,** 100µg of **1** and 5000µg of **2f,** 100µg of **1** and 6000µg of **2f,** 100µg of **1** and 7000µg of **2f,** 100µg of **1** and 8000µg of **2f,** 100µg of **1** and 9000µg of **2f,** 100µg of **1** and 10000µg of **2f,**
200µg of **1** and 1000µg of **2f,** 200µg of **1** and 1500µg of **2f,** 200µg of **1** and 2000µg of **2f,** 200µg of **1** and 2500µg of **2f,** 200µg of **1** and 3000µg of **2f,** 200µg of **1** and 3500µg of **2f,** 200µg of **1** and 4000µg of **2f,** 200µg of **1** and 4500µg of **2f,** 200µg of **1** and 5000µg of **2f,** 200µg of **1** and 6000µg of **2f,** 200µg of **1** and 7000µg of **2f,** 200µg of **1** and 8000µg of **2f,** 200µg of **1** and 9000µg of **2f,** 200µg of **1** and 10000µg of **2f,**
500µg of **1** and 1000µg of **2f,** 500µg of **1** and 1500µg of **2f,** 500µg of **1** and 2000µg of **2f,** 500µg of **1** and 2500µg of **2f,** 500µg of **1** and 3000µg of **2f,** 500µg of **1** and 3500µg of **2f,** 500µg of **1** and 4000µg of **2f,** 500µg of **1** and 4500µg of **2f,** 500µg of **1** and 5000µg of **2f,** 500µg of **1** and 6000µg of **2f,** 500µg of **1** and 7000µg of **2f,** 500µg of **1** and 8000µg of **2f,** 500µg of **1** and 9000µg of **2f,** 500µg of **1** and 10000µg of **2f,**
1000µg of **1** and 1000µg of **2f,** 1000µg of **1** and 1500µg of **2f,** 1000µg of **1** and 2000µg of **2f,** 1000µg of **1** and 2500µg of **2f,** 1000µg of **1** and 3000µg of **2f,** 1000µg of **1** and 3500µg of **2f,** 1000µg of **1** and 4000µg of **2f,** 1000µg of **1** and 4500µg of **2f,** 1000µg of **1** and 5000µg of **2f,** 1000µg of **1** and 6000µg of **2f,** 1000µg of **1** and 7000µg of **2f,** 1000µg of **1** and 8000µg of **2f,** 1000µg of **1** and 9000µg of **2f,** 1000µg of **1** and 10000µg of **2f,**
5000µg of **1** and 1000µg of **2f,** 5000µg of **1** and 1500µg of **2f,** 5000µg of **1** and 2000µg of **2f,** 5000µg of **1** and 2500µg of **2f,** 5000µg of **1** and 3000µg of **2f,** 5000µg of **1** and 3500µg of **2f,** 5000µg of **1** and 4000µg of **2f,** 5000µg of **1** and 4500µg of **2f,** 5000µg of **1** and 5000µg of **2f,** 5000µg of **1** and 6000µg of **2f,** 5000µg of **1** and 7000µg of **2f,** 5000µg of **1** and 8000µg of **2f,** 5000µg of **1** and 9000µg of **2f,** 5000µg of **1** and 10000µg of **2f,**
10000µg of **1** and 1000µg of **2f,** 10000µg of **1** and 1500µg of **2f,** 10000µg of **1** and 2000µg of **2f,** 10000µg of **1** and 2500µg of **2f,** 10000µg of **1** and 3000µg of **2f,** 10000µg of **1** and 3500µg of **2f,** 10000µg of **1** and 4000µg of **2f,** 10000µg of **1** and 4500µg of **2f,** 10000µg of **1** and 5000µg of **2f,** 10000µg of **1** and 6000µg of **2f,** 10000µg of **1** and 7000µg of **2f,** 10000µg of **1** and 8000µg of **2f,** 10000µg of **1** and 9000µg of **2f,** 10000µg of **1** and 10000µg of **2f,**
25000µg of **1** and 1000µg of **2f,** 25000µg of **1** and 1500µg of **2f,** 25000µg of **1** and 2000µg of **2f,** 25000µg of **1** and 2500µg of **2f,** 25000µg of **1** and 3000µg of **2f,** 25000µg of **1** and 3500µg of **2f,** 25000µg of **1** and 4000µg of **2f,** 25000µg of **1** and 4500µg of **2f,** 25000µg of **1** and 5000µg of **2f,** 25000µg of **1** and 6000µg of **2f,** 25000µg of **1** and 7000µg of **2f,** 25000µg of **1** and 8000µg of **2f,** 25000µg of **1** and 9000µg of **2f,** 25000µg of **1** and 10000µg of **2f,**
50000µg of **1** and 1000µg of **2f,** 50000µg of **1** and 1500µg of **2f,** 50000µg of **1** and 2000µg of **2f,** 50000µg of **1** and 2500µg of **2f,** 50000µg of **1** and 3000µg of **2f,** 50000µg of **1** and 3500µg of **2f,** 50000µg of **1** and 4000µg of **2f,** 50000µg of **1** and 4500µg of **2f,** 50000µg of **1** and 5000µg of **2f,** 50000µg of **1** and 6000µg of **2f,** 50000µg of **1** and 7000µg of **2f,** 50000µg of **1** and 8000µg of **2f,** 50000µg of **1** and 9000µg of **2f,** 50000µg of **1** and 10000µg of **2f,** are administered.

The suggested dosages per single dose specified above are not to be regarded as being limited to the numerical values actually stated, but are intended as dosages which are disclosed by way of example. Of course, dosages which may fluctuate about the abovementioned numerical values within a range of about +/- 2.5 µg are also included in the values given above by way of example. In these dosage ranges, the active substances **1** and **2f** may be present in the weight ratios given above.

The actives of the combinations according to the invention may be administered simultaneously, separately or sequentially. The preferred route of administration depends on the indication to be treated. In case of gastrointestinal indications, inflammatory joint, skin and eyes disorders both components **1** and **2** may be administered orally, intravenously or rectally, using suitable formulations known in the art, such as tablets, coated tablets, pills, granules or granular powder, syrups, emulsions, suspensions, solutions or suppositories, optionally together with inert and non-toxic pharmaceutically acceptable excipients or solvents. In case of inflammatory joint or skin disorders both components **1** and **2** also may be may be administered topically, using suitable formulations known in the art, such as ointments or transdermal patches. Furthermore, in case of inflammatory disorders of the eye both components **1** and **2** preferably are administered topically using suitable formulations such as ophthalmic solutions, eye drops or viscoelastic gels.

In case of respiratory indications and if administered separately or sequentially preferably at least one of components **1** and **2** is given by inhalative route. If component **1** is administered by inhalation component **2**, administered separately, may be given for instance orally, intravenously, subcutaneously, by intramuscular injection, intraperitoneally, intranasally or transdermally, using suitable formulations known in the art, such as tablets, coated tablets, pills, granules or granular powder, aerosols, syrups, emulsions, suspensions, powders, solutions or transdermal patches, optionally together with inert and non-toxic pharmaceutically acceptable excipients or solvents. The same applies with respect to component **1**, vice versa, if component **2** is administered by inhalation.

In case of respiratory indications both components **1** and **2** of the pharmaceutical combinations according to the invention preferably are administered by inhalation. Inhalable preparations according to the invention include inhalable powders, propellant-containing metered dose aerosols or propellant-free inhalable solutions. Inhalable powders according to the invention containing the combination of active substances **1** and **2** may consist of the active substances on their own or of a mixture of the active substances with physiologically acceptable excipients. Within the scope of the present invention, the term carrier may optionally be used instead of the term excipient. Within the scope of the present invention, the term propellant-free inhalable solutions also includes concentrates or sterile inhalable solutions ready for use. The preparations according to the invention may contain the combination of active substances **1** and **2** either together in one formulation or in two separate formulations. These formulations which may be used within the scope of the present invention are described in more detail in the next part of the specification.

Any aforementioned possible doses applicable for the combinations according to the invention are to be understood as referring to doses per single application. However, these examples are not be understood as excluding the possibility of administering the combinations according to the invention multiple times. Depending on the medical need patients may receive also multiple inhalative applications. As an example patients may receive the combinations according to the invention for instance two or three times (e.g. two or three puffs with a powder inhaler, an MDI etc.) in the morning of each treatment day. As the aforementioned dose examples are only to be understood as dose examples per single application (i.e. per puff) multiple application of the combinations according to the invention leads to multiple doses of the aforementioned examples. The application of the compositions according to the invention can be for instance once a day, or depending on the duration of action of the agents twice a day, or once every 2 or 3 days.

Moreover it is emphazised that the aforementioned dosages are to be understood as examples of metered doses only. In other terms, the aforementioned doses are not to be understood as the effective doses of the combinations according to the invention that do in fact reach the lung. It is clear for the person of ordinary skill in the art that the delivered dose to the lung is generally lower than the metered dose of the administered active ingredients.

### A) Inhalable powder containing the combinations of active substances 1 and 2 according to the invention:

The inhalable powders according to the invention may contain **1** and **2** either on their own or in admixture with suitable physiologically acceptable excipients.
If the active substances **1** and **2** are present in admixture with physiologically acceptable excipients, the following physiologically acceptable excipients may be used to prepare these inhalable powders according to the invention: monosaccharides (e.g. glucose or arabinose), disaccharides (e.g. lactose, saccharose, maltose, trehalose), oligo- and polysaccharides (e.g. dextran), polyalcohols (e.g. sorbitol, mannitol, xylitol), cyclodextrines (e.g. α-cyclodextrine, β-cyclodextrine, χ-cyclodextrine, methyl-β-cyclodextrine, hydroxypropyl-β-cyclodextrine), salts (e.g. sodium chloride, calcium carbonate) or mixtures of these excipients with one another. Preferably, mono- or disaccharides are used, while the use of lactose, trehalose or glucose is preferred, particularly, but not exclusively, in the form of their hydrates.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipient mentioned above. These finer excipients are also selected from the group of possible excipients listed hereinbefore. Finally, in order to prepare the inhalable powders according to the invention, micronised active substance **1** and **2**, preferably with an average particle size of 0.5 to 10µm, more preferably from 1 to 6µm, is added to the excipient mixture. Processes for producing the inhalable powders according to the invention by grinding and micronising and by finally mixing the ingredients together are known from the prior art. The inhalable powders according to the invention may be prepared and administered either in the form of a single powder mixture which contains both **1** and **2** or in the form of separate inhalable powders which contain only **1** or **2**.

The inhalable powders according to the invention may be administered using inhalers known from the prior art. Inhalable powders according to the invention which contain one or more physiologically acceptable excipients in addition to **1** and **2** may be administered, for example, by means of inhalers which deliver a single dose from a supply using a measuring chamber as described in US 4570630A, or by other means as described in DE 36 25 685 A. The inhalable powders according to the invention which contain **1** and **2** optionally in conjunction with a physiologically acceptable excipient may be administered, for example, using the inhaler known by the name Turbuhaler^{®} or using inhalers as disclosed for example in EP 237507 A. Preferably, the inhalable powders according to the invention which contain physiologically acceptable excipient in addition to **1** and **2** are packed into capsules (to produce so-called inhalettes) which are used in inhalers as described, for example, in WO 94/28958.

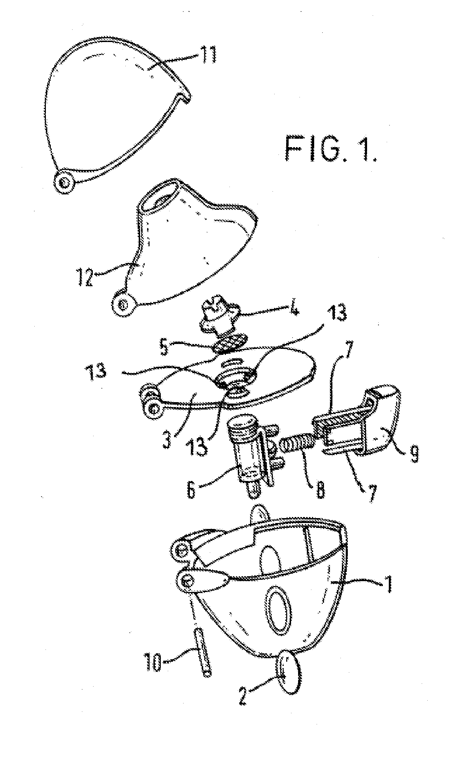

A particularly preferred inhaler for using the pharmaceutical combination according to the invention in inhalettes is shown in Figure 1.
This inhaler (Handyhaler) for inhaling powdered pharmaceutical compositions from capsules is characterised by a housing 1 containing two windows 2, a deck 3 in which there are air inlet ports and which is provided with a screen 5 secured via a screen housing 4, an inhalation chamber 6 connected to the deck 3 on which there is a push button 9 provided with two sharpened pins 7 and movable counter to a spring 8, and a mouthpiece 12 which is connected to the housing 1, the deck 3 and a cover 11 via a spindle 10 to enable it to be flipped open or shut, as well as airholes 13 for adjusting the flow resistance.

If the inhalable powders according to the invention are packed into capsules (inhalers) for the preferred use described above, the quantities packed into each capsule should be 1 to 30mg per capsule. These capsules contain, according to the invention, either together or separately, the doses of **1** and **2** or **2'** mentioned hereinbefore for each single dose.

### B) Propellant gas-driven inhalation aerosols containing the combinations of active substances 1 and 2:

Inhalation aerosols containing propellant gas according to the invention may contain substances **1** and **2** dissolved in the propellant gas or in dispersed form. **1** and **2** may be present in separate formulations or in a single preparation, in which **1** and **2** are either both dissolved, both dispersed or only one component is dissolved and the other is dispersed. The propellant gases which may be used to prepare the inhalation aerosols according to the invention are known from the prior art. Suitable propellant gases are selected from among hydrocarbons such as n-propane, n-butane or isobutane and halohydrocarbons such as fluorinated derivatives of methane, ethane, propane, butane, cyclopropane or cyclobutane. The propellant gases mentioned above may be used on their own or in mixtures thereof. Particularly preferred propellant gases are halogenated alkane derivatives selected from TG11, TG12, TG134a (1,1,1,2-tetrafluoroethane) and TG227 (1,1,1,2,3,3,3-heptafluoropropane) and mixtures thereof, of which the propellant gases TG134a, TG227 and mixtures thereof are preferred.

The propellant-driven inhalation aerosols according to the invention may also contain other ingredients such as co-solvents, stabilisers, surfactants, antioxidants, lubricants and pH adjusters. All these ingredients are known in the art.

The inhalation aerosols containing propellant gas according to the invention may contain up to 5 wt.-% of active substance **1** and/or **2**. Aerosols according to the invention contain, for example, 0.002 to 5 wt.-%, 0.01 to 3 wt.-%, 0.015 to 2 wt.-%, 0.1 to 2 wt.-%, 0.5 to 2 wt.-% or 0.5 to 1 wt.-% of active substance **1** and/or **2**.

If the active substances **1** and/or **2** are present in dispersed form, the particles of active substance preferably have an average particle size of up to 10µm, preferably from 0.1 to 6µm, more preferably from 1 to 5µm.

The propellant-driven inhalation aerosols according to the invention mentioned above may be administered using inhalers known in the art (MDIs = metered dose inhalers). Accordingly, in another aspect, the present invention relates to pharmaceutical compositions in the form of propellant-driven aerosols as hereinbefore described combined with one or more inhalers suitable for administering these aerosols. In addition, the present invention relates to inhalers which are **characterised in that** they contain the propellant gas-containing aerosols described above according to the invention. The present invention also relates to cartridges fitted with a suitable valve which can be used in a suitable inhaler and which contain one of the above-mentioned propellant gas-containing inhalation aerosols according to the invention. Suitable cartridges and methods of filling these cartridges with the inhalable aerosols containing propellant gas according to the invention are known from the prior art.

### C) Propellant-free inhalable solutions or suspensions containing the combinations of active substances 1 and 2 according to the invention:

Propellant-free inhalable solutions and suspensions according to the invention contain, for example, aqueous or alcoholic, preferably ethanolic solvents, optionally ethanolic solvents mixed with aqueous solvents. If aqueous/ethanolic solvent mixtures are used the relative proportion of ethanol compared with water is not limited but preferably the maximum is up to 70 percent by volume, more particularly up to 60 percent by volume of ethanol. The remainder of the volume is made up of water. The solutions or suspensions containing **1** and **2**, separately or together, are adjusted to a pH of 2 to 7, preferably 2 to 5, using suitable acids. The pH may be adjusted using acids selected from inorganic or organic acids. Examples of particularly suitable inorganic acids include hydrochloric acid, hydrobromic acid, nitric acid, sulphuric acid and/or phosphoric acid. Examples of particularly suitable organic acids include ascorbic acid, citric acid, malic acid, tartaric acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g. as flavourings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium editate, as stabiliser or complexing agent is unnecessary in the present formulation. Other embodiments may contain this compound or these compounds. In a preferred embodiment the content based on sodium editate is less than 100mg/100ml, preferably less than 50mg/100 ml, more preferably less than 20mg/100 ml. Generally, inhalable solutions in which the content of sodium editate is from 0 to 10mg/100ml are preferred.

Co-solvents and/or other excipients may be added to the propellant-free inhalable solutions according to the invention. Preferred co-solvents are those which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation. Preferably, these substances have no pharmacological effect or, in connection with the desired therapy, no appreciable or at least no undesirable pharmacological effect. The excipients and additives include, for example, surfactants such as soya lecithin, oleic acid, sorbitan esters, such as polysorbates, polyvinylpyrrolidone, other stabilisers, complexing agents, antioxidants and/or preservatives which guarantee or prolong the shelf life of the finished pharmaceutical formulation, flavourings, vitamins and/or other additives known in the art. The additives also include pharmacologically acceptable salts such as sodium chloride as isotonic agents. The preferred excipients include antioxidants such as ascorbic acid, for example, provided that it has not already been used to adjust the pH, vitamin A, vitamin E, tocopherols and similar vitamins and provitamins occurring in the human body.

Preservatives may be used to protect the formulation from contamination with pathogens. Suitable preservatives are those which are known in the art, particularly cetyl pyridinium chloride, benzalkonium chloride or benzoic acid or benzoates such as sodium benzoate in the concentration known from the prior art. The preservatives mentioned above are preferably present in concentrations of up to 50mg/100ml, more preferably between 5 and 20mg/100ml.

Preferred formulations contain, in addition to the solvent water and the combination of active substances **1** and **2**, only benzalkonium chloride and sodium editate. In another preferred embodiment, no sodium editate is present.

The propellant-free inhalable solutions according to the invention are administered in particular using inhalers of the kind which are capable of nebulising a small amount of a liquid formulation in the therapeutic dose within a few seconds to produce an aerosol suitable for therapeutic inhalation. Within the scope of the present invention, preferred inhalers are those in which a quantity of less than 100µL, preferably less than 50µL, more preferably between 20 and 30µL of active substance solution can be nebulised in preferably one spray action to form an aerosol with an average particle size of less than 20µm, preferably less than 10µm, in such a way that the inhalable part of the aerosol corresponds to the therapeutically effective quantity.

An apparatus of this kind for propellant-free delivery of a metered quantity of a liquid pharmaceutical composition for inhalation is described for example in International Patent Application WO 91/14468 and also in WO 97/12687 (cf. in particular Figures 6a and 6b). The nebulisers (devices) described therein are known by the name Respimat®.
This nebuliser (Respimat®) can advantageously be used to produce the inhalable aerosols according to the invention containing the combination of active substances **1** and **2**. Because of its cylindrical shape and handy size of less than 9 to 15 cm long and 2 to 4 cm wide, this device can be carried at all times by the patient. The nebuliser sprays a defined volume of pharmaceutical formulation using high pressures through small nozzles so as to produce inhalable aerosols.

The preferred atomiser essentially consists of an upper housing part, a pump housing, a nozzle, a locking mechanism, a spring housing, a spring and a storage container, characterised by
- a pump housing which is secured in the upper housing part and which comprises at one end a nozzle body with the nozzle or nozzle arrangement,
- a hollow plunger with valve body,
- a power takeoff flange in which the hollow plunger is secured and which is located in the upper housing part,
- a locking mechanism situated in the upper housing part,
- a spring housing with the spring contained therein, which is rotatably mounted on the upper housing part by means of a rotary bearing,
- a lower housing part which is fitted onto the spring housing in the axial direction.

The hollow plunger with valve body corresponds to a device disclosed in WO 97/12687. It projects partially into the cylinder of the pump housing and is axially movable within the cylinder. Reference is made in particular to Figures 1 to 4, especially Figure 3, and the relevant parts of the description. The hollow plunger with valve body exerts a pressure of 5 to 60 Mpa (about 50 to 600 bar), preferably 10 to 60 Mpa (about 100 to 600 bar) on the fluid, the measured amount of active substance solution, at its high pressure end at the moment when the spring is actuated. Volumes of 10 to 50 microlitres are preferred, while volumes of 10 to 20 microlitres are particularly preferred and a volume of 15 microlitres per spray is most particularly preferred.

The valve body is preferably mounted at the end of the hollow plunger facing the valve body.

The nozzle in the nozzle body is preferably microstructured, i.e. produced by microtechnology. Microstructured nozzle bodies are disclosed for example in WO 94/07607; reference is hereby made to the contents of this specification, particularly Figure 1 therein and the associated description.

The nozzle body consists for example of two sheets of glass and/or silicon firmly joined together, at least one of which has one or more microstructured channels which connect the nozzle inlet end to the nozzle outlet end. At the nozzle outlet end there is at least one round or non-round opening 2 to 10 microns deep and 5 to 15 microns wide, the depth preferably being 4.5 to 6.5 microns while the length is preferably 7 to 9 microns.
In the case of a plurality of nozzle openings, preferably two, the directions of spraying of the nozzles in the nozzle body may extend parallel to one another or may be inclined relative to one another in the direction of the nozzle opening. In a nozzle body with at least two nozzle openings at the outlet end the directions of spraying may be at an angle of 20 to 160° to one another, preferably 60 to 150°, most preferably 80 to 100°. The nozzle openings are preferably arranged at a spacing of 10 to 200 microns, more preferably at a spacing of 10 to 100 microns, most preferably 30 to 70 microns. Spacings of 50 microns are most preferred. The directions of spraying will therefore meet in the vicinity of the nozzle openings.

The liquid pharmaceutical preparation strikes the nozzle body with an entry pressure of up to 600 bar, preferably 200 to 300 bar, and is atomised into an inhalable aerosol through the nozzle openings. The preferred particle or droplet sizes of the aerosol are up to 20 microns, preferably 3 to 10 microns.

The locking mechanism contains a spring, preferably a cylindrical helical compression spring, as a store for the mechanical energy. The spring acts on the power takeoff flange as an actuating member the movement of which is determined by the position of a locking member. The travel of the power takeoff flange is precisely limited by an upper and lower stop. The spring is preferably biased, via a power step-up gear, e.g. a helical thrust gear, by an external torque which is produced when the upper housing part is rotated counter to the spring housing in the lower housing part. In this case, the upper housing part and the power takeoff flange have a single or multiple V-shaped gear.

The locking member with engaging locking surfaces is arranged in a ring around the power takeoff flange. It consists, for example, of a ring of plastic or metal which is inherently radially elastically deformable. The ring is arranged in a plane at right angles to the atomiser axis. After the biasing of the spring, the locking surfaces of the locking member move into the path of the power takeoff flange and prevent the spring from relaxing. The locking member is actuated by means of a button. The actuating button is connected or coupled to the locking member. In order to actuate the locking mechanism, the actuating button is moved parallel to the annular plane, preferably into the atomiser; this causes the deformable ring to deform in the annular plane. Details of the construction of the locking mechanism are given in WO 97/20590.

The lower housing part is pushed axially over the spring housing and covers the mounting, the drive of the spindle and the storage container for the fluid.
When the atomiser is actuated the upper housing part is rotated relative to the lower housing part, the lower housing part taking the spring housing with it. The spring is thereby compressed and biased by means of the helical thrust gear and the locking mechanism engages automatically. The angle of rotation is preferably a whole-number fraction of 360 degrees, e.g. 180 degrees. At the same time as the spring is biased, the power takeoff part in the upper housing part is moved along by a given distance, the hollow plunger is withdrawn inside the cylinder in the pump housing, as a result of which some of the fluid is sucked out of the storage container and into the high pressure chamber in front of the nozzle.

If desired, a number of exchangeable storage containers which contain the fluid to be atomised may be pushed into the atomiser one after another and used in succession. The storage container contains the aqueous aerosol preparation according to the invention.
The atomising process is initiated by pressing gently on the actuating button. As a result, the locking mechanism opens up the path for the power takeoff member. The biased spring pushes the plunger into the cylinder of the pump housing. The fluid leaves the nozzle of the atomiser in atomised form.

Further details of construction are disclosed in PCT Applications WO 97/12683 and WO 97/20590, to which reference is hereby made.
The components of the atomiser (nebuliser) are made of a material which is suitable for its purpose. The housing of the atomiser and, if its operation permits, other parts as well, are preferably made of plastics, e.g. by injection moulding. For medicinal purposes, physiologically safe materials are used.

Figures 6a/b of WO 97/12687, show the nebuliser (Respimat®) which can advantageously be used for inhaling the aqueous aerosol preparations according to the invention.
Figure 6a of WO 97/12687 shows a longitudinal section through the atomiser with the spring biased while Figure 6b of WO 97/12687 shows a longitudinal section through the atomiser with the spring relaxed.
The upper housing part (51) contains the pump housing (52) on the end of which is mounted the holder (53) for the atomiser nozzle. In the holder is the nozzle body (54) and a filter (55). The hollow plunger (57) fixed in the power takeoff flange (56) of the locking mechanism projects partially into the cylinder of the pump housing. At its end the hollow plunger carries the valve body (58). The hollow plunger is sealed off by means of the seal (59). Inside the upper housing part is the stop (60) on which the power takeoff flange abuts when the spring is relaxed. On the power takeoff flange is the stop (61) on which the power takeoff flange abuts when the spring is biased. After the biasing of the spring the locking member (62) moves between the stop (61) and a support (63) in the upper housing part. The actuating button (64) is connected to the locking member. The upper housing part ends in the mouthpiece (65) and is sealed off by means of the protective cover (66) which can be placed thereon.
The spring housing (67) with compression spring (68) is rotatably mounted on the upper housing part by means of the snap-in lugs (69) and rotary bearing. The lower housing part (70) is pushed over the spring housing. Inside the spring housing is the exchangeable storage container (71) for the fluid (72) which is to be atomised. The storage container is sealed off by the stopper (73) through which the hollow plunger projects into the storage container and is immersed at its end in the fluid (supply of active substance solution).
The spindle (74) for the mechanical counter is mounted in the covering of the spring housing. At the end of the spindle facing the upper housing part is the drive pinion (75). The slider (76) sits on the spindle.

The nebuliser described above is suitable for nebulising the aerosol preparations according to the invention to produce an aerosol suitable for inhalation.
If the formulation according to the invention is nebulised using the method described above (Respimat®) the quantity delivered should correspond to a defined quantity with a tolerance of not more than 25%, preferably 20% of this amount in at least 97%, preferably at least 98% of all operations of the inhaler (spray actuations). Preferably, between 5 and 30 mg of formulation, most preferably between 5 and 20 mg of formulation are delivered as a defined mass on each actuation.

However, the formulation according to the invention may also be nebulised by means of inhalers other than those described above, e.g. jet stream inhalers or other stationary nebulisers.

Accordingly, in a further aspect, the invention relates to pharmaceutical formulations in the form of propellant-free inhalable solutions or suspensions as described above combined with a device suitable for administering these formulations, preferably in conjunction with the Respimat®. Preferably, the invention relates to propellant-free inhalable solutions or suspensions characterised by the combination of active substances **1** and **2** according to the invention in conjunction with the device known by the name Respimat®. In addition, the present invention relates to the above-mentioned devices for inhalation, preferably the Respimat®, **characterised in that** they contain the propellant-free inhalable solutions or suspensions according to the invention as described hereinbefore.

According to the invention, inhalable solutions which contain the active substances **1** and **2** in a single preparation are preferred. The term "single preparation" also includes preparations which contain the two ingredients **1** and **2** in two-chamber cartridges, as disclosed for example in WO 00/23037. Reference is hereby made to this publication in its entirety.

The propellant-free inhalable solutions or suspensions according to the invention may take the form of concentrates or sterile inhalable solutions or suspensions ready for use, as well as the above-mentioned solutions and suspensions designed for use in a Respimat®. Formulations ready for use may be produced from the concentrates, for example, by the addition of isotonic saline solutions. Sterile formulations ready for use may be administered using energy-operated free-standing or portable nebulisers which produce inhalable aerosols by means of ultrasound or compressed air by the Venturi principle or other principles.

Accordingly, in another aspect, the present invention relates to pharmaceutical compositions in the form of propellant-free inhalable solutions or suspensions as described hereinbefore which take the form of concentrates or sterile formulations ready for use, combined with a device suitable for administering these solutions, **characterised in that** the device is an energy-operated free-standing or portable nebuliser which produces inhalable aerosols by means of ultrasound or compressed air by the Venturi principle or other methods.

The Examples which follow serve to illustrate the present invention in more detail without restricting the scope of the invention to the following embodiments by way of example.

### Examples of Formulations

The following examples of formulations, which may be obtained analogously to methods known in the art, serve to illustrate the present invention more fully without restricting it to the contents of these examples. Examples of formulations comprising an EGFR kinase inhibitor **1** selected from compounds **1.1 to 1.105** as the only active ingredient are disclosed in the prior art, e.g in WO 96/30347; WO 97/02266; WO 99/35146; WO 00/31048; WO 00/78735; WO 01/34574; WO 01/61816; WO 01/77104; WO02/18351; WO 02/18372; WO 02/18373; WO 02/18376; WO 02/50043; WO 03/082290; Cancer Research 2004, 64:11 (3958-3965); Am J Health-Syst Pharm 2000, 57(15), 2063-2076; Clinical Therapeutics 1999, 21(2), 309-318; WO 98/50433; and WO 95/20045.

Formulations comprising an EGFR kinase inhibitor **1** and a beta-2 mimetic **2a**

### Inhalable Formulations:

Examples of binary formulations comprising an EGFR kinase inhibitor **1** selected from compounds **1.1 to 1.105** and a beta-2 mimetic **2a** as the active ingredients:
A) Inhalable powders:

**Example 1:**

| **Ingredients** | **µg per capsule** |
|---|---|
| **1** | 150 |
| Formoterol fumarate dihydrate (**2a.1**) | 50 |
| Lactose | 12300 |
| **Total** | 12500 |

**Example 2:**

| **Ingredients** | **µg per capsule** |
|---|---|
| **1** | 150 |
| Salmeterol xinafoate (**2a.2**) | 50 |
| Lactose | 12300 |
| **Total** | 12500 |

**Example 3:**

| **Ingredients** | **µg per capsule** |
|---|---|
| **1** | 1500 |
| Salmeterol xinafoate (**2a.2**) | 150 |
| Lactose | 12200 |
| **Total** | 13850 |

**Example 4:**

| **Ingredients** | **µg per capsule** |
|---|---|
| **1** | 200 |
| Formoterol fumarate dihydrate (**2a.1**) | 50 |
| Lactose | 24750 |
| **Total** | 25000 |

B) Propellant-containing inhalable aerosols (suspension aerosols):

**Example 5:**

| **Ingredients** | **% by weight** |
|---|---|
| **1** | 0.50 |
| Salmeterol xinafoate (**2a.2**) | 0.066 |
| Soya lecithin | 0.2 |
| TG 134a : TG 227 = 2:3 | ad 100 |

**Example 6:**

| **Ingredients** | **% by weight** |
|---|---|
| **1** | 0.080 |
| Salmeterol xinafoate (**2a.2**) | 0.033 |
| Absolute ethanol | 0.5 |
| Isopropyl myristate | 0.1 |
| TG 227 | ad 100 |

**Example 7:**

| **Ingredients** | **% by weight** |
|---|---|
| **1** | 0.050 |
| Formoterol fumarate dihydrate (**2a.1**) | 0.035 |
| Soya lecithin | 0.2 |
| TG 134a : TG 227 = 2:3 | ad 100 |

Ternary or quartenary formulations containing three or four actives may be prepared in analogy to the binary formulations described in examples 1 to 7, e.g. by incorporating two components **1** and/or two components **2a** instead of only one of these components. The amounts given in the tables should in this case be understood as the sum of amounts for the corresponding actives.

Formulations comprising an EGFR kinase inhibitor **1** and a steroid **2b**

### Inhalable Formulations:

Examples of binary formulations comprising an EGFR kinase inhibitor **1** selected from compounds **1.1 to 1.105** and a steroid **2b** as the active ingredients:
A) Inhalable powders

**Example 8**

| **Ingredients** | **µg per capsule** |
|---|---|
| **1** | 100 |
| budesonide **2b.1** | 200 |
| lactose | 4750 |
| **Total** | 5050 |

**Example 9**

| **Ingredients** | **µg per capsule** |
|---|---|
| **1** | 100 |
| Fluticasone-propionate **2b.2** | 125 |
| lactose | 4825 |
| **Total** | 5050 |

**Example 10**

| **Ingredients** | **µg per capsule** |
|---|---|
| **1** | 200 |
| Mometasone-furoate X H₂O **2b.3** | 250 |
| lactose | 4700 |
| **Total** | 5150 |

**Example 11**

| **Ingredients** | **µg per capsule** |
|---|---|
| **1** | 2000 |
| Ciclesonide **2b.4** | 250 |
| lactose | 4700 |
| **Total** | 6950 |

**Example 12**

| **Ingredients** | **µg per capsule** |
|---|---|
| **1** | 500 |
| budesonide **2b.1** | 125 |
| lactose | 4855 |
| **Total** | 5480 |

**Example 13**

| **Ingredients** | µ**g per capsule** |
|---|---|
| **1** | 500 |
| Fluticasone-propionate **2b.2** | 200 |
| lactose | 4780 |
| **Total** | 5480 |

**Example 14**

| **Ingredients** | µ**g per capsule** |
|---|---|
| **1** | 250 |
| Mometasone-furoate X H₂O **2b.3** | 250 |
| lactose | 4725 |
| **Total** | 5225 |

**Example 15**

| **Ingredients** | µ**g per capsule** |
|---|---|
| **1** | 750 |
| Ciclesonide **2b.4** | 250 |
| lactose | 4725 |
| **Total** | 5725 |

**Example 16**

| **Ingredients** | µ**g per capsule** |
|---|---|
| **1** | 1000 |
| budesonide **2b.1** | 125 |
| lactose | 4865 |
| **Total** | 5990 |

**Example 17**

| **Ingredients** | µ**g per capsule** |
|---|---|
| **1** | 1000 |
| Fluticasone-propionate **2b.2** | 200 |
| lactose | 4790 |
| **Total** | 5990 |

**Example 18**

| **Ingredients** | µ**g per capsule** |
|---|---|
| **1** | 1500 |
| Mometasone-furoate X H₂O **2b.3** | 250 |
| lactose | 4735 |
| **Total** | 6485 |

B) Propellant-containing aerosols for inhalation:

**Example 19**

| **Ingredients** | **% by weight** |
|---|---|
| **1** | 1 |
| budesonide **2b.1** | 0.4 |
| soya lecithin | 0.2 |
| TG 134a : TG227 = 2:3 | ad 100 |

**Example 20**

| **Ingredients** | **% by weight** |
|---|---|
| **1** | 1 |
| Fluticasone-propionate **2b.2** | 0.3 |
| Isopropyl myristate | 0.1 |
| TG 227 | ad 100 |

**Example 21**

| **Ingredients** | **% by weight** |
|---|---|
| **1** | 1 |
| Mometasone-furoate X H₂O **2b.3** | 0.6 |
| Isopropyl myristate | 0.1 |
| TG 227 | ad 100 |

**Example 22**

| **Ingredients** | **% by weight** |
|---|---|
| 1 | 1 |
| Ciclesonide **2b.4** | 0.4 |
| Isopropyl myristate | 0.1 |
| TG 134a : TG227 = 2:3 | ad 100 |

Ternary or quartenary formulations containing three or four actives may be prepared in analogy to the binary formulations described in examples 30 to 33, e.g. by incorporating two components **1** and/or two components **2b** instead of only one of these components. The amounts given in the tables should in this case be understood as the sum of amounts for the corresponding actives.

Examples: Inhalable ternary formulations comprising an EGFR kinase inhibitor **1** selected from compounds **1.1 to 1.101,** a beta₂ agonist **2a** and a steroid **2b**
A) Inhalable powders:

**Example 23**

| **Ingredients** | µ**g per capsule** |
|---|---|
| **1** | 1000 |
| budesonide | 200 |
| salmeterol x ½ H₂SO4 | 55.9 |
| lactose | 4744.1 |
| **Total** | 6000 |

**Example 24**

| **Ingredients** | µ**g per capsule** |
|---|---|
| **1** | 500 |
| fluticasone propionate | 125 |
| salmeterol xinafoate | 50 |
| lactose | 4825 |
| **Total** | 5500 |

**Example 25**

| **Ingredients** | µ**g per capsule** |
|---|---|
| **1** | 1000 |
| mometasone furoate | 250 |
| formoterol fumarate dihydrate | 12 |
| lactose | 4738 |
| **Total** | 6000 |

**Example 26**

| **Ingredients** | µ**g per capsule** |
|---|---|
| **1** | 1000 |
| fluticasone propionate | 250 |
| formoterol fumarate dihydrate | 12 |
| lactose | 4738 |
| **Total** | 6000 |

**Example 27**

| **Ingredients** | µ**g per capsule** |
|---|---|
| **1** | 5000 |
| formoterol fumarate dihydrate | 12 |
| fluticasone propionate | 250 |
| lactose | 24738 |
| **Total** | 30000 |

B) Inhalable aerosols containing propellant gas (suspension aerosols):

**Example 28**

| **Ingredients** | **Wt-%** |
|---|---|
| **1** | 2.9 |
| budesonide | 0.4 |
| salmeterol x ½ H2SO4 | 0.066 |
| soya lecithin | 0.2 |
| TG 134a : TG227 = 2:3 | ad 100 |

**Example 29**

| **Ingredients** | **Wt-%** |
|---|---|
| **1** | 2.9 |
| fluticasone propionate | 0.3 |
| salmeterol xinafoate | 0.033 |
| isopropyl myristate | 0.1 |
| TG 227 | ad 100 |

**Example 30**

| **Ingredients** | **Wt-%** |
|---|---|
| **1** | 2.9 |
| mometasone furoate | 0.6 |
| salmeterol x ½ H2SO4 | 0.066 |
| isopropyl myristate | 0.1 |
| TG 227 | ad 100 |

**Example 31**

| **Ingredients** | **Wt-%** |
|---|---|
| **1** | 2.0 |
| fluticasone propionate | 0.3 |
| salmeterol x ½ H₂SO4 | 0.066 |
| soya lecithin | 0.2 |
| TG 11 : TG12 = 2:3 | ad 100 |

**Example 32**

| **Ingredients** | **Wt-%** |
|---|---|
| **1** | 3.9 |
| salmeterol xinafoate | 0.033 |
| budesonide | 0.4 |
| absolute ethanol | 0.5 |
| isopropyl myristate | 0.1 |
| TG 227 | ad 100 |

**Example 33**

| **Ingredients** | **Wt-%** |
|---|---|
| **1** | 1.17 |
| budesonide | 0.4 |
| salmeterol x ½ H₂SO4 | 0.047 |
| absolute ethanol | 30 |
| purified water | 1.5 |
| anhydrous citric acid | 0.002 |
| TG 134a | ad 100 |

Formulations comprising an EGFR kinase inhibitor **1** and a PDE-IV inhibitor **2c**

### Inhalable Formulations:

Examples of binary formulations comprising an EGFR kinase inhibitor **1** selected from compounds **1.1** to **1.101** and a PDE-IV inhibitor **2c** as the active ingredients:
A) Inhalable powders

**Example 34**

| **Ingredients** | µ**g per capsule** |
|---|---|
| **1** | 250 |
| AWD-12-281 | 200 |
| Lactose | 12050 |
| **Total** | 12500 |

**Example 35**

| **Ingredients** | µ**g per capsule** |
|---|---|
| **1** | 300 |
| AWD-12-281 | 100 |
| Lactose | 12100 |
| Total | 12500 |

**Example 36**

| **Ingredients** | µ**g per capsule** |
|---|---|
| **1** | 100 |
| AWD-12-281 | 300 |
| Lactose | 12100 |
| **Total** | 12500 |

**Example 37**

| **Ingredients** | µ**g per capsule** |
|---|---|
| **1** | 500 |
| ariflo | 200 |
| Lactose | 12050 |
| **Total** | 12750 |

**Example 38**

| **Ingredients** | µ**g per capsule** |
|---|---|
| **1** | 1000 |
| ariflo | 100 |
| Lactose | 12100 |
| **Total** | 13200 |

**Example 39**

| **Ingredients** | µ**g per capsule** |
|---|---|
| **1** | 100 |
| ariflo | 300 |
| Lactose | 12100 |
| **Total** | 12500 |

**Example 40**

| **Ingredients** | µ**g per capsule** |
|---|---|
| **1** | 250 |
| roflumilast | 200 |
| Lactose | 12050 |
| **Total** | 12500 |

**Example 41**

| **Ingredients** | µ**g per capsule** |
|---|---|
| **1** | 300 |
| roflumilast | 100 |
| Lactose | 12100 |
| **Total** | 12500 |

**Example 42**

| **Ingredients** | µ**g per capsule** |
|---|---|
| **1** | 100 |
| roflumilast | 300 |
| Lactose | 12100 |
| **Total** | 12500 |

**Example 43**

| **Ingredients** | µ**g per capsule** |
|---|---|
| **1** | 250 |
| Z-15370 | 200 |
| Lactose | 12050 |
| **Total** | 12500 |

**Example 44**

| **Ingredients** | µ**g per capsule** |
|---|---|
| **1** | 300 |
| Z-15370 | 100 |
| Lactose | 12100 |
| **Total** | 12500 |

**Example 45**

| **Ingredients** | µ**g per capsule** |
|---|---|
| **1** | 100 |
| Z-15370 | 300 |
| Lactose | 12100 |
| **Total** | 12500 |

**Example 46**

| **Ingredients** | µ**g per capsule** |
|---|---|
| **1** | 300 |
| AWD-12-281 | 2000 |
| Lactose | 12800 |
| **Total** | 15000 |

**Example 47**

| **Ingredients** | µ**g per capsule** |
|---|---|
| **1** | 100 |
| AWD-12-281 | 2000 |
| Lactose | 12900 |
| **Total** | 15000 |

**Example 48**

| **Ingredients** | µ**g per capsule** |
|---|---|
| **1** | 300 |
| **2c'**^{*)} | 100 |

| Lactose | 12100 |
|---|---|
| **Total** | 12500 |

| | |
|---|---|
| *) **2c'** = each of the following compounds: 2-(4-fluoro-phenoxy)-N-{4-[(2hydroxy-3-methyl-benzoylamino)-methyl]-benzyl}-nicotinamide, 3-(3-{4-[(3-Hydroxy-benzoylamino)-methyl]-benzylcarbamoyl}-pyridin-2-yl-oxy)-benzoic acid ethylester, 2-(4-fluoro-phenoxy)-N-{4-[(6-fluoro-2-hydroxy-benzoylamino)-methyl]-benzyl}-nicotinamide, 2-(4-fluoro-phenoxy)-N-{4-[(5-fluoro-2-hydroxy-benzoylamino)-methyl]-benzyl}-nicotinamide, 2-(4-fluoro-phenoxy)-N-{4-[(3-hydroxy-4-methyl-benzoylamino)methyl]benzyl}-nicotinamide, 2-(4-fluoro-phenoxy)-N-{4-[(3-hydroxy-benzoylamino)-methyl]-benzyl}-nicotinamide, 2-(4-fluoro-phenoxy)-N-{4-[(2-hydroxy-benzoylamino)methyl]-benzyl}-nicotinamide, 2-(4-fluoro-phenoxy)-N-{4-[(4-hydroxy-benzoylamino)methyl]-benzyl}-nicotinamide, 2-(4-fluoro-phenoxy)-N-{4-[(2-hydroxy-4-methyl-benzoylamino)methyl]-benzyl}-nicotinamide, 2-(4-fluoro-phenoxy)-N-{4-[(3-hydroxy-2-methyl-benzoylamino)methyl]-benzyl}-nicotinamide, 2-(4-fluoro-phenoxy)-N-{4-[(2-hydroxy-5-methyl-benzoylamino)methyl]-benzyl}-nicotinamide, 5-fluoro-2-(4-fluoro-phenoxy)-N-{4-[(2-hydroxy-benzoylamino)methyl]-benzyl}-nicotinamide, 5-fluoro-2-(4-fluoro-phenoxy)-N-{4-[(2-hydroxy-acetylamino)methyl]-benzyl}-nicotinamide, 5-fluoro-2-(4-fluoro-phenoxy)-N-{4-[(4-hydroxy-benzoylamino)methyl]-benzyl} nicotinamide, 3-(3-{4-[(3-hydroxy-benzoylamino)methyl]-benzylcarbamoyl}-pyridin-2-yloxy)benzoic acid ethylester, 3-(3-{4-[(2-hydroxy-phenacetylamino)methyl]-benzylcarbamoyl}-pyridin-2-yloxy)benzoic acid ethylester, 3-(3-{4-[(3-hydroxy-phenacetylamino)methyl]-benzylcarbamoyl}-pyridin-2-yloxy)benzoic acid ethyl ester, and 3-(3-{4-[(4-hydroxy-phenacetylamino)methyl]-benzylcarbamoyl}-pyridin-2-yloxy)benzoic acid ethyl ester, compound **2c.4** | |

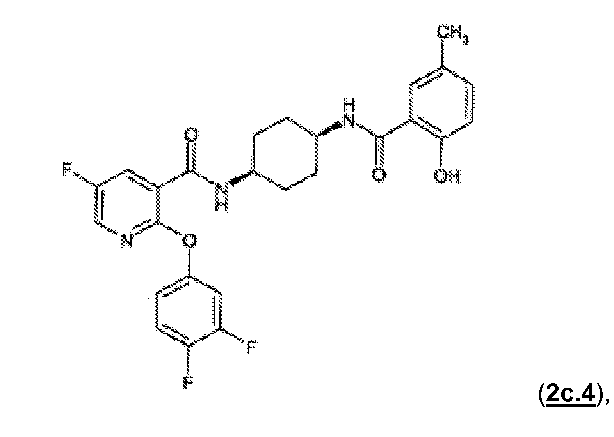

and
compound **2c.5** 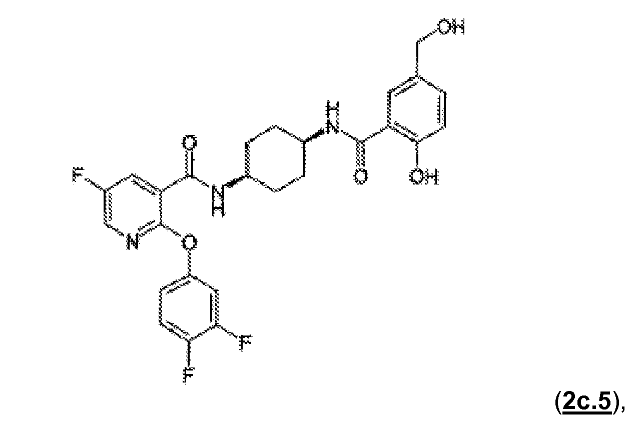 optionally in the form of the racemates, the enantiomers, the diastereomers and optionally the pharmacologically acceptable acid addition salts thereof, and the hydrates thereof.
B) Inhalable aerosols containing propellant gas (suspension aerosols):

**Example 49**

| **Ingredients** | **% by weight** |
|---|---|
| **1** | 0.050 |
| AWD-12-281 | 0.060 |
| Soya lecithin | 0.2 |
| TG 134a : TG 227 = 2:3 | ad 100 |

**Example 50**

| **Ingredients** | **% by weight** |
|---|---|
| **1** | 0.050 |
| ariflo | 0.035 |
| TG 134a | ad 100 |

**Example 51**

| **Ingredients** | **% by weight** |
|---|---|
| **1** | 0.050 |
| Z-15370 | 0.035 |
| TG 134a | ad 100 |

Formulations comprising an EGFR kinase inhibitor **1** and a p38 MAP kinase inhibitor **2d**

### Inhalable Formulations:

Examples of binary formulations comprising an EGFR kinase inhibitor **1** selected from compounds **1.1** to **1.101** and a p38 MAP kinase inhibitor **2d** as the active ingredients:
A) Inhalable powders

**Example 52**

| **Ingredients** | µ**g per capsule** |
|---|---|
| **1** | 100 |
| **2d.1** | 3500 |
| Lactose | 3480 |
| Total | 7080 |

**Example 53**

| **Ingredients** | µ**g per capsule** |
|---|---|
| **1** | 260 |
| **2d.1** | 3000 |
| Lactose | 3940 |
| **Total** | 720 |

**Example 54**

| **Ingredients** | µ**g per capsule** |
|---|---|
| **1** | 1000 |
| **2d.1** | 5000 |
| Lactose | 4900 |
| **Total** | 10900 |

**Example 55**

| **Ingredients** | µ**g per capsule** |
|---|---|
| **1** | 5000 |
| **2d.2** | 5000 |
| Lactose | 2000 |
| **Total** | 12000 |

**Example 56**

| **Ingredients** | µ**g per capsule** |
|---|---|
| **1** | 6000 |
| **2d.1** | 5000 |
| **Total** | 11000 |

**Example 57**

| **Ingredients** | µ**g per capsule** |
|---|---|
| **1** | 10000 |
| **2d.2** | 5000 |
| **Total** | 15000 |

**Example 58**

| **Ingredients** | µ**g per capsule** |
|---|---|
| **1** | 500 |
| **2d.2** | 3500 |
| Lactose | 3500 |
| **Total** | 7500 |

**Example 59**

| **Ingredients** | µ**g per capsule** |
|---|---|
| **1** | 1000 |
| **2d.2** | 3000 |
| Lactose | 4000 |
| **Total** | 8000 |

**Example 60**

| **Ingredients** | µ**g per capsule** |
|---|---|
| **1** | 800 |
| **2d.3** | 5000 |
| **Total** | 5800 |

Formulations comprising an EGFR kinase inhibitor **1** and a NK₁ antagonist **2e**

### Inhalable Formulations:

Examples of binary formulations comprising an EGFR kinase inhibitor **1** selected from compounds **1.1** to **1.101** and a NK₁ antagonist **2e** as the active ingredients:
A) Inhalable powders

**Example 61**

| **Ingredients** | µ**g per capsule** |
|---|---|
| **1** | 200 |
| **2e.1** | 150 |
| Lactose | 12150 |
| **Total** | 12500 |

**Example 62**

| **Ingredients** | µ**g per capsule** |
|---|---|
| **1** | 100 |
| **2e.1** | 125 |
| Lactose | 12350 |
| **Total** | 12500 |

**Example 63**

| **Ingredients** | µ**g per capsule** |
|---|---|
| **1** | 1000 |
| **2e.1** | 250 |
| Lactose | 12250 |
| **Total** | 13500 |

**Example 64**

| **Ingredients** | µ**g per capsule** |
|---|---|
| **1** | 5000 |
| **2e.1** | 400 |
| Lactose | 24600 |
| **Total** | 30000 |

Formulations comprising an EGFR kinase inhibitor **1** and an endothelin-antagonist **2f**

### Inhalable Formulations:

Examples of binary formulations comprising an EGFR kinase inhibitor **1** selected from compounds **1.1** to **1.101** and an endothelin-antagonist selected from **2f.1** to **2f.13** as the active ingredients:
A) Inhalable powders

**Example 65**

| **Ingredients** | µ**g per capsule** |
|---|---|
| **1** | 100 |
| **2f** | 125 |
| Lactose | 4775 |
| **Total** | 5000 |

**Example 66**

| **Ingredients** | µ**g per capsule** |
|---|---|
| **1** | 200 |
| **2f** | 150 |
| Lactose | 4650 |
| **Total** | 5000 |

**Example 67**

| **Ingredients** | µ**g per capsule** |
|---|---|
| **1** | 500 |
| **2f** | 400 |
| Lactose | 6100 |
| **Total** | 7000 |

**Example 68**

| **Ingredients** | µ**g per capsule** |
|---|---|
| **1** | 1000 |
| **2f** | 250 |
| Lactose | 5750 |
| **Total** | 7000 |

**Example 69**

| **Ingredients** | µ**g per capsule** |
|---|---|
| **1** | 5000 |
| **2f** | 5000 |
| Lactose | 24500 |
| **Total** | 34500 |

**Example 70**

| **Ingredients** | µ**g per capsule** |
|---|---|
| **1** | 10000 |
| **2f** | 1000 |
| Lactose | 24000 |
| **Total** | 35000 |

B) Propellant-containing inhalable aerosols (suspension aerosols):

**Example 71**

| **Ingredients** | **Wt-%** |
|---|---|
| **1** | 1,5 |
| **2f** | 1.2 |
| Sojalecithin | 0.3 |
| TG 134a : TG227 = 2:3 | ad 100 |

**Example 72**

| **Ingredients** | **Wt-%** |
|---|---|
| **1** | 2.9 |
| **2f** | 1.4 |
| Ethanol, absolute | 0.5 |
| Isopropylmyristate | 0.1 |
| TG 227 | ad 100 |

**Example 73**

| **Ingredients** | **Wt-%** |
|---|---|
| **1** | 0.29 |
| **2f** | 0.3 |
| Ethanol, absolute | 0.5 |
| Isopropylmyristate | 0.1 |
| TG 227 | ad 100 |

## Claims

1. Pharmaceutical composition comprising at least one EGFR kinase inhibitor **1** selected from the group consisting of compounds
(**1.1**) 4-[(3-chloro-4-fluorophenyl)amino]-6-{[4-(morpholin-4-yl)-1-oxo-2-buten-1-yl]-amino}-7-cyclopropylmethoxy-quinazoline,
(**1.2**) 4-[(3-chloro-4-fluorophenyl)amino]-6-{[4-(N,N-diethylamino)-1-oxo-2-buten-1-yl]amino}-7-cyclopropylmethoxy-quinazoline,
(**1.3**) 4-[(3-chloro-4-fluorophenyl)amino]-6-{[4-(N,N-dimethylamino)-1-oxo-2-buten-1-yl]amino}-7-cyclopropylmethoxy-quinazoline,
(**1.4**) 4-[(R)-(1-phenyl-ethyl)amino]-6-{[4-(morpholin-4-yl)-1-oxo-2-buten-1-yl]-amino}-7-cyclopentyloxy-quinazoline,
(**1.5**) 4-[(3-chloro-4-fluoro-phenyl)amino]-6-{[4-((R)-6-methyl-2-oxo-morpholin-4-yl)-1-oxo-2-buten-1-yl]amino}-7-cyclopropylmethoxy-quinazoline,
(**1.6**) 4-[(3-chloro-4-fluoro-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-quinazoline,
(**1.7**) 4-[(3-chloro-4-fluoro-phenyl)amino]-6-{[4-((R)-2-methoxymethyl-6-oxo-morpholin-4-yl)-1-oxo-2-buten-1-yl]amino}-7-cyclopropylmethoxy-quinazoline,
(**1.8**) 4-[(3-chloro-4-fluoro-phenyl)amino]-6-[2-((S)-6-methyl-2-oxo-morpholin-4-yl)-ethoxy]-7-methoxy-quinazoline,
(**1.9**) 4-[(3-chloro-4-fluorophenyl)amino]-6-({4-[N-(2-methoxy-ethyl)-N-methylamino]-1-oxo-2-buten-1-yl}amino)-7-cyclopropylmethoxy-quinazoline,
(**1.10**) 4-[(3-chloro-4-fluorophenyl)amino]-6-{[4-(N,N-dimethylamino)-1-oxo-2-buten-1-yl]amino}-7-cyclopentyloxy-quinazoline,
(**1.11**) 4-[(R)-(1-phenyl-ethyl)amino]-6-{[4-(N,N-bis-(2-methoxy-ethyl)-amino)-1-oxo-2-buten-1-yl]amino}-7-cyclopropylmethoxy-quinazoline,
(**1.12**) 4-[(R)-(1-phenyl-ethyl)amino]-6-({4-[N-(2-methoxy-ethyl)-N-ethyl-amino]-1-oxo-2-buten-1-yl}amino)-7-cyclopropylmethoxy-quinazoline,
(**1.13**) 4-[(R)-(1-phenyl-ethyl)amino]-6-({4-[N-(2-methoxy-ethyl)-N-methyl-amino]-1-oxo-2-buten-1-yl}amino)-7-cyclopropylmethoxy-quinazoline,
(**1.14**) 4-[(R)-(1-phenyl-ethyl)amino]-6-({4-[N-(tetrahydropyran-4-yl)-N-methyl-amino]-1-oxo-2-buten-1-yl}amino)-7-cyclopropylmethoxy-quinazoline,
(**1.15**) 4-[(3-chloro-4-fluorophenyl)amino]-6-{[4-(N,N-dimethylamino)-1-oxo-2-buten-1-yl]amino}-7-((R)-tetrahydrofuran-3-yloxy)-quinazoline,
(**1.16**) 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline,
(**1.17**) 4-[(3-chloro-4-fluorophenyl)amino]-6-({4-[N-(2-methoxy-ethyl)-N-methylamino]-1-oxo-2-buten-1-yl}amino)-7-cyclopentyloxy-quinazoline,
(**1.18**) 4-[(3-chloro-4-fluorophenyl)amino]-6-{[4-(N-cyclopropyl-N-methyl-amino)-1-oxo-2-buten-1-yl]amino}-7-cyclopentyloxy-quinazoline,
(**1.19**) 4-[(3-chloro-4-fluorophenyl)amino]-6-{[4-(N,N-dimethylamino)-1-oxo-2-buten-1-yl]amino}-7-[(R)-(tetrahydrofuran-2-yl)methoxy]-quinazoline,
(**1.20**) 4-[(3-chloro-4-fluorophenyl)amino]-6-{[4-(N,N-dimethylamino)-1-oxo-2-buten-1-yl]amino}-7-[(S)-(tetrahydrofuran-2-yl)methoxy]-quinazoline,
(**1.21**) 4-[(3-ethinyl-phenyl)amino]-6,7-bis-(2-methoxy-ethoxy)-quinazoline,
(**1.22**) 4-[(3-chloro-4-fluorophenyl)amino]-7-[3-(morpholin-4-yl)-propyloxy]-6-[(vinyl-carbonyl)amino]-quinazoline,
(**1.23**) 4-[(R)-(1-phenyl-ethyl)amino]-6-(4-hydroxy-phenyl)-7H-pyrrolo[2,3-d]pyrimidine,
(**1.24**) 3-cyano-4-[(3-chlor-4-fluorophenyl)amino]-6-{[4-(N,N-dimethylamino)-1-oxo-2-buten-1-yl]amino}-7-ethoxy-quinoline,
(**1.25**) 3-cyano-4-[(3-chlor-4-(pyridin-2-yl-methoxy)-phenyl)amino]-6-{[4-(N,N-dimethylamino)-1-oxo-2-buten-1-yl]amino}-7-ethoxy-quinoline,
(**1.26**) 4-{[3-chloro-4-(3-fluoro-benzyloxy)-phenyl]amino}-6-(5-{[(2-methansulfonyl-ethyl)amino]methyl}-furan-2-yl)quinazoline,
(**1.27**) 4-[(R)-(1-phenyl-ethyl)amino]-6-{[4-((R)-6-methyl-2-oxo-morpholin-4-yl)-1-oxo-2-buten-1-yl]amino}-7-methoxy-quinazoline,
(**1.28**) 4-[(3-chloro-4-fluorophenyl)amino]-6-{[4-(morpholin-4-yl)-1-oxo-2-buten-1-yl]-amino}-7-[(tetrahydrofuran-2-yl)methoxy]-quinazoline,
(**1.29**) 4-[(3-chloro-4-fluorophenyl)amino]-6-({4-[N,N-bis-(2-methoxy-ethyl)-amino]-1-oxo-2-buten-1-yl}amino)-7-[(tetrahydrofuran-2-yl)methoxy]-quinazoline,
(**1.30**) 4-[(3-ethinyl-phenyl)amino]-6-{[4-(5,5-dimethyl-2-oxo-morpholin-4-yl)-1-oxo-2-buten-1-yl]amino}-quinazoline,
(**1.31**) 4-[(3-chloro-4-fluoro-phenyl)amino]-6-[2-(2,2-dimethyl-6-oxo-morpholin-4-yl)-ethoxy]-7-methoxy-quinazoline,
(**1.32**) 4-[(3-chloro-4-fluoro-phenyl)amino]-6-[2-(2,2-dimethyl-6-oxo-morpholin-4-yl)-ethoxy]-7-[(R)-(tetrahydrofuran-2-yl)methoxy]-quinazoline,
(**1.33**) 4-[(3-chloro-4-fluoro-phenyl)amino]-7-[2-(2,2-dimethyl-6-oxo-morpholin-4-yl)-ethoxy]-6-[(S)-(tetrahydrofuran-2-yl)methoxy]-quinazoline,
(**1.34**) 4-[(3-chloro-4-fluoro-phenyl)amino]-6-{2-[4-(2-oxo-morpholin-4-yl)-piperidin-1-yl]-ethoxy}-7-methoxy-quinazoline,
(**1.35**) 4-[(3-chloro-4-fluoro-phenyl)amino]-6-[1-(tert.-butyloxycarbonyl)-piperidin-4-yloxy]-7-methoxy-quinazoline,
(**1.36**) 4-[(3-chloro-4-fluoro-phenyl)amino]-6-(trans-4-amino-cyclohexan-1-yloxy)-7-methoxy-quinazoline,
(**1.37**) 4-[(3-chloro-4-fluoro-phenyl)amino]-6-(trans-4-methansulfonylamino-cyclo-hexan-1-yloxy)-7-methoxy-q u inazol ine,
(**1.38**) 4-[(3-chloro-4-fluoro-phenyl)amino]-6-(tetrahydropyran-3-yloxy)-7-methoxyquinazoline,
(**1.39**) 4-[(3-chloro-4-fluoro-phenyl)amino]-6-(1-methyl-piperidin-4-yloxy)-7-methoxyquinazoline,
(**1.40**) 4-[(3-chloro-4-fluoro-phenyl)amino]-6-{1-[(morpholin-4-yl)carbonyl]-piperidin-4-yloxy}-7-methoxy-quinazoline,
(**1.41**) 4-[(3-chloro-4-fluoro-phenyl)amino]-6-{1-[(methoxymethyl)carbonyl]-piperidin-4-yloxy}-7-methoxy-quinazoline,
(**1.42**) 4-[(3-chloro-4-fluoro-phenyl)amino]-6-(piperidin-3-yloxy)-7-methoxyquinazoline,
(**1.43**) 4-[(3-chloro-4-fluoro-phenyl)amino]-6-[1-(2-acetylamino-ethyl)-piperidin-4-yl-oxy]-7-methoxy-quinazoline,
(**1.44**) 4-[(3-chloro-4-fluoro-phenyl)amino]-6-(tetrahydropyran-4-yloxy)-7-ethoxy-quinazoline
(**1.45**) 4-[(3-chloro-4-fluoro-phenyl)amino]-6-((S)-tetrahydrofuran-3-yloxy)-7-hydroxy-quinazoline,
(**1.46**) 4-[(3-chloro-4-fluoro-phenyl)amino]-6-(tetrahydropyran-4-yloxy)-7-(2-methoxy-ethoxy)-quinazoline,
(**1.47**) 4-[(3-chloro-4-fluoro-phenyl)amino]-6-{trans-4-[(dimethylamino)sulfonyl-amino]-cyclohexan-1-yloxy}-7-methoxy-quinazoline,
(**1.48**) 4-[(3-chloro-4-fluoro-phenyl)amino]-6-{trans-4-[(morpholin-4-yl)carbonylamino]-cyclohexan-1-yloxy}-7-methoxy-quinazoline,
(**1.49**) 4-[(3-chloro-4-fluoro-phenyl)amino]-6-{trans-4-[(morpholin-4-yl)sulfonylamino]-cyclohexan-1-yloxy}-7-methoxy-quinazoline,
(**1.50**) 4-[(3-chloro-4-fluoro-phenyl)amino]-6-(tetrahydropyran-4-yloxy)-7-(2-acetylamino-ethoxy)-quinazoline,
(**1.51**) 4-[(3-chloro-4-fluoro-phenyl)amino]-6-(tetrahydropyran-4-yloxy)-7-(2-methan-sulfonylamino-ethoxy)-quinazoline,
(**1.52**) 4-[(3-chloro-4-fluoro-phenyl)amino]-6-{1-[(piperidin-1-yl)carbonyl]-piperidin-4-yloxy}-7-methoxy-quinazoline,
(**1.53**) 4-[(3-chloro-4-fluoro-phenyl)amino]-6-(1-aminocarbonylmethyl-piperidin-4-yl-oxy)-7-methoxy-quinazoline,
(**1.54**) 4-[(3-chloro-4-fluoro-phenyl)amino]-6-(cis-4-{N-[(tetrahydropyran-4-yl)carbonyl]-N-methyl-amino}-cyclohexan-1-yloxy)-7-methoxy-quinazoline,
(**1.55**) 4-[(3-chloro-4-fluoro-phenyl)amino]-6-(cis-4-{N-[(morpholin-4-yl)carbonyl]-N-methyl-amino}-cyclohexan-1-yloxy)-7-methoxy-quinazoline,
(**1.56**) 4-[(3-chloro-4-fluoro-phenyl)amino]-6-(cis-4-{N-[(morpholin-4-yl)sulfonyl]-N-methyl-amino}-cyclohexan-1-yloxy)-7-methoxy-quinazoline,
(**1.57**) 4-[(3-chloro-4-fluoro-phenyl)amino]-6-(trans-4-ethansulfonylamino-cyclohexan-1-yloxy)-7-methoxy-quinazoline,
(**1.58**) 4-[(3-chloro-4-fluoro-phenyl)amino]-6-(1-methansulfonyl-piperidin-4-yloxy)-7-ethoxy-quinazoline,
(**1.59**) 4-[(3-chloro-4-fluoro-phenyl)amino]-6-(1-methansulfonyl-piperidin-4-yloxy)-7-(2-methoxy-ethoxy)-quinazoline,
(**1.60**) 4-[(3-chloro-4-fluoro-phenyl)amino]-6-[1-(2-methoxy-acetyl)-piperidin-4-yloxy]-7-(2-methoxy-ethoxy)-quinazoline,
(**1.61**) 4-[(3-chloro-4-fluoro-phenyl)amino]-6-(cis-4-acetylamino-cyclohexan-1-yloxy)-7-methoxy-quinazoline,
(**1.62**) 4-[(3-ethinyl-phenyl)amino]-6-[1-(tert.-butyloxycarbonyl)-piperidin-4-yloxy]-7-methoxy-quinazoline,
(**1.63**) 4-[(3-ethinyl-phenyl)amino]-6-(tetrahydropyran-4-yloxy]-7-methoxyquinazoline,
(**1.64**) 4-[(3-chloro-4-fluoro-phenyl)amino]-6-(cis-4-{N-[(piperidin-1-yl)carbonyl]-N-methyl-amino}-cyclohexan-1-yloxy)-7-methoxy-quinazoline,
(**1.65**) 4-[(3-chloro-4-fluoro-phenyl)amino]-6-(cis-4-{N-[(4-methyl-piperazin-1-yl)-carbonyl]-N-methyl-amino}-cyclohexan-1-yloxy)-7-methoxy-quinazoline,
(**1.66**) 4-[(3-chloro-4-fluoro-phenyl)amino]-6-{cis-4-[(morpholin-4-yl)carbonylamino]-cyclohexan-1-yloxy}-7-methoxy-quinazoline,
(**1.67**) 4-[(3-chloro-4-fluoro-phenyl)amino]-6-{1-[2-(2-oxopyrrolidin-1-yl)ethyl]-piperidin-4-yloxy}-7-methoxy-quinazoline,
(**1.68**) 4-[(3-chloro-4-fluoro-phenyl)amino]-6-{1-[(morpholin-4-yl)carbonyl]-piperidin-4-yloxy}-7-(2-methoxy-ethoxy)-quinazoline,
(**1.69**) 4-[(3-ethinyl-phenyl)amino]-6-(1-acetyl-piperidin-4-yloxy)-7-methoxyquinazoline,
(**1.70**) 4-[(3-ethinyl-phenyl)amino]-6-(1-methyl-piperidin-4-yloxy)-7-methoxyquinazoline,
(**1.71**) 4-[(3-ethinyl-phenyl)amino]-6-(1-methansulfonyl-piperidin-4-yloxy)-7-methoxy-quinazoline,
(**1.72**) 4-[(3-chloro-4-fluoro-phenyl)amino]-6-(1-methyl-piperidin-4-yloxy)-7(2-methoxy-ethoxy)-quinazoline,
(**1.73**) 4-[(3-chloro-4-fluoro-phenyl)amino]-6-(1-isopropyloxycarbonyl-piperidin-4-yl-oxy)-7-methoxy-quinazoline,
(**1.74**) 4-[(3-chloro-4-fluoro-phenyl)amino]-6-(cis-4-methylamino-cyclohexan-1-yloxy)-7-methoxy-quinazoline,
(**1.75**) 4-[(3-chloro-4-fluoro-phenyl)amino]-6-{cis-4-[N-(2-methoxy-acetyl)-N-methylamino]-cyclohexan-1-yloxy}-7-methoxy-quinazoline,
(**1.76**) 4-[(3-ethinyl-phenyl)amino]-6-(piperidin-4-yloxy)-7-methoxy-quinazoline,
(**1.77**) 4-[(3-ethinyl-phenyl)amino]-6-[1-(2-methoxy-acetyl)-piperidin-4-yloxy]-7-methoxy-quinazoline,
(**1.78**) 4-[(3-ethinyl-phenyl)amino]-6-{1-[(morpholin-4-yl)carbonyl]-piperidin-4-yloxy}-7-methoxy-quinazoline,
(**1.79**) 4-[(3-chloro-4-fluoro-phenyl)amino]-6-{1-[(cis-2,6-dimethyl-morpholin-4-yl)carbonyl]-piperidin-4-yloxy}-7-methoxy-quinazoline,
(**1.80**) 4-[(3-chloro-4-fluoro-phenyl)amino]-6-{1-[(2-methyl-morpholin-4-yl)carbonyl]-piperidin-4-yloxy}-7-methoxy-quinazoline,
(**1.81**) 4-[(3-chloro-4-fluoro-phenyl)amino]-6-{1-[(S,S)-(2-oxa-5-aza-bicyclo[2.2.1]-hept5-yl)carbonyl]-piperidin-4-yloxy}-7-methoxy-quinazoline,
(**1.82**) 4-[(3-chloro-4-fluoro-phenyl)amino]-6-{1-[(N-methyl-N-2-methoxyethyl-amino)carbonyl]-piperidin-4-yloxy}-7-methoxy-quinazoline,
(**1.83**) 4-[(3-chloro-4-fluoro-phenyl)amino]-6-(1-ethyl-piperidin-4-yloxy)-7-methoxyquinazoline,
(**1.84**) 4-[(3-chloro-4-fluoro-phenyl)amino]-6-{1-[(2-methoxyethyl)carbonyl]-piperidin-4-yloxy}-7-methoxy-quinazoline,
(**1.85**) 4-[(3-chloro-4-fluoro-phenyl)amino]-6-{1-[(3-methoxypropyl-amino)-carbonyl]-piperidin-4-yloxy}-7-methoxy-quinazoline,
(**1.86**) 4-[(3-chloro-4-fluoro-phenyl)amino]-6-[cis-4-(N-methansulfonyl-N-methyl-amino)-cyclohexan-1-yloxy]-7-methoxy-quinazoline,
(**1.87**) 4-[(3-chloro-4-fluoro-phenyl)amino]-6-[cis-4-(N-acetyl-N-methyl-amino)-cyclo-hexan-1-yloxy]-7-methoxy-q u inazol ine,
(**1.88**) 4-[(3-chloro-4-fluoro-phenyl)amino]-6-(trans-4-methylamino-cyclohexan-1-yl-oxy)-7-methoxy-quinazoline ,
(**1.89**) 4-[(3-chloro-4-fluoro-phenyl)amino]-6-[trans-4-(N-methansulfonyl-N-methylamino)-cyclohexan-1-yloxy]-7-methoxy-quinazoline,
(**1.90**) 4-[(3-chloro-4-fluoro-phenyl)amino]-6-(trans-4-dimethylamino-cyclohexan-1-yloxy)-7-methoxy-quinazoline ,
(**1.91**) 4-[(3-chloro-4-fluoro-phenyl)amino]-6-(trans-4-{N-[(morpholin-4-yl)carbonyl]-N-methyl-amino}-cyclohexan-1-yloxy)-7-methoxy-quinazoline,
(**1.92**) 4-[(3-chloro-4-fluoro-phenyl)amino]-6-{1-[2-(2-oxo-3-methyl-imidazolidin-1-yl)-ethyl]-piperidin-4-yloxy}-7-methoxy-quinazoline ,
(**1.93**) 4-[(3-chloro-4-fluoro-phenyl)amino]-6-{1-[2-(2-oxo-hexahydropyrimidin-1-yl)-ethyl]-piperidin-4-yloxy}-7-methoxy-quinazoline,
(**1.94**) 4-[(3-chloro-4-fluoro-phenyl)amino]-6-[2-(2,2-dimethyl-6-oxo-morpholin-4-yl)-ethoxy]-7-[(S)-(tetrahydrofuran-2-yl)methoxy]-quinazoline,
(**1.95**) 4-[(3-chloro-4-fluoro-phenyl)amino]-6-(1-methansulfonyl-piperidin-4-yloxy)-7-methoxy-quinazoline,
(**1.96**) 4-[(3-chloro-4-fluoro-phenyl)amino]-6-(1-cyano-piperidin-4-yloxy)-7-methoxyquinazoline,
**(1.97)** 4-[(3-chloro-4-fluoro-phenyl)amino]-6-(tetrahydropyran-4-yloxy)-7-methoxyquinazoline,
**(1.98)** 4-[(3-chloro-4-fluoro-phenyl)amino]-6-(1-methylcarbonyl-piperidin-4-yloxy)-7-methoxy-quinazoline,
**(1.99)** 4-[(3-chloro-4-fluoro-phenyl)amino]-6-(1-dimethylaminoacetyl-piperidin-4-yloxy)-7-methoxy-quinazoline,
**(1.100)** 4-[(3-chloro-4-fluoro-phenyl)amino]-6-{1-[(dimethylamino)carbonylmethyl]-piperidin-4-yloxy}-7-methoxy-quinazoline,
**(1.101)** 4-[(3-chloro-4-fluoro-phenyl)amino]-6-(1-methansulfonyl-piperidin-4-yloxy)-quinazoline,
(**1.102**) Cetuximab, (**1.103**) Trastuzumab, (**1.104**) ABX-EGF and (**1.105**) Mab ICR-62,
optionally in the form of tautomers, racemates, enantiomers, diastereomers, pharmacologically acceptable acid addition salts, solvates or hydrates thereof,
and further comprising one or more additional active compounds **2** selected from the classes consisting of steroids **2b,** optionally together with one or more pharmaceutically acceptable excipients or carriers.

2. The pharmaceutical composition of claim 1, wherein the steroid **2b** is selected from the group consisting of methyl prednisolone, prednisone, butixocort propionate, RPR-106541, flunisolide, beclomethasone, triamcinolone, budesonide, fluticasone, mometasone, ciclesonide, rofleponide, ST-126, dexamethasone, 6α,9α-difluoro-17α-[(2-furanylcarbonyl)oxy]-11β-hydroxy-16α-methyl-3-oxo-androsta-1,4-diene-17β-carbothioic acid (S)-fluoromethyl ester, and 6α,9α-difluoro-11β-hydroxy-16a-methyl-3-oxo-17α-propionyloxy-androsta-1,4-diene-17β-carbothioic acid (S)-(2-oxo-tetrahydro-furan-3S-yl) ester.

3. Pharmaceutical composition according to claim 1 or 2, **characterised in that** it is in the form of a preparation suitable for inhalative, oral, intravenous, topical, subcutaneous, intramuscular, intraperitoneal, intranasal, transdermal or rectal administration.

4. Pharmaceutical composition according to one of claims 1 to 3, **characterised in that** it is in the form of a preparation suitable for inhalation.

5. Pharmaceutical composition according to claim 4, **characterised in that** it is a preparation selected from among the inhalable powders, propellant-containing metered-dose aerosols and propellant-free inhalable solutions.

6. Pharmaceutical composition according to claim 4, **characterised in that** it is an inhalable powder which contains **1** and **2** in admixture with suitable physiologically acceptable excipients selected from among the monosaccharides, disaccharides, oligo- and polysaccharides, polyalcohols, salts, or mixtures of these excipients with one another.

7. Pharmaceutical composition in form of an inhalable powder according to claim 6, **characterised in that** the excipient has a maximum average particle size of up to 250µm, preferably between 10 and 150µm.

8. Pharmaceutical composition according to claim 5, **characterised in that** it is an inhalable powder which contains only the active substances **1** and **2** as its ingredients.

9. Pharmaceutical composition according to claim 5, **characterised in that** it is a propellant-containing inhalable aerosol which contains **1** and **2** in dissolved or dispersed form.

10. Pharmaceutical composition in form of a propellant-containing inhalable aerosol according to claim 9, **characterised in that** it contains, as propellant gas, hydrocarbons such as n-propane, n-butane or isobutane or halohydrocarbons such as chlorinated and/or fluorinated derivatives of methane, ethane, propane, butane, cyclopropane or cyclobutane.

11. Pharmaceutical composition in form of an inhalable solution according to claim 10, **characterised in that** it optionally contains other co-solvents and/or excipients.

12. The use of a pharmaceutical composition according to one of claims 3 to 11 for the manufacture of a medicament for treating an indication selected from indications (A):
prevention and treatment of diseases of the airways and lungs which are accompanied by increased or altered production of mucus and/or inflammatory and/or obstructive diseases of the airways such as
acute bronchitis, chronic bronchitis, chronic obstructive bronchitis (COPD), cough, pulmonary emphysema,
allergic or non-allergic rhinitis or sinusitis, chronic sinusitis or rhinitis,
nasal polyposis, chronic rhinosinusitis, acute rhinosinusitis,
asthma, allergic bronchitis, alveolitis, Farmers'disease, hyperreactive airways,
bronchitis or pneumonits caused by infection, e.g. by bacteria or viruses or helminthes or fungi or protozoons or other pathogens,
pediatric asthma, bronchiectasis,
pulmonary fibrosis,
adult respiratory distress syndrome, bronchial and pulmonary edema,
bronchitis or pneumonitis or interstitial pneumonitis caused by different origins, e.g. aspiration, inhalation of toxic gases, vapors,
bronchitis or pneumonitis or interstitial pneumonitis caused by heart failure, X-rays, radiation, chemotherapy,
bronchitis or pneumonitis or interstitial pneumonitis associated with collagenosis, e.g. lupus erythematodes, systemic scleroderma,
lung fibrosis, idiopathic pulmonary lung fibrosis (IPF), interstitial lung diseases or interstitial pneumonitis of different origin, including asbestosis, silicosis, M. Boeck
or sarcoidosis, granulomatosis,
cystic fibrosis or mucoviscidosis, or α1-antitrypsin deficiency.

13. The use of pharmaceutical composition according to claim 3 to 11, wherein the EGFR kinase inhibitor is selected from compounds **1.1** to **1.101,** preferably from compounds
**1.1, 1.4, 1.6, 1.8, 1.9, 1.14, 1.17, 1.19, 1.21, 1.23, 1.24, 1.27, 1.28, 1.30, 1.34, 1.35, 1.37, 1.38, 1.40, 1.42, 1.43, 1.44, 1.48, 1.52, 1.55, 1.57, 1.59, 1.60, 1.63, 1.64, 1.66, 1.67, 1.69, 1.70, 1.71, 1.72, 1.78, 1.82, 1.83, 1.84, 1.88, 1.90, 1.91, 1.94** and **1.95.**
